# EUROPEAN PATENT APPLICATION

(11) **EP 4 606 793 A1**
(43) Date of publication of application: **27.08.2025**
(21) Application number: 23880252.4
(22) Date of filing: 19.10.2023
(51) Int. Cl.: C07D 251/24, C07D 239/26, C07D 213/16, C07D 405/04, C07D 409/04, C07D 405/10, C07D 409/10, C07D 239/70, C07D 239/74, C07D 495/04

(54) **ORGANIC COMPOUND AND ORGANIC ELECTROLUMINESCENT DEVICE COMPRISING SAME**

(30) Priority: 20.10.2022 KR 20220136034
(71) Applicant: Solus Advanced Materials Co., Ltd., Iksan-si, Jeollabuk-do 54584 (KR)
(72) Inventor: SON, Hyosuk, Seongnam-si, Gyeonggi-do 13636 (KR); SIM, Jaeyi, Seongnam-si, Gyeonggi-do 13636 (KR); LEE, Yonghwan, Seongnam-si, Gyeonggi-do 13636 (KR); BAE, Hyungchan, Seongnam-si, Gyeonggi-do 13636 (KR)
(74) Representative: Office Freylinger
(86) International application number: PCT/KR2023/016238
(87) International publication number: WO 2024/085676

(57) **Abstract**

The present invention relates to a novel organic compound and an organic electroluminescent device using the same, and, more specifically, to an organic compound having excellent electron injection and transport ability and thermal stability, and an organic electroluminescent device including the compound in one or more organic layers, thereby having improved characteristics such as luminous efficiency, driving voltage and lifespan.

## Description

### Technical Field

The present disclosure relates to a novel organic compound and an organic electroluminescent device including the same and, more specifically, to an organic compound exhibiting excellent electron injection and transport capabilities as well as thermal stability, and an organic electroluminescent device in which at least one organic layer includes the organic compound, thereby improving characteristics such as luminous efficiency, driving voltage, and lifespan.

### Background Art

In an organic electroluminescent device (hereinafter referred to as "organic EL device"), the application of voltage between two electrodes injects holes from the anode and electrons from the cathode into an organic layer. When the injected holes and electrons recombine, excitons are formed, and light is emitted as the excitons return to the ground state. The materials used in the organic layer are classified based on their function into emissive materials, hole injection materials, hole transport materials, electron transport materials, and electron injection materials.

The luminescent layer materials of organic EL devices are categorized based on emission color into blue, green, and red luminescent materials. Additionally, yellow and orange luminescent materials are used to achieve better full-color implementation. Furthermore, to enhance color purity and luminous efficiency via energy transfer, a host/dopant system can be employed as the luminescent material. Dopant materials are classified into fluorescent dopants, which use organic compounds, and phosphorescent dopants, which employ metal complex compounds containing heavy atoms such as Ir and Pt. The development of phosphorescent materials is of particular interest, as they can theoretically enhance luminous efficiency up to four times that of fluorescence. Consequently, significant attention has been directed not only toward phosphorescent dopants but also toward phosphorescent host materials.

To date, materials widely known as hole injection layers, hole transport layers, hole blocking layers, and electron transport layers include compounds such as NPB, BCP, and Alq₃, which are represented by the following chemical Formulas. Anthracene derivatives have been reported as fluorescent dopant/host materials for luminescent applications. Among luminescent materials, phosphorescent materials offer substantial advantages in terms of efficiency improvement. Examples of Ir-containing metal complex compounds used as blue, green, and red dopants include Firpic, Ir(ppy)₃, and (acac)Ir(btp)₂. Currently, CBP exhibits excellent properties as a phosphorescent host material.

However, conventional organic layer materials, despite their advantageous luminescent properties, suffer from low glass transition temperatures and poor thermal stability, which severely limit the lifespan of organic EL devices. Therefore, the development of high-performance organic layer materials is in high demand.

### Disclosure of Invention

### Technical Problem

The present disclosure aims to provide a novel compound that exhibits improved electron injection and transport properties as well as excellent thermal stability, thus finding applications as an organic layer material in an organic electroluminescent device, specifically as an electron transport layer material or an electron transport auxiliary layer material.

Also, the present disclosure is to provide an organic electroluminescent device including the the above-described novel compound, thereby achieving a lower driving voltage, higher luminous efficiency, and enhanced lifespan characteristics.

### Solution to Problem

To achieve the goals mentioned above, the present discloses provides a compound represented by any one of the following Chemical Formulas 1 to 5: 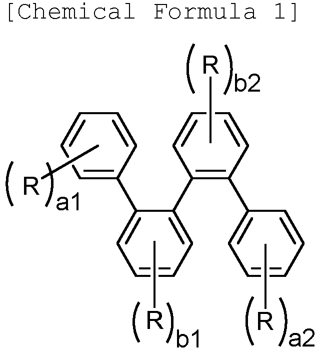 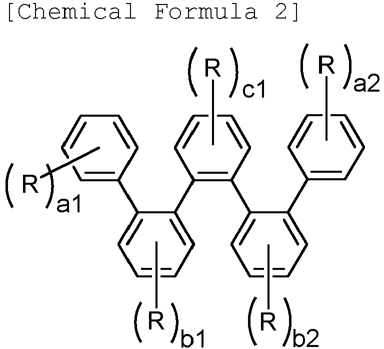 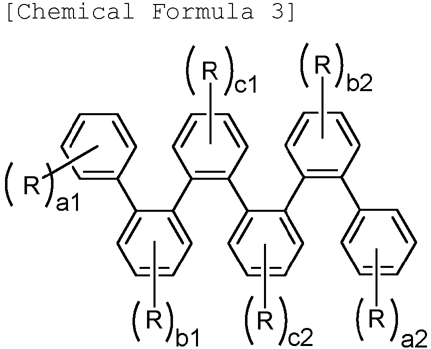 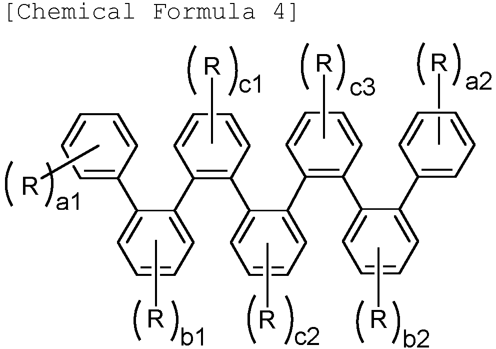 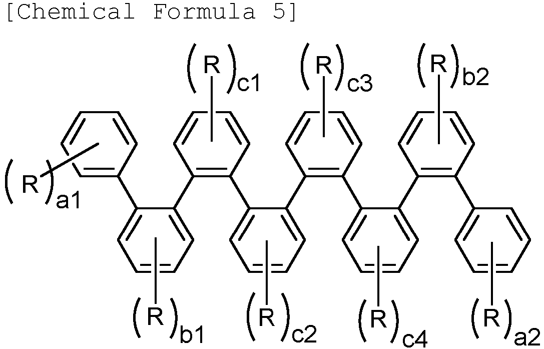 (wherein,
a1 and a2 are each an integer of 0 to 4,
b1 and b2 are each an integer of 0 to 3,
c1, c2, c3, and c4 are each an integer of 0 to 2,
with a proviso of a1+a2+b1+b2≥1, a1+a2+b1+b2+c1≥1, a1+a2+b1+b2+c1+c2≥1, a1+a2+b1+b2+c1+c2+c3≥1, and a1+a2+b1+b2+c1+c2+c3+c4≥1,
the plural R's, which are the the same or different from each other from each other, are each independently selected from the group consisting of a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, or may form a fused ring with an adjacent group,
with a proviso that at least one of the plural R's is a moiety A represented by the following Chemical Formula A1 or A2, 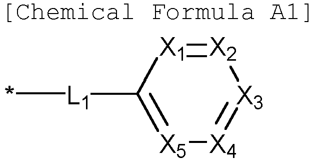 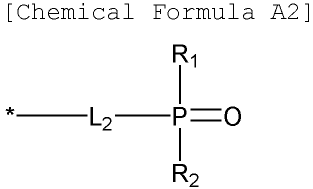
wherein,
L₁ and L₂ are each independently a single bond or a C₆-C₆₀ arylene group,
X₁ to X₅, which are the same or different from each other, are each independently N or C(R₃), with a proviso that at least one of X₁ to X₅ being N, wherein when two or more C(R₃) are present, the plural R₃'s the same or different from each other,
R₁ to R₃, which are the same or different from each other, are each independently selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, or may form a fused ring with an adjacent group,
the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, alkylphosphine oxide group, arylphosphine group, alkylphosphine group, arylphosphine oxide group, and arylamine group and the fused ring in R and R₁ to R₃, and the arylene group in L₁ and L₂ are unsubstituted or substituted with at least one substituent selected from the group consisting of a deuterium atom, a halogen group, a cyano group, a nitro group, a C₂-C₄₀ alkenyl group, a C₂-C₄₀ alkynyl group, a C₃-C₄₀ cycloalkyl group, a heterocycloalkyl group having 3 to 40 nuclear atoms, a C₁-C₄₀ alkyl group, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, wherein when the substituent is present in a plurality of numbers, a plurality of substituents are the same or different from each other).

Moreover, the present disclosure provides an organic electroluminescent device including: an anode; a cathode; and at least one organic layer interposed between the anode and the cathode, wherein at least one of the one or more organic layers includes the aforementioned organic compound.

The organic layer including the organic compound may serve as an electron transport layer and/or an electron transport auxiliary layer.

### Advantageous Effects of Invention

The compound of the present disclosure have excellent electron transport and injection capabilities, heat resistance, and electrochemical stability, and thus can be used as a material in the organic layer of an organic electroluminescent device. In particular, when the compound of the present disclosure is used as at least one of the electron transport layer material and the electron transport auxiliary layer material, an organic electroluminescent device with superior luminescent performance, lower driving voltage, higher efficiency, fast mobility, and prolonged lifespan can be produced. Furthermore, a full-color display panel with enhanced performance and lifespan can be fabricated.

### Brief Description of Drawings

FIG. 1 is a schematic cross-sectional view of an organic electroluminescent device according to the first embodiment of the present disclosure.
FIG. 2 is a schematic cross-sectional view of an organic electroluminescent device according to the second embodiment of the present disclosure.
FIG. 3 is a schematic cross-sectional view of an organic electroluminescent device according to the third embodiment of the present disclosure.

### **Description of Reference Numerals**

100: Anode, 200: Cathode
300: Organic layer, 310: Hole injection layer
320: Hole transport layer, 330: Emissive layer
340: Electron transport layer, 350: Electron injection layer
360: Electron transport auxiliary layer

### Mode for Carrying out the Invention

Below, a detailed description will be given of the present disclosure.

### <Novel Organic Compound>

The compound according to the present disclosure has a structure including a fundamental skeleton composed of repetitively bonded ortho-phenylene groups, with a nitrogen-containing heterocyclic moiety (e.g., pyridine group, pyrimidine group, triazine group, etc.), an arylphosphine oxide moiety, or an alkylphosphine oxide moiety linked thereto, either directly or via a linker group, and is represented by any one of Chemical Formulas 1 to 5. The compound of the present disclosure exhibits excellent electron injection and transport capabilities, electrochemical stability, and thermal stability, and thus can enhance key characteristics of organic electroluminescent devices, such as high efficiency, long lifespan, and low driving voltage.

Specifically, in the compound of the present disclosure, the ortho-phenylene group in the skeleton exhibits n-conjugation, and then delocalization of the electron density due to doping, thereby enhancing electron conductivity. Since the skeleton has the ortho-phenylene groups repeatedly bonded in a spiral form, the compound of the present disclosure possesses a bulky structure. Moreover, because the fundamental skeleton consists of repetitively bonded ortho-phenylene groups, the compound of the present disclosure has a higher triplet energy level (T1) compared to compounds having meta-phenylene or para-phenylene backbones. Therefore, when the compound of the present disclosure is included as an electron transport auxiliary layer material, the compound of the present disclosure can enhance the efficiency of the organic electroluminescent device due to the triplet-triplet fusion (TTF) effect. Additionally, the compound of the present disclosure can suppress the diffusion of excitons or holes from the emissive layer into the adjacent electron transport layer, thereby increasing the number of excitons contributing to luminescence within the emissive layer. This ultimately improves luminescence efficiency of the device, and enhances the device's stability and durability, leading to a longer device lifespan.

Furthermore, in the compound of the present disclosure, the nitrogen-containing heterocyclic moiety introduced into the skeleton serves as a strong electron-withdrawing group (EWG) with high electron affinity. This nitrogen-containing heterocyclic moiety enhances electron mobility, thereby improving electron transport and injection properties of the compounds. Accordingly, when the compound of the present disclosure is applied as a material for the electron transport layer or electron transport auxiliary layer of an organic electroluminescent device, electrons can be efficiently transferred from the cathode (or electron injection layer) to the emissive layer, leading to a reduction in the device's driving voltage and the realization of high efficiency and long lifespan characteristics.

Moreover, when containing an arylphosphine oxide moiety or an alkylphosphine oxide moiety, the compound of the present disclosure can form coordination bonds with metal complexes such as Liq (8-Quinolinolato Lithium), thereby maximizing electron mobility. As a result, when applied as a material for the electron transport layer or electron transport auxiliary layer in an organic electroluminescent device, the compound facilitates the efficient transfer of electrons from the cathode (or electron injection layer) to the emissive layer, leading to lower driving voltage, higher efficiency, and extended device lifespan.

As described above, the compound represented by any one of Chemical Formulas 1 to 5 in the present disclosure exhibits superior electron injection and transport properties, thermal stability, and electrochemical stability. Therefore, the compound of the present disclosure is suitable for use in the organic layer of an organic electroluminescent device, preferably as an electron transport/injection layer material or an electron transport auxiliary layer material, and more preferably as an electron transport layer material or an electron transport auxiliary layer material. An organic electroluminescent device incorporating the compound of the present disclosure can exhibit significantly improved performance and longevity, and a full-color organic light-emitting panel incorporating such a device can achieve maximized performance.

In the compound of the present disclosure, a1 and a2 are each an integer from 0 to 4, b1 and b2 are each an integer from 0 to 3, and c1, c2, c3, and c4 are each an integer from 0 to 2. In this regard, at least one of the plural R's must be a moiety A represented by Chemical Formula A1 or A2. Therefore, the following conditions must be met: a1 + a2 + b1 + b2 ≥ 1, a1 + a2 + b1 + b2 + c1 ≥ 1, a1 + a2 + b1 + b2 + c1 + c2 ≥ 1, a1 + a2 + b1 + b2 + c1 + c2 + c3 ≥ 1, or a1 + a2 + b1 + b2 + c1 + c2 + c3 + c4 ≥ 1.

Here, when a1, a2, b1, b2, c1, c2, c3, and c4 are each 0, this means that the hydrogen atom is not substituted with substituent R. When a1 and a2 are each an integer from 1 to 4, b1 and b2 are each an integer from 1 to 3, and c1, c2, c3, and c4 are each an integer from 1 to 2, one or more R may be the same or different from each other and are each independently selected from the group consisting of a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group or may form a fused ring with an adjacent group. Here, the fused ring may be at least one selected from the group consisting of a C₃-C₆₀ fused aliphatic ring (specifically, C₃-C₃₀ fused aliphatic ring), a C₆-C₆₀ fused aromatic ring (specifically, a C₆-C₃₀ fused aromatic ring), a 5- to 60-membered, fused heteroaromatic ring (specifically, 5- to 30-membered, fused heteroaromatic ring), a C₃-C₆₀ spiro ring, and a combination thereof.

According to an embodiment, the plural R's are the same or different from each other and may each be independently selected from the group consisting of a deuterium atom (D), a cyano group, a C₁-C₄₀ alkyl group, a C₆-C₆₀ aryl group, a heteroaryl group having 5 to 60 nuclear atoms, a C₁-C₄₀ alkylphosphine oxide group, and a C₆-C₆₀ arylphosphine oxide or may form a fused ring with an adjacent group. In this regard, at least one of the plural R's is a moiety A represented by Chemical Formula A1 or A2.

The alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, alkylphosphine group, arylphosphine group, alkylphosphine oxide group, arylphosphine oxide group, and arylamine group and fused ring in R may each be independently unsubstituted or substituted with at least one substituent selected from the group consisting of a deuterium atom, a halogen group, a cyano group, a nitro group, a C₂-C₄₀ alkenyl group, a C₂-C₄₀ alkynyl group, a C₃-C₄₀ cycloalkyl group, a heterocycloalkyl group having 3 to 40 nuclear atoms, a C₁-C₄₀ alkyl group, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group. In this regard, when present, a plurality of substituents may be the same or different from each other.

At least one of the plural R's is a moiety A represented by Chemical Formula A1 or A2.

According to an embodiment, the moiety A represented by Chemical Formula A1 may be selected from the group consisting of the following moieties S1-1 to S1-10, but with no limitations thereto: 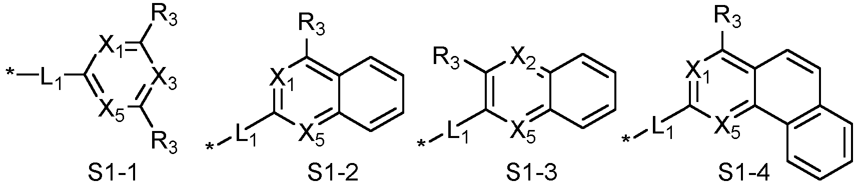 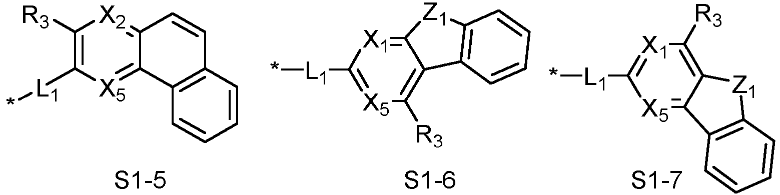 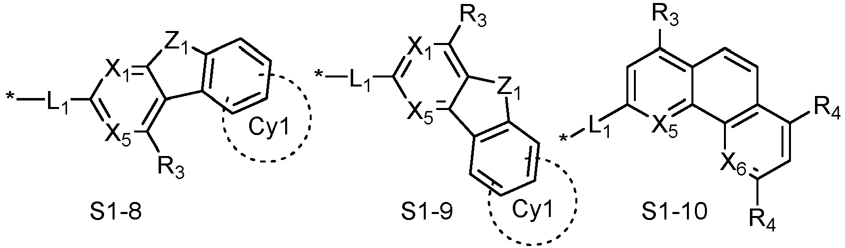 in moiety S1-1,
X₁, X₃, and X₅, which are the same or different from each other, are each independently N or C(R₃), with a proviso that at least one of X₁, X₃, and X₅ is N,
in moieties S1-2, S1-4, and S1-6 to S1-9,
X₁ and X₅, which are the same or different from each other, are each independently N or C(R₃), with a proviso that at least one of X₁ and X₅ is N,
in moieties S1-3 and S1-5,
X₂ and X₅, which are the same or different from each other, are each independently N or C(R₃), with a proviso that at least one of X₂ and X₅ is N,
in moieties S1-1 to S1-9,
Z₁ is O or S,
Cy1 is a C₆-C₃₀ fused aromatic ring and specifically, a C₆-C₁₈ fused aromatic ring,
in moiety S1-10, X₅ and X₆, which are the same or different from each other, are each independently N or C(R₃), with a proviso that at least one of X₅ and X₆ is N,
L₁ and R₃ are each as defined in Chemical Formula A1,
the plural R₄'s are the same or different from each other,
R₄ is selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group or may form a fused ring with an adjacent group.

In the moiety A represented by Chemical Formulas A1 or A2, L₁ and L₂ are each independently a single bond or a C₆-C₆₀ arylene and specifically each independently a single bond or may be selected from the group consisting of a phenylene group, a biphenylene group, and a divalent naphthalene group (naphthylene group).

In this regard, the arylene group in L₁ and L₂ may be each independently unsubstituted or substituted with at least one substituent selected from the group consisting of a deuterium atom, a halogen group, a cyano group, a nitro group, a C₂-C₄₀ alkenyl group, a C₂-C₄₀ alkynyl group, a C₃-C₄₀ cycloalkyl group, a heterocycloalkyl group having 3 to 40 nuclear atoms, a C₁-C₄₀ alkyl group, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group. When a plurality of substituents are present, they may be the same or different from each other.

According to an embodiment, L₁ and L₂ in Chemical Formulas A1 and A2 may each be independently a single bond or be selected from the group consisting of the following linkers L-1 to L-19, but with no limitations thereto: 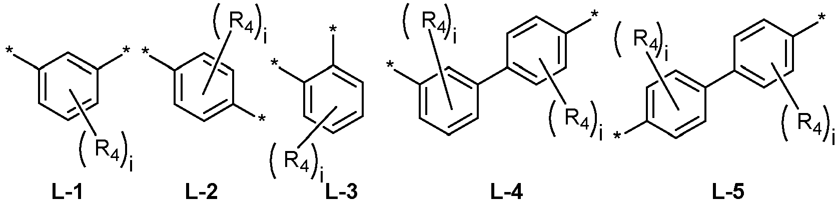 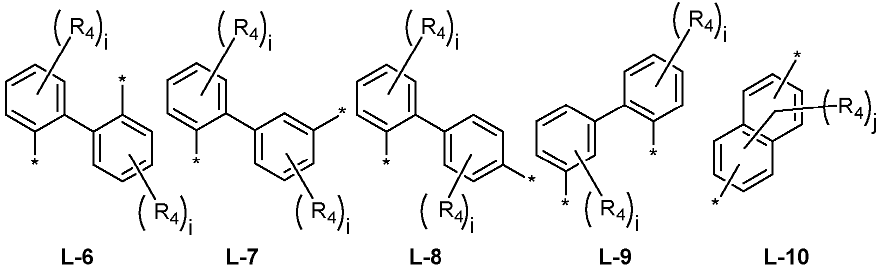 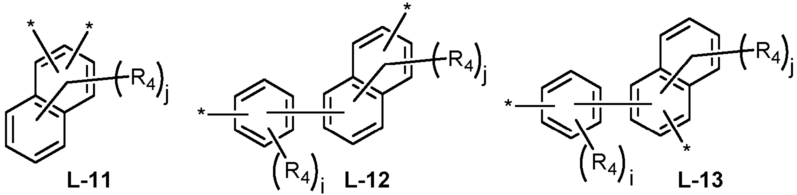 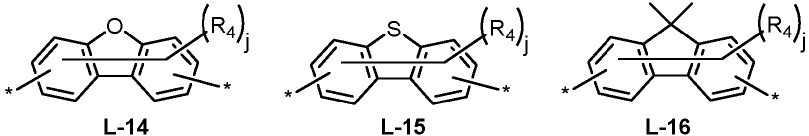 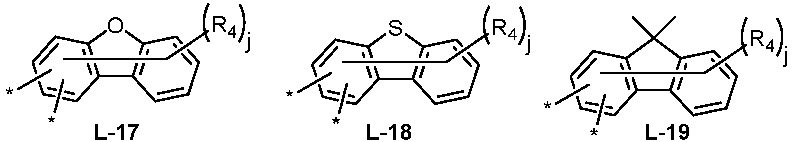 wherein,
i is an integer of 0 to 4,
j is an integer of 0 to 6,
plural R₄'s are the same or different from each other,
R₄ is selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group or may form a fused ring with an adjacent group.

In Chemical Formulas A1 and A2, X₁ to X₅, which are the same or different from each other, are each independently N or C(R₃), with a proviso that at least one of X₁ to X₅ is N, wherein when two or more C(R₃) are present, the plural R₃'s are the same or different from other,
R₁ to R₃, which are the same or different from each other, are each independently selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, or may form a fused ring with an adjacent group,
the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, alkylphosphine group, arylphosphine group, alkylphosphine oxide group, arylphosphine oxide group, arylamine group and the fused ring in R₁ to R₃ remaining unsubstituted or being each independently substituted with at least one substituent selected from the group consisting of a deuterium atom, a halogen group, a cyano group, a nitro group, a C₂-C₄₀ alkenyl group, a C₂-C₄₀ alkynyl group, a C₃-C₄₀ cycloalkyl group, a heterocycloalkyl group having 3 to 40 nuclear atoms, a C₁-C₄₀ alkyl group, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, wherein when a plurality of substituents are present, they are the same or different from each other.

According to an embodiment, the plural R₃'s, which are the same or different from each other, may each be selected from the group consisting of the following substituents S1 to S23, but with no limitations thereto:

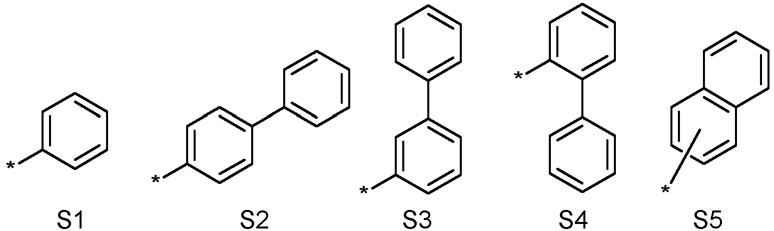

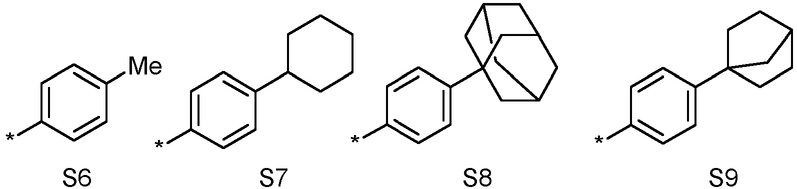

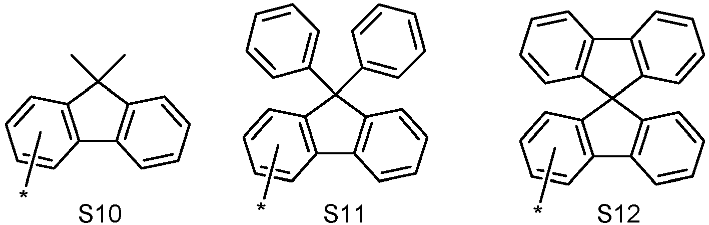

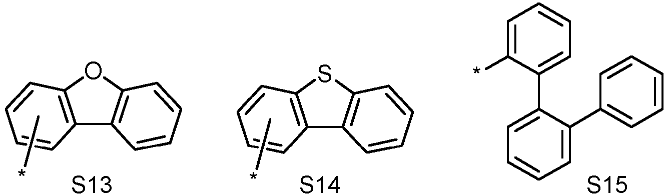

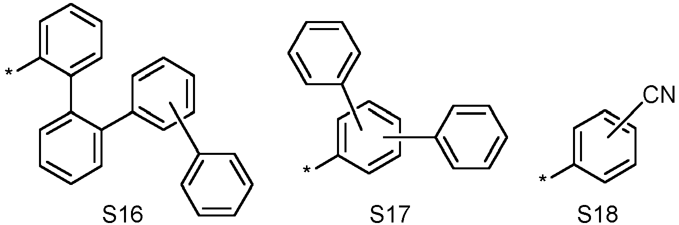

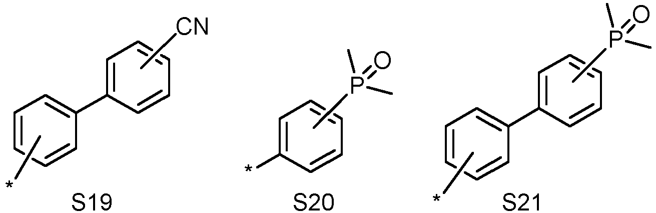

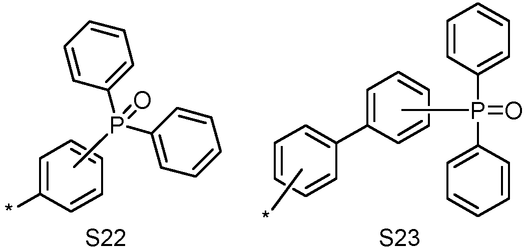

Depending on the position of incorporation of the aforementioned moiety A, the compound of the present disclosure may be represented by any one of Chemical Formulas 1a to 5k, but is not limited thereto. 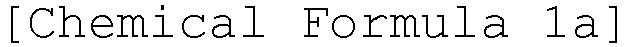 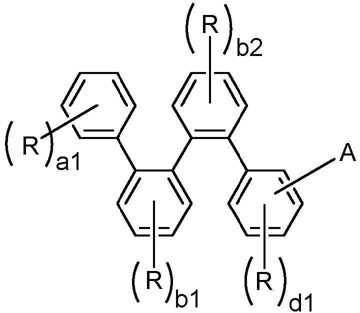 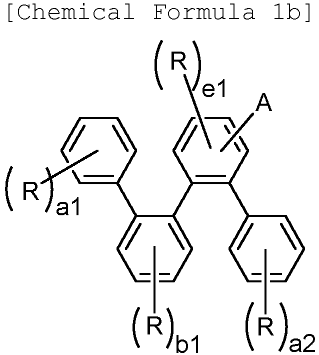 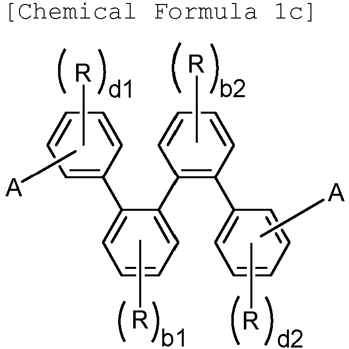 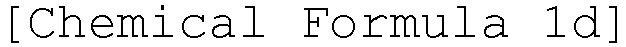 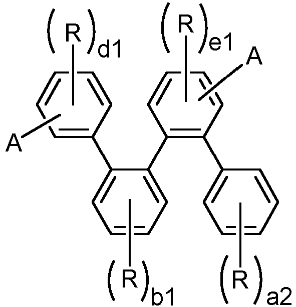 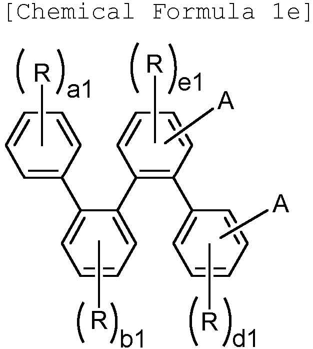 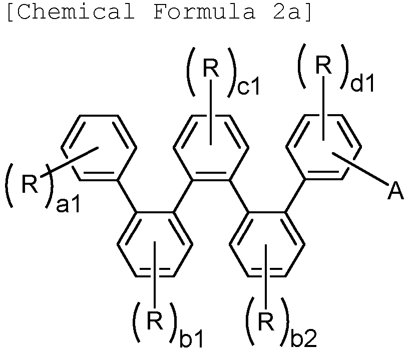 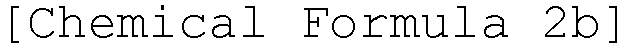 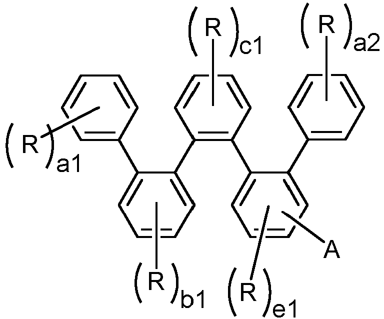 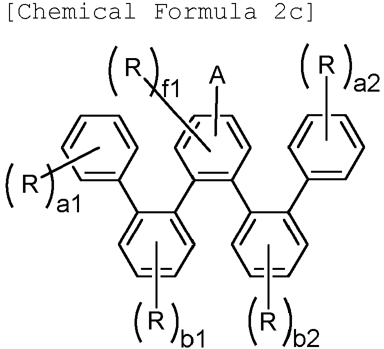 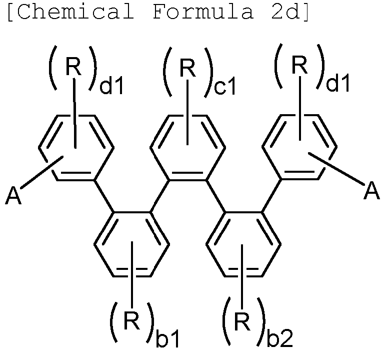 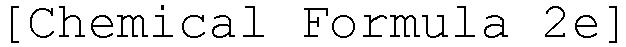 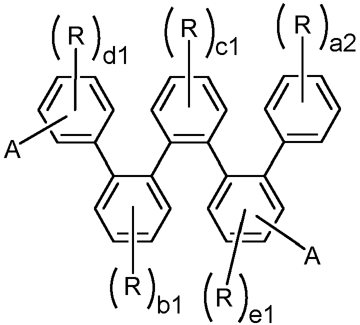 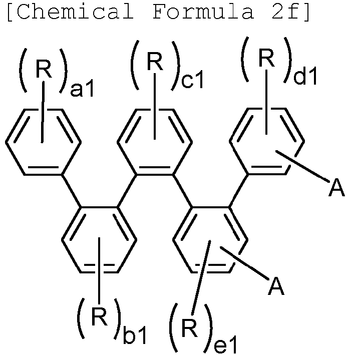 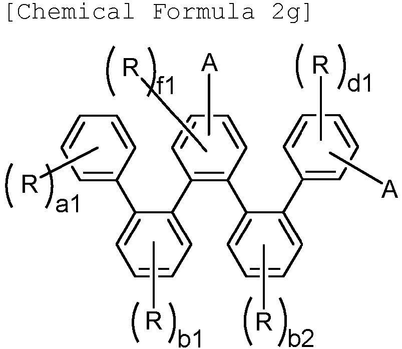 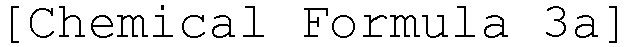 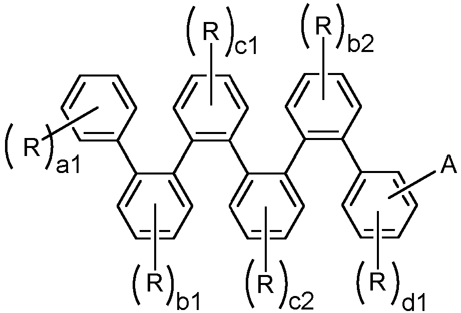 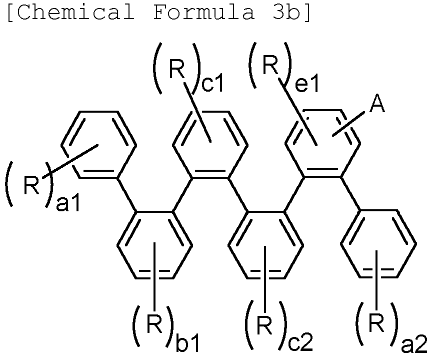 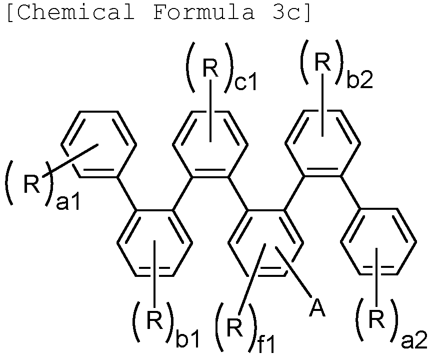 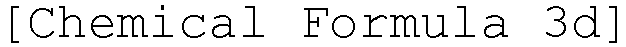 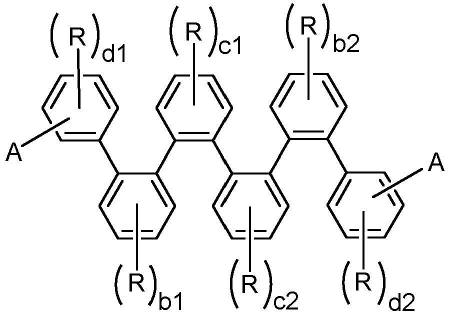 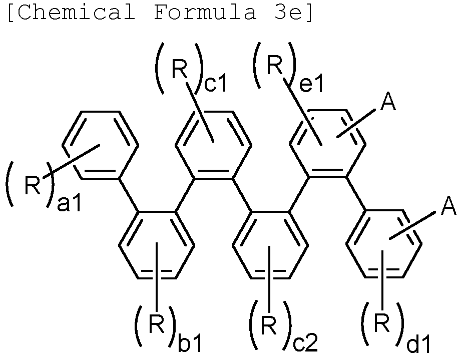 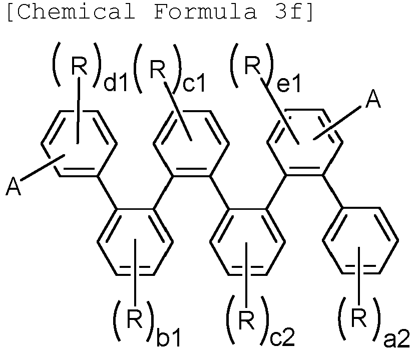 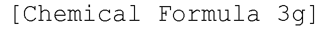 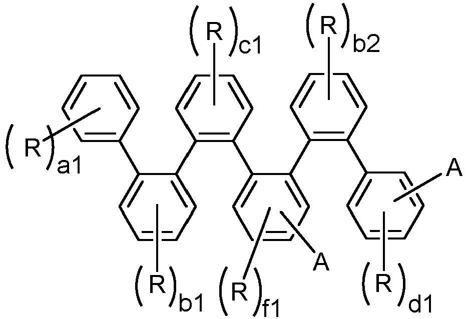 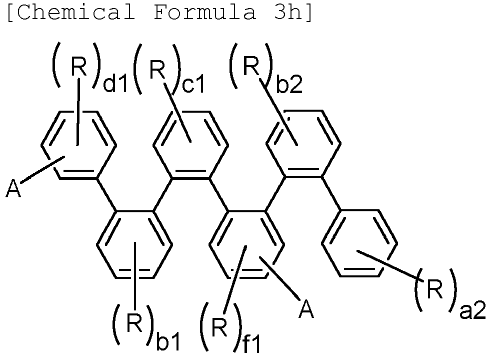 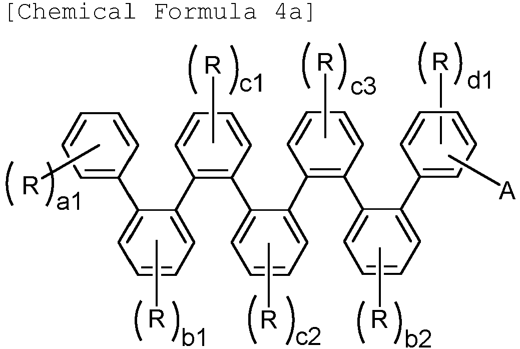 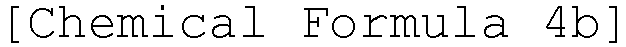 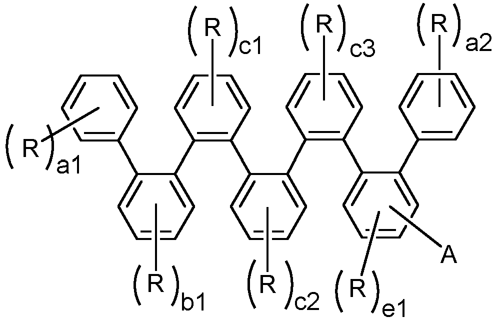 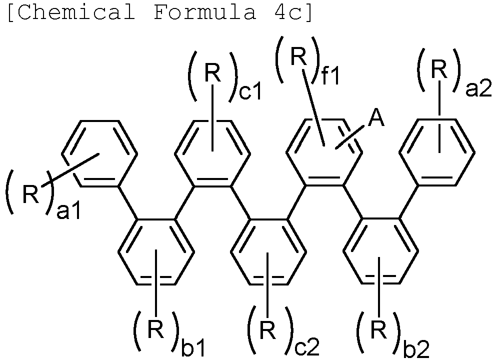 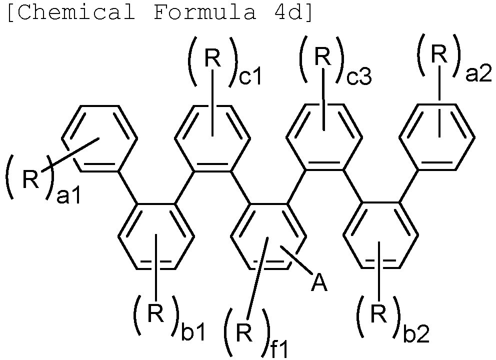 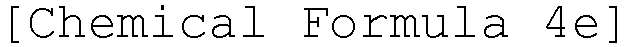 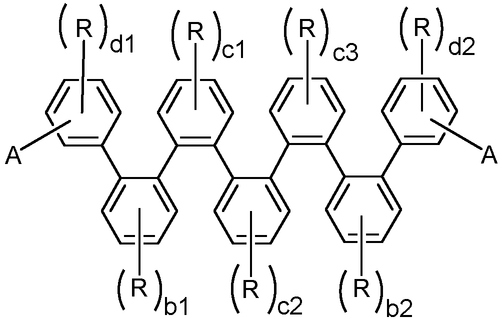 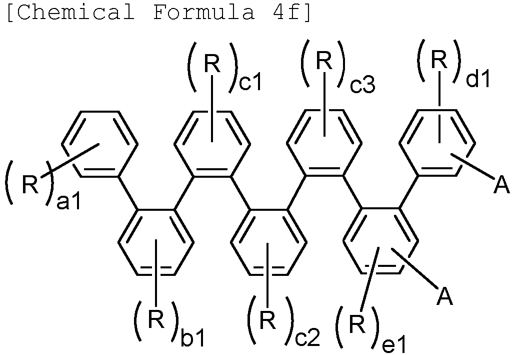 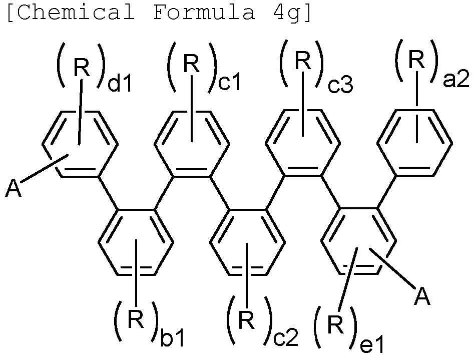 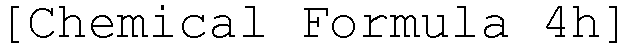 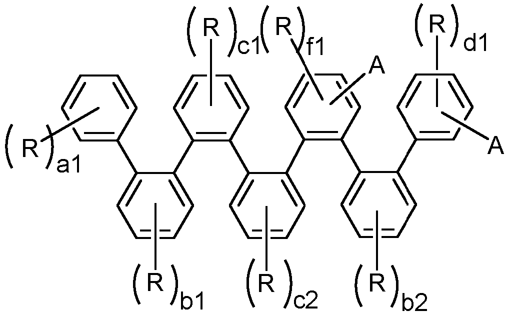 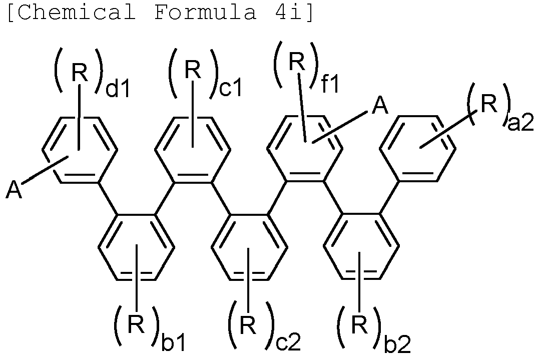 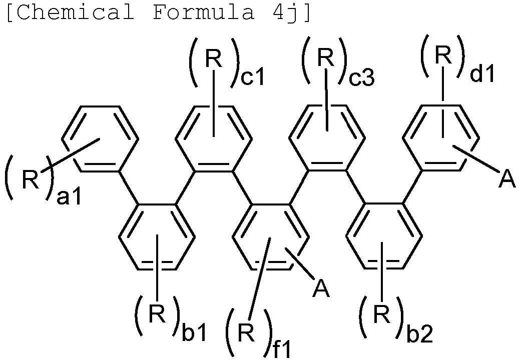 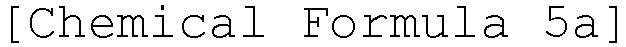 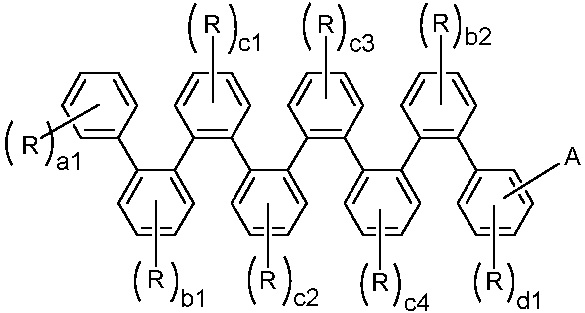 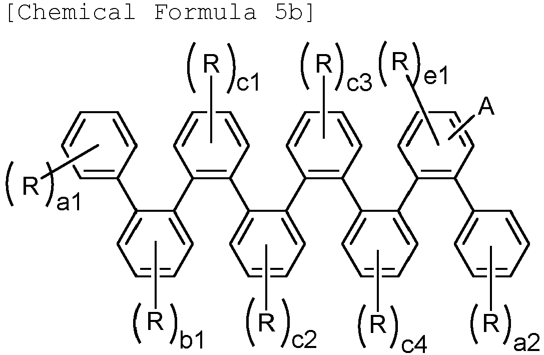 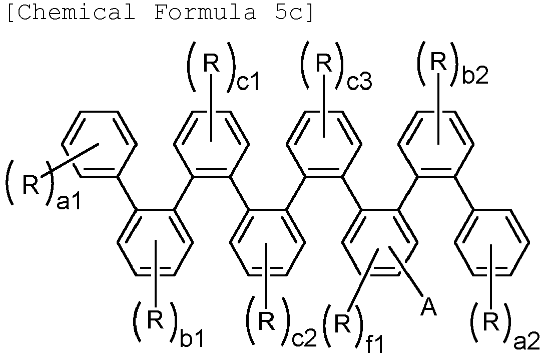 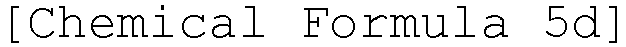 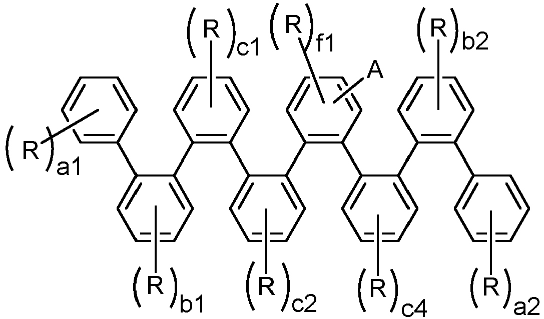 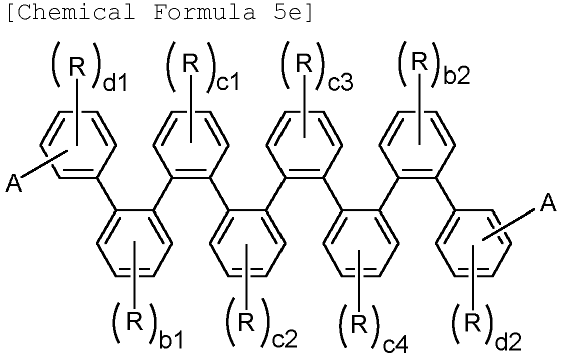 [Chemical Formula 5f] 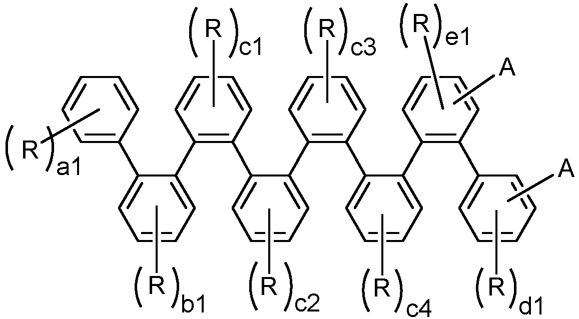 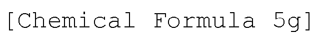 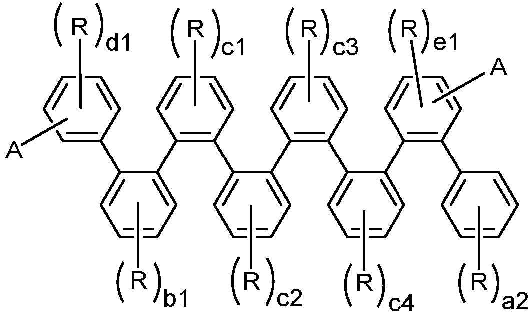 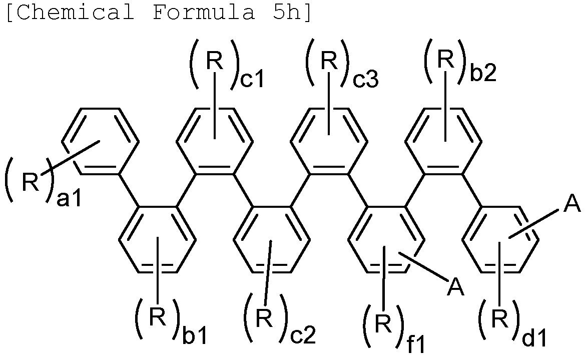 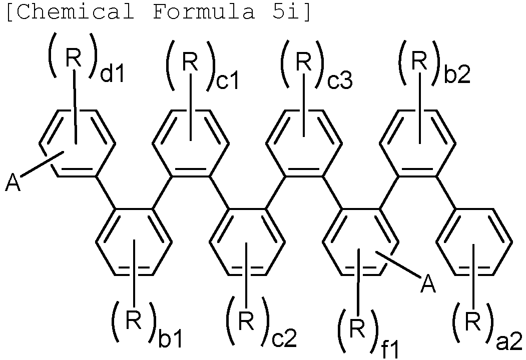 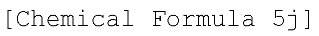 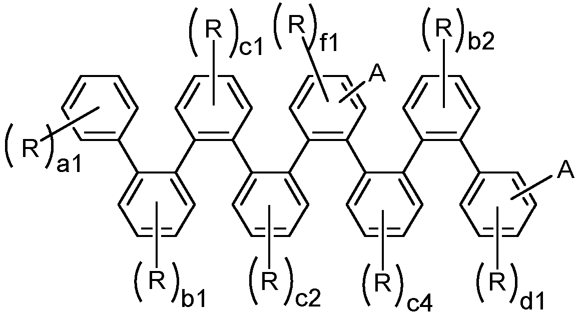 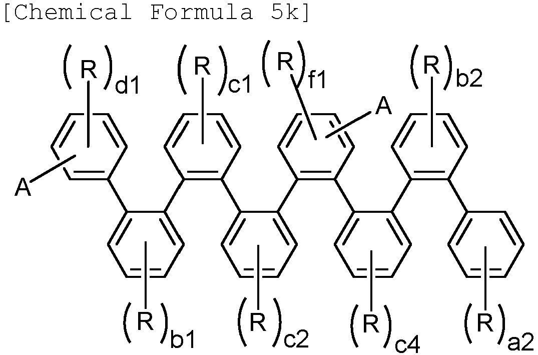 wherein,
A represents the moiety A, which is as defined in Chemical Formulas 1 to 5, with plural A's being the same or different from each other,
a1, a2, b1, b2, c1, c2, c3, c4, and R are each as defined in Chemical Formulas 1 to 5,
d1 and d2 are each independently an integer of 0 to 3,
e1 is an integer of 0 to 2, and
f1 is 0 or 1.

Concrete examples of the organic compound represented by any one of Chemical Formulas 1 to 5 include, but are not limited to, the compounds represented by the following Chemical Formulas 6 to 58: 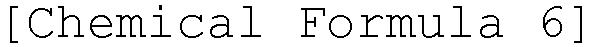 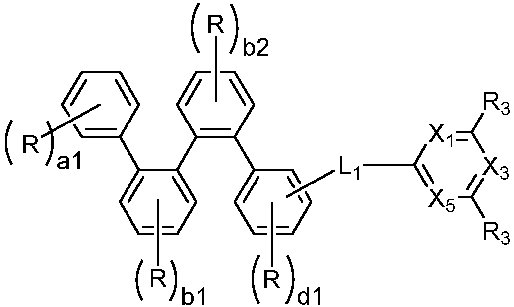 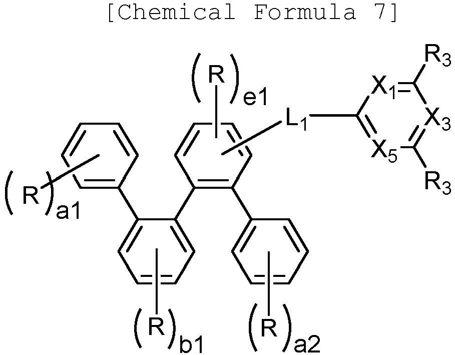 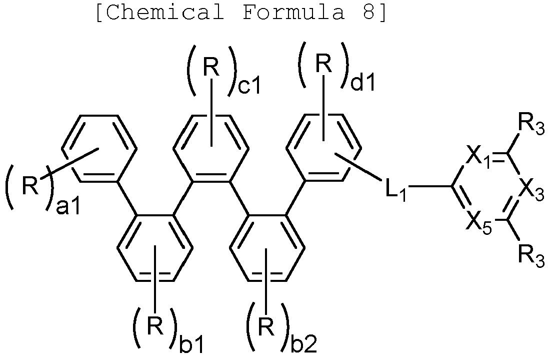 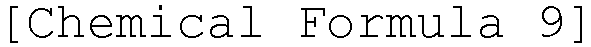 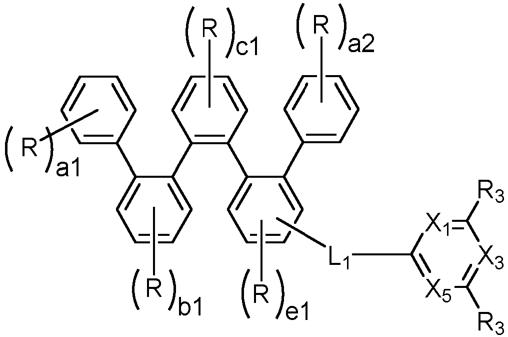 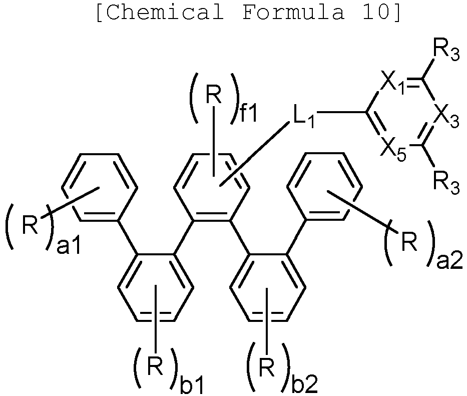 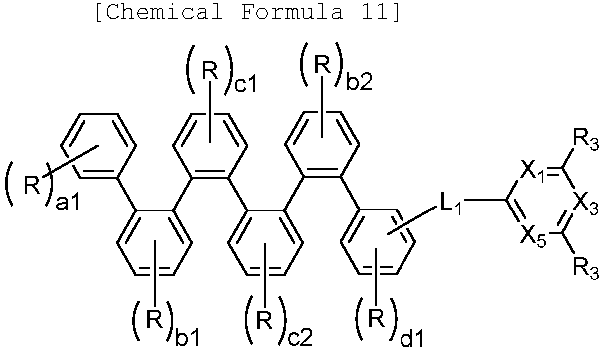 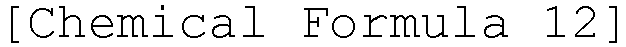 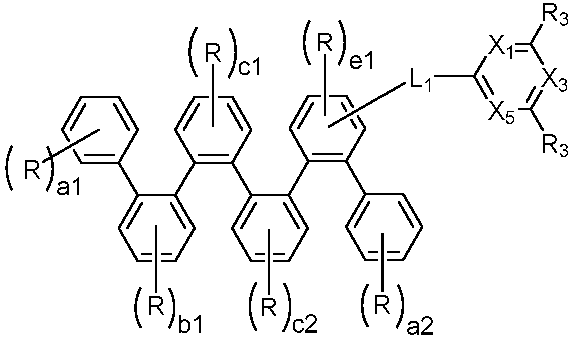 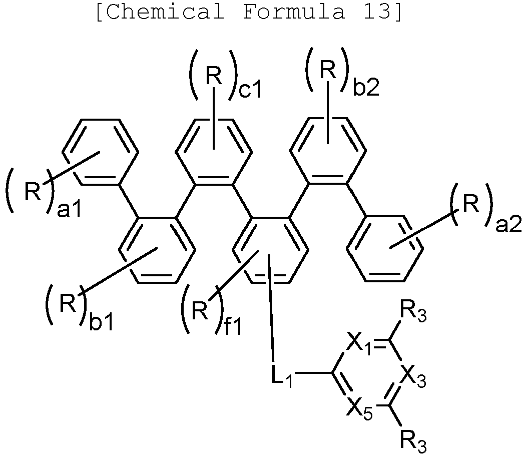 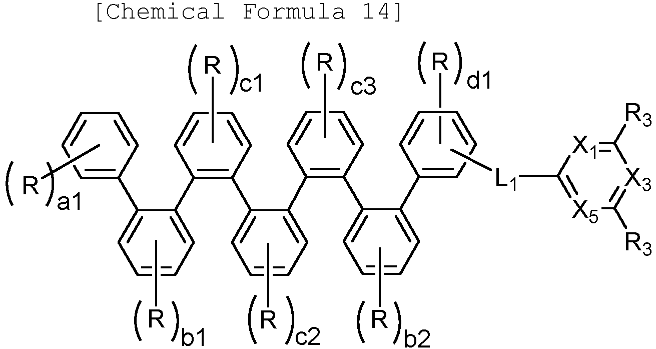 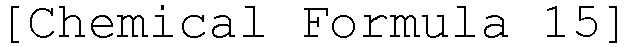 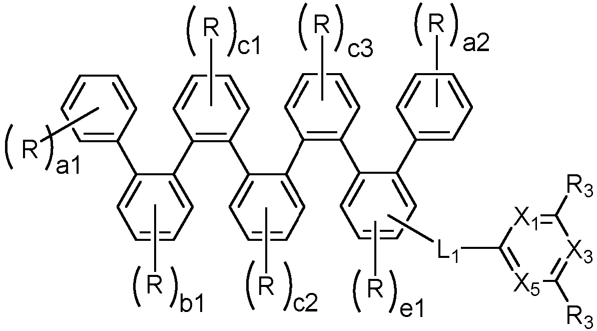 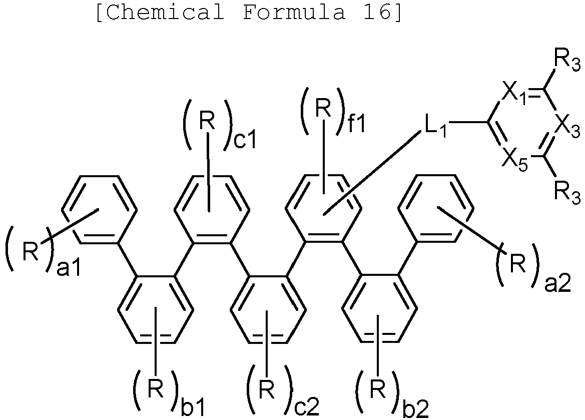 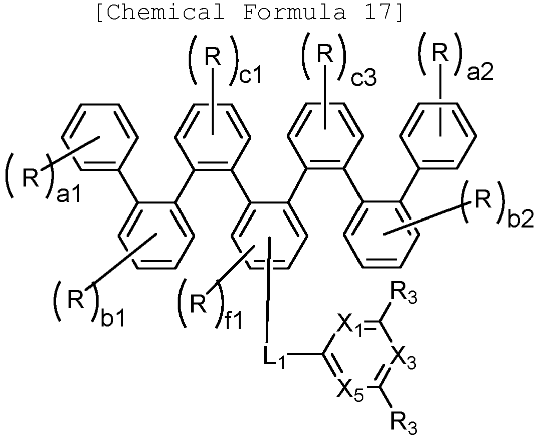 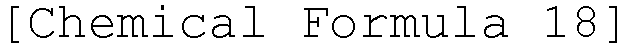 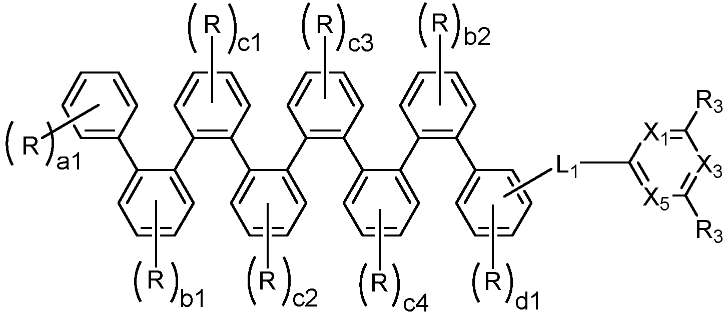 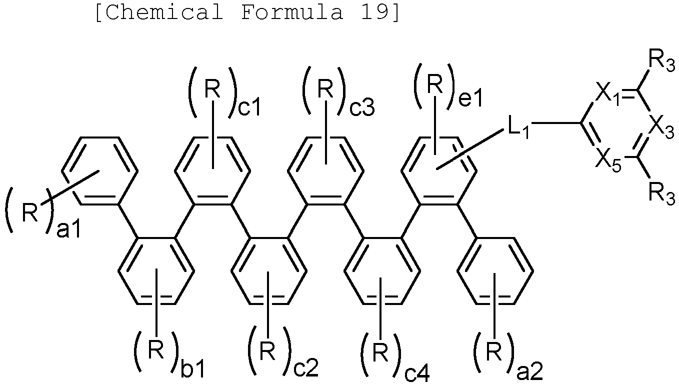 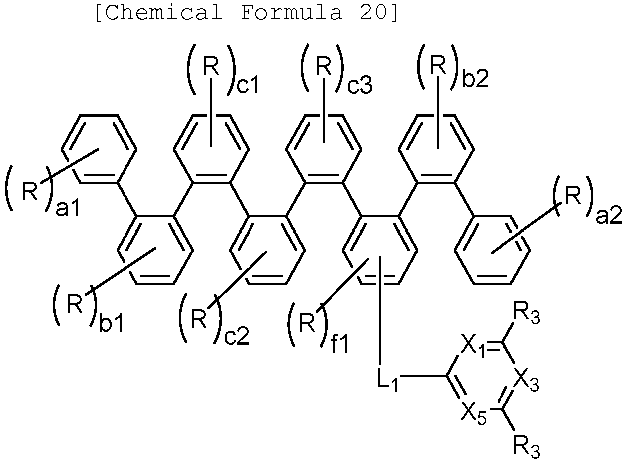 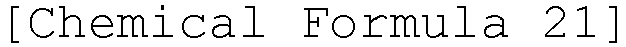 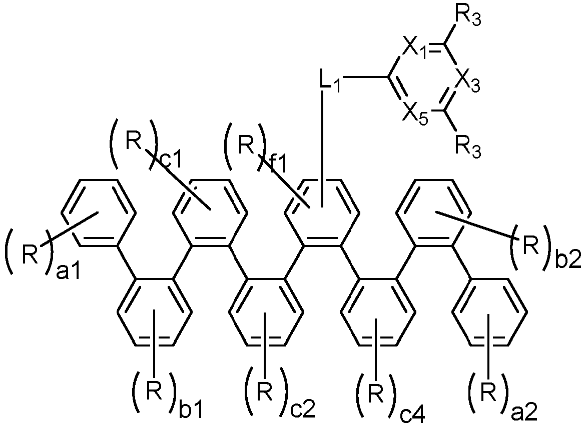 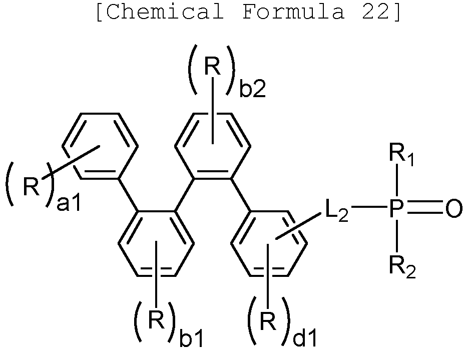 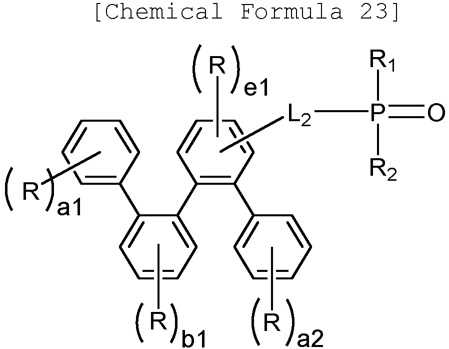 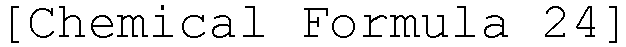 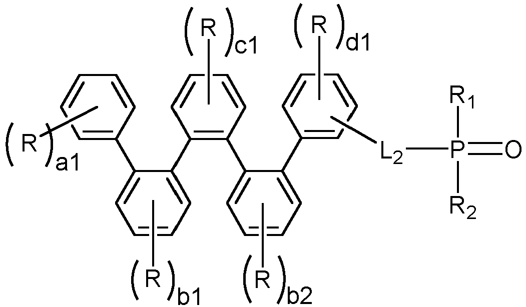 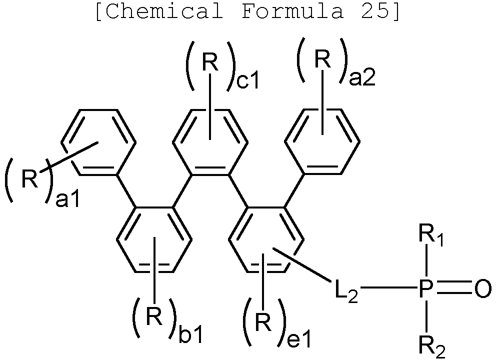 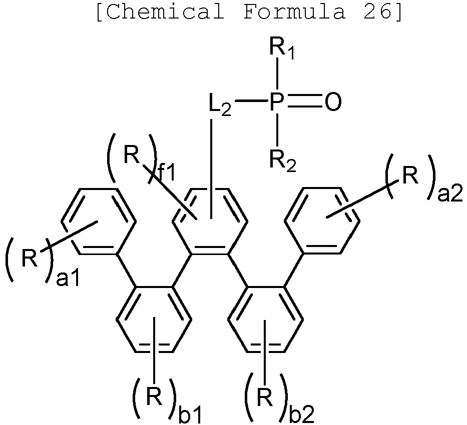 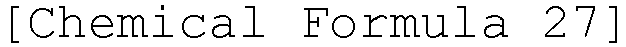 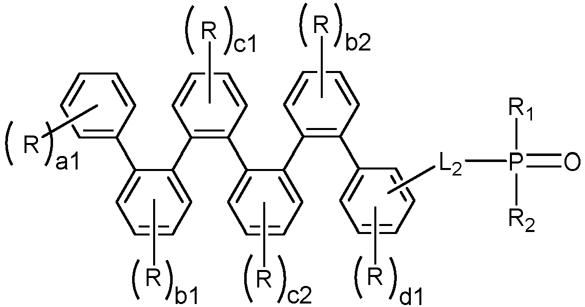 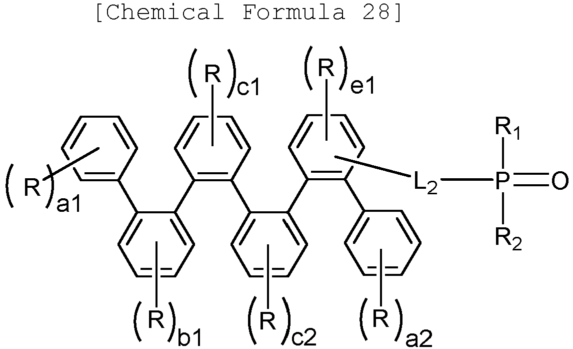 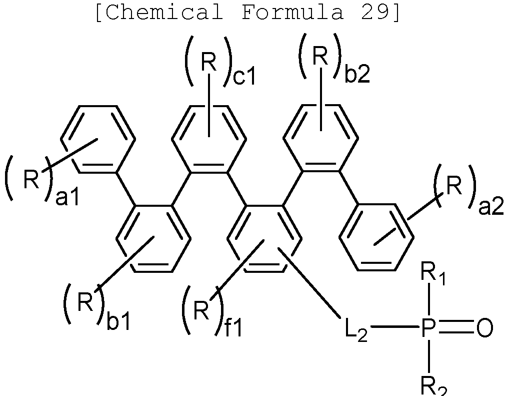 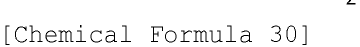 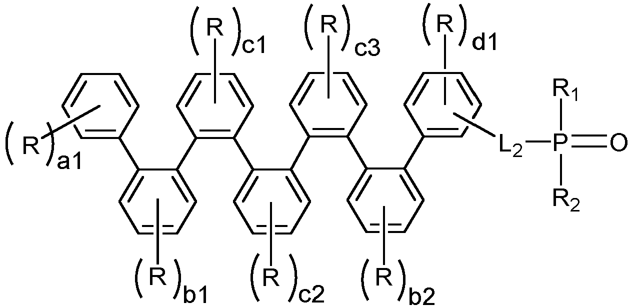 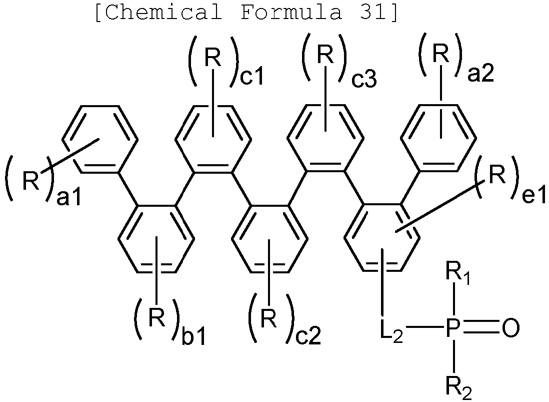 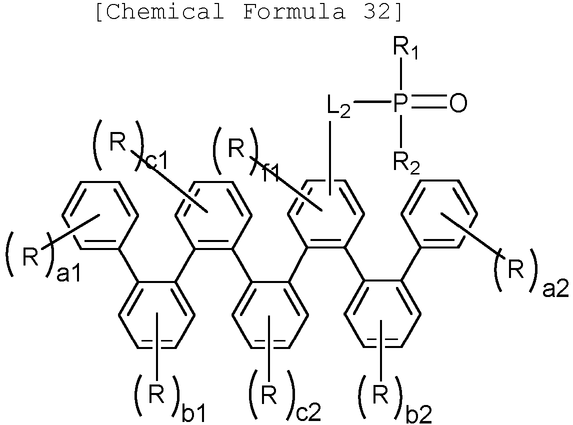 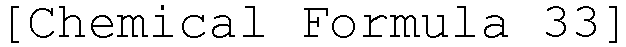 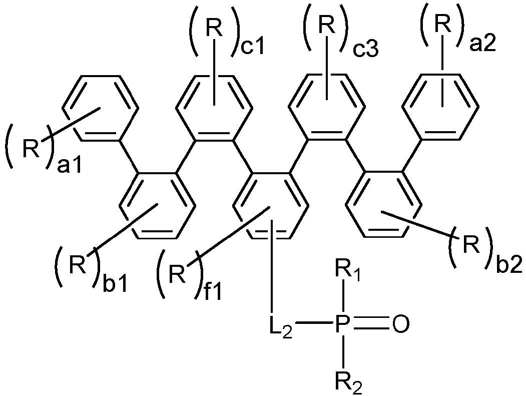 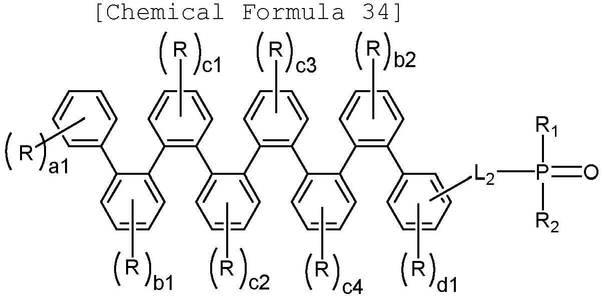 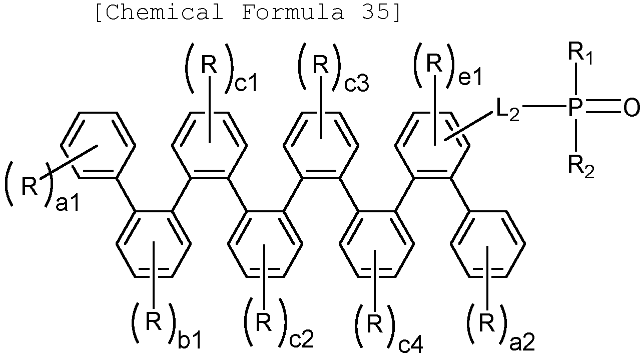 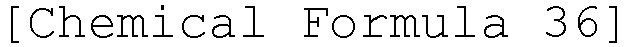 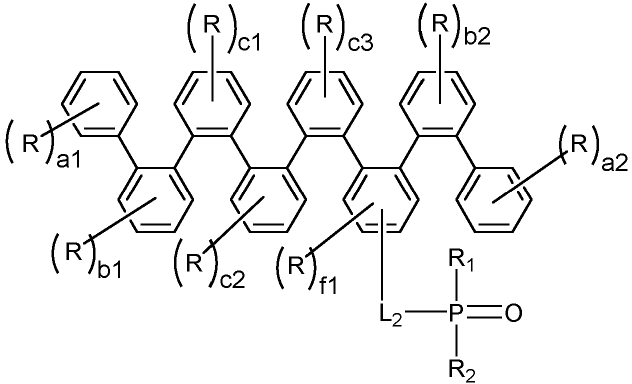 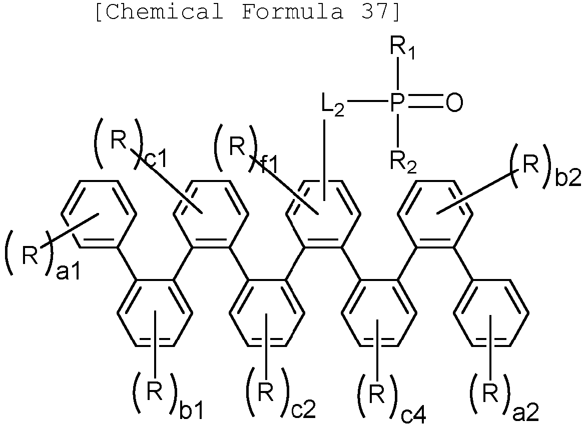 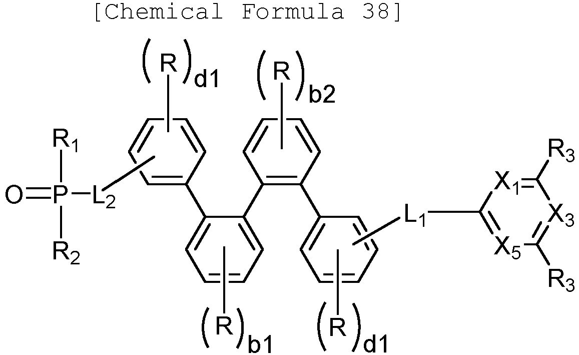 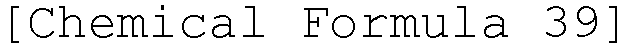 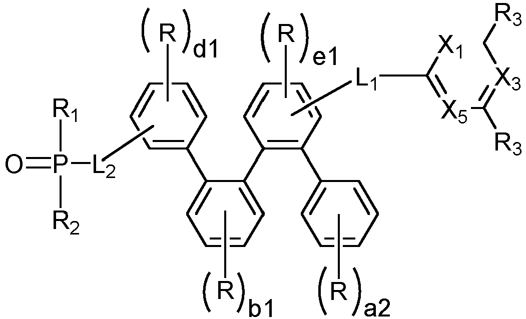 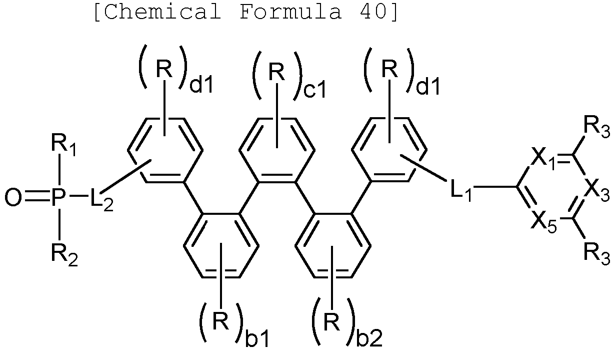 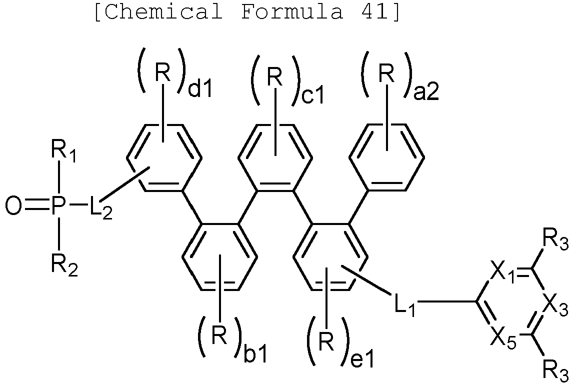 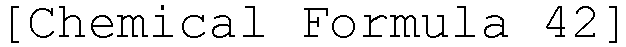 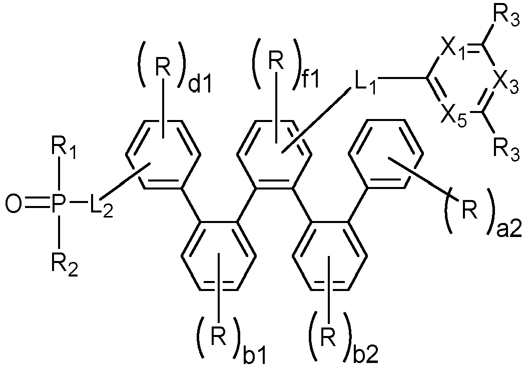 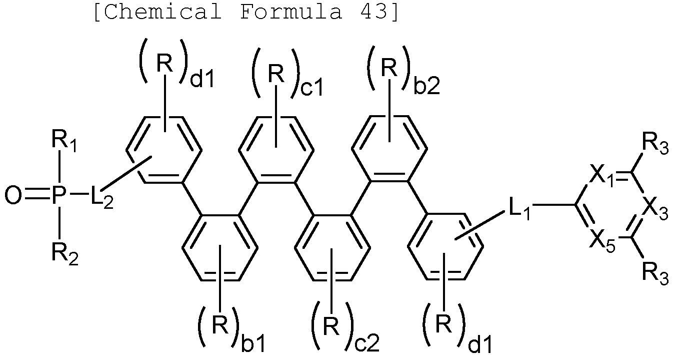 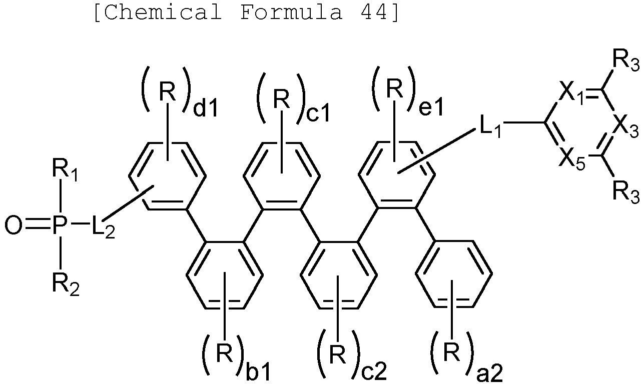 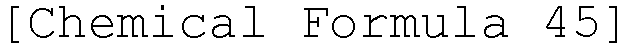 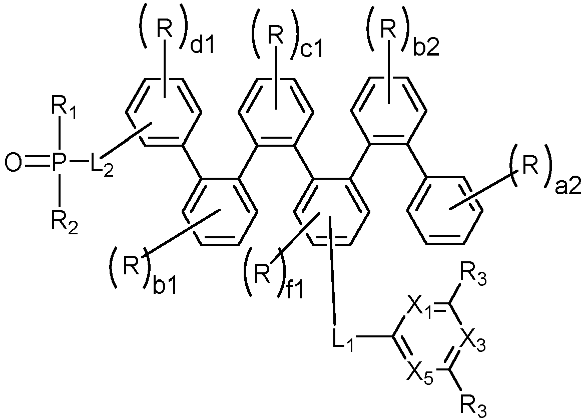 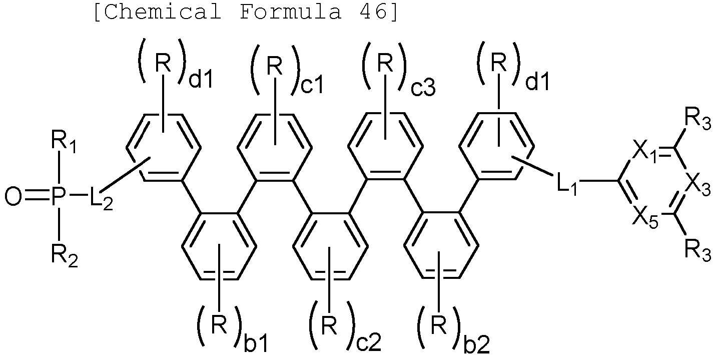 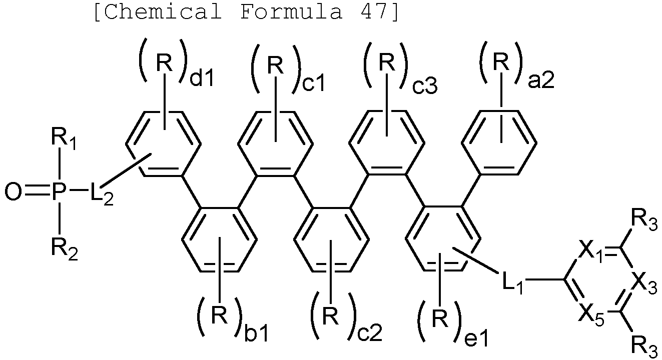 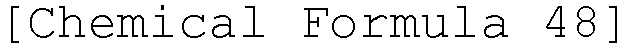 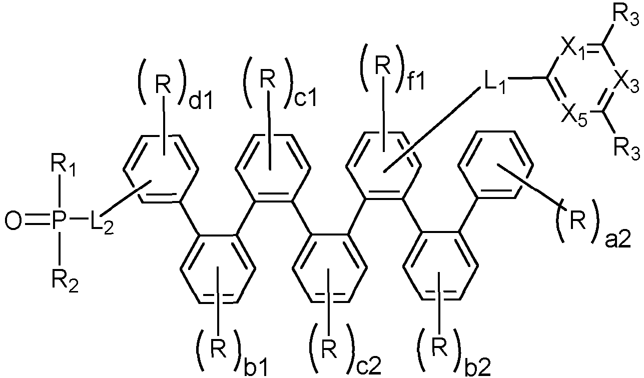 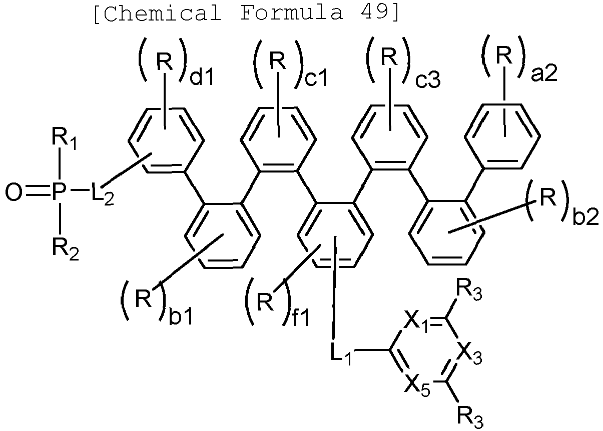 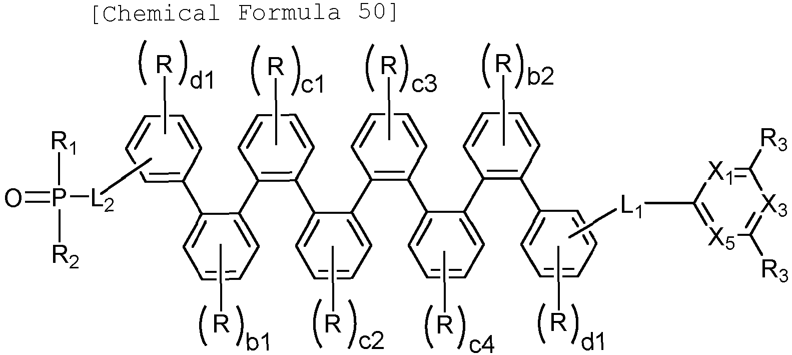 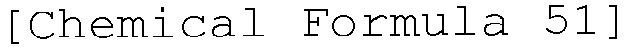 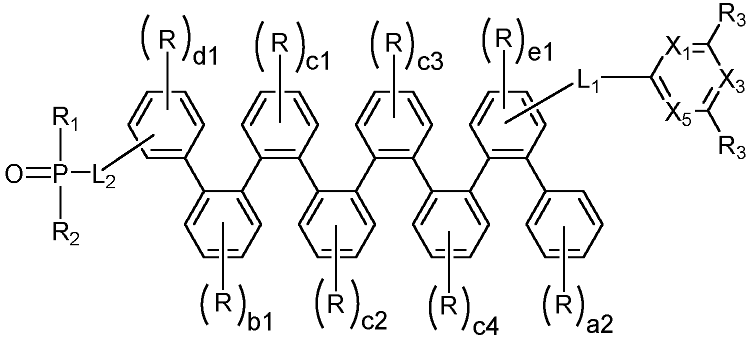 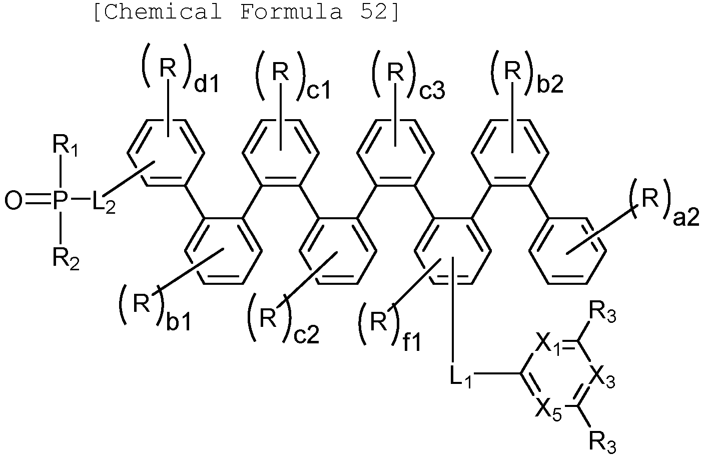 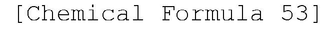 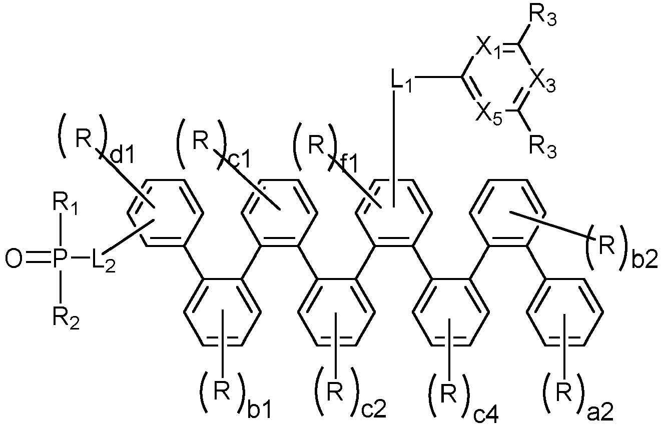 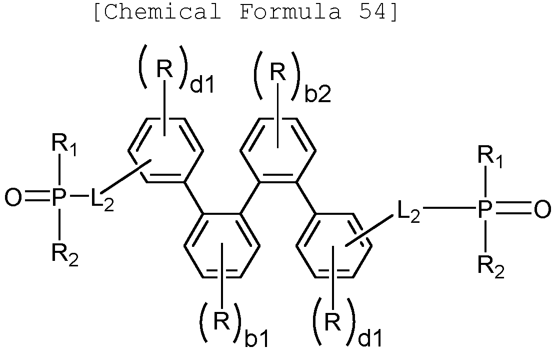 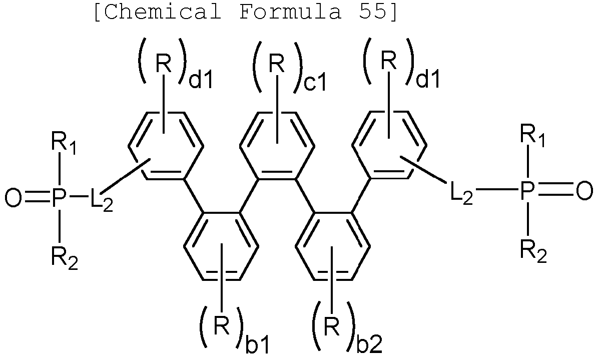 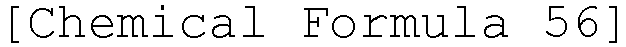 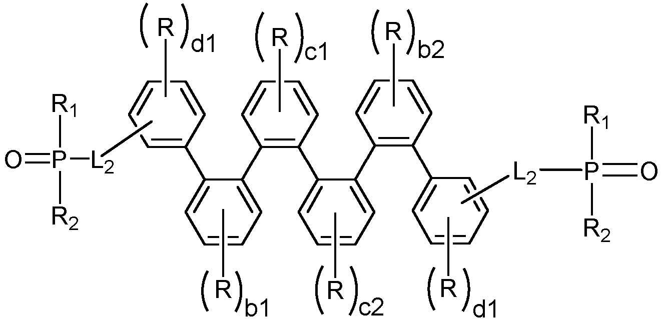 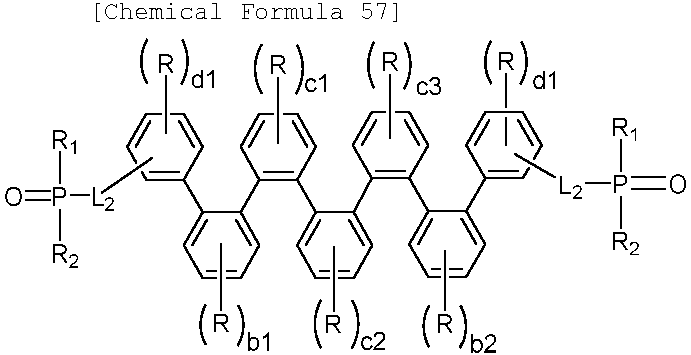 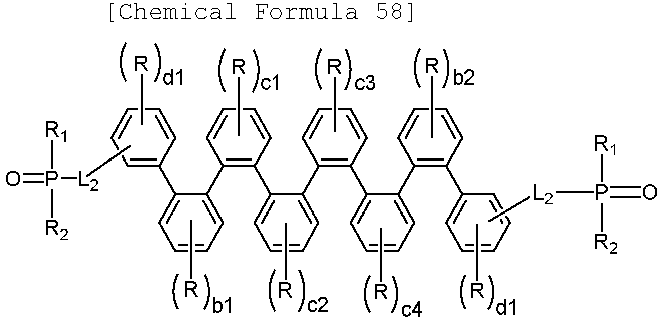 wherein,
a1, b1, b2, c1, c2, c3, c4, R, R₁, R₂, L₁, and L₂ are each as defined in Chemical Formulas 1 to 5,
d1 is an integer of 0 to 3,
e1 is an integer of 0 to 2,
f1 is 0 or 1,
X₁, X₃, and X₅, which are the same or different from each other, are each independently N or C(R₃), with a proviso that at least one of X₁, X₃, and X₅ is N, and
R₃ is as defined in Chemical Formulas 1 to 5.

Depending on the aforementioned R and moiety A, the organic compound represented by any one of Chemical Formulas 1 to 5 may further be represented by any one of Chemical Formulas 59 to 126, but is not limited thereto: 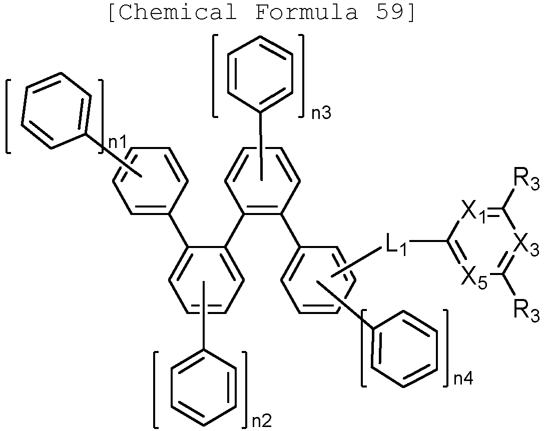 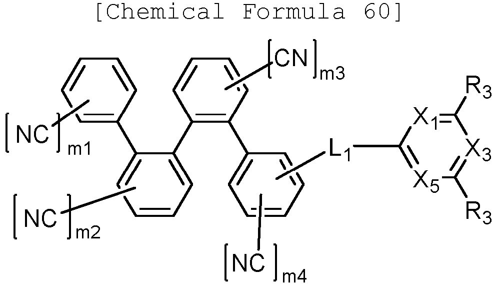 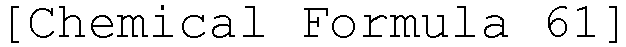 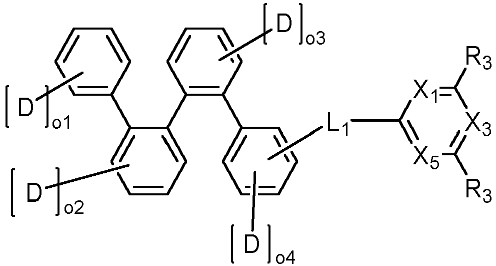 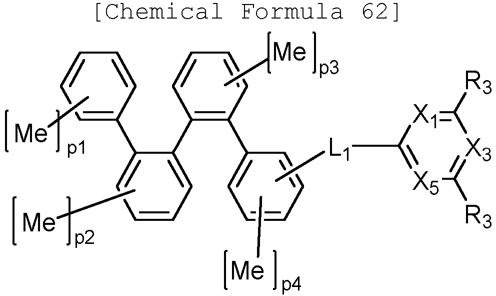 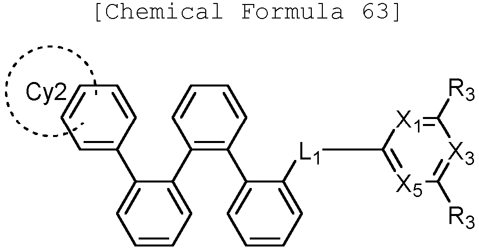 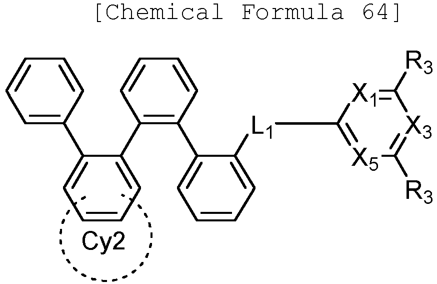 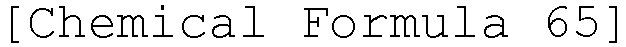 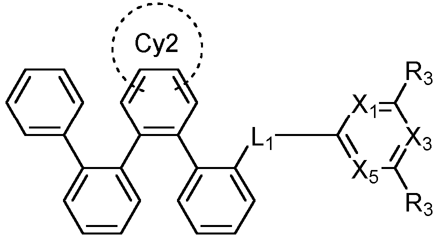 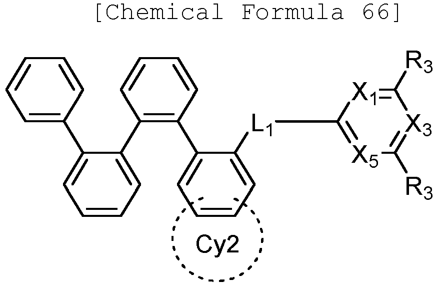 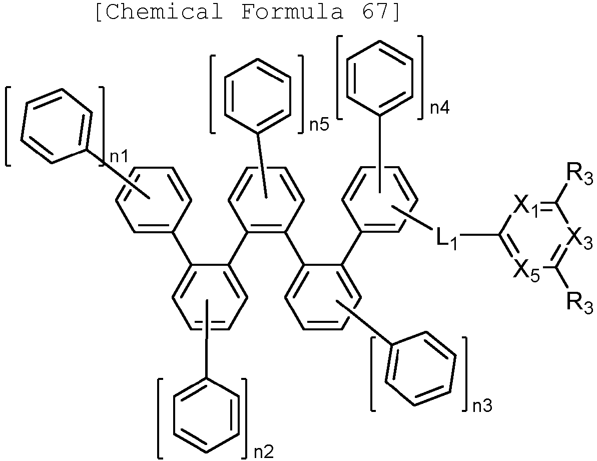 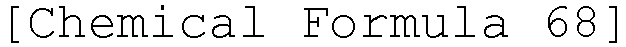 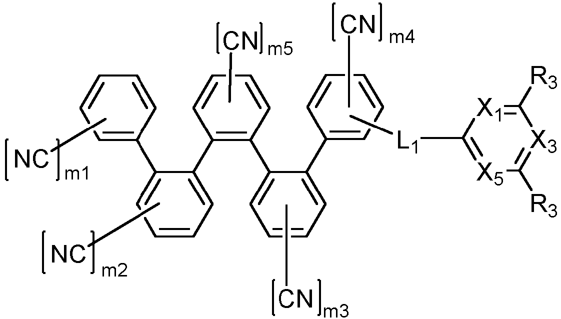 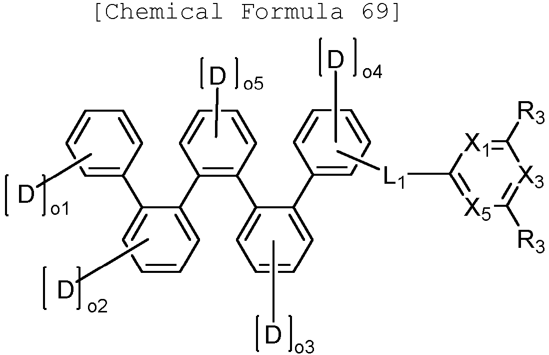 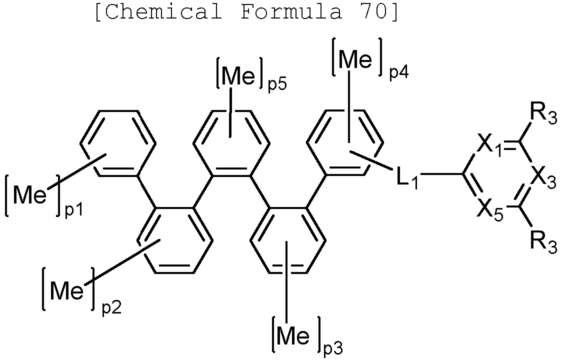 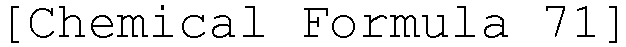 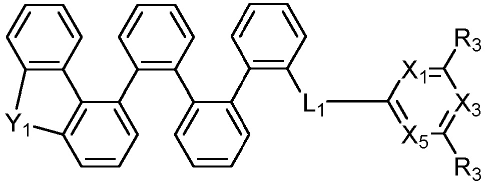 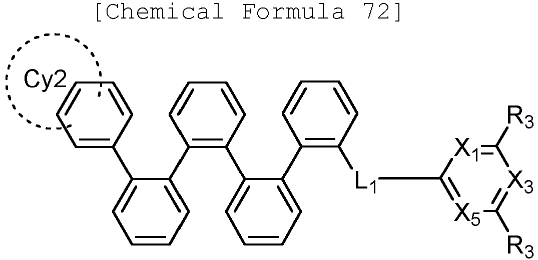 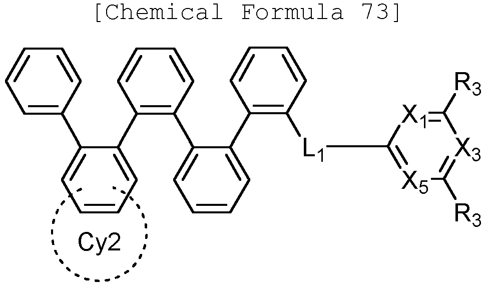 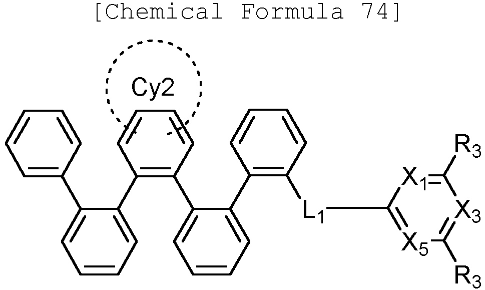 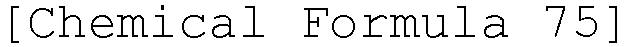 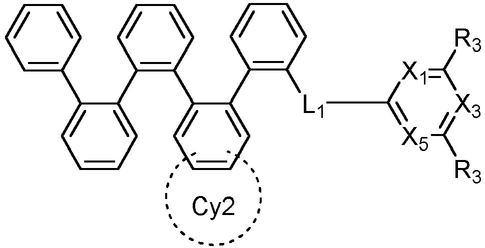 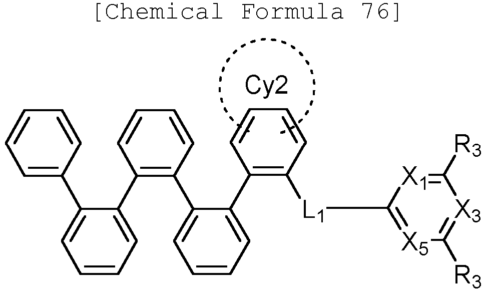 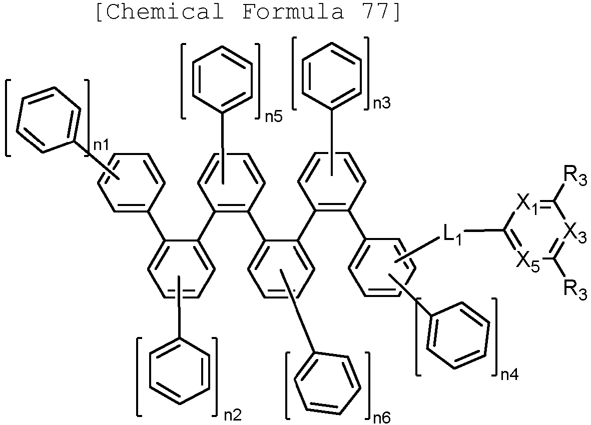 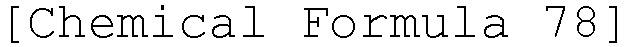 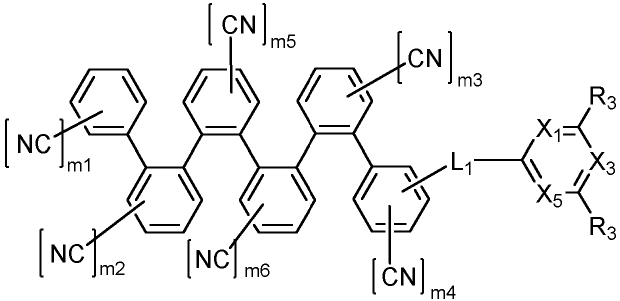 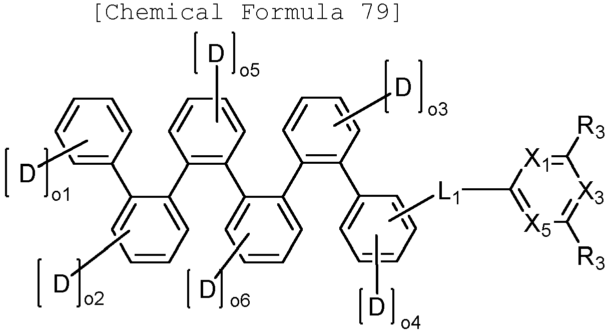 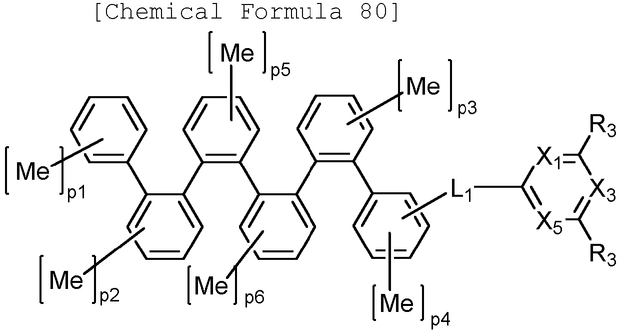 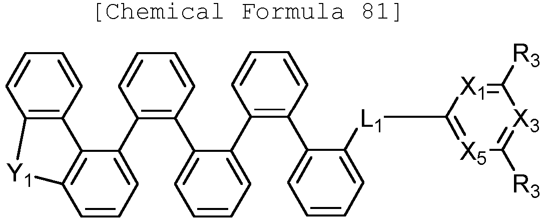 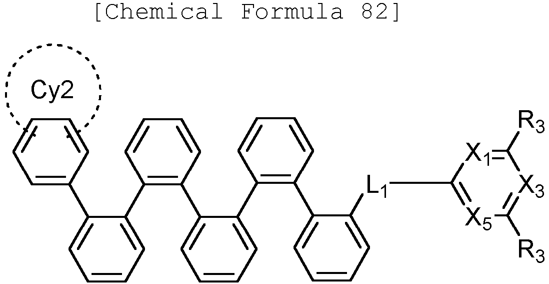 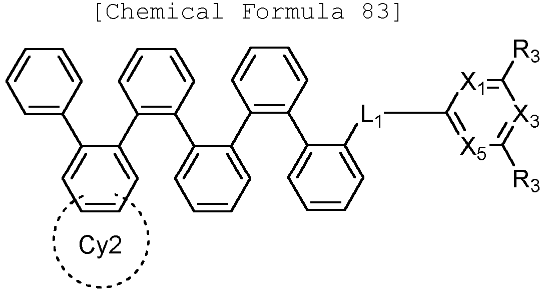 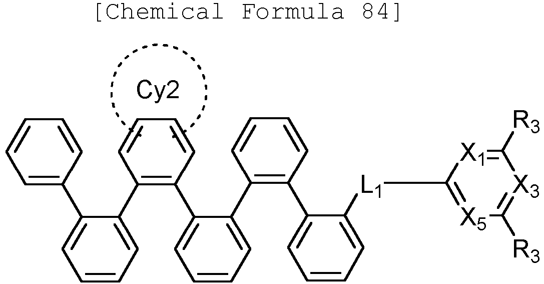 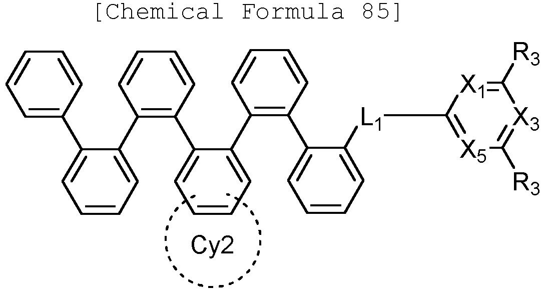 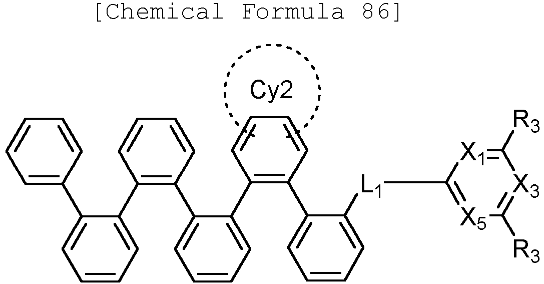 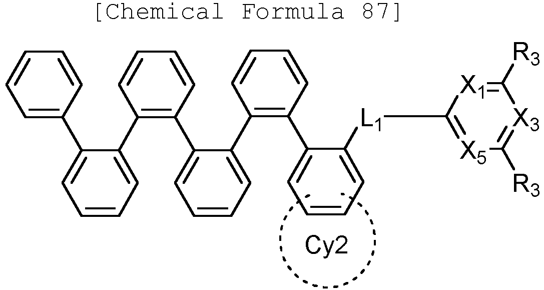 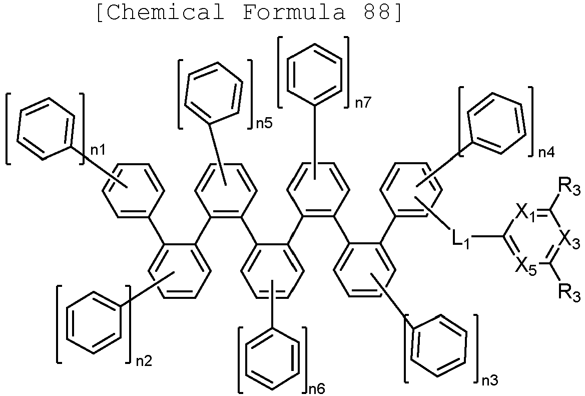 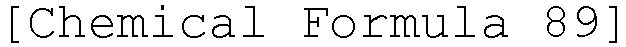 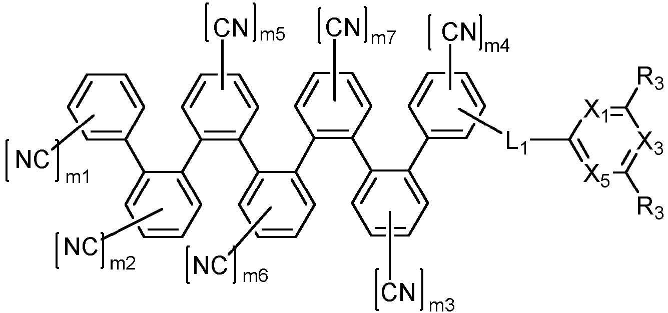 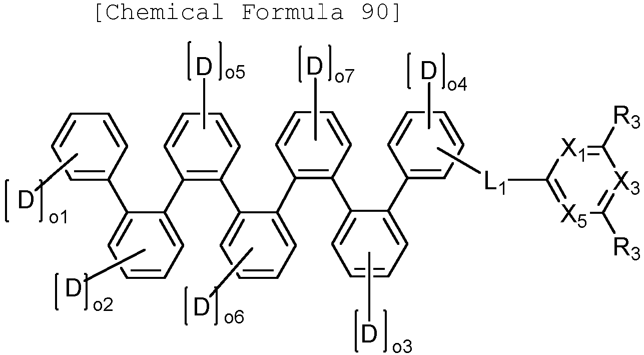 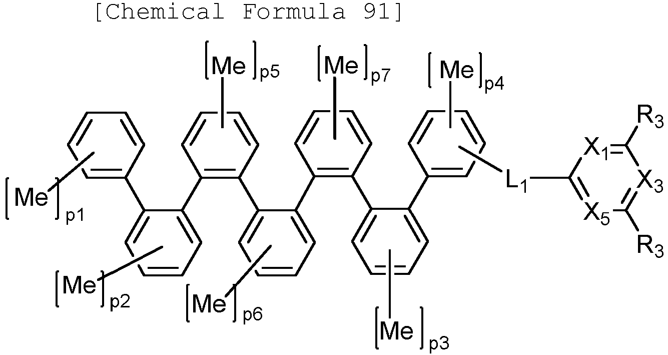 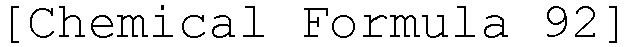 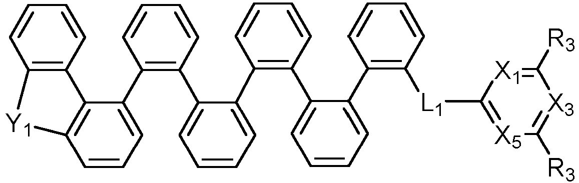 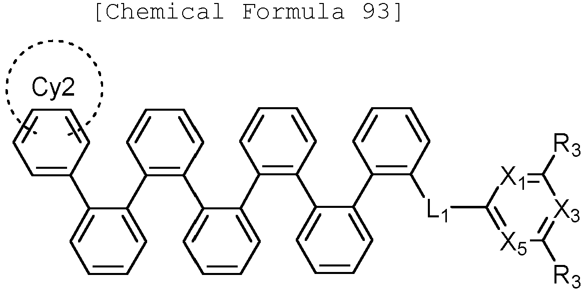 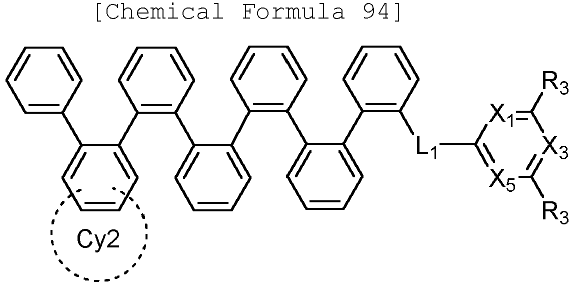 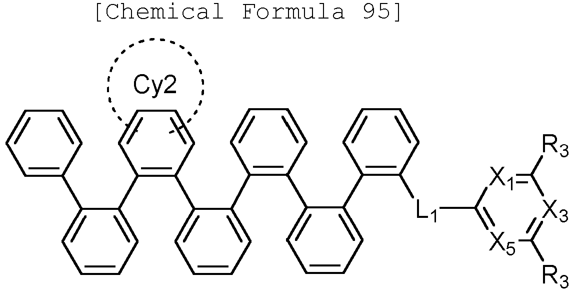 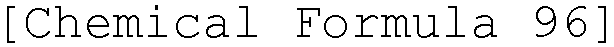 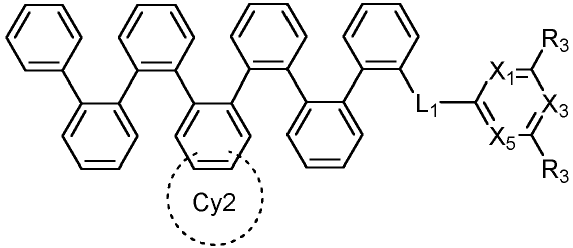 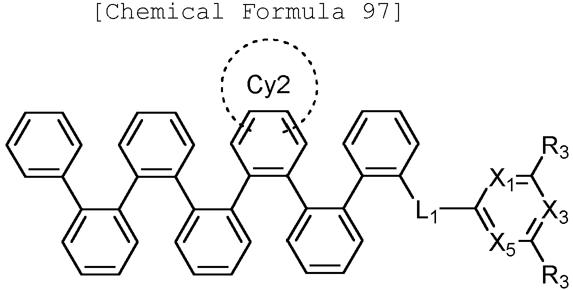 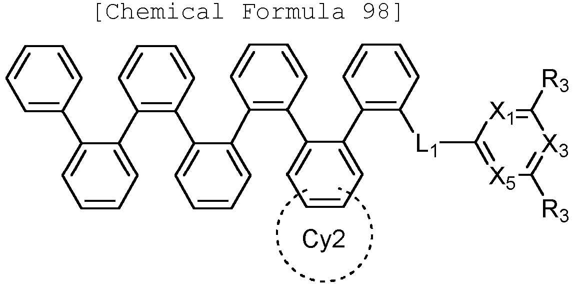 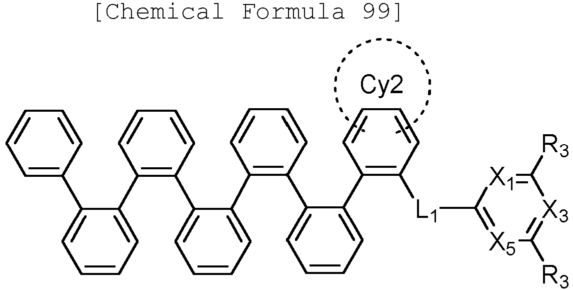 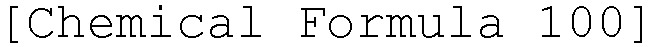 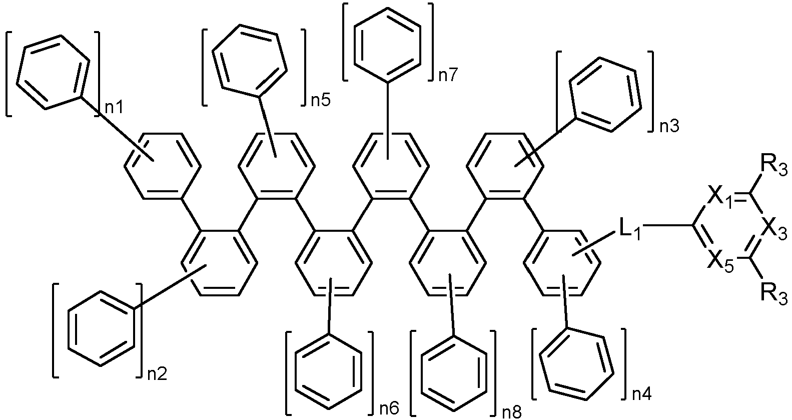 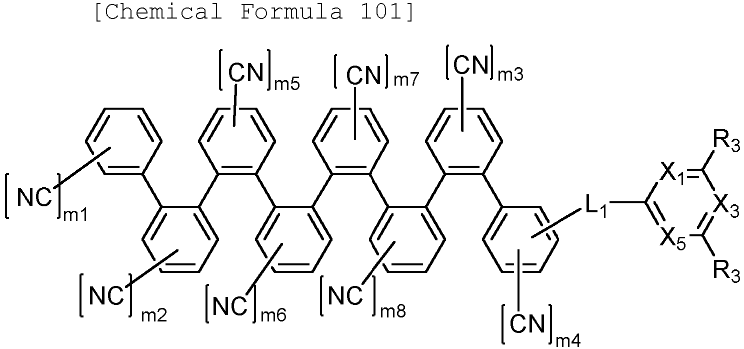 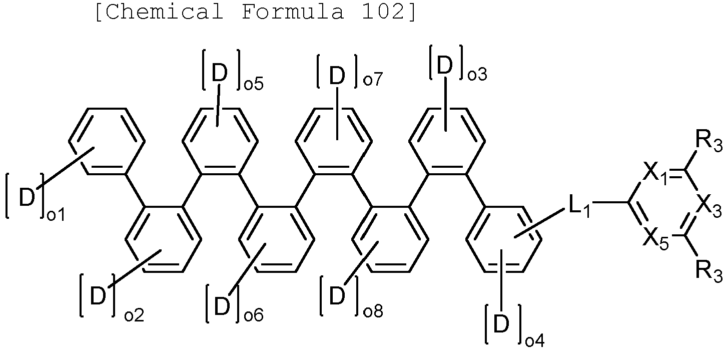 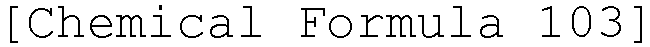 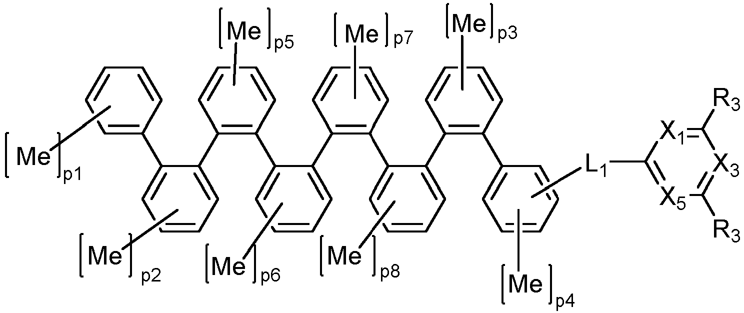 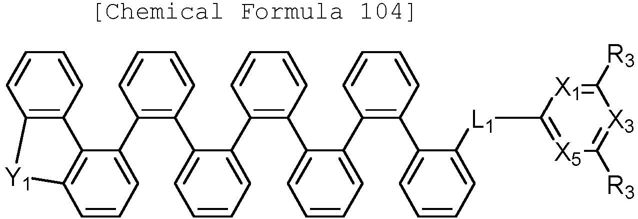 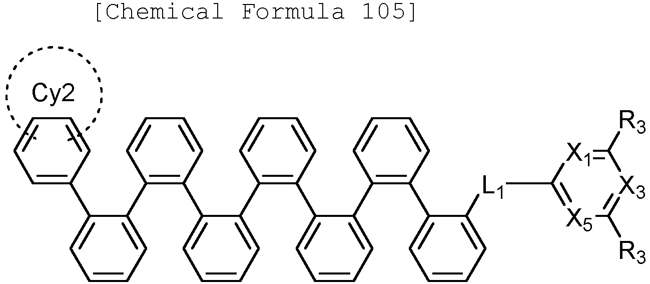 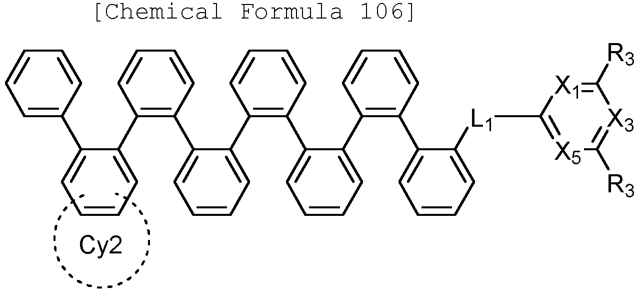 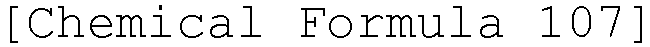 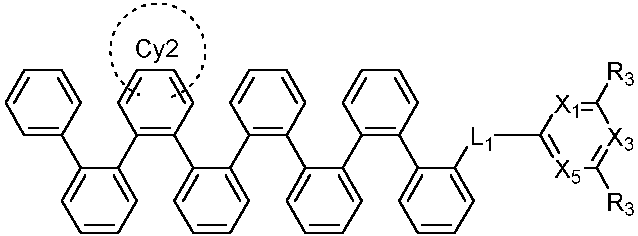 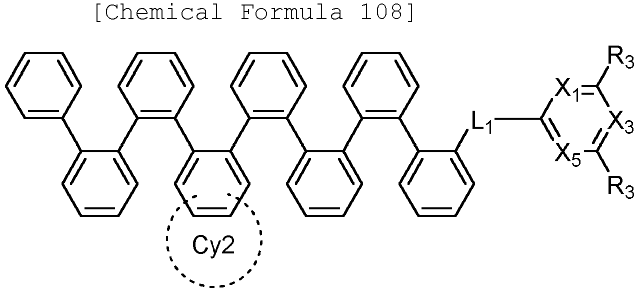 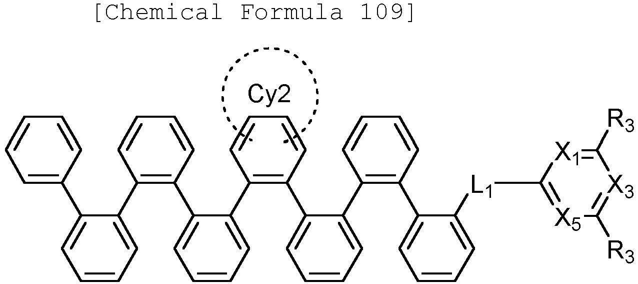 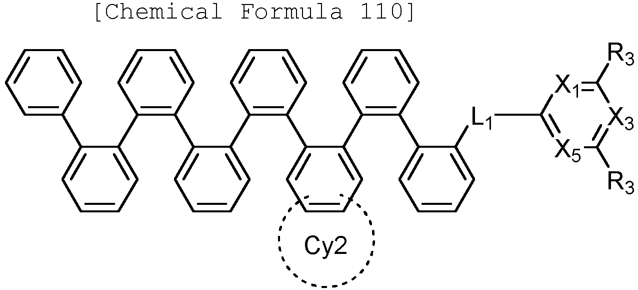 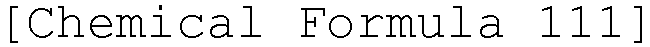 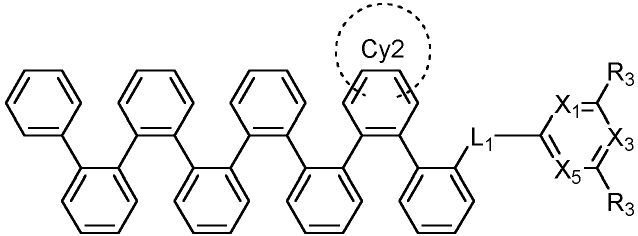 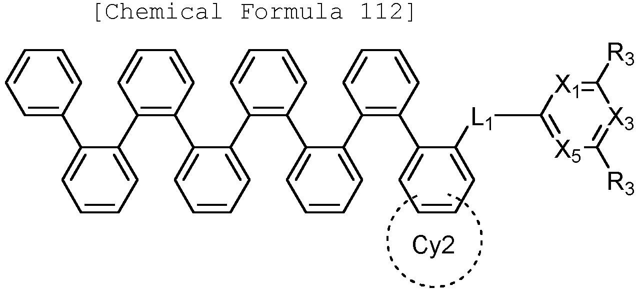 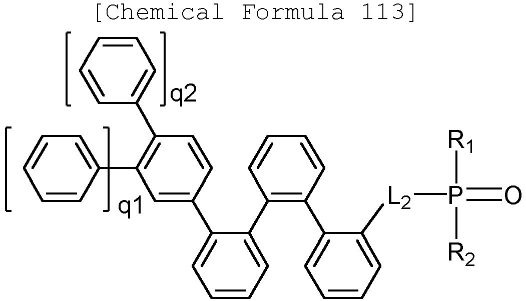 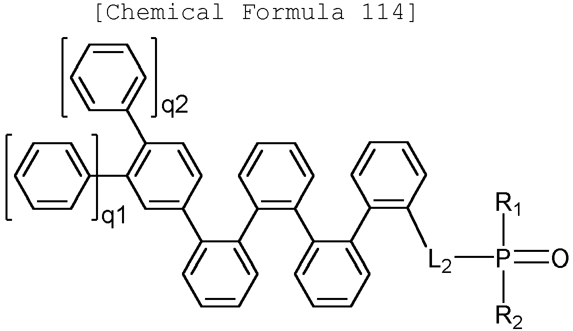 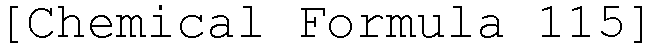 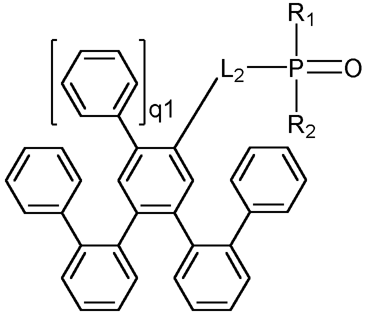 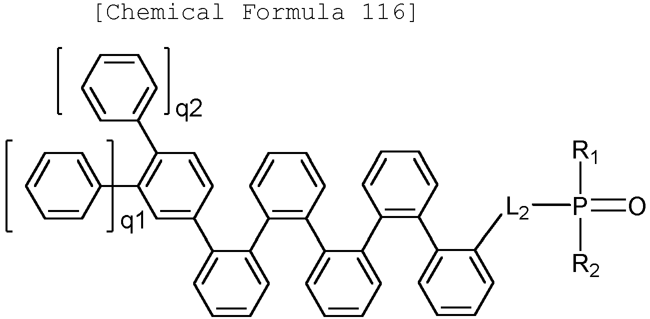 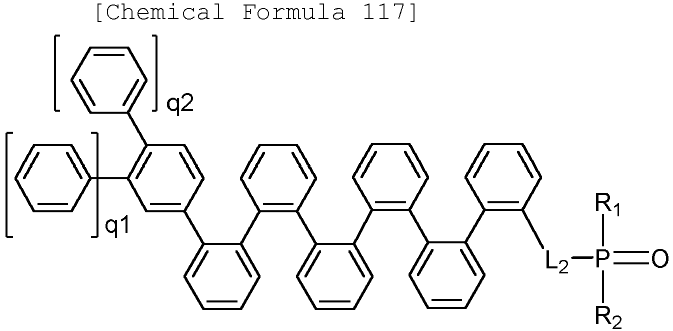 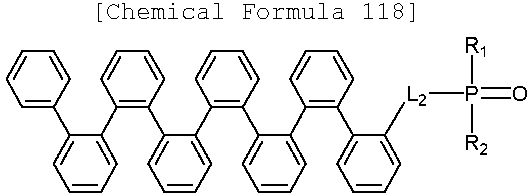 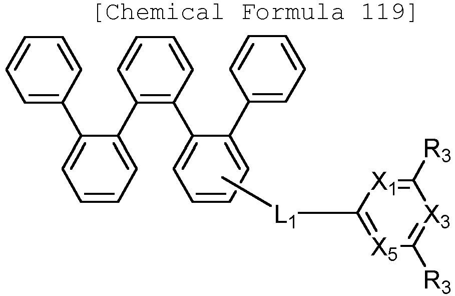 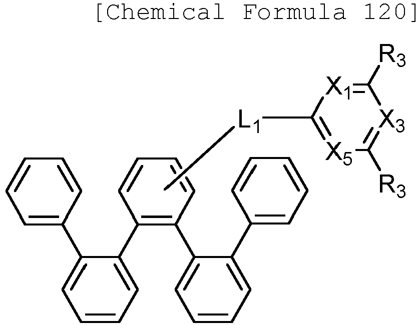 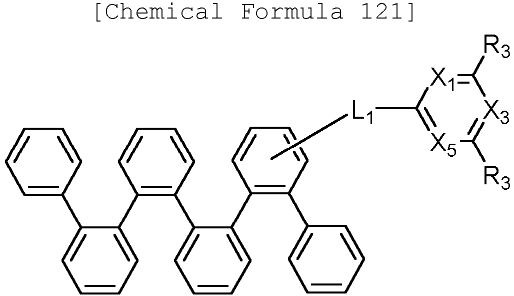 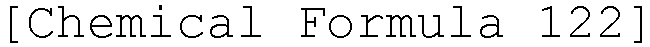 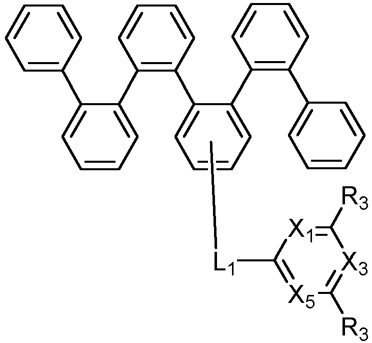 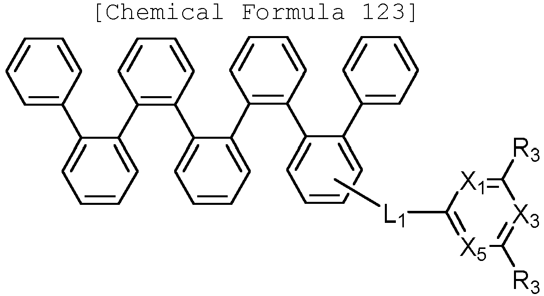 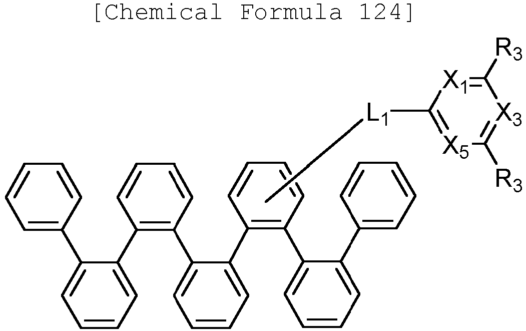 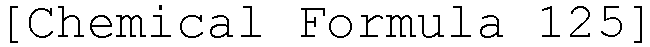 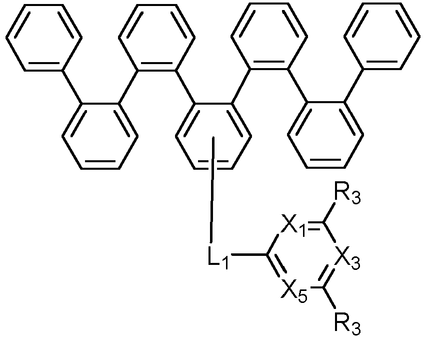 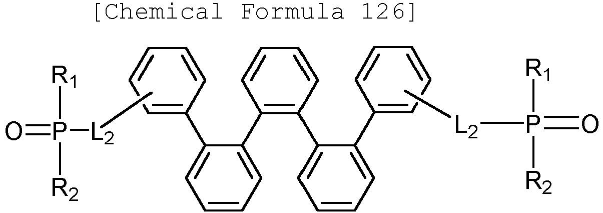 wherein,
n1, m1, o1, and p1 are each independently an integer of 0 to 4,
n2, n3, n4, m2, m3, m4, o2, o3, o4, p2, p3, and p4 are each independently an integer of 0 to 3,
n5, n6, n7, n8, m5, m6, m7, m8, o5, o6, o7, o8, p5, p6, p7, and p8 are each independently an integer of 0 to 2,
CN represents a cyano group,
D represents a deuterium atom,
Me represents a methyl group,
q1 and q2 are each independently 0 or 1,
Y₁ is O, S, or C(R₅) (R₆),
Cy2 is selected from the group consisting of the following fused rings Cy2-1 to Cy2-7, 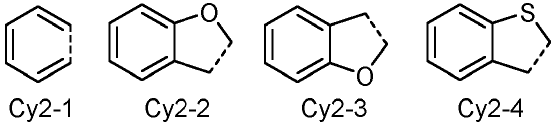 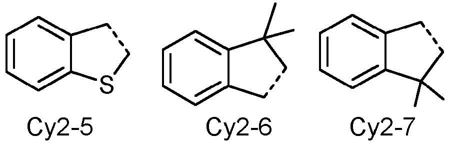
X₁, X₃, and X₅, which are the same or different from each other, are each independently N or C(R₃), with a proviso that at least one of X₁, X₃, and X₅ is N,
L₁, L₂, R₁, R₂, and R₃ are each as defined in Chemical Formulas 1 to 5,
R₅ and R₆, which are the same or different from each other, are each independently selected from the group consisting of a hydrogen atom, a deuterium atom (D), a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, and a heteroaryl group having 5 to 60 nuclear atoms or may form a fused ring with an adjacent group.

The organic compound according to the present disclosure, as described above, may be further exemplified by specific compounds, such as Compounds A1-A120, B1-B120, C1-C120, D1-D104, E1-E100, F1-F74, G1-G88, H1-H90, I1-I58, J1-J120, K1-K80, L1-L39, M1-M45, N1-N50, and 01-064, but is not limited thereto:

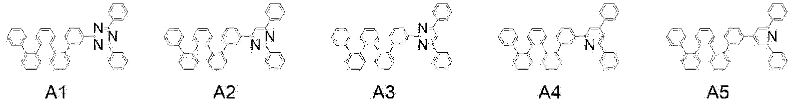

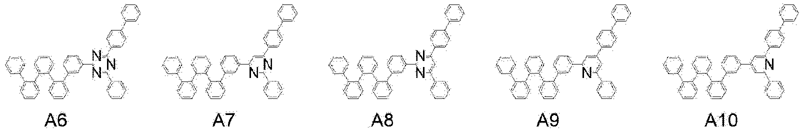

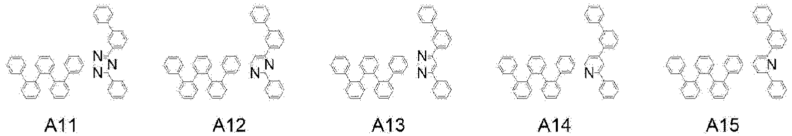

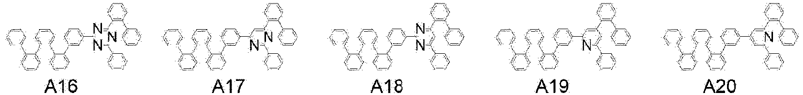

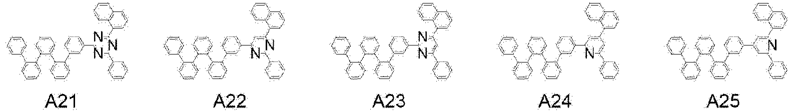

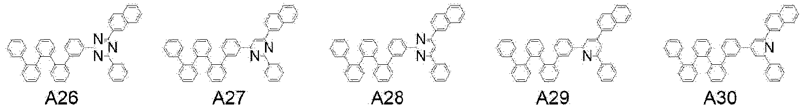

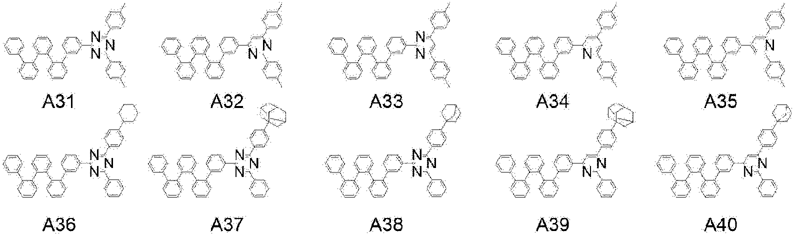

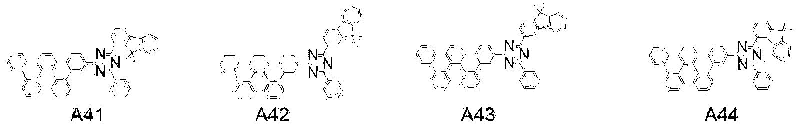

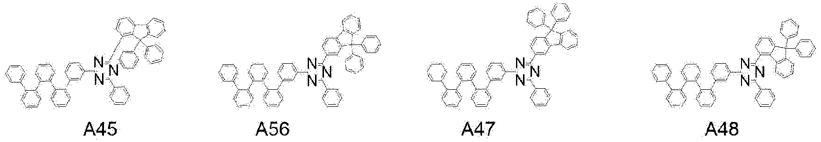

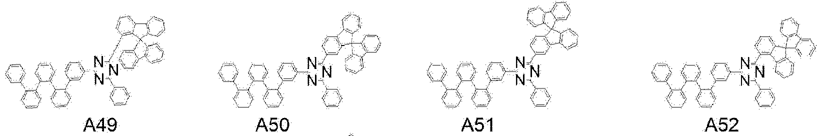

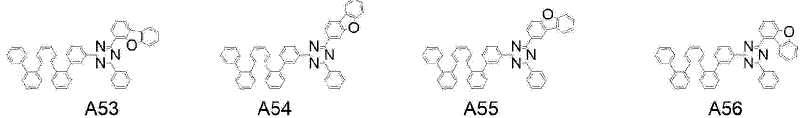

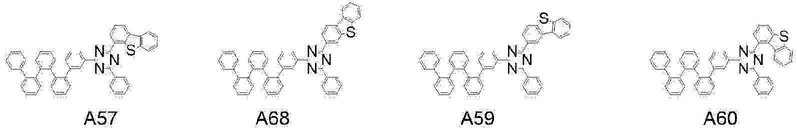

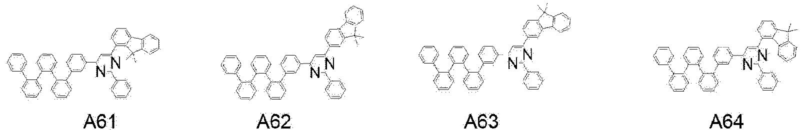

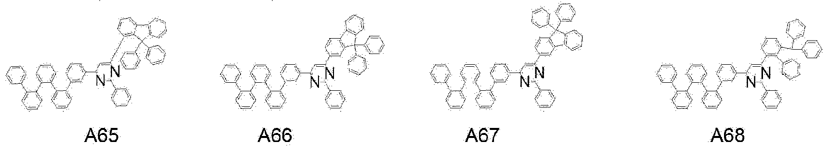

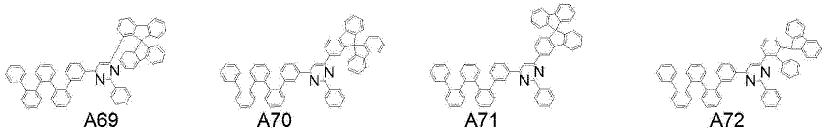

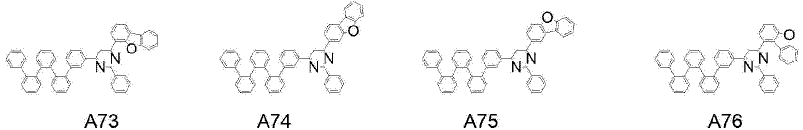

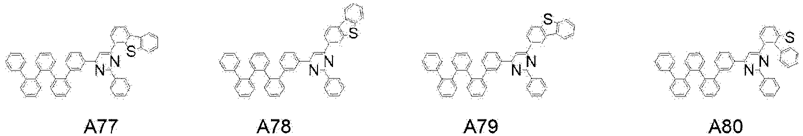

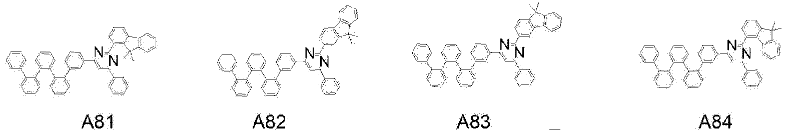

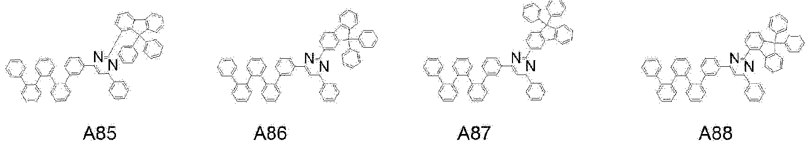

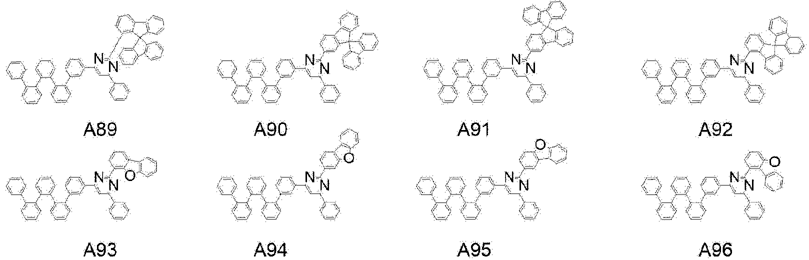

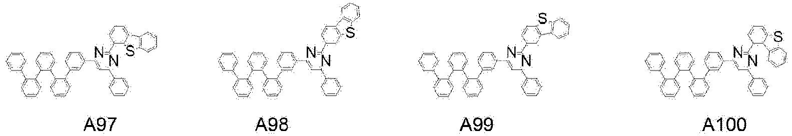

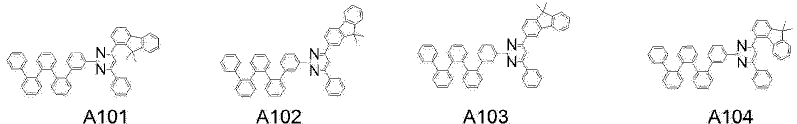

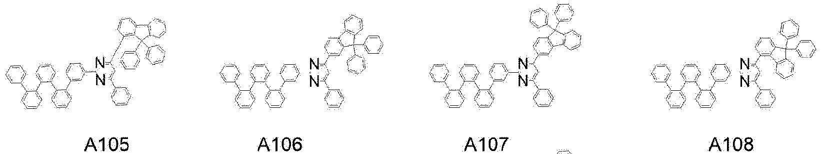

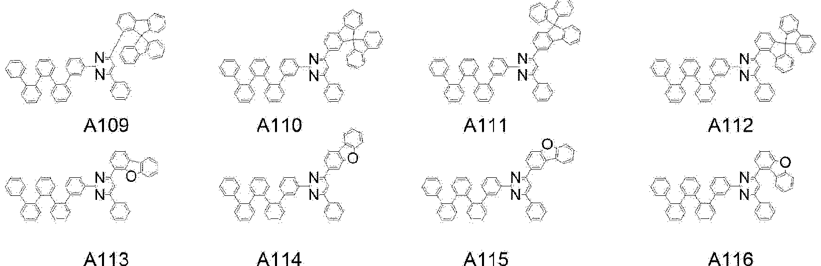

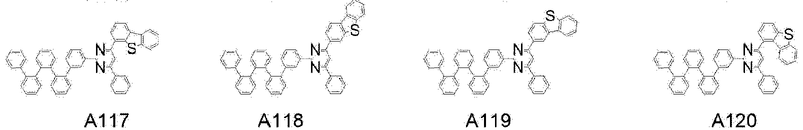

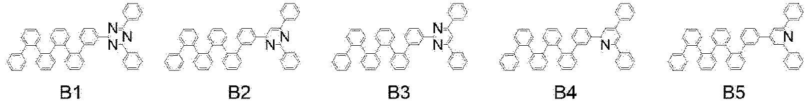

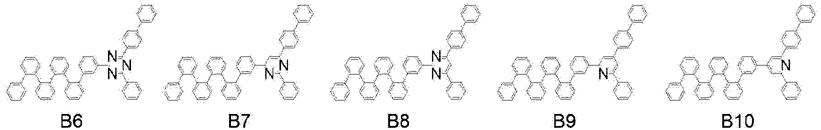

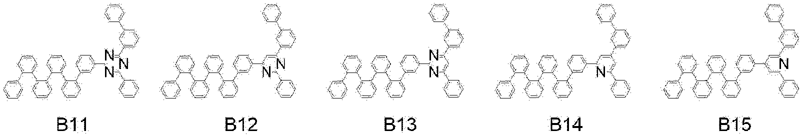

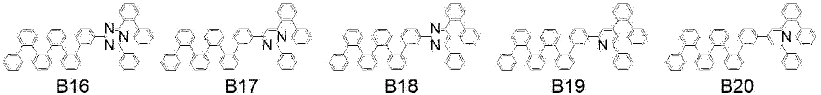

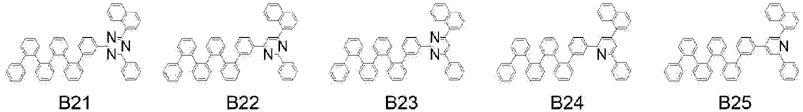

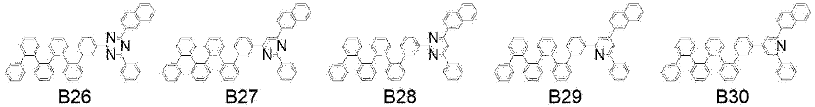

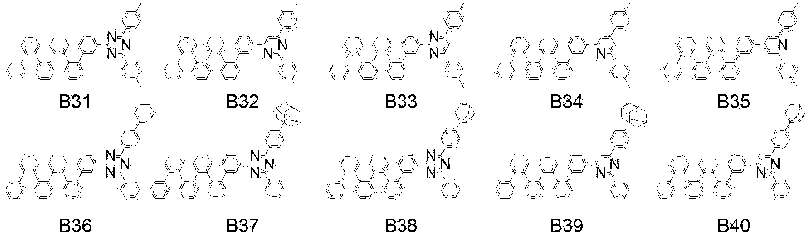

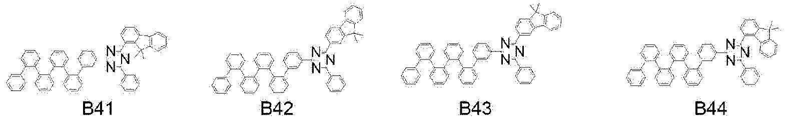

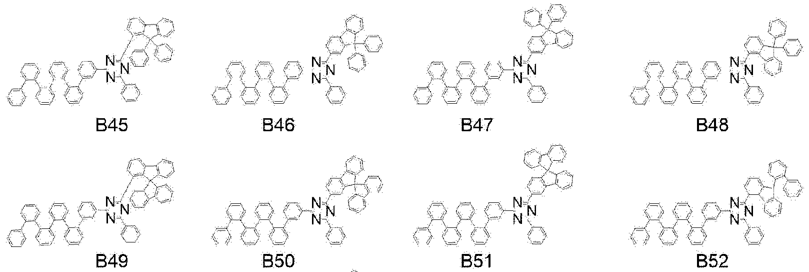

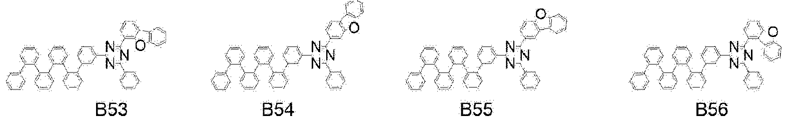

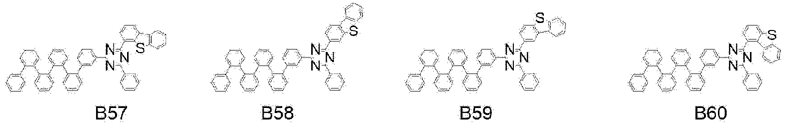

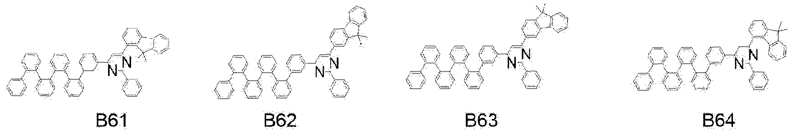

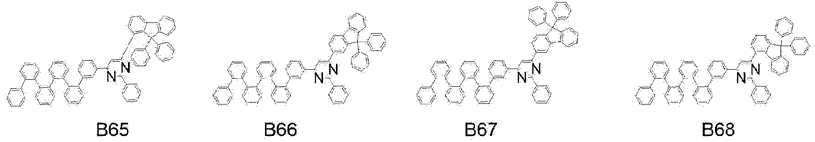

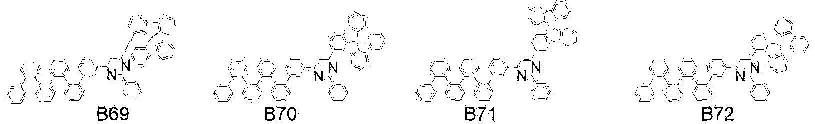

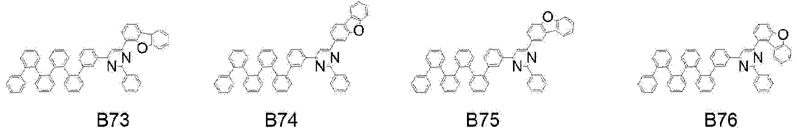

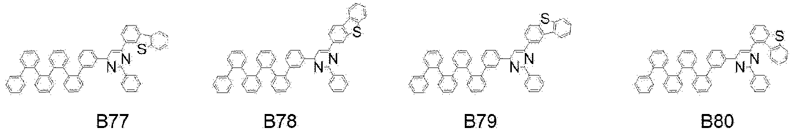

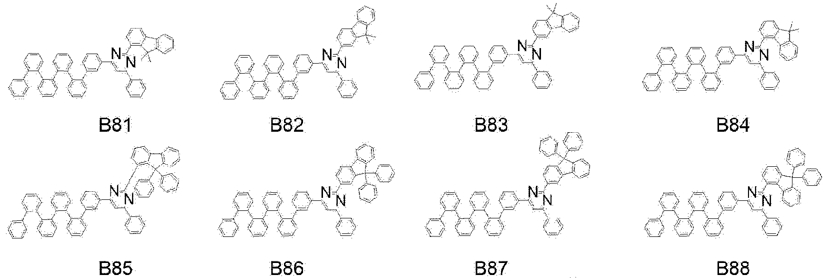

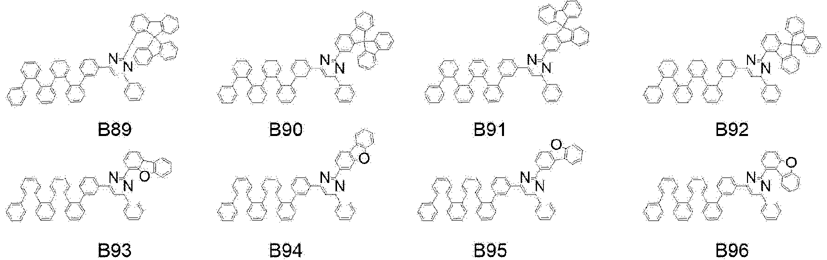

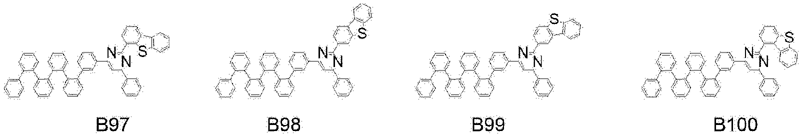

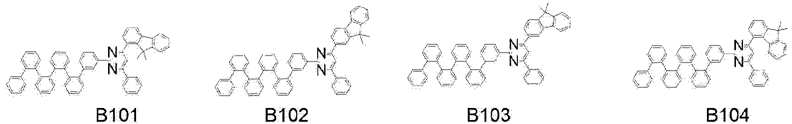

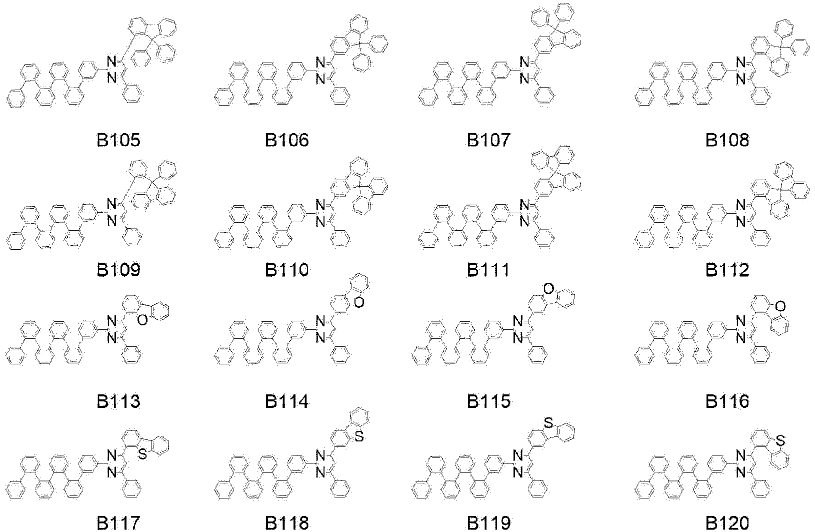

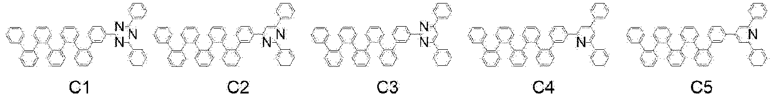

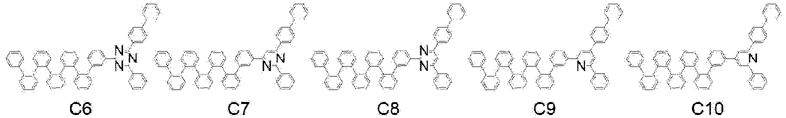

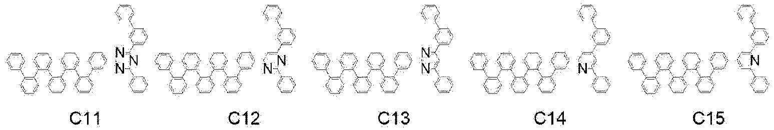

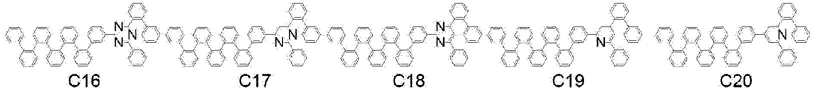

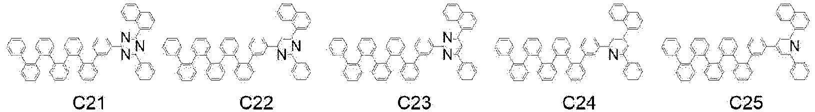

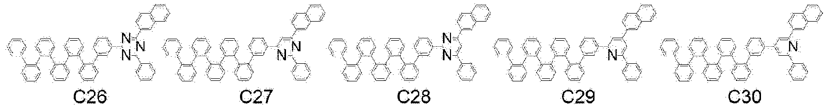

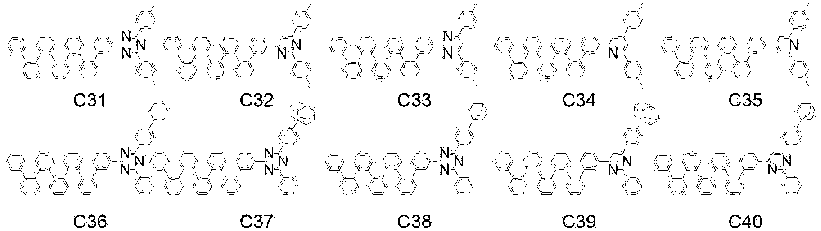

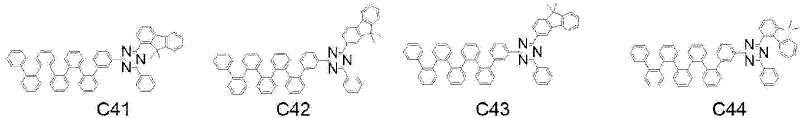

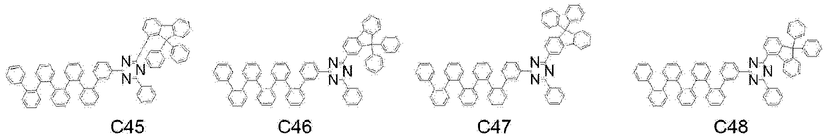

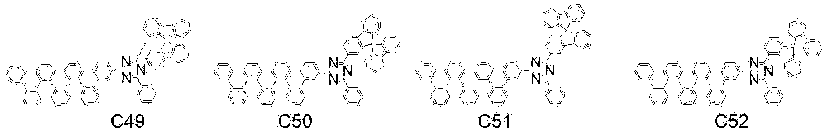

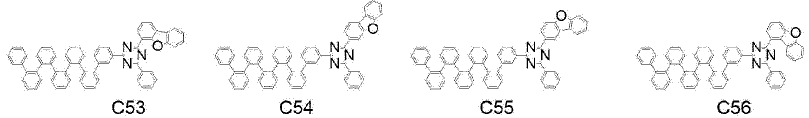

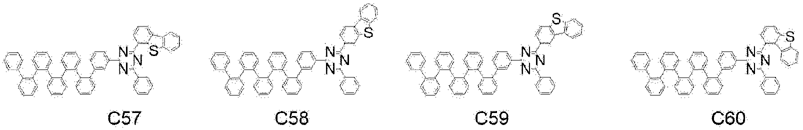

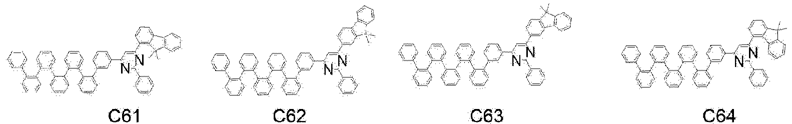

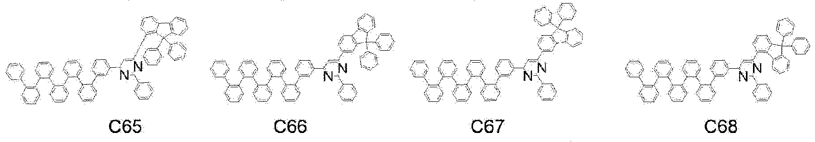

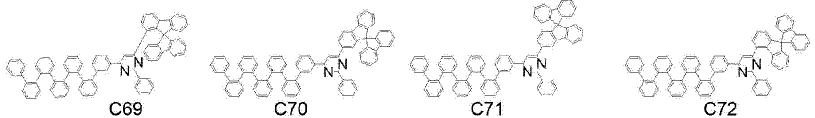

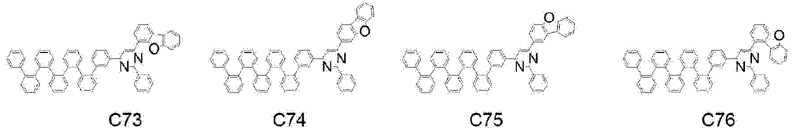

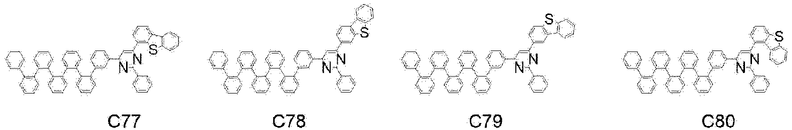

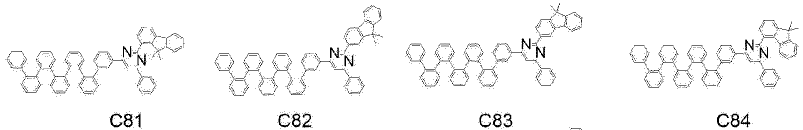

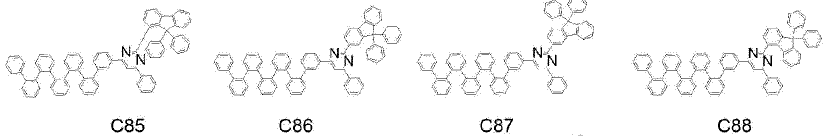

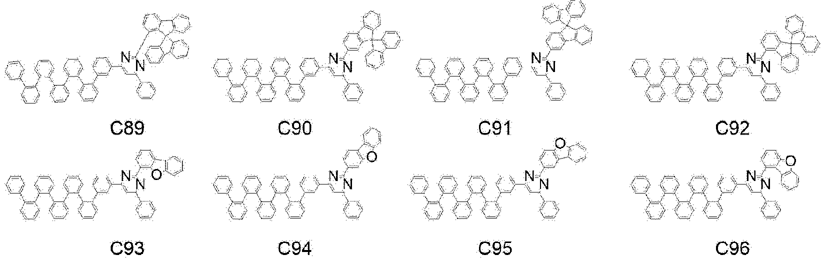

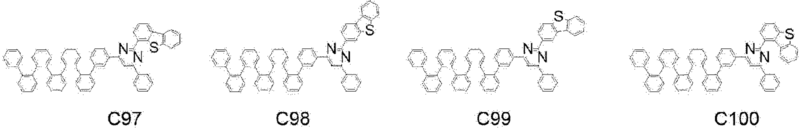

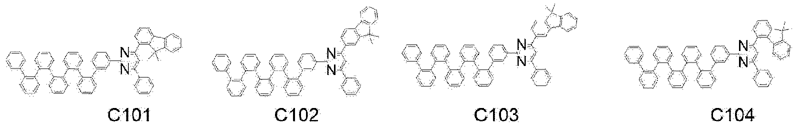

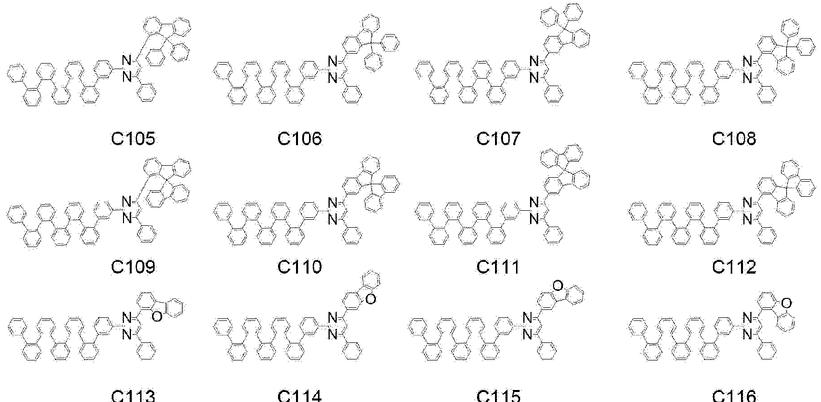

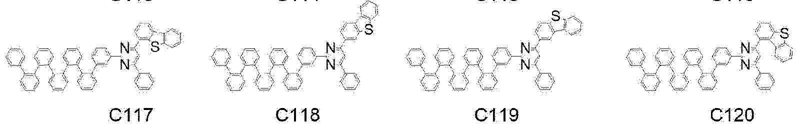

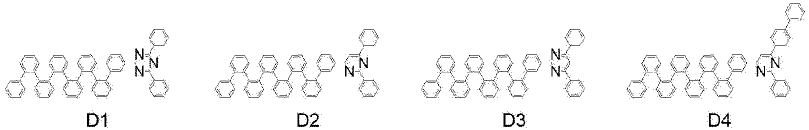

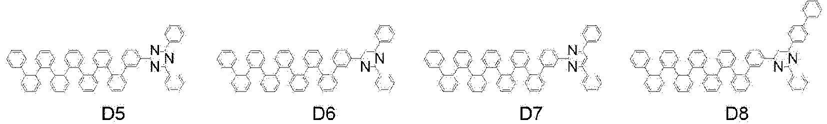

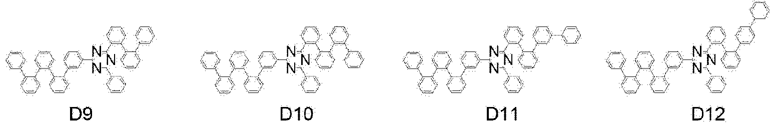

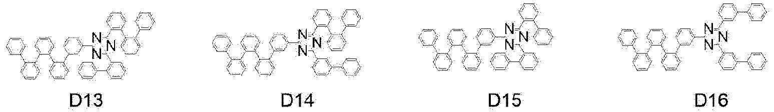

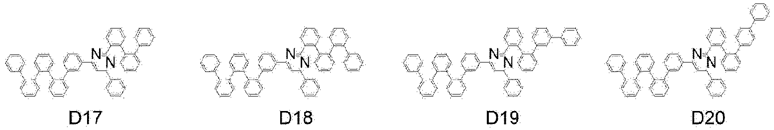

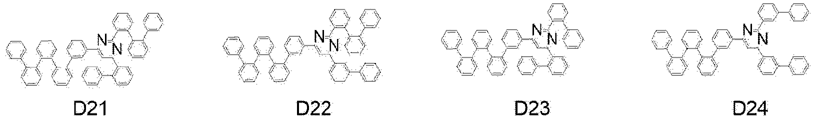

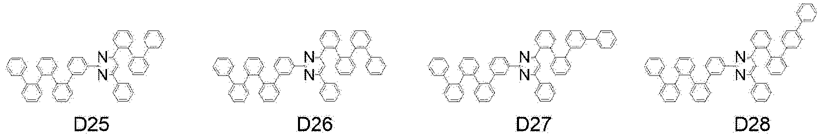

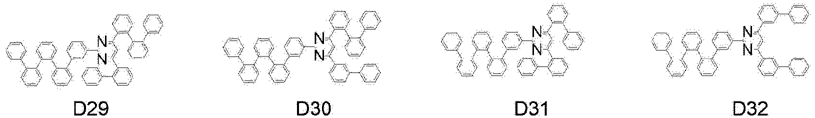

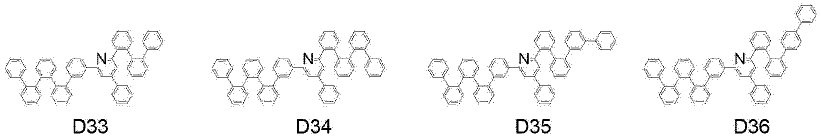

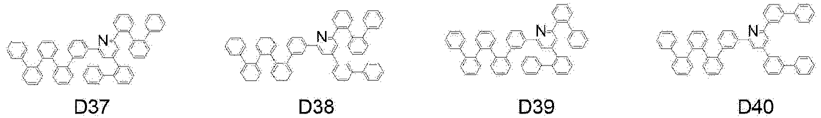

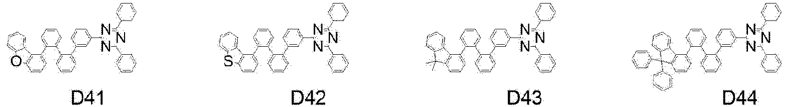

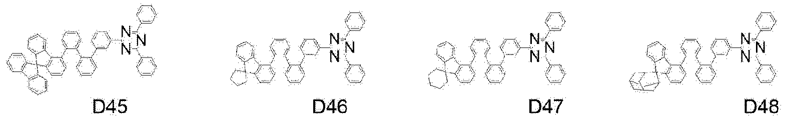

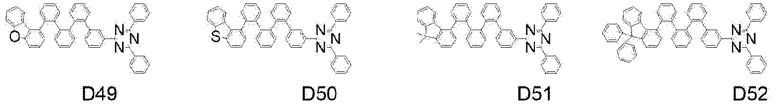

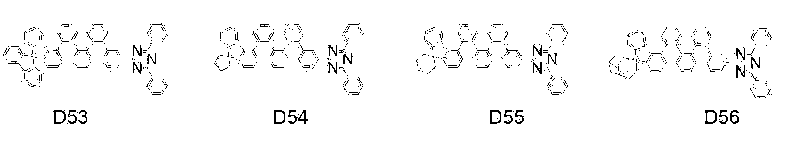

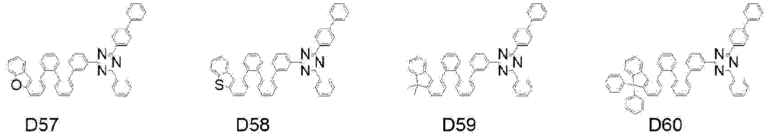

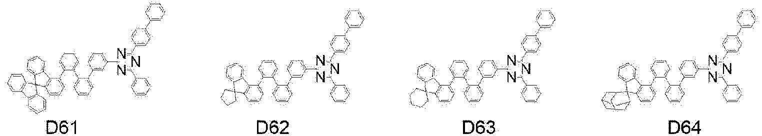

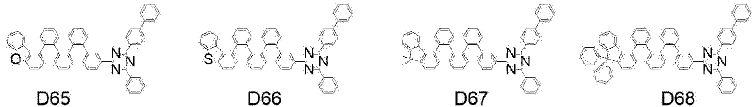

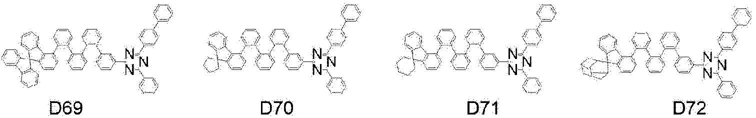

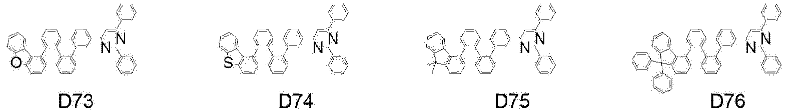

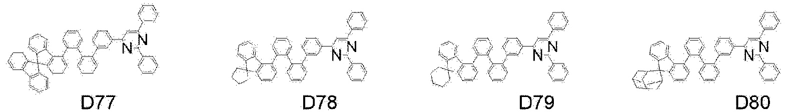

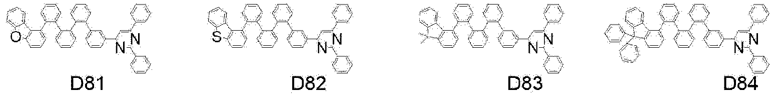

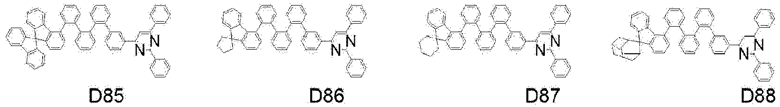

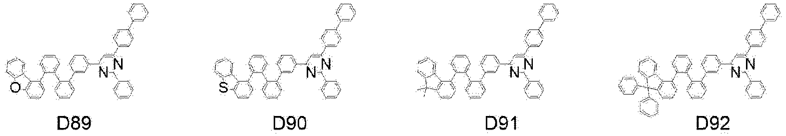

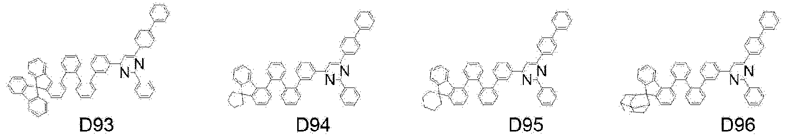

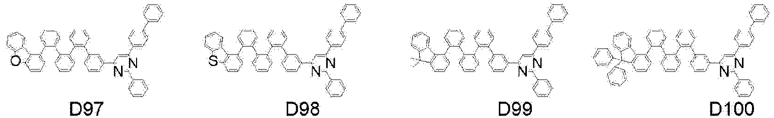

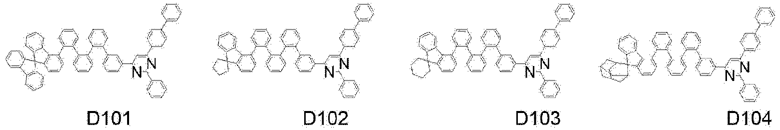

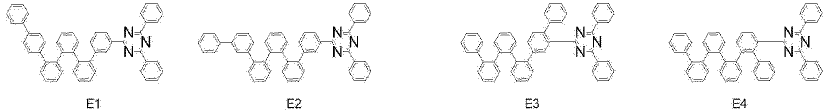

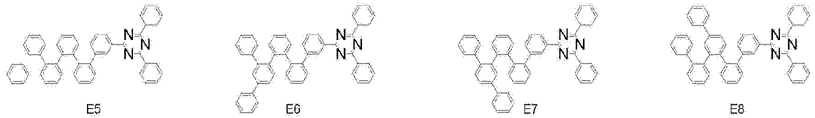

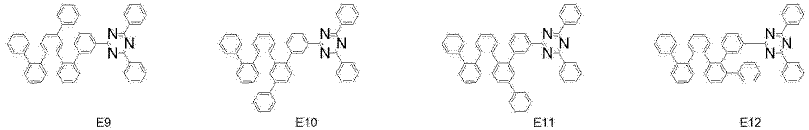

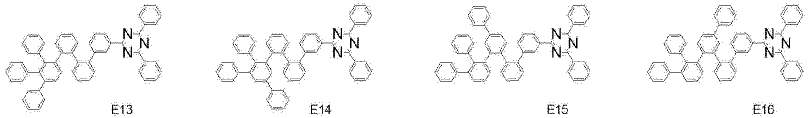

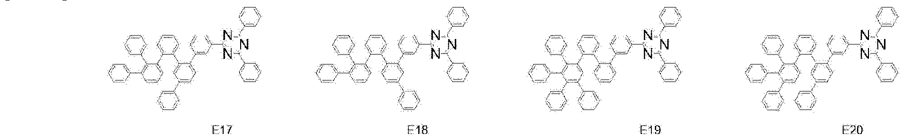

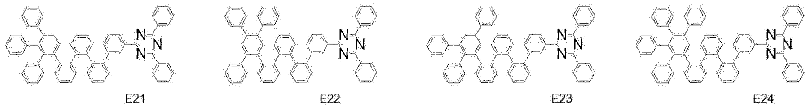

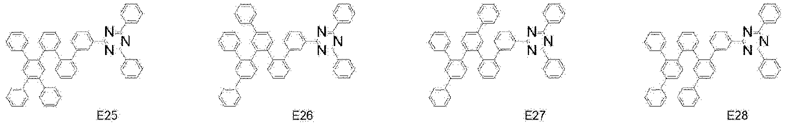

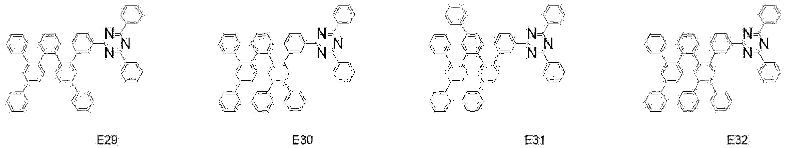

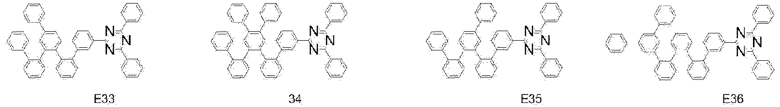

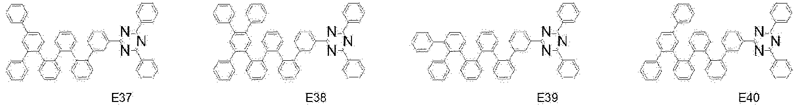

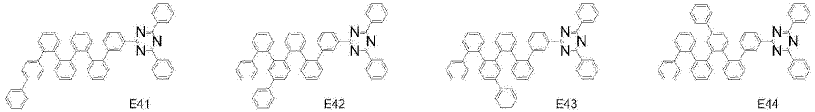

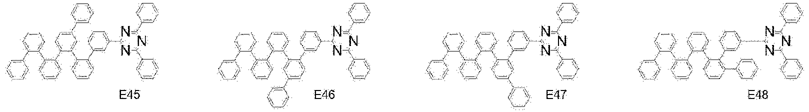

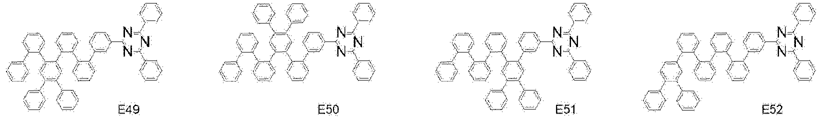

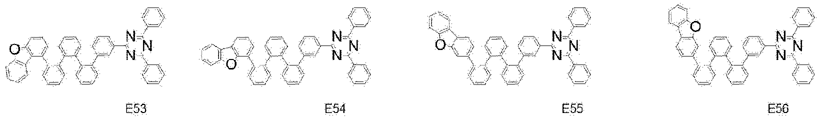

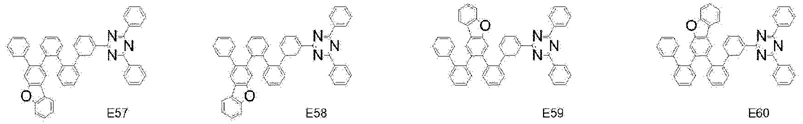

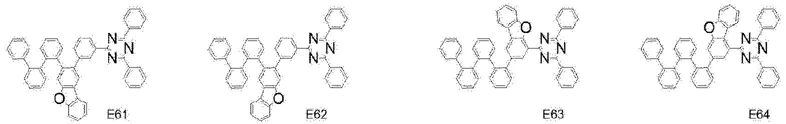

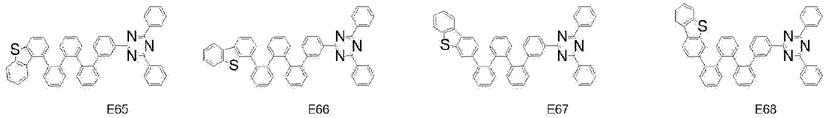

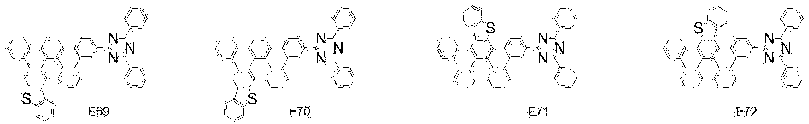

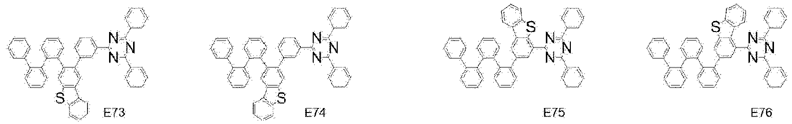

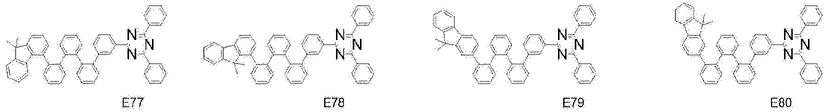

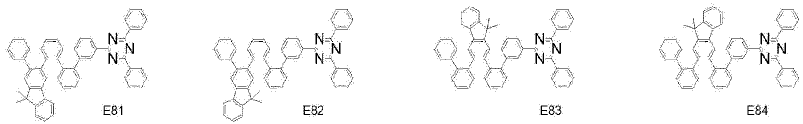

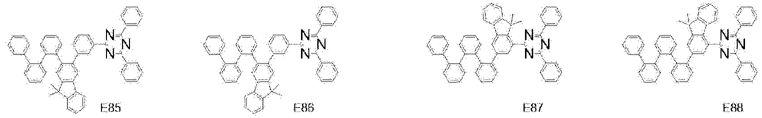

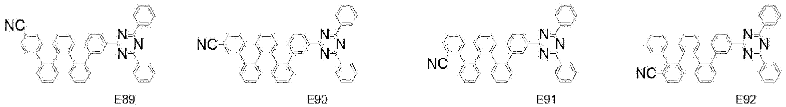

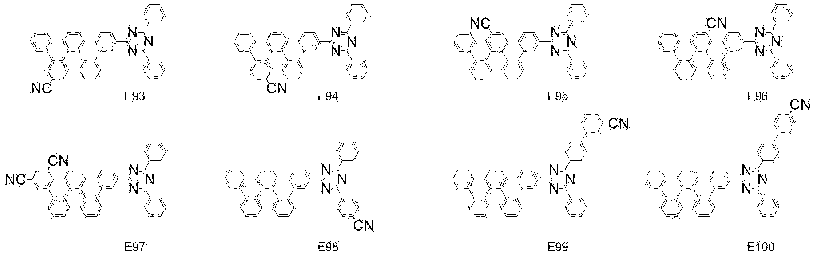

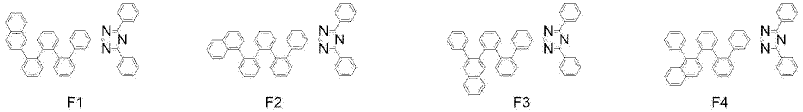

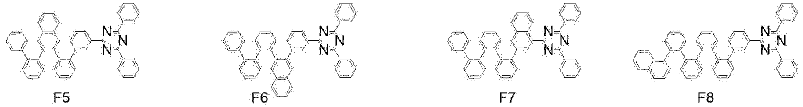

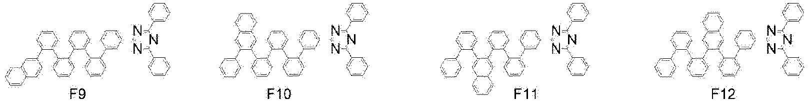

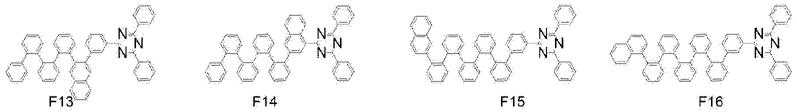

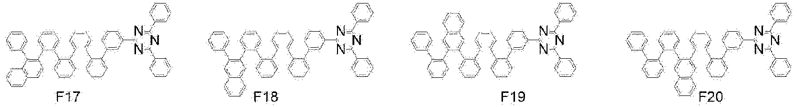

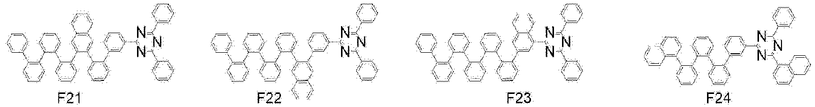

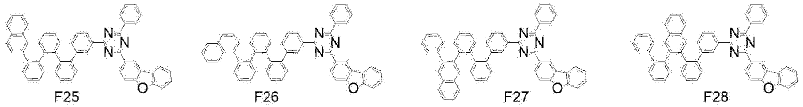

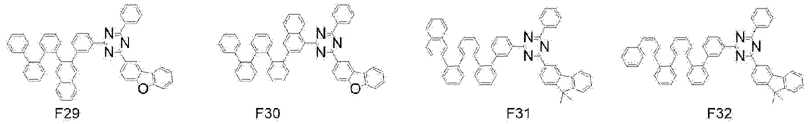

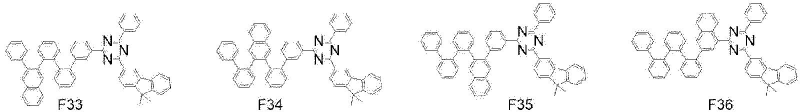

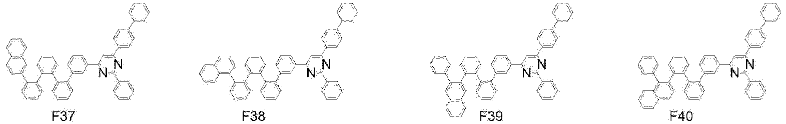

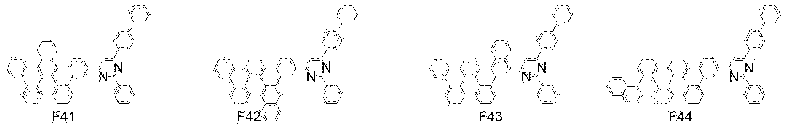

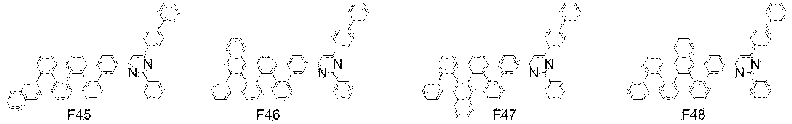

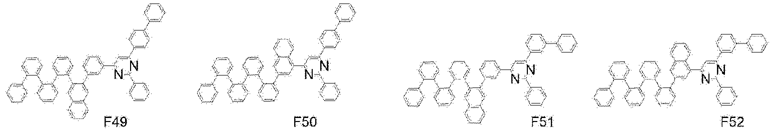

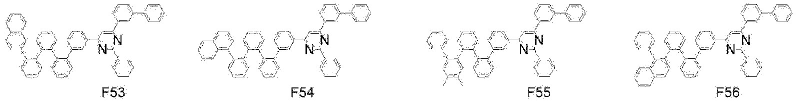

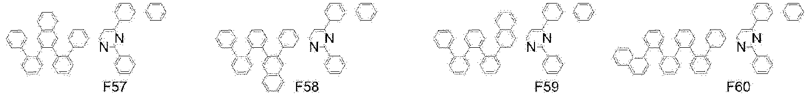

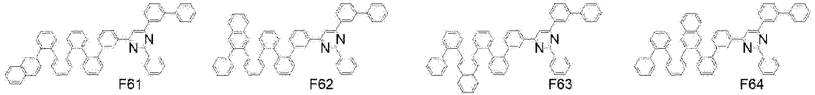

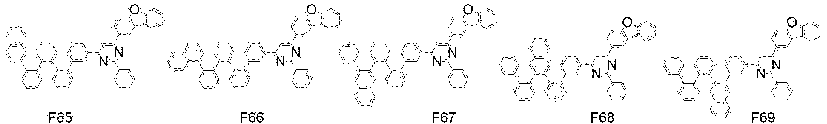

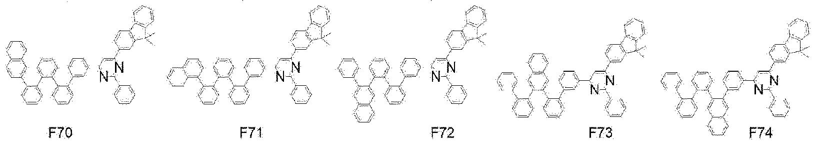

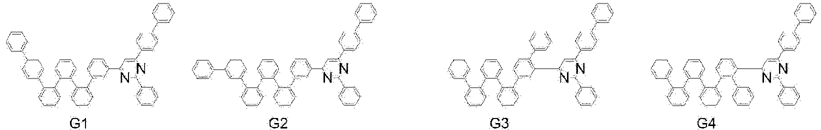

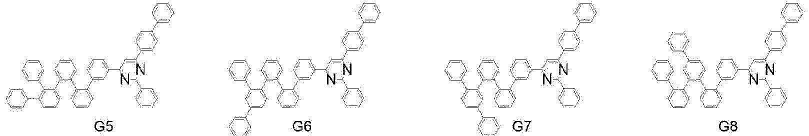

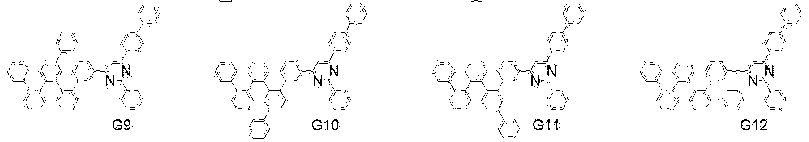

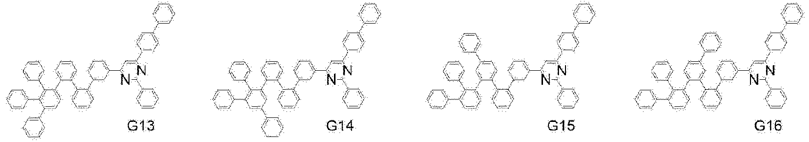

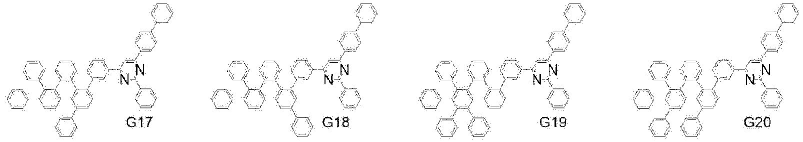

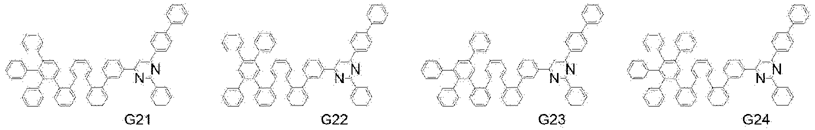

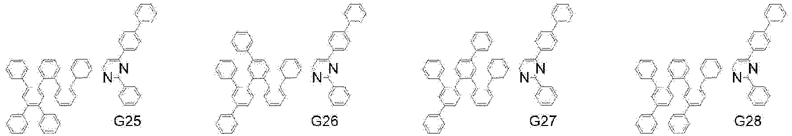

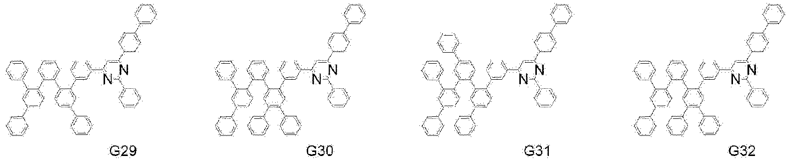

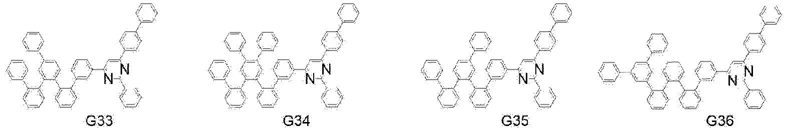

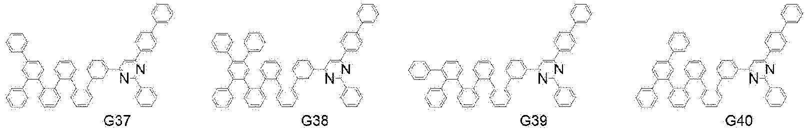

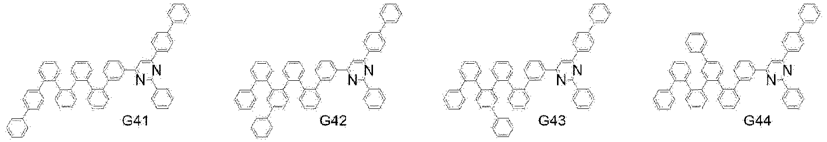

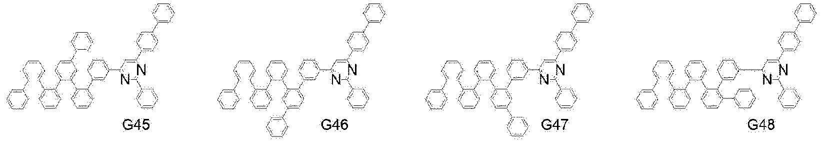

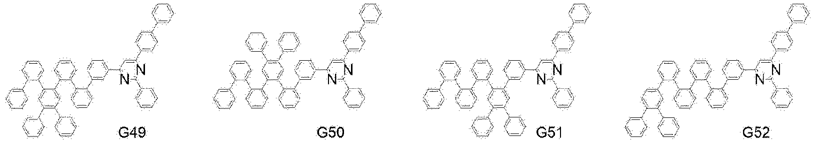

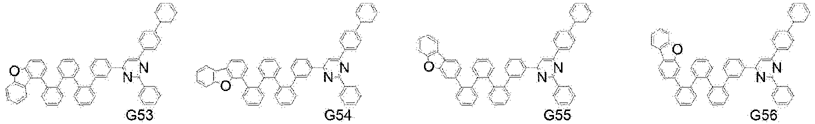

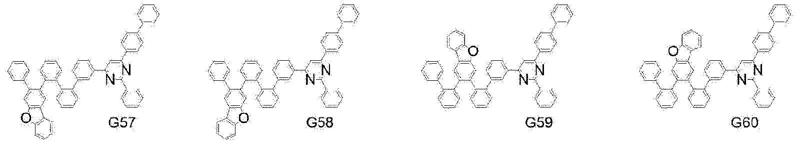

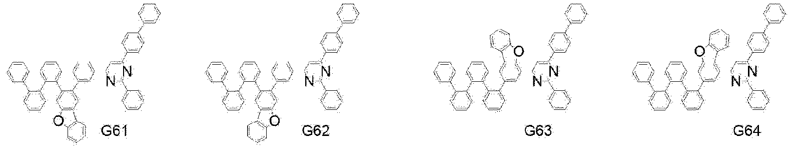

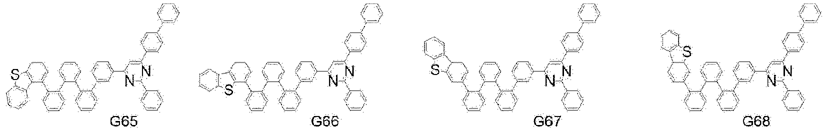

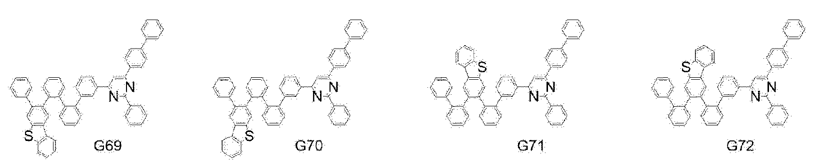

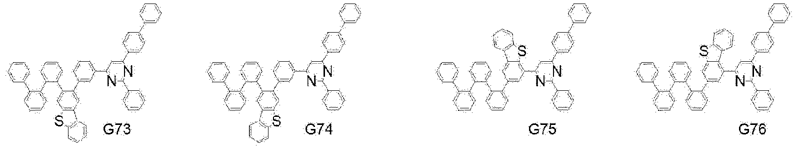

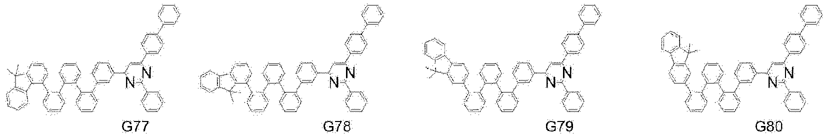

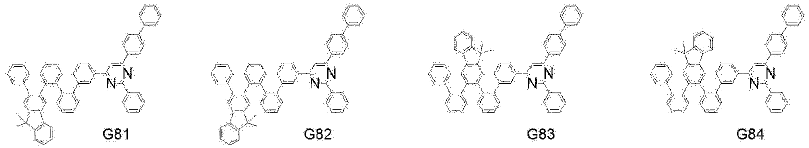

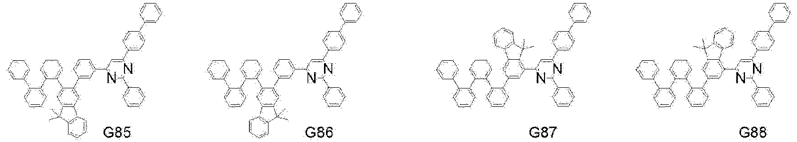

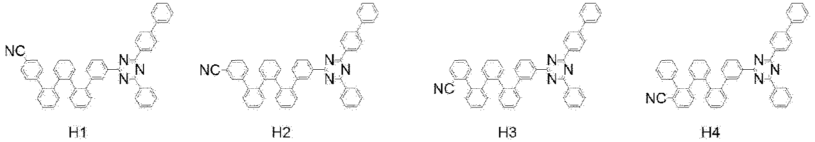

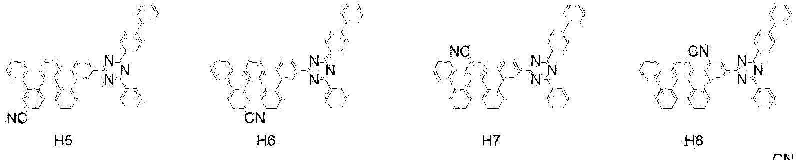

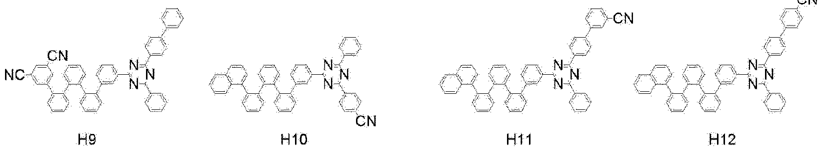

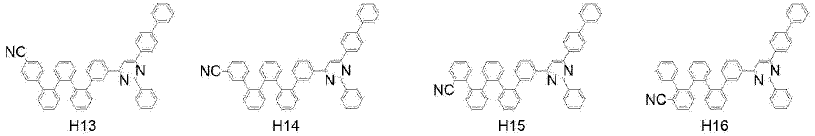

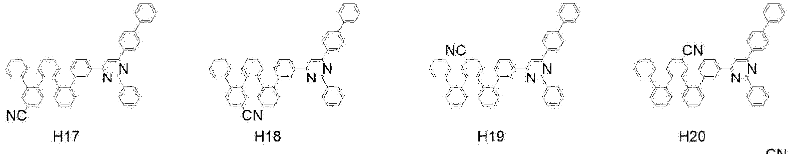

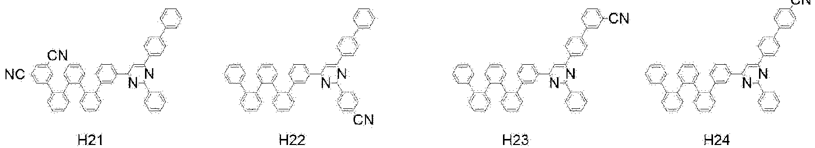

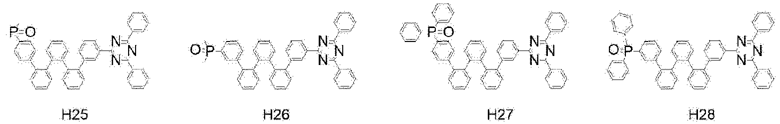

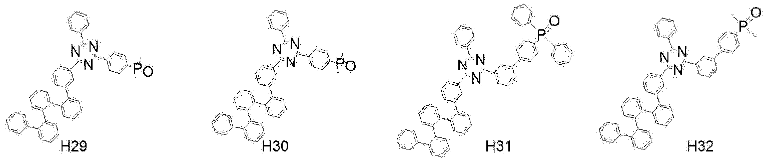

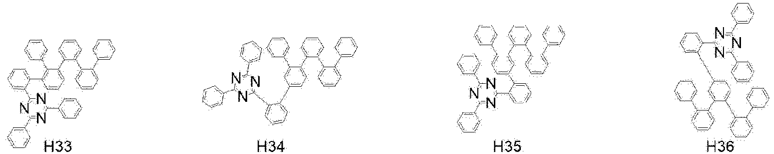

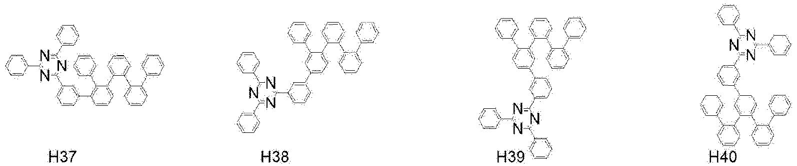

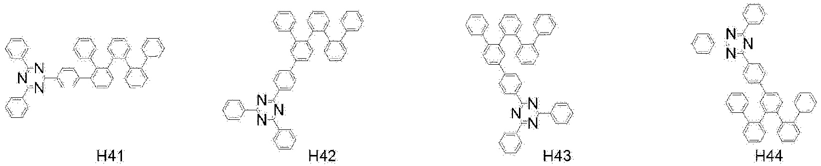

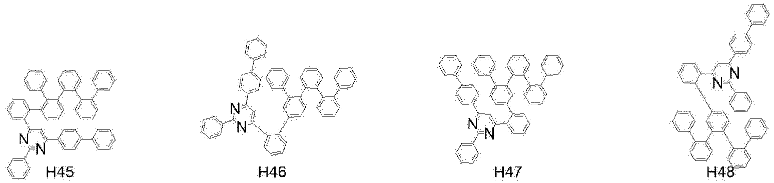

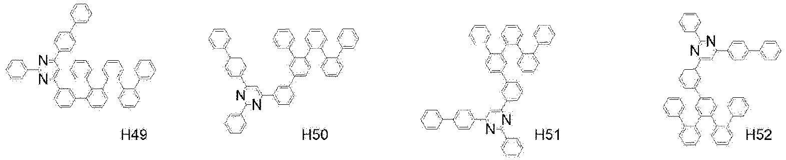

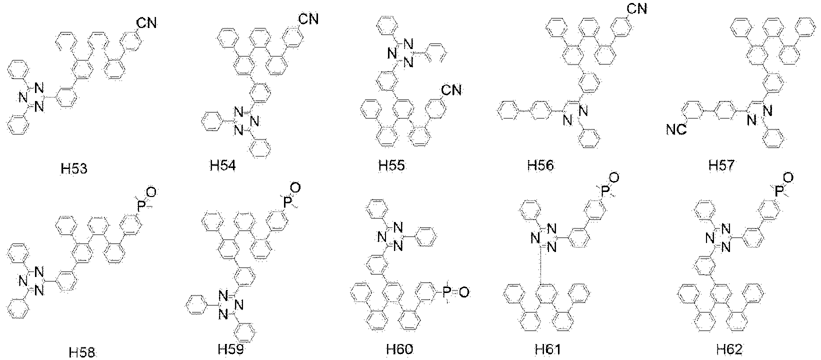

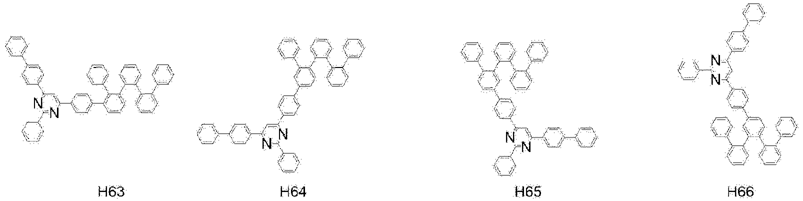

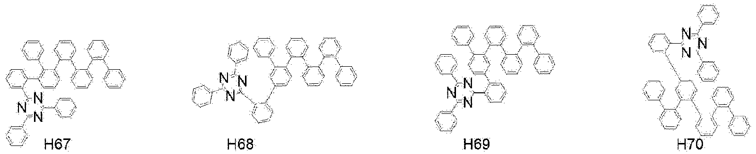

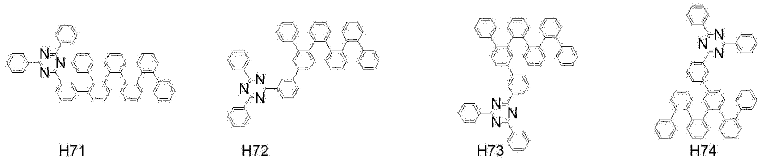

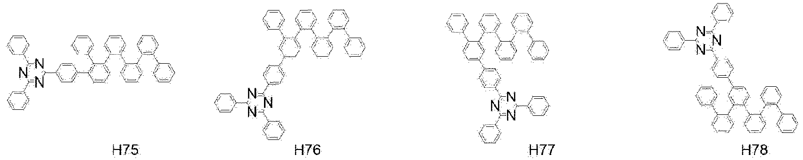

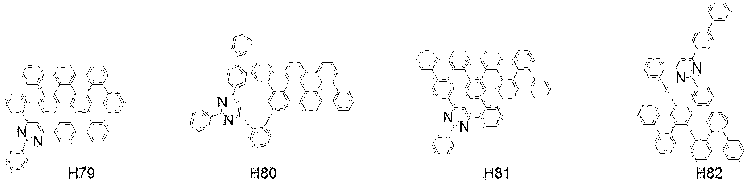

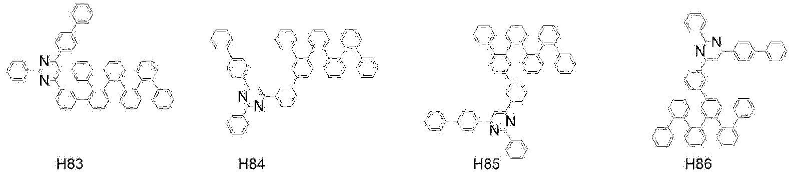

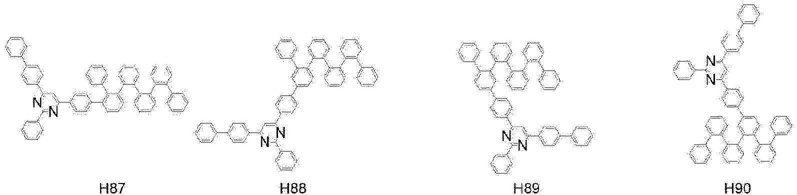

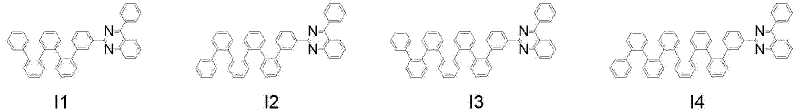

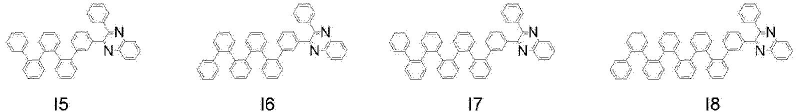

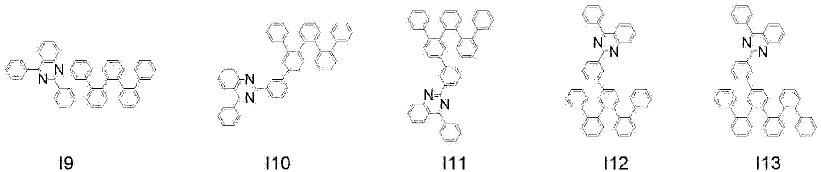

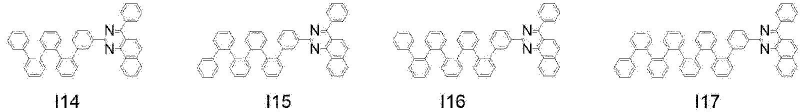

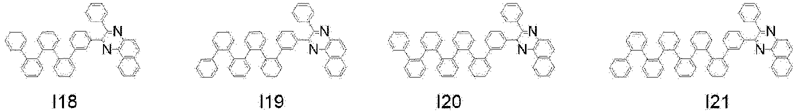

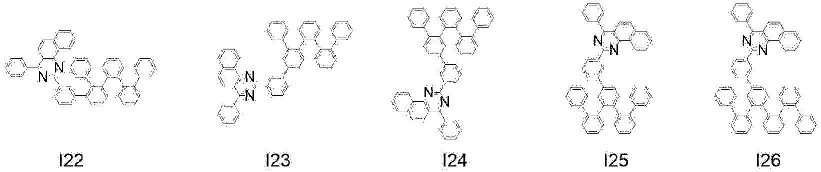

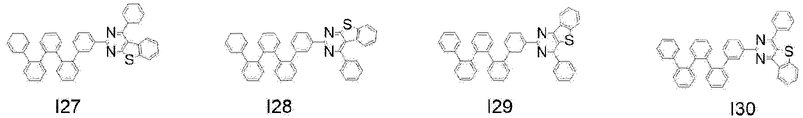

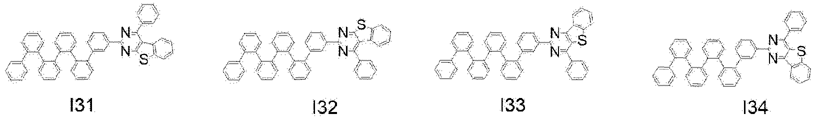

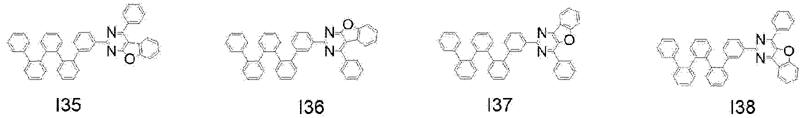

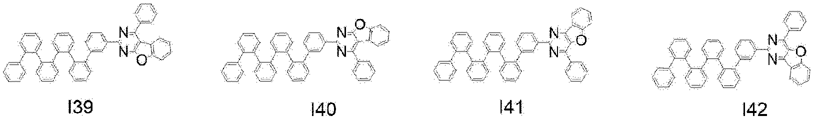

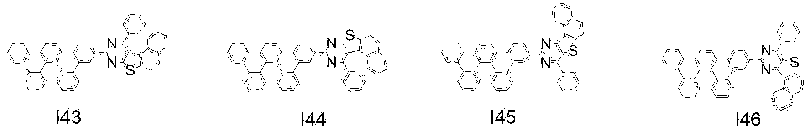

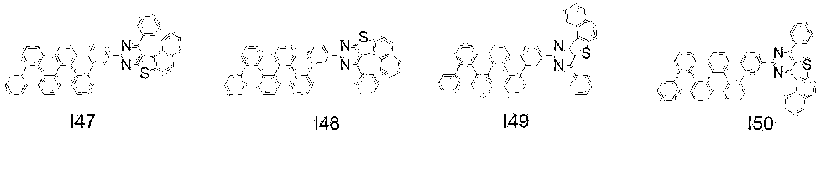

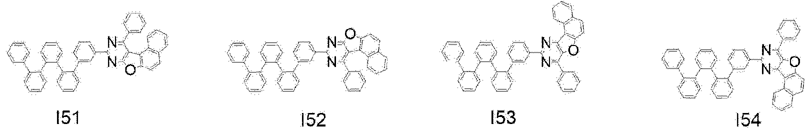

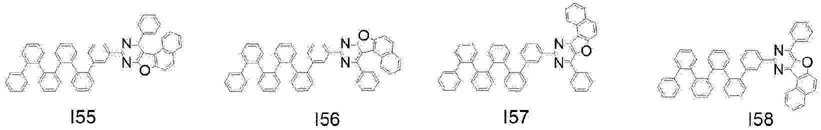

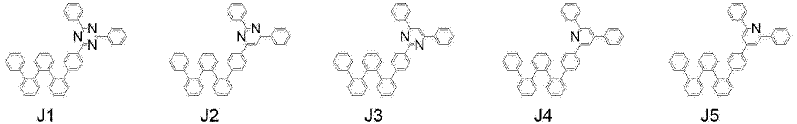

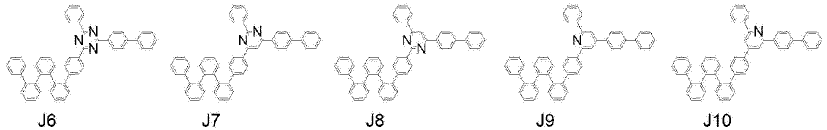

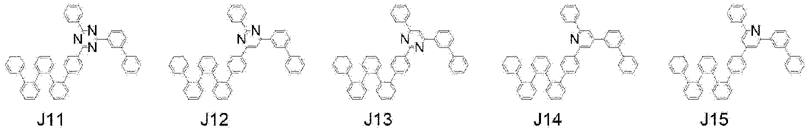

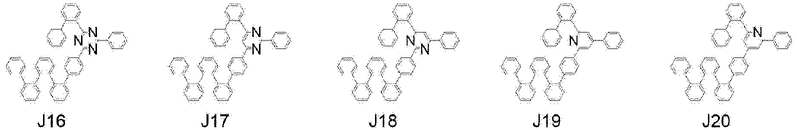

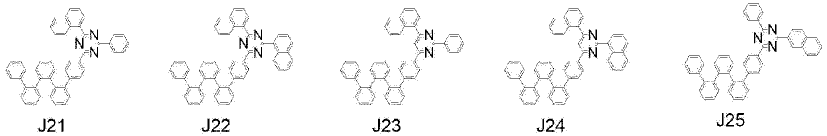

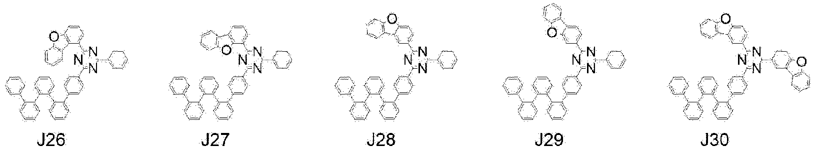

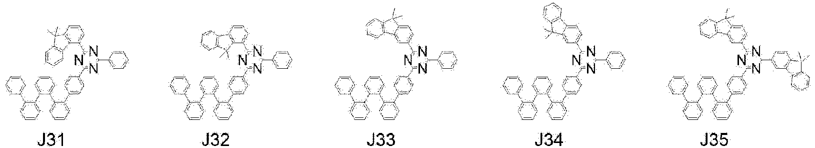

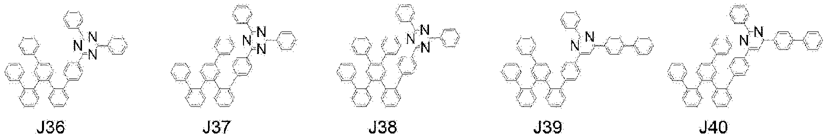

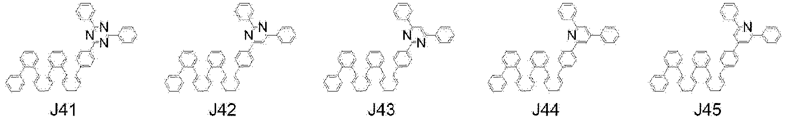

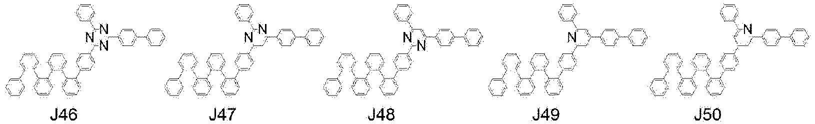

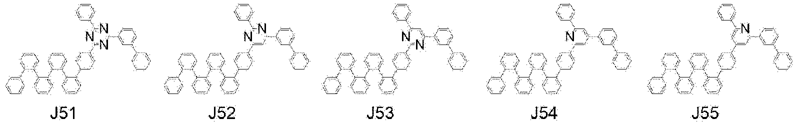

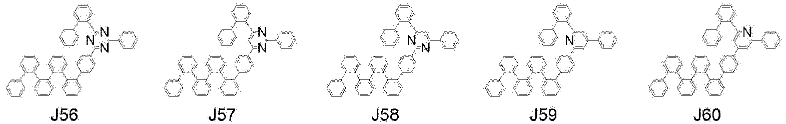

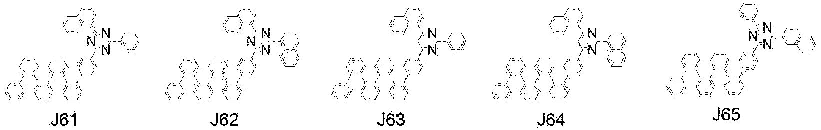

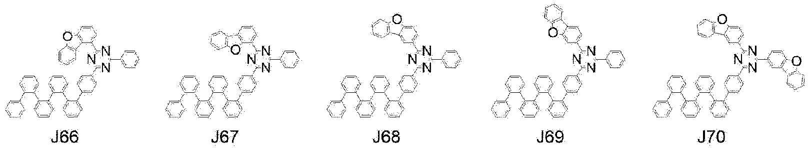

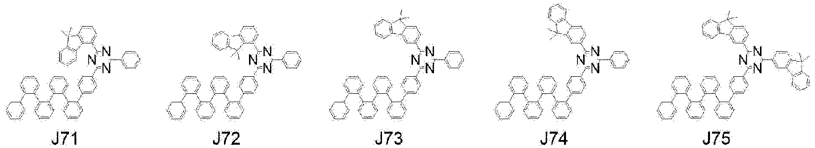

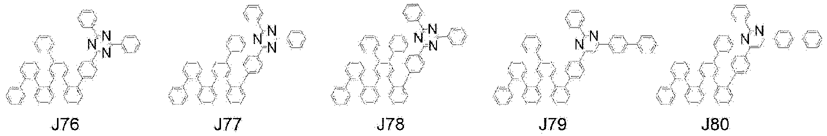

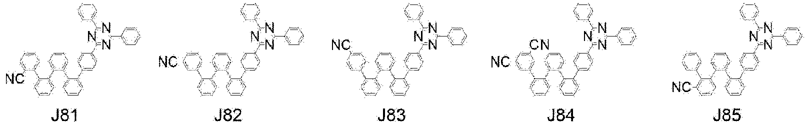

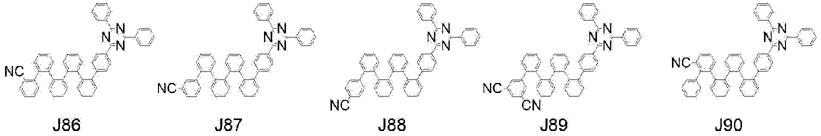

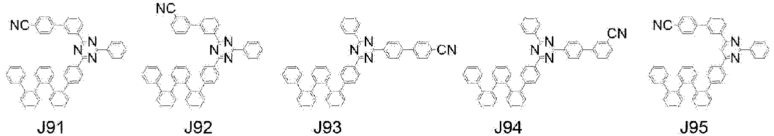

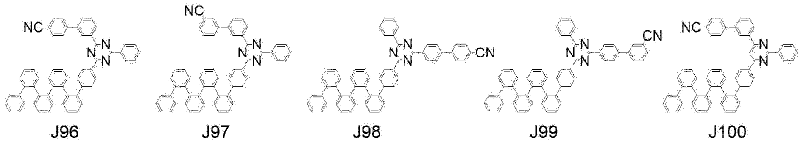

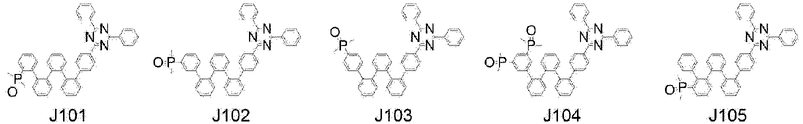

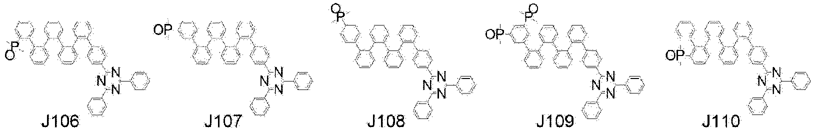

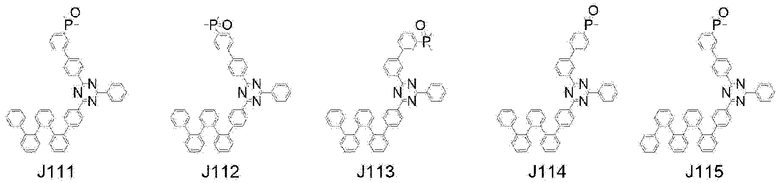

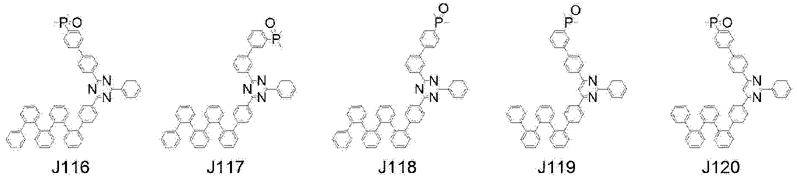

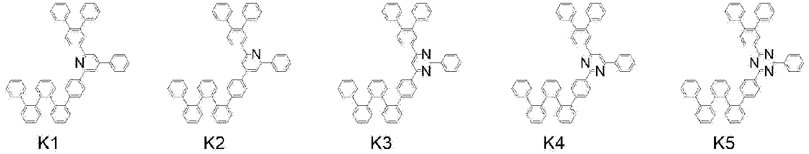

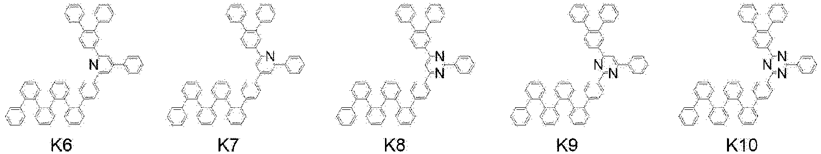

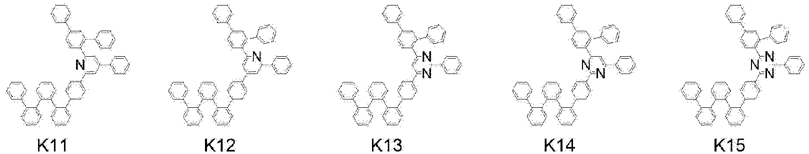

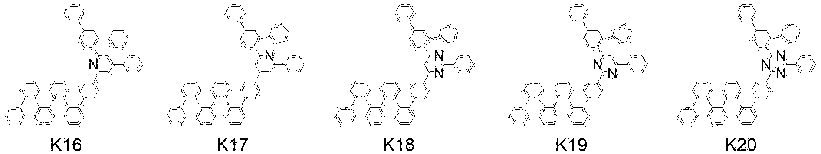

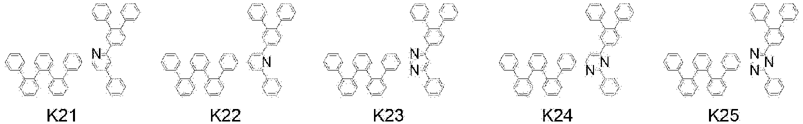

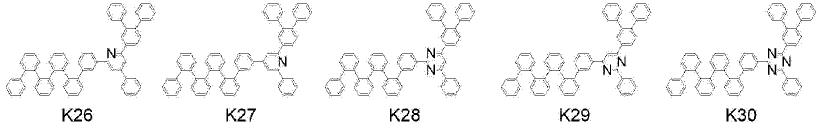

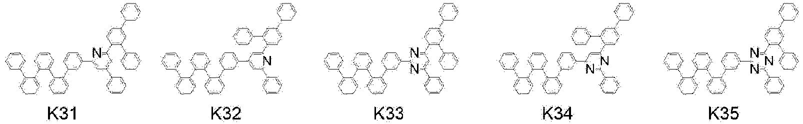

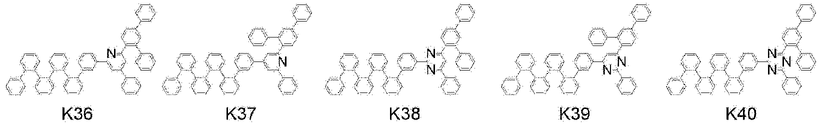

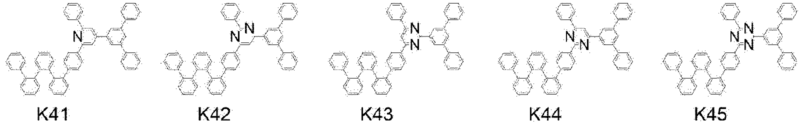

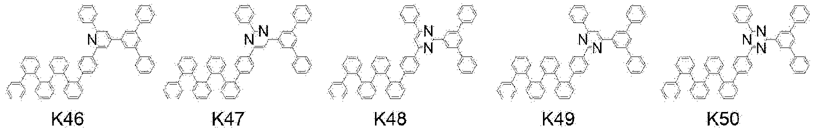

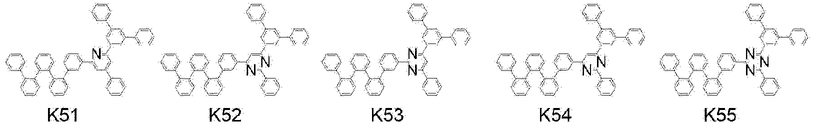

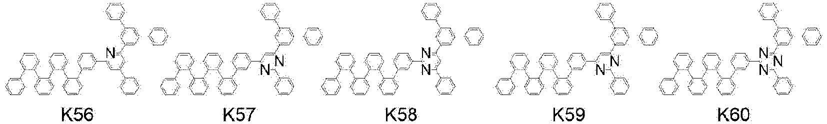

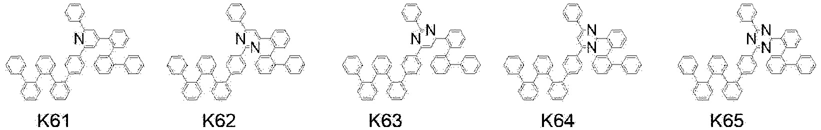

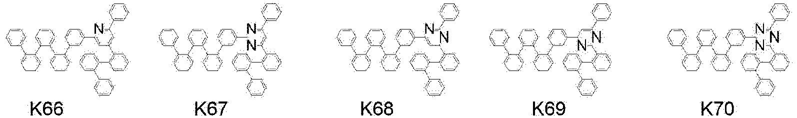

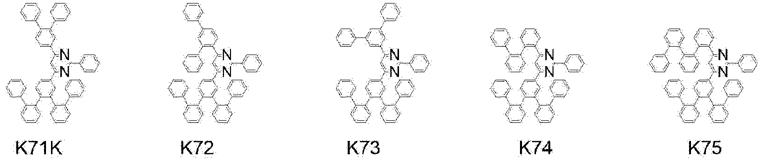

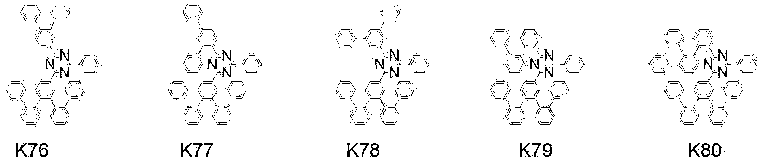

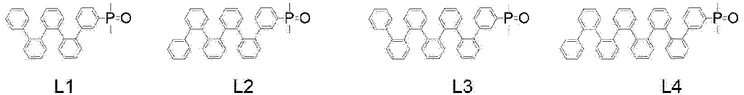

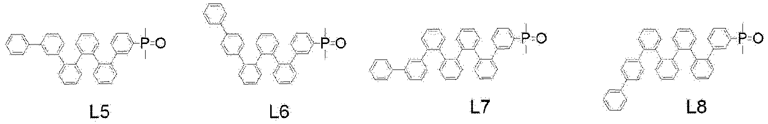

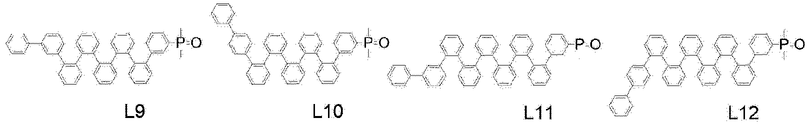

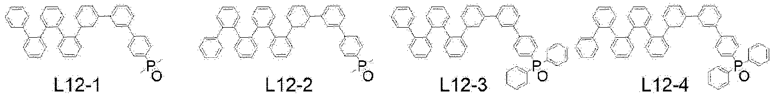

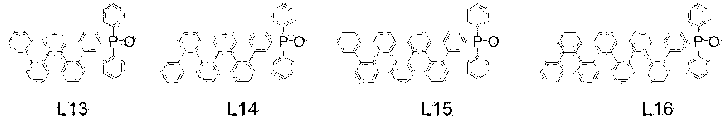

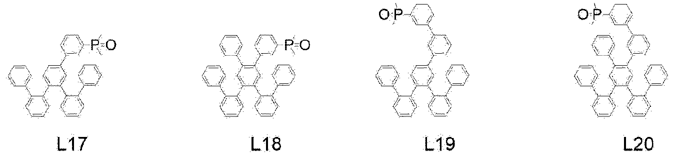

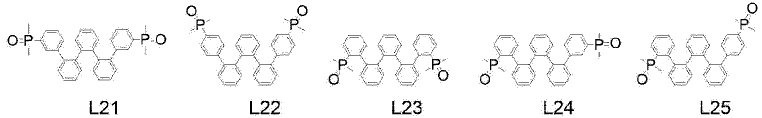

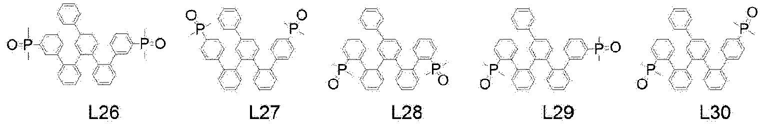

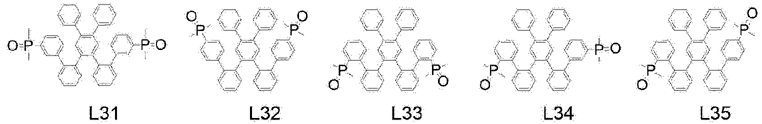

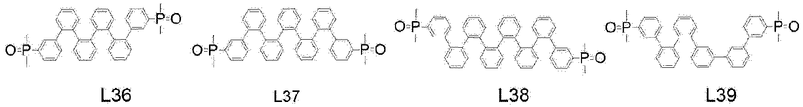

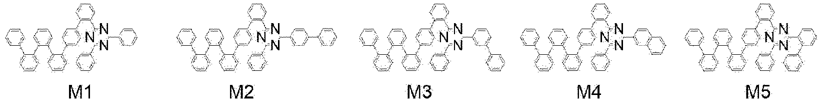

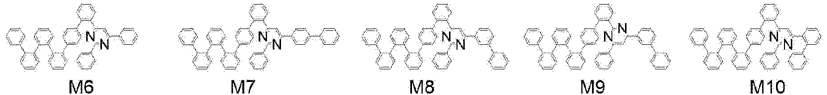

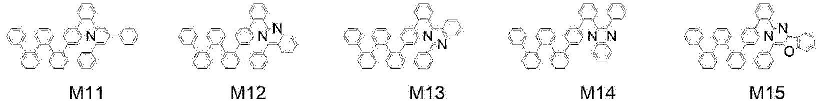

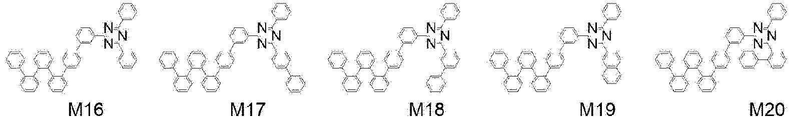

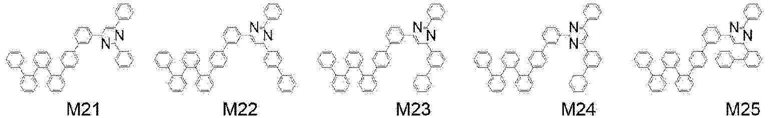

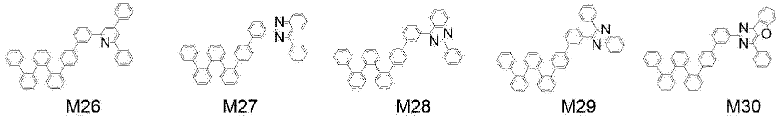

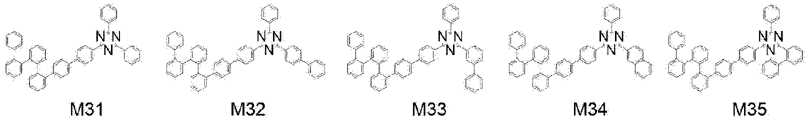

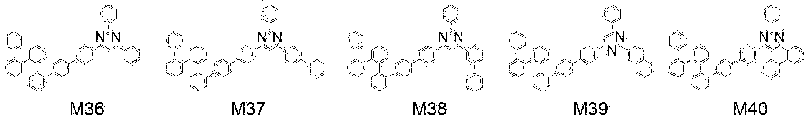

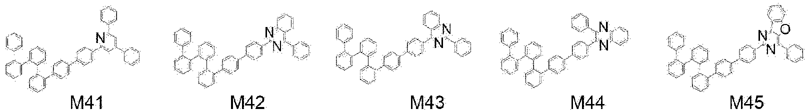

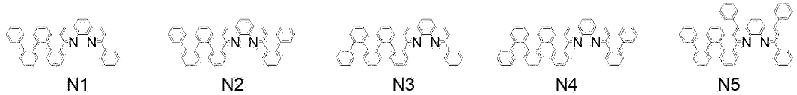

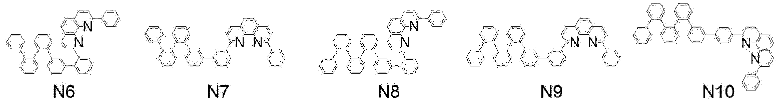

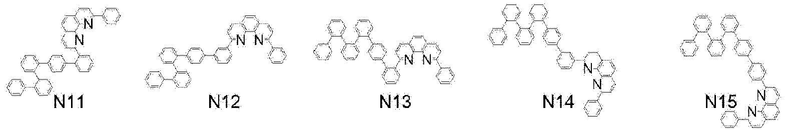

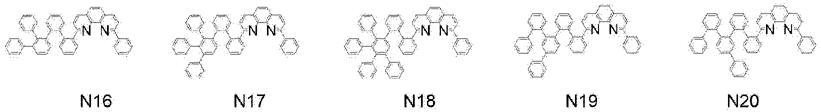

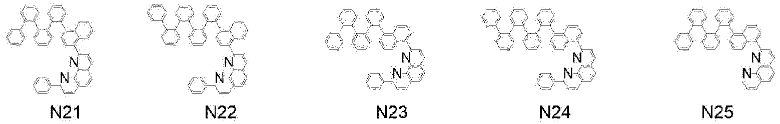

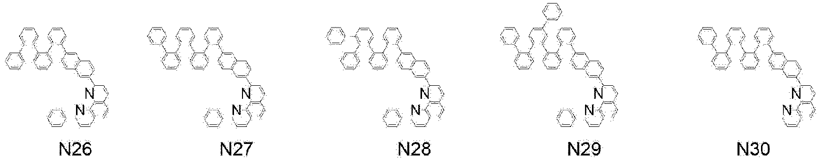

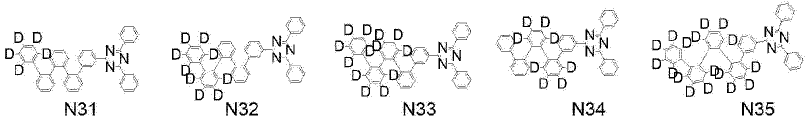

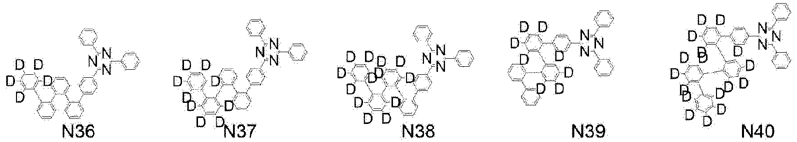

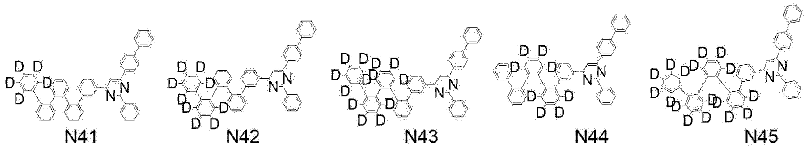

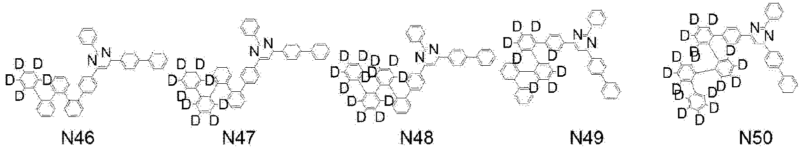

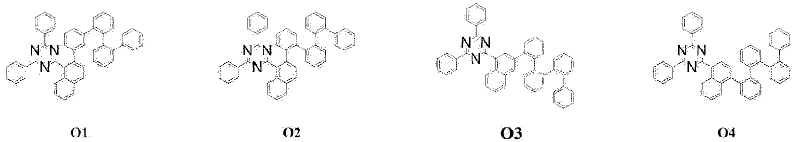

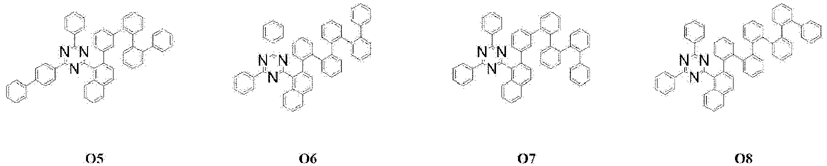

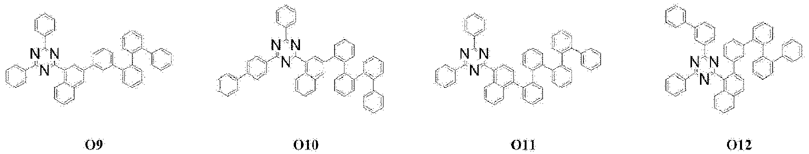

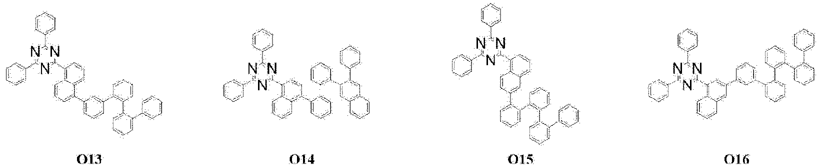

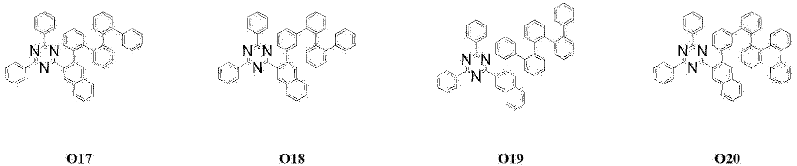

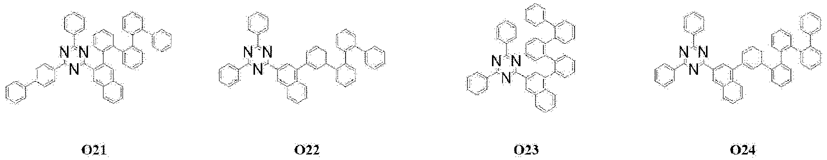

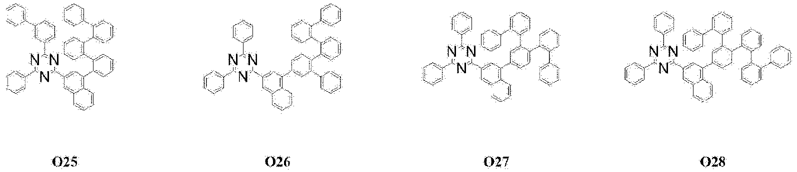

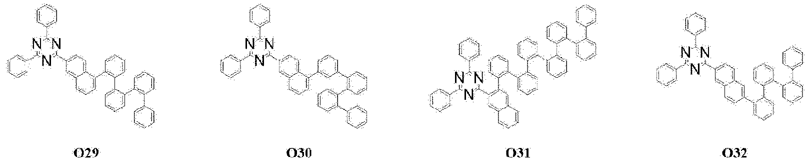

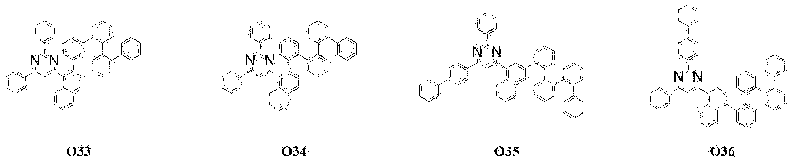

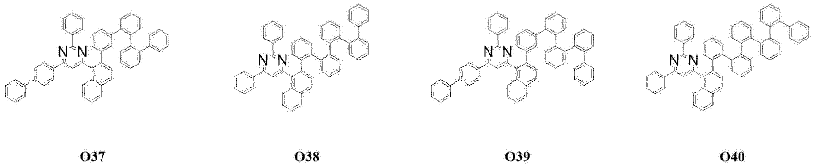

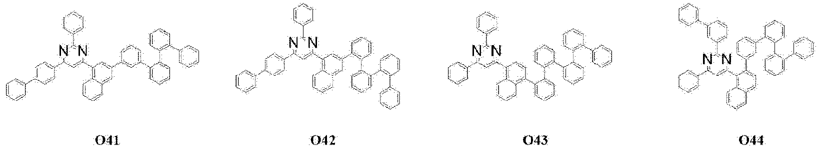

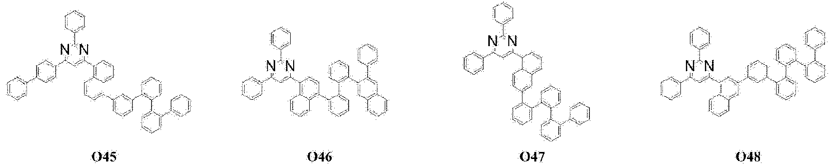

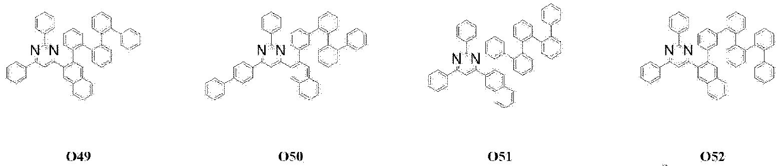

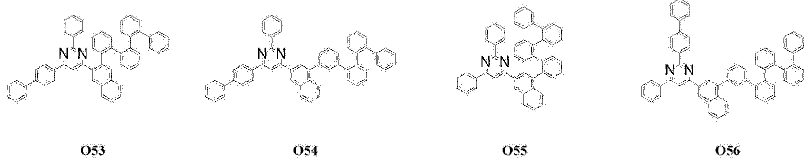

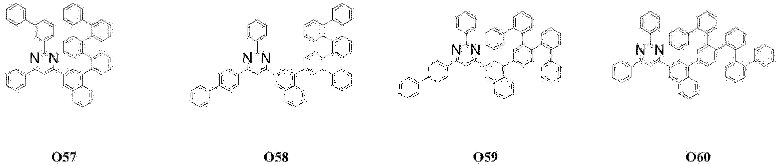

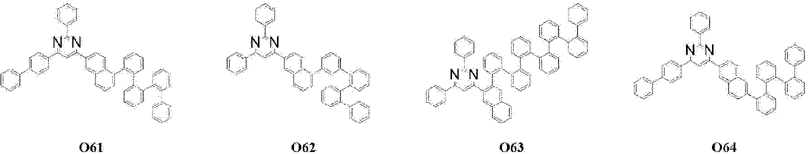

As used herein, the term "alkyl" refers to a monovalent substituent derived from a saturated, linear or branched hydrocarbon of 1 to 40 carbon atoms. Examples of such alkyl include, but are not limited to, methyl, ethyl, propyl, isobutyl, sec-butyl, pentyl, iso-amyl, hexyl or the like.

As used herein, the term "alkenyl" refers to a monovalent substituent derived from an unsaturated, linear or branched hydrocarbon of 2 to 40 carbon atoms, with at least one carbon-carbon double bond therein. Examples of such alkenyl include, but are not limited to, vinyl, allyl, isopropenyl, 2-butenyl or the like.

As used herein, the term "alkynyl" refers to a monovalent substituent derived from an unsaturated, linear or branched hydrocarbon of 2 to 40 carbon atoms, with at least one carbon-carbon triple bond therein. Examples of such alkynyl include, but are not limited to, ethynyl, 2-propynyl or the like.

As used herein, the term "cycloalkyl" refers to a monovalent substituent derived from a monocyclic or polycyclic non-aromatic hydrocarbon of 3 to 40 carbon atoms. Examples of such cycloalkyl include, but are not limited to, cyclopropyl, cyclopentyl, cyclohexyl, norbornyl, adamantine or the like.

As used herein, the term "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl include, but are not limited to, morpholine group, piperazine group or the like.

As used herein, the term "aryl" refers to a monovalent substituent derived from an aromatic hydrocarbon of 6 to 60 carbon atoms and is intended to encompass structures in which two or more rings are simply pendant to or condensed with each other. Examples of such aryl include, but are not limited to, phenyl, naphthyl, phenanthryl, anthryl or the like.

As used herein, the term "heteroaryl" refers to a monovalent substituent derived from a monoheterocyclic or polyheterocyclic aromatic hydrocarbon of 5 to 60 nuclear atoms. In this regard, one or more carbons in the ring, preferably one to three carbons, are substituted with a heteroatom such as N, O, S or Se. In addition, the heteroaryl is intended to encompass structures in which two or more rings are pendant to or condensed with each other or further condensed with an aryl group. Examples of such heteroaryl include, but are not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole group, and carbazolyl; 2-furanyl; N-imidazolyl; 2-isoxazolyl; 2-pyridinyl; 2-pyrimidinyl or the like.

As used herein, the term "alkyloxy" refers to a monovalent substituent represented by R'O-, where R' is an alkyl of 1 to 40 carbon atoms. Such alkyloxy may include a linear, branched or cyclic structure. Examples of such alkyloxy include, but are not limited to, methoxy, ethoxy, n-propoxy, 1-propoxy, t-butoxy, n-butoxy, pentoxy or the like.

As used herein, the term "aryloxy" refers to a monovalent substituent represented by RO-, where R is an aryl of 5 to 40 carbon atoms. Examples of such aryloxy may include, but are not limited to, phenyloxy, naphthyloxy, diphenyloxy or the like.

As used herein, the term "alkylsilyl" refers to silyl substituted with an alkyl of 1 to 40 carbon atoms and is intended to encompass di- and tri-alkylsilyl as well as mono-alkylsilyl. In addition, "arylsilyl" refers to silyl substituted with an aryl of 5 to 60 carbon atoms and is intended to encompass poly-arylsilyl such as di- and triarylsilyl as well as mono-arylsilyl.

As used herein, the term "alkylboron group" refers to a boron group substituted with alkyl having 1 to 40 carbon atoms, and "arylboron group" refers to a boron group substituted with aryl having 6 to 60 carbon atoms.

As used herein, the term "alkylphosphinyl" refers to phosphine substituted with an alkyl of 1 to 40 carbon atoms and is intended to encompass di-alkylphosphinyl as well as mono-alkylphosphinyl. As used herein, the term "arylphosphinyl group" refers to phosphine substituted with a mono- or diaryl of 6 to 60 carbon atoms and is intended to encompass di-arylphosphinyl as well as mono-arylphosphinyl.

The term "alkylphosphine oxide group," as used herein, refers to phosphine oxide substituted with an alkyl of 1 to 40 carbon atoms and is intended to encompass mono- and dialkyl phosphine oxide. In addition, as used herein, the term "arylphosphine oxide" refers to phosphine oxide substituted with a mono- or diaryl of 6 to 60 carbon atoms and is intended to encompass mono- and di-arylphosphine oxide.

As used herein, the term "arylamine" refers to amine substituted with an aryl of 6 to 60 carbon atoms and is intended to encompass mono- and diarylamine.

The term "heteroarylamine" as used herein, refers to amine substituted with a heteroaryl group having 5 to 60 nuclear atoms and is intended to encompass mono- and diheteroarylamine.

The term "(aryl) (heteroaryl)amine" as used herein, refers to amine substituted with an aryl of 6 to 60 carbon atoms and a heteroaryl group having 5 to 60 nuclear atoms.

As used herein, the term "fused ring" refers to a fused aliphatic ring of 3 to 40 carbon atoms, a fused aromatic ring of 6 to 60 carbon atoms, a fused heteroaliphatic ring of 3 to 60 carbon atoms, a fused heteroaromatic ring of 5 to 60 nuclear atoms, or a combination thereof

### <Organic Electroluminescence Device>

The present disclosure provides an organic electroluminescent device (hereinafter referred to as "organic EL device") including a compound represented by any one of Chemical Formulas 1 to 5, as described above.

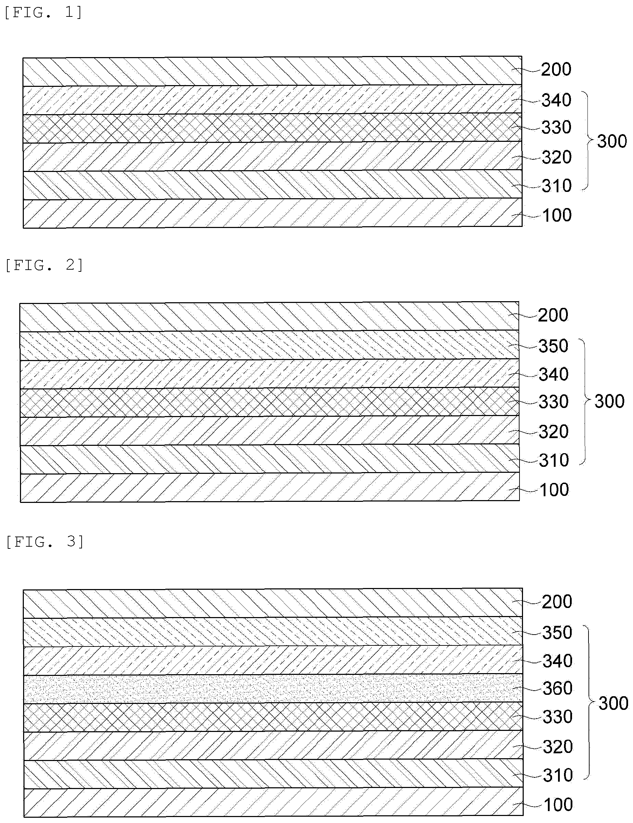

Specifically, as illustrated in FIGS. 1 to 3, the organic electroluminescent device according to the present disclosure includes an anode (100), a cathode (200), and at least one organic layer (300) disposed between the anode and the cathode, wherein at least one of the organic layers includes a compound represented by any one of Chemical Formulas 1 to 5. The compound may be used alone or in combination with two or more compounds.

The at least one organic layer (300) may include at least one selected from a hole injection layer (310), a hole transport layer (320), an emission layer (330), an electron transport auxiliary layer (360), an electron transport layer (340), and an electron injection layer (350). Among these, at least one organic layer (300) contains a compound represented by any one of Chemical Formulas 1 to 5. Specifically, the organic layer including the compound represented by any one of Chemical Formulas 1 to 5 may be at least one of the electron transport layer (340) and the electron transport auxiliary layer (360).

According to an embodiment, the at least one organic layer may include a hole injection layer, a hole transport layer, an emission layer, an electron transport layer, and an electron injection layer, and optionally, an electron transport auxiliary layer. The electron transport layer contains a compound represented by any one of Chemical Formulas 1 to 5. In this regard, the compound represented by any one of Chemical Formulas 1 to 5 is included in the organic electroluminescent device as an electron transport layer material. In this organic electroluminescent device, the presence of the compound represented by any one of Chemical Formulas 1 to 5 facilitates the injection of electrons from the cathode or the electron injection layer into the electron transport layer, and the electrons can also move rapidly from the electron transport layer to the emission layer, thereby enhancing the recombination efficiency of holes and electrons in the emission layer. As a result, the organic electroluminescent device of the present disclosure exhibits excellent luminescence efficiency, power efficiency, and luminance. Furthermore, the compound represented by any one of Chemical Formulas 1 to 5 exhibits excellent thermal stability and electrochemical stability, thereby improving the overall performance of the organic electroluminescent device.

The compound represented by any one of Chemical Formulas 1 to 5 may be used alone or in combination with an electron transport layer material known in the field.

In the present disclosure, the electron transport layer materials that may be used in combination with any one of the compounds represented by Chemical Formulas 1 to 5 include electron transport materials conventionally known in the field. Non-limiting examples of usable electron transport materials include oxazole-based compounds, isoxazole-based compounds, triazole-based compounds, isothiazole-based compounds, oxadiazole-based compounds, thiadiazole-based compounds, perylene-based compounds, aluminum complexes (e.g., Alq₃, tris(8-quinolinolato)-aluminium), and gallium complexes (e.g., Gaq'2OPiv, Gaq'2OAc, 2(Gaq'2). These materials may be used alone or in combination of two or more.

In the present disclosure, when any one of the compounds represented by Chemical Formulas 1 to 5 is blended with an electron transport layer material, the mixing ratio is not particularly limited and may be appropriately adjusted within the range known in the field.

According to another embodiment, the at least one organic layer may include a hole injection layer, a hole transport layer, an emission layer, an electron transport auxiliary layer, an electron transport layer, and an electron injection layer, wherein the electron transport auxiliary layer contains a compound represented by any one of Chemical Formulas 1 to 5. In this regard, the compound represented by any one of Chemical Formulas 1 to 5 is included in the organic electroluminescent device as an electron transport auxiliary layer material. Additionally, the compound represented by any one of Chemical Formulas 1 to 5 has a high triplet energy level. Therefore, when any one of the compounds represented by Chemical Formulas 1 to 5 is included as an electron transport auxiliary layer material, the efficiency of the organic electroluminescent device may be enhanced due to the triplet-triplet fusion (TTF) effect. Furthermore, the compound represented by any one of Chemical Formulas 1 to 5 prevents excitons or holes generated in the emission layer from diffusing into the adjacent electron transport layer. Consequently, the number of excitons contributing to luminescence within the emission layer increases, thereby improving the luminous efficiency of the device. Additionally, the durability and stability of the device are improved, resulting in a significant increase in device lifetime.

The compound represented by any one of Chemical Formulas 1 to 5 may be used alone or in combination with an electron transport auxiliary layer material known in the field.

In the present disclosure, the electron transport auxiliary layer material that may be used in combination with any one of the compounds represented by Chemical Formulas 1 to 5 includes electron transport materials conventionally known in the field, such as oxadiazole derivatives, triazole derivatives, phenanthroline derivatives (e.g., BCP), and heterocyclic derivatives containing nitrogen, but is not limited thereto.

No particular limitations are imparted to the structure of the organic electroluminescent (EL) device according to the present disclosure. For instance, as illustrated in FIGS. 1 to 3, the device may have a laminated structure in which an anode (100), one or more organic layers (300), and a cathode (200) are sequentially stacked on a substrate. Furthermore, although not illustrated, an insulating layer or an adhesive layer may be inserted between the electrode and the organic layer interface.

According to an embodiment, as illustrated in FIG. **1****,** the organic electroluminescent device may have a laminated structure in which an anode (100), a hole injection layer (310), a hole transport layer (320), an emission layer (330), an electron transport layer (340), and a cathode (200) are sequentially stacked on a substrate. Optionally, as illustrated in FIG. 2, an electron injection layer (350) may be positioned between the electron transport layer (340) and the cathode (200). Additionally, an electron transport auxiliary layer (360) may be positioned between the emission layer (330) and the electron transport layer (340) (see FIG. 3).

The organic electroluminescent (EL) device of the present disclosure may be fabricated by forming organic layers and electrodes using materials and methods known in the art, except that at least one organic layer (300) [e.g., an emission layer (330), an electron transport layer (340), or an electron transport auxiliary layer (360)] includes a compound represented by any one of Chemical Formulas 1 to 5.

The organic layer may be formed using vacuum deposition or solution coating methods. Examples of solution coating methods include spin coating, dip coating, doctor blading, inkjet printing, or thermal transfer methods, but with no limitations thereto.

The substrate usable in the present disclosure is not particularly limited, and non-limiting examples include silicon wafers, quartz, glass plates, metal plates, plastic films, and sheets.

Examples of anode materials include metals such as vanadium, chromium, copper, zinc, and gold, or alloys thereof; metal oxides such as zinc oxide, indium oxide, indium tin oxide (ITO), and indium zinc oxide (IZO); combinations of metals and oxides such as ZnO:Al or SnO₂:Sb; conductive polymers such as polythiophene, poly(3-methylthiophene), poly[3,4-(ethylene-1,2-dioxy)thiophene] (PEDT), polypyrrole, or polyaniline; and carbon black, but with no limitations thereto.

Examples of cathode materials include, but are not limited to, metals such as magnesium, calcium, sodium, potassium, titanium, indium, yttrium, lithium, gadolinium, aluminum, silver (Ag), tin, or lead, or alloys thereof; and multilayer structured materials such as LiF/Al or LiO₂/Al.

Additionally, the hole injection layer, hole transport layer, emission layer, and electron injection layer are not particularly limited and may be formed using conventional materials known in the industry.

A better understanding of the present disclosure may be obtained through the following Examples, which are set forth to illustrate, but are not to be construed to limit, the present disclosure.

### [SYNTHESIS EXAMPLE 1] - Synthesis of Compound A1

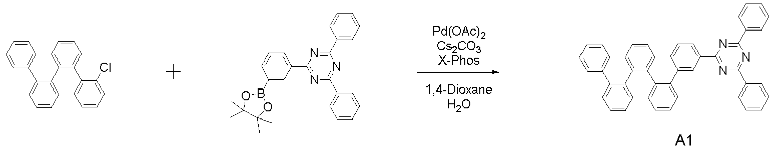

Under a nitrogen stream, 2-chloro-1,1':2',1":2'',1‴-quaterphenyl (15.00 g, 44.01 mmol), 2,4-diphenyl-6-(3-(4,4,5,5-tetramethyl-1,3,2-dioxaborolan-2-yl)phenyl)-1,3,5-triazine (19.16 g, 44.01 mmol), Pd(OAc)₂ (0.30 g, 1.32 mmol), Cs₂CO₃ (28.68 g, 88.02 mmol), X-Phos (2.10g, 4.40 mmol), 1,4-dioxane (200 ml), and H₂O (50 ml) were mixed and stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (22 g, yield: 85 %).
GC-Mass (Calcd.: 613.76 g/mol, Found: 613 g/mol)

### [SYNTHESIS EXAMPLE 2] - Synthesis of Compound A6

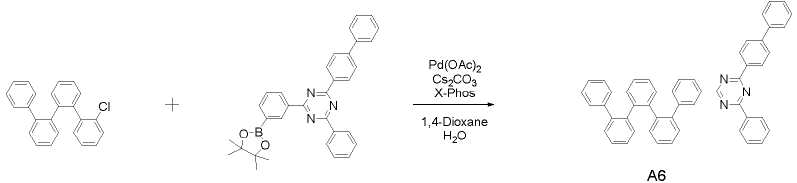

The same procedure as in Synthesis Example 1, with the exception of using 2-([1,1'-biphenyl]-4-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (26 g, yield: 87 %).
GC-Mass (Cacld.: 689.86 g/mol, Found: 689 g/mol)

### [SYNTHESIS EXAMPLE 3] - Synthesis of Compound A7

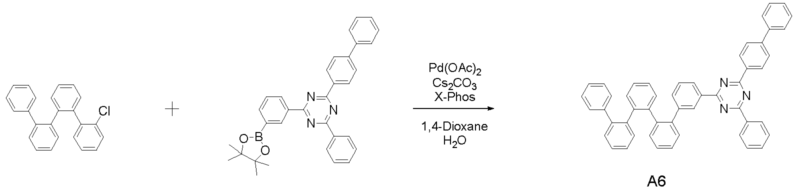

The same procedure as in Synthesis Example 1, with the exception of using 4-([1,1'-biphenyl]-4-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (27 g, yield: 90 %).
GC-Mass (Cacld.: 688.87 g/mol, Found: 688 g/mol)

### [SYNTHESIS EXAMPLE 4] - Synthesis of Compound A12

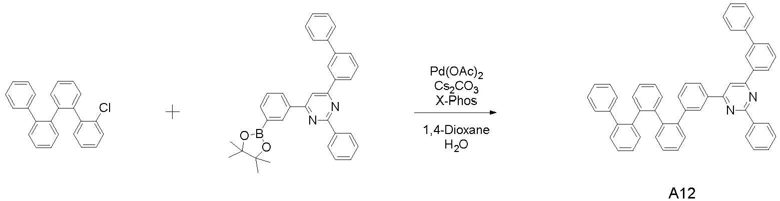

The same procedure as in Synthesis Example 1, with the exception of using 4-([1,1'-biphenyl]-3-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (25 g, yield: 85 %).
GC-Mass (Cacld.: 688.87 g/mol, Found: 688 g/mol)

### [SYNTHESIS EXAMPLE 5] - Synthesis of Compound A93

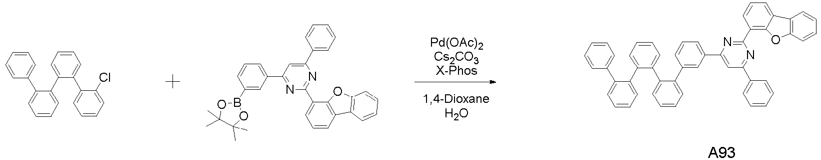

The same procedure as in Synthesis Example 1, with the exception of using 2-(dibenzo[b,d]furan-4-yl)-4-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (27 g, yield: 90 %).
GC-Mass (Cacld.: 702.86 g/mol, Found: 702 g/mol)

### [SYNTHESIS EXAMPLE 6] - Synthesis of Compound B12

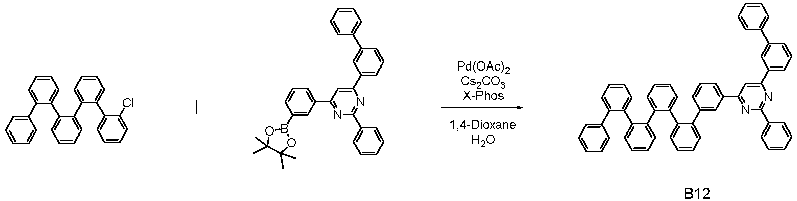

Under nitrogen stream, 2-chloro-1,1':2',1'':2'',1‴:2‴,1ʺʺ-quinquephenyl (15.00 g, 35.98 mmol), 4-([1,1'-biphenyl]-3-yl)-2-phenyl-6-(3-(4,4,5,5-tetramethyl-1,3,2-dioxaborolan-2-yl)phenyl)pyrimidine (18.36 g, 35.98 mmol), Pd(OAc)₂ (0.24 g, 1.08 mmol), Cs₂CO₃ (23.44 g, 71.95 mmol), X-Phos (1.72g, 3.60 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (27 g, yield: 80 %).
GC-Mass (Cacld.: 764.97 g/mol, Found: 764 g/mol)

### [SYNTHESIS EXAMPLE 7] - Synthesis of Compound B11

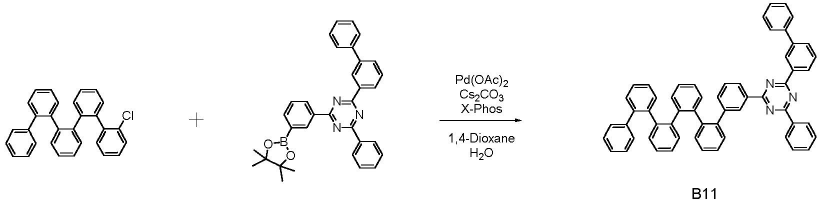

The same procedure as in Synthesis Example 6, with the exception of using 2-([1,1'-biphenyl]-3-yl)-4-phenyl-6-(3-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (23 g, yield: 85 %).
GC-Mass (Cacld.: 765.96 g/mol, Found: 765 g/mol)

### [SYNTHESIS EXAMPLE 8] - Synthesis of Compound C1

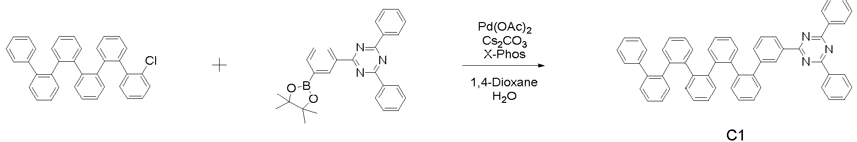

Under nitrogen stream, 2-chloro-1,1':2',1'':2'',1‴:2‴,1ʺʺ:2ʺʺ,1‴ʺ-sexiphenyl (15.00 g, 30.42 mmol), 2,4-diphenyl-6-(3-(4,4,5,5-tetramethyl-1,3,2-dioxaborolan-2-yl)phenyl)-1,3,5-triazine (13.24 g, 30.42 mmol), Pd(OAc)₂ (0.20 g, 0.91 mmol), Cs₂CO₃ (19.82 g, 60.85 mmol), X-Phos (1.45g, 3.04 mmol), 1,4-dioxane (200 ml) and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (17 g, yield: 75 %).
GC-Mass (Cacld.: 765.96 g/mol, Found: 765 g/mol)

### [SYNTHESIS EXAMPLE 9] - Synthesis of Compound C11

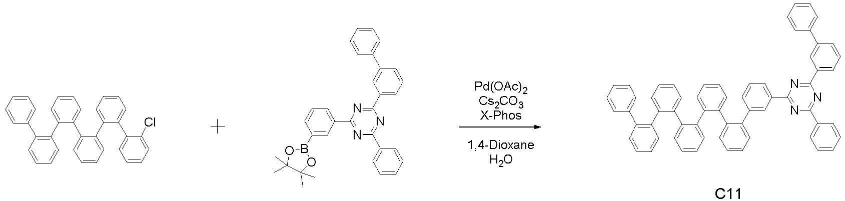

The same procedure as in Synthesis Example 8, with the exception of using 2-([1,1'-biphenyl]-3-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 8, was carried out to afford the target organic compound (18 g, yield: 73 %).
GC-Mass (Cacld.: 842.06 g/mol, Found: 842 g/mol)

### [SYNTHESIS EXAMPLE 10] - Synthesis of Compound C17

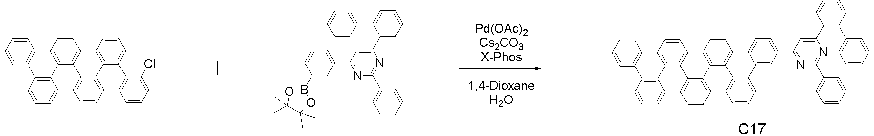

The same procedure as in Synthesis Example 8, with the exception of using 4-([1,1'-biphenyl]-2-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 8, was carried out to afford the target organic compound (19 g, yield: 75 %).
GC-Mass (Cacld.: 841.07 g/mol, Found: 841 g/mol)

### [SYNTHESIS EXAMPLE 11] - Synthesis of Compound D41

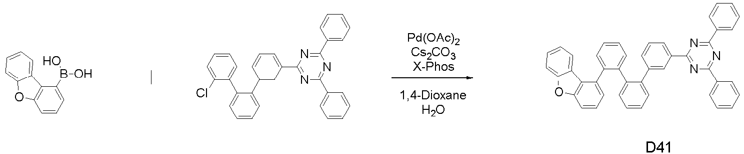

Under nitrogen stream, dibenzo[b,d]furan-1-ylboronic acid (10.00 g, 47.17 mmol), 2-(2''-chloro-[1,1':2',1''-terphenyl]-3-yl)-4,6-diphenyl-1,3,5-triazine (23.40 g, 47.17 mmol), Pd(OAc)₂ (0.32 g, 1.42 mmol), Cs₂CO₃ (30.74 g, 94.34 mmol), X-Phos (2.25g, 4.72 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (23 g, yield: 80 %).
GC-Mass (Cacld.: 627.75 g/mol, Found: 627 g/mol)

### [SYNTHESIS EXAMPLE 12] - Synthesis of Compound D89

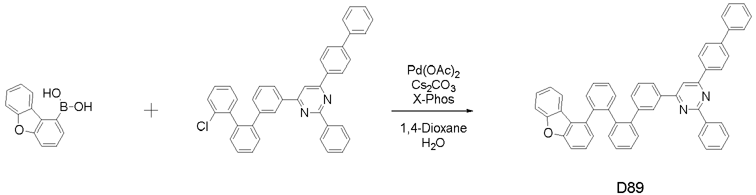

The same procedure as in Synthesis Example 11, with the exception of using 4-([1,1'-biphenyl]-4-yl)-6-(2"-chloro-[1,1':2',1"-terphenyl]-3-yl)-2-phenylpyrimidine instead of 2-(2"-chloro-[1,1':2',1"-terphenyl]-3-yl)-4,6-diphenyl-1,3,5-triazine used in Synthesis Example 11, was carried out to afford the target organic compound (26 g, yield: 80 %).
GC-Mass (Cacld.: 702.86 g/mol, Found: 702 g/mol)

### [SYNTHESIS EXAMPLE 13] - Synthesis of Compound D43

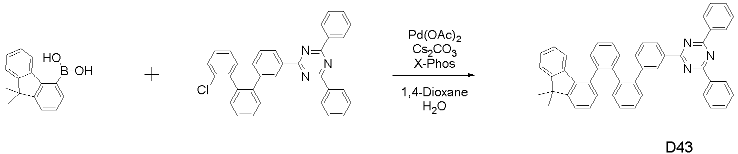

Under nitrogen stream, (9,9-dimethyl-9H-fluoren-4-yl)boronic acid (10.00 g, 42.00 mmol), 2-(2"-chloro-[1,1':2',1"-terphenyl]-3-yl)-4,6-diphenyl-1,3,5-triazine (20.83 g, 42.00 mmol), Pd(OAc)₂ (0.28 g, 1.26 mmol), Cs₂CO₃ (27.37 g, 84.00 mmol), X-Phos (2.00g, 4.20 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (25 g, yield: 92 %).
GC-Mass (Cacld.: 653.83 g/mol, Found: 653 g/mol)

### [SYNTHESIS EXAMPLE 14] - Synthesis of Compound D91

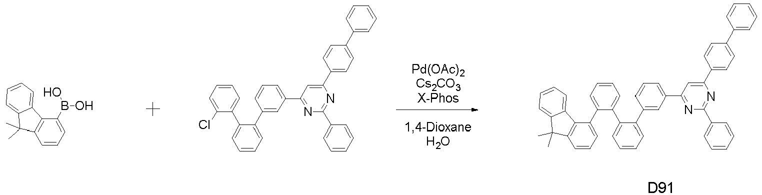

The same procedure as in Synthesis Example 13, with the exception of using 4-([1,1'-biphenyl]-4-yl)-6-(2"-chloro-[1,1':2',1"-terphenyl]-3-yl)-2-phenylpyrimidine instead of 2-(2"-chloro-[1,1':2',1"-terphenyl]-3-yl)-4,6-diphenyl-1,3,5-triazine used in Synthesis Example 13, was carried out to afford the target organic compound (26 g, yield: 80 %).
GC-Mass (Cacld.: 728.94 g/mol, Found: 728 g/mol)

### [SYNTHESIS EXAMPLE 15] - Synthesis of Compound E33

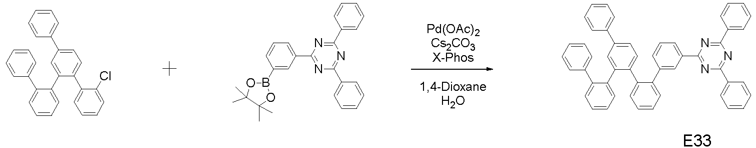

Under nitrogen stream, 2-chloro-4'-phenyl-1,1':2',1":2",1‴-quaterphenyl (15.00 g, 35.98 mmol), 2,4-diphenyl-6-(3-(4,4,5,5-tetramethyl-1,3,2-dioxaborolan-2-yl)phenyl)-1,3,5-triazine (15.66 g, 35.98 mmol), Pd(OAc)₂ (0.24 g, 1.08 mmol), Cs₂CO₃ (23.44 g, 71.95 mmol), X-Phos (1.72g, 3.60 mmol), 1,4-dioxane (200 ml) and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (21 g, yield: 85 %).
GC-Mass (Cacld.: 689.88 g/mol, Found: 689 g/mol)

### [SYNTHESIS EXAMPLE 16] - Synthesis of Compound G33

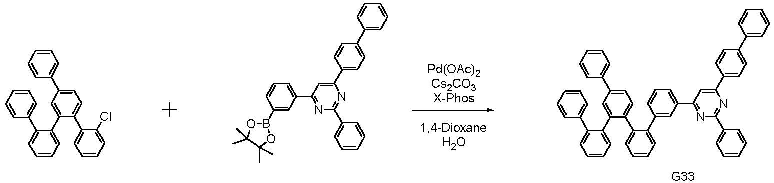

The same procedure as in Synthesis Example 13, with the exception of using 4-([1,1'-biphenyl]-4-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 13, was carried out to afford the target organic compound (22 g, yield: 80 %).
GC-Mass (Cacld.: 764.97 g/mol, Found: 764 g/mol)

### [SYNTHESIS EXAMPLE 17] - Synthesis of Compound E90

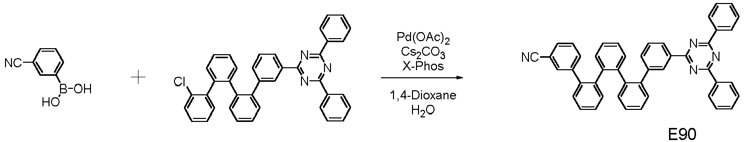

Under nitrogen stream, (3-cyanophenyl)boronic acid (5.00 g, 34.03 mmol), 2-(2‴-chloro-[1,1':2',1'':2'',1‴-quaterphenyl]-3-yl)-4,6-diphenyl-1,3,5-triazine (19.47 g, 34.03 mmol), Pd(OAc)₂ (0.23 g, 1.02 mmol), Cs₂CO₃ (22.17 g, 68.05 mmol), X-Phos (1.62g, 3.40 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (17 g, yield: 80 %).
GC-Mass (Cacld.: 638.77 g/mol, Found: 638 g/mol)

### [SYNTHESIS EXAMPLE 18] - Synthesis of Compound E89

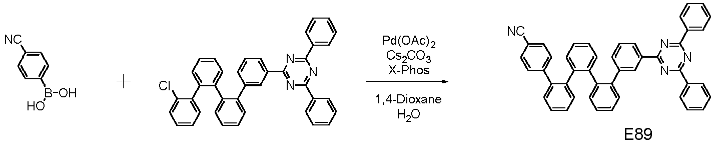

The same procedure as in Synthesis Example 17, with the exception of using (4-cyanophenyl)boronic acid instead of (3-cyanophenyl)boronic acid used in Synthesis Example 17, was carried out to afford the target organic compound (16 g, yield: 75 %).
GC-Mass (Cacld.: 638.77 g/mol, Found: 638 g/mol)

### [SYNTHESIS EXAMPLE 19] - Synthesis of Compound L2

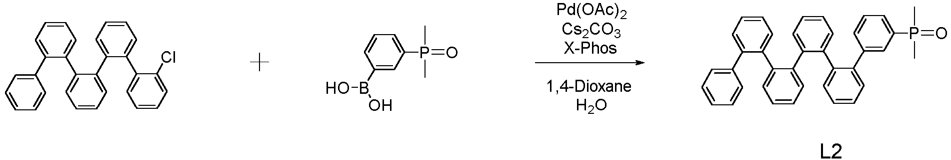

The same procedure as in Synthesis Example 6, with the exception of using (3-(dimethylphosphoryl)phenyl)boronic acid instead of 4-([1,1'-biphenyl]-3-yl)-2-phenyl-6-(3-(4,4,5,5-tetramethyl-1,3,2-dioxaborolan-2-yl)phenyl)pyrimidine, was carried out to afford the target organic compound (20 g, yield: 85 %) .
GC-Mass (Cacld.: 534.64 g/mol, Found: 534 g/mol)

### [SYNTHESIS EXAMPLE 20] - Synthesis of Compound L3

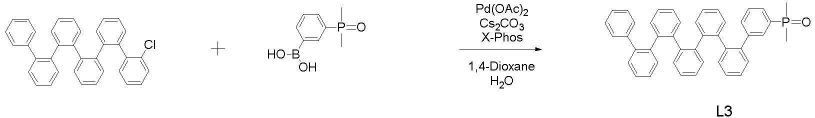

The same procedure as in Synthesis Example 8, with the exception of using (3-(dimethylphosphoryl)phenyl)boronic acid instead of 2,4-diphenyl-6-(3-(4,4,5,5-tetramethyl-1,3,2-dioxaborolan-2-yl)phenyl)-1,3,5-triazine, was carried out to afford the target compound (18 g, yield: 70 %).
GC-Mass (Cacld.: 610.74 g/mol, Found: 610 g/mol)

### [SYNTHESIS EXAMPLE 21] - Synthesis of Compound L21

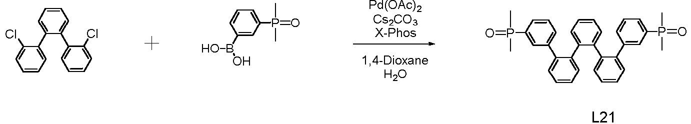

Under nitrogen stream, 2,2''-dichloro-1,1':2',1''-terphenyl (5 g, 16.71 mmol), (3-(dimethylphosphoryl)phenyl)boronic acid (9.92 g, 50.14 mmol), Pd(OAc)₂ (0.15 g, 0.67 mmol), Cs₂CO₃ (21.78 g, 66.85 mmol), X-Phos (4.78g, 10.03 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (6.25 g, yield: 70 %).
GC-Mass (Cacld.: 534.58 g/mol, Found: 534 g/mol)

### [SYNTHESIS EXAMPLE 22] - Synthesis of Compound A2

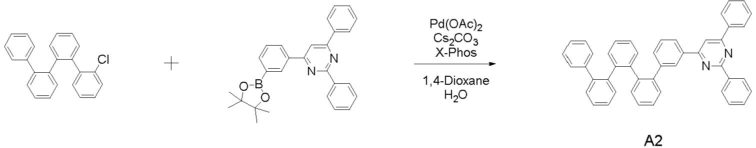

The same procedure as in Synthesis Example 1, with the exception of using 2,4-diphenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (26 g, yield: 75 %).
GC-Mass (Cacld.: 612.78 g/mol, Found: 612 g/mol)

### [SYNTHESIS EXAMPLE 23] - Synthesis of Compound A21

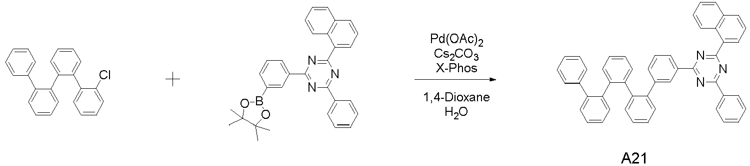

The same procedure as in Synthesis Example 1, with the exception of using 2-(naphthalen-1-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (27 g, yield: 70 %).
GC-Mass (Cacld.: 663.82 g/mol, Found: 663 g/mol)

### [SYNTHESIS EXAMPLE 24] - Synthesis of Compound A16

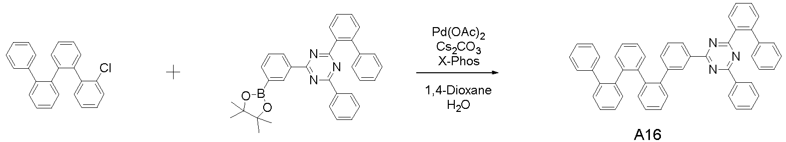

The same procedure as in Synthesis Example 1, with the exception of using 2-([1,1'-biphenyl]-2-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (29 g, yield: 72 %).
GC-Mass (Cacld.: 689.86 g/mol, Found: 689 g/mol)

### [SYNTHESIS EXAMPLE 25] - Synthesis of Compound A26

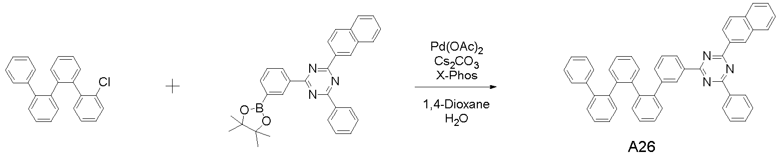

The same procedure as in Synthesis Example 1, with the exception of using 2-(naphthalen-2-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (29 g, yield: 75 %).
GC-Mass (Cacld.: 663.82 g/mol, Found: 663 g/mol)

### [SYNTHESIS EXAMPLE 26] - Synthesis of Compound A54

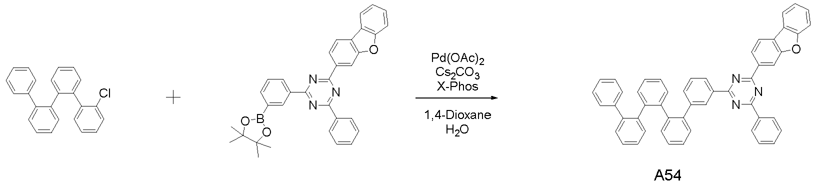

The same procedure as in Synthesis Example 1, with the exception of using 2-(dibenzo[b,d]furan-3-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (29 g, yield: 71 %).
GC-Mass (Cacld.: 703.84 g/mol, Found: 703 g/mol)

### [SYNTHESIS EXAMPLE 27] - Synthesis of Compound A17

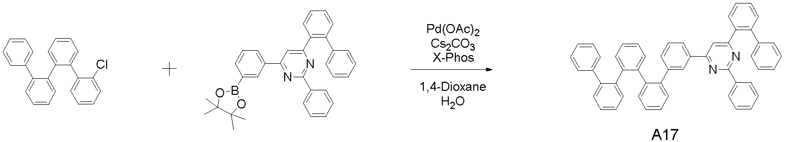

The same procedure as in Synthesis Example 1, with the exception of using 4-([1,1'-biphenyl]-2-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (31 g, yield: 78 %).
GC-Mass (Cacld.: 688.87 g/mol, Found: 688 g/mol)

### [SYNTHESIS EXAMPLE 28] - Synthesis of Compound A22

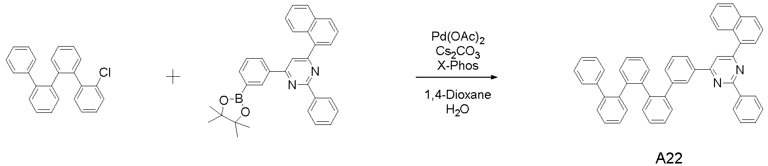

The same procedure as in Synthesis Example 1, with the exception of using 4-(naphthalen-1-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (28 g, yield: 72 %).
GC-Mass (Cacld.: 662.84 g/mol, Found: 662 g/mol)

### [SYNTHESIS EXAMPLE 29] - Synthesis of Compound A27

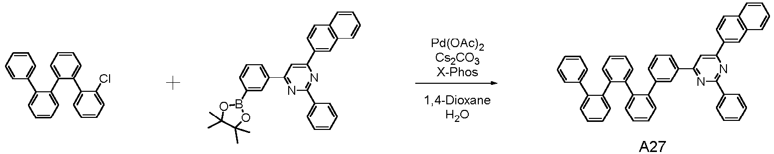

The same procedure as in Synthesis Example 1, with the exception of using 4-(naphthalen-2-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (28 g, yield: 72 %).
GC-Mass (Cacld.: 662.84 g/mol, Found: 662 g/mol)

### [SYNTHESIS EXAMPLE 30] - Synthesis of Compound A74

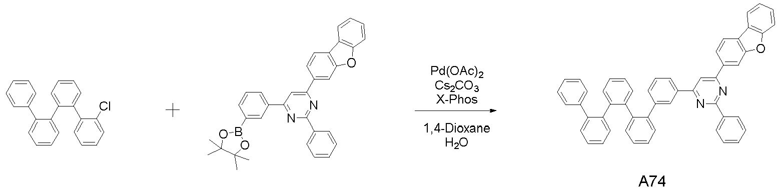

The same procedure as in Synthesis Example 1, with the exception of using 4-(dibenzo[b,d]furan-3-yl)-2-phenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (28 g, yield: 70 %).
GC-Mass (Cacld.: 702.86 g/mol, Found: 702 g/mol)

### [SYNTHESIS EXAMPLE 31] - Synthesis of Compound A9

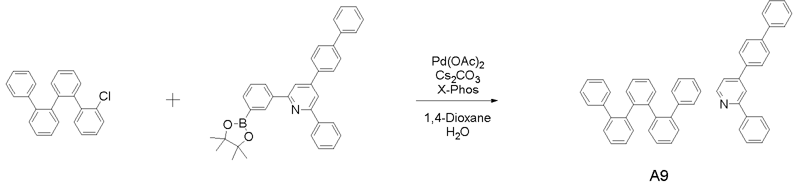

The same procedure as in Synthesis Example 1, with the exception of using 4-([1,1'-biphenyl]-4-yl)-2-phenyl-6-(3-(4,4,5,5-tetramethyl-1,3,2-dioxaborolan-2-yl)phenyl)pyridine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (29 g, yield: 72 %).
GC-Mass (Cacld.: 687.89 g/mol, Found: 687 g/mol)

### [SYNTHESIS EXAMPLE 32] - Synthesis of Compound B1

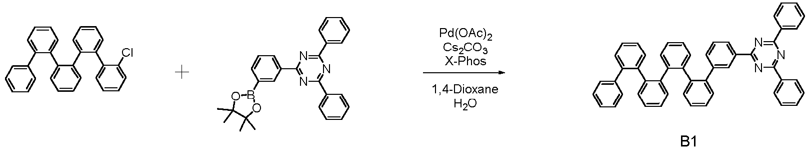

The same procedure as in Synthesis Example 6, with the exception of using 2,4-diphenyl-6-(3-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (23 g, yield: 72 %).
GC-Mass (Cacld.: 689.86 g/mol, Found: 689 g/mol)

### [SYNTHESIS EXAMPLE 33] - Synthesis of Compound B6

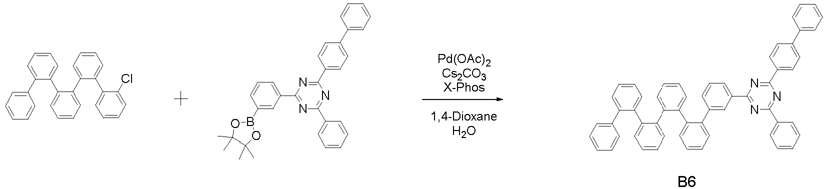

The same procedure as in Synthesis Example 6, with the exception of using 2-([1,1'-biphenyl]-4-yl)-4-phenyl-6-(3-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (25 g, yield: 70 %).
GC-Mass (Cacld.: 765.96 g/mol, Found: 765 g/mol)

### [SYNTHESIS EXAMPLE 34] - Synthesis of Compound B26

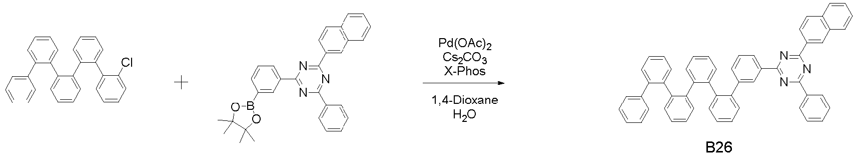

The same procedure as in Synthesis Example 6, with the exception of using 2-(naphthalen-2-yl)-4-phenyl-6-(3-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (26 g, yield: 75 %).
GC-Mass (Cacld.: 739.92 g/mol, Found: 739 g/mol)

### [SYNTHESIS EXAMPLE 35] - Synthesis of Compound B54

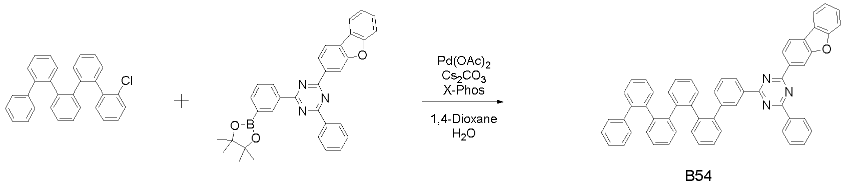

The same procedure as in Synthesis Example 6, with the exception of using 2-(dibenzo[b,d]furan-3-yl)-4-phenyl-6-(3-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (26 g, yield: 70 %).
GC-Mass (Cacld.: 779.94 g/mol, Found: 779 g/mol)

### [SYNTHESIS EXAMPLE 36] - Synthesis of Compound B2

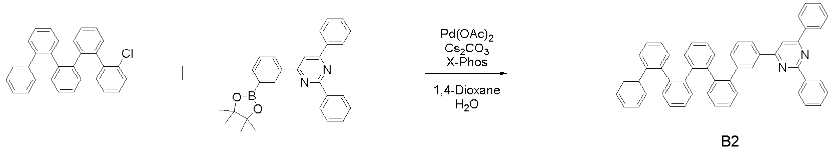

The same procedure as in Synthesis Example 6, with the exception of using 2,4-diphenyl-6-(3-(4,4,5,5-tetramethyl-1,3,2-dioxaborolan-2-yl)phenyl)pyrimidine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (26 g, yield: 80 %).
GC-Mass (Cacld.: 688.87 g/mol, Found: 688 g/mol)

### [SYNTHESIS EXAMPLE 37] - Synthesis of Compound B7

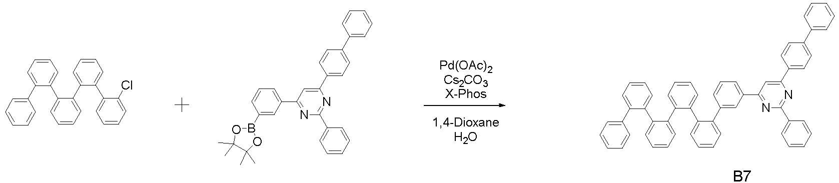

The same procedure as in Synthesis Example 6, with the exception of using 4-([1,1'-biphenyl]-4-yl)-2-phenyl-6-(3-(4,4,5,5-tetramethyl-1,3,2-dioxaborolan-2-yl)phenyl)pyrimidine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (28 g, yield: 77 %).
GC-Mass (Cacld.: 764.97 g/mol, Found: 764 g/mol)

### [SYNTHESIS EXAMPLE 38] - Synthesis of Compound B17

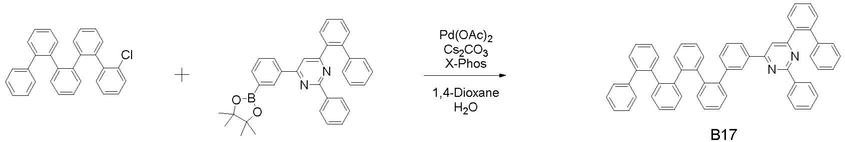

The same procedure as in Synthesis Example 6, with the exception of using 4-([1,1'-biphenyl]-2-yl)-2-phenyl-6-(3-(4,4,5,5-tetramethyl-1,3,2-dioxaborolan-2-yl)phenyl)pyrimidine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (27 g, yield: 75 %).
GC-Mass (Cacld.: 764.97 g/mol, Found: 764 g/mol)

### [SYNTHESIS EXAMPLE 39] - Synthesis of Compound B27

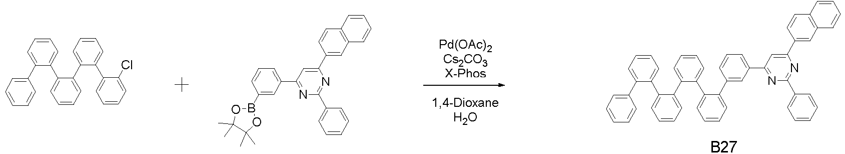

The same procedure as in Synthesis Example 6, with the exception of using 4-(naphthalen-2-yl)-2-phenyl-6-(3-(4,4,5,5-tetramethyl-1,3,2-dioxaborolan-2-yl)phenyl)pyrimidine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (26 g, yield: 75 %).
GC-Mass (Cacld.: 738.93 g/mol, Found: 738 g/mol)

### [SYNTHESIS EXAMPLE 40] - Synthesis of Compound B20

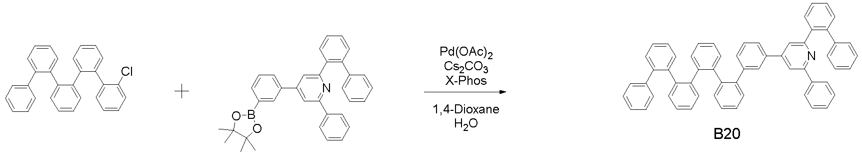

The same procedure as in Synthesis Example 6, with the exception of using 2-([1,1'-biphenyl]-2-yl)-6-phenyl-4-(3-(4,4,5,5-tetramethyl-1,3,2-dioxaborolan-2-yl)phenyl)pyridine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (23 g, yield: 65 %).
GC-Mass (Cacld.: 763.98 g/mol, Found: 763 g/mol)

### [SYNTHESIS EXAMPLE 41] - Synthesis of Compound D1

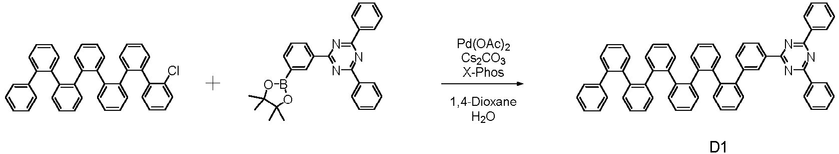

Under nitrogen stream, 2-chloro-1,1':2',1":2",1‴:2‴,1ʺʺ:2ʺʺ,1‴ʺ:2‴ʺ,1‴‴-septiphenyl, (20.00 g, 35.14 mmol), 2,4-diphenyl-6-(3-(4,4,5,5-tetramethyl-1,3,2-dioxaborolan-2-yl)phenyl)-1,3,5-triazine (15.30 g, 35.14 mmol), Pd(OAc)₂ (0.24 g, 1.05 mmol), Cs₂CO₃ (22.90 g, 70.28 mmol), X-Phos (1.68g, 3.51 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (22 g, yield: 75 %).
GC-Mass (Cacld.: 764.97 g/mol, Found: 764 g/mol)

### [SYNTHESIS EXAMPLE 42] - Synthesis of Compound D2

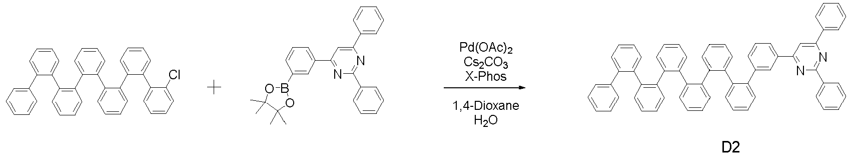

The same procedure as in Synthesis Example 6, with the exception of using 2,4-diphenyl-6-(3-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 41, was carried out to afford the target organic compound (22 g, yield: 75 %).
GC-Mass (Cacld.: 841.07 g/mol, Found: 841 g/mol)

### [SYNTHESIS EXAMPLE 43] - Synthesis of Compound D9

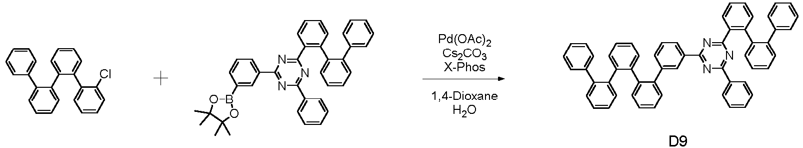

The same procedure as in Synthesis Example 1, with the exception of using 2-([1,1':2',1"-terphenyl]-2-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (36 g, yield: 80 %).
GC-Mass (Cacld.: 765.96g/mol, Found: 765 g/mol)

### [SYNTHESIS EXAMPLE 44] - Synthesis of Compound D10

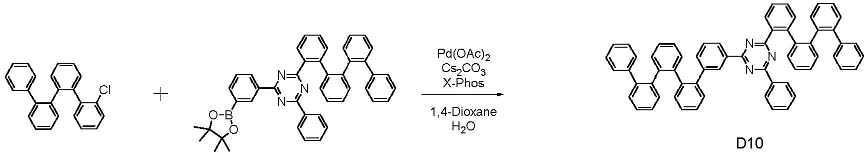

The same procedure as in Synthesis Example 1, with the exception of using 2-([1,1':2',1":2",1‴-quaterphenyl]-2-yl)-4-phenyl-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (35 g, yield: 72 %).
GC-Mass (Cacld.: 842.06g/mol, Found: 842 g/mol)

### [SYNTHESIS EXAMPLE 45] - Synthesis of Compound D15

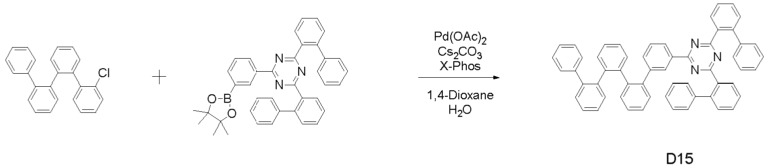

The same procedure as in Synthesis Example 1, with the exception of using 2,4-di([1,1'-biphenyl]-2-yl)-6-(3-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (34 g, yield: 77 %).
GC-Mass (Cacld.: 765.96g/mol, Found: 765 g/mol)

### [SYNTHESIS EXAMPLE 46] - Synthesis of Compound D49

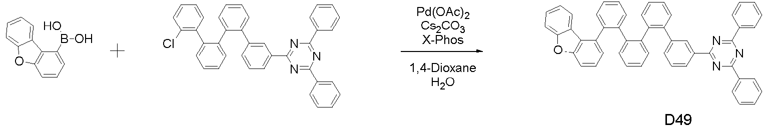

Under nitrogen stream, 2-(2‴-chloro-[1,1':2',1":2",1‴-quaterphenyl]-3-yl)-4,6-diphenyl-1,3,5-triazine, (20.00 g, 34.96 mmol), dibenzo[b,d]furan-1-ylboronic acid (7.41 g, 34.96 mmol), Pd(OAc)₂ (0.24 g, 1.05 mmol), Cs₂CO₃ (22.78 g, 69.92 mmol), X-Phos (1.67g, 3.50 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (16 g, yield: 65 %).
GC-Mass (Cacld.: 703.84 g/mol, Found: 703 g/mol)

### [SYNTHESIS EXAMPLE 47] - Synthesis of Compound D50

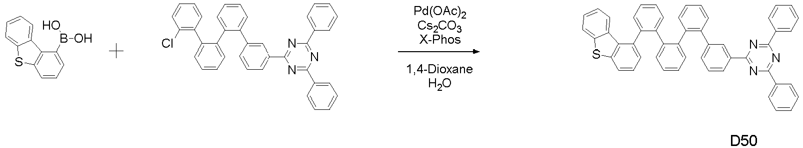

The same procedure as in Synthesis Example 46, with the exception of using dibenzo[b,d]thiophen-1-ylboronic acid instead of dibenzo[b,d]furan-1-ylboronic acid used in Synthesis Example 1, was carried out to afford the target organic compound (18 g, yield: 68 %).
GC-Mass (Cacld.: 719.91g/mol, Found: 719 g/mol)

### [SYNTHESIS EXAMPLE 48] - Synthesis of Compound F3

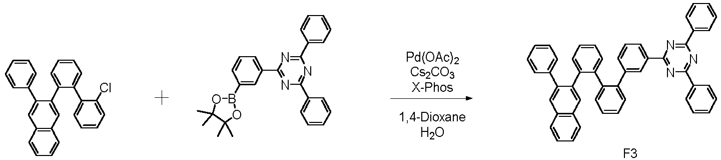

Under nitrogen stream, 2,4-diphenyl-6-(3-(4,4,5,5-tetramethyl-1,3,2-dioxaborolan-2-yl)phenyl)-1,3,5-triazine, (20.00 g, 45.94 mmol), 2-(2'-chloro-[1,1'-biphenyl]-2-yl)-3-phenylnaphthalene (17.96 g, 45.94 mmol), Pd(OAc)₂ (0.31 g, 1.38 mmol), Cs₂CO₃ (29.94 g, 91.88 mmol), X-Phos (2.19g, 4.59 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (23 g, yield: 75 %).
GC-Mass (Cacld.: 663.82 g/mol, Found: 663 g/mol)

### [SYNTHESIS EXAMPLE 49] - Synthesis of Compound F5

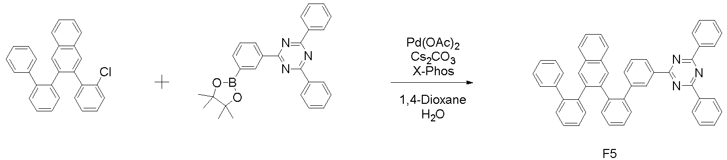

The same procedure as in Synthesis Example 48, with the exception of using 2-([1,1'-biphenyl]-2-yl)-3-(2-chlorophenyl)naphthalene instead of 2-(2'-chloro-[1,1'-biphenyl]-2-yl)-3-phenylnaphthalene used in Synthesis Example 48, was carried out to afford the target organic compound (23 g, yield: 77 %).
GC-Mass (Cacld.: 719.91g/mol, Found: 719 g/mol)

### [SYNTHESIS EXAMPLE 50] - Synthesis of Compound F6

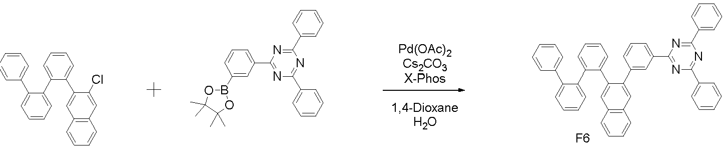

The same procedure as in Synthesis Example 48, with the exception of using 2-([1,1':2',1"-terphenyl]-2-yl)-3-chloronaphthalene instead of 2-(2'-chloro-[1,1'-biphenyl]-2-yl)-3-phenylnaphthalene used in Synthesis Example 48, was carried out to afford the target organic compound (23 g, yield: 75 %).
GC-Mass (Cacld.: 719.91g/mol, Found: 719 g/mol)

### [SYNTHESIS EXAMPLE 51] - Synthesis of Compound F7

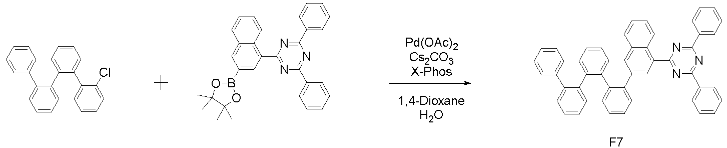

The same procedure as in Synthesis Example 1, with the exception of using 2,4-diphenyl-6-(3-(4,4,5,5-tetramethyl-1,3,2-dioxaborolan-2-yl)naphthalen-1-yl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (28 g, yield: 72 %).
GC-Mass (Cacld.: 663.82 g/mol, Found: 663 g/mol)

### [SYNTHESIS EXAMPLE 52] - Synthesis of Compound H52

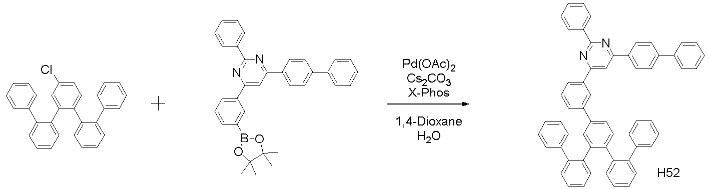

Under nitrogen stream, 4-([1,1'-biphenyl]-4-yl)-2-phenyl-6-(3-(4,4,5,5-tetramethyl-1,3,2-dioxaborolan-2-yl)phenyl)pyrimidine, (20.00 g, 39.18 mmol), 4"-chloro-1,1':2',1":2",1‴:2‴,1ʺʺ-quinquephenyl (16.34 g, 39.18 mmol), Pd(OAc)₂ (0.26 g, 1.18 mmol), Cs₂CO₃ (25.53 g, 78.36 mmol), X-Phos (1.87g, 3.92 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (22 g, yield: 72 %).
GC-Mass (Cacld.: 764.97 g/mol, Found: 764 g/mol)

### [SYNTHESIS EXAMPLE 53] - Synthesis of Compound H64

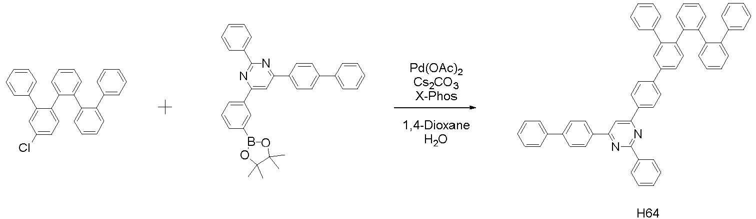

The same procedure as in Synthesis Example 52, with the exception of using 4‴-chloro-1,1':2',1":2",1‴:2‴,1ʺʺ-quinquephenyl instead of 4''-chloro-1.1':2',1":2",1‴,2‴,1ʺʺ-quinquephenyl used in Synthesis Example 52, was carried out to afford the target organic compound (21 g, yield: 70 %).
GC-Mass (Cacld.: 719.91g/mol, Found: 719 g/mol)

### [SYNTHESIS EXAMPLE 54] - Synthesis of Compound I1

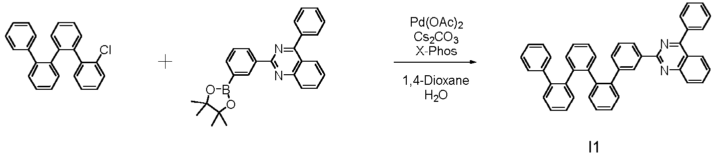

The same procedure as in Synthesis Example 1, with the exception of using 4-phenyl-2-(3-(4,4,5,5-tetramethyl-1,3,2-dioxaborolan-2-yl)phenyl)quinazoline instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (26 g, yield: 76 %).
GC-Mass (Cacld.: 586.74 g/mol, Found: 586 g/mol)

### [SYNTHESIS EXAMPLE 55] - Synthesis of Compound I37

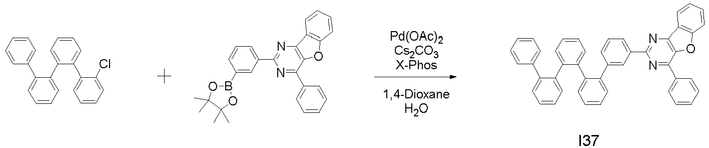

The same procedure as in Synthesis Example 1, with the exception of using 4-phenyl-2-(3-(4,4,5,5-tetramethyl-1,3,2-dioxaborolan-2-yl)phenyl)-313-benzofuro[3,2-d][1,3]chlorazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (29 g, yield: 80 %).
GC-Mass (Cacld.: 626.76 g/mol, Found: 626 g/mol)

### [SYNTHESIS EXAMPLE 56] - Synthesis of Compound J1

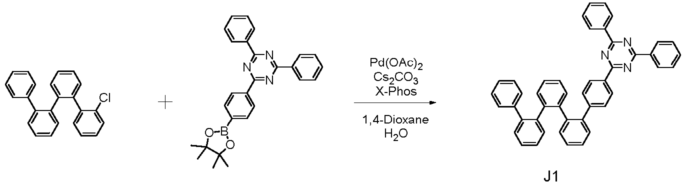

The same procedure as in Synthesis Example 1, with the exception of using 2,4-diphenyl-6-(4-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (27 g, yield: 77 %).
GC-Mass (Cacld.: 613.76 g/mol, Found: 613 g/mol)

### [SYNTHESIS EXAMPLE 57] - Synthesis of Compound J7

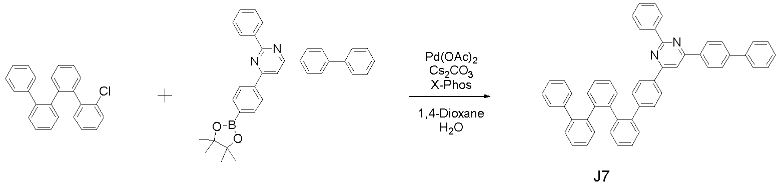

The same procedure as in Synthesis Example 1, with the exception of using 4-([1,1'-biphenyl]-4-yl)-2-phenyl-6-(4-(4,4,5,5-tetramethyl-1,3,2-dioxaborolan-2-yl)phenyl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (30 g, yield: 75 %).
GC-Mass (Cacld.: 688.87 g/mol, Found: 688 g/mol)

### [SYNTHESIS EXAMPLE 58] - Synthesis of Compound J25

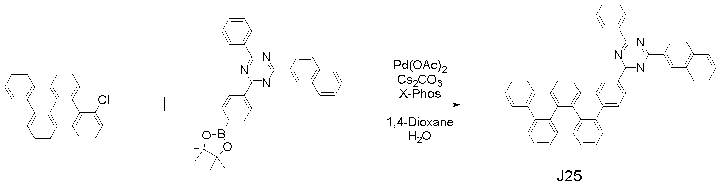

The same procedure as in Synthesis Example 1, with the exception of using 2-(naphthalen-2-yl)-4-phenyl-6-(4-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (30 g, yield: 75 %).
GC-Mass (Cacld.: 663.82 g/mol, Found: 663 g/mol)

### [SYNTHESIS EXAMPLE 59] - Synthesis of Compound J21

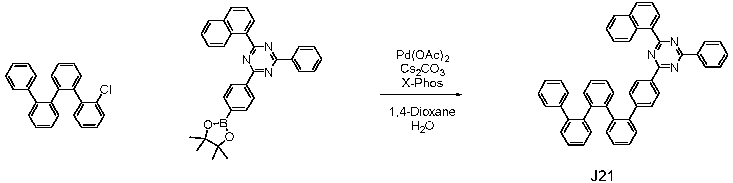

The same procedure as in Synthesis Example 1, with the exception of using 2-(naphthalen-1-yl)-4-phenyl-6-(4-(4,4,5,5-tetramethyl-1,3,2-dioxaborolan-2-yl)phenyl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (27 g, yield: 70 %).
GC-Mass (Cacld.: 663.82 g/mol, Found: 663 g/mol)

### [SYNTHESIS EXAMPLE 60] - Synthesis of Compound J41

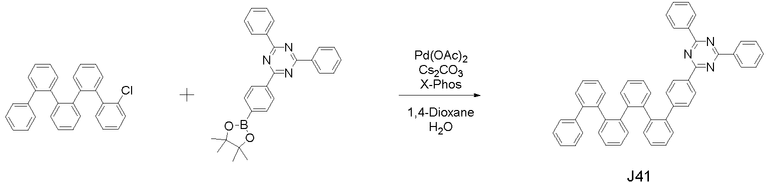

The same procedure as in Synthesis Example 6, with the exception of using 2,4-diphenyl-6-(4-(4,4,5,5-tetramethyl-1,3,2-dioxaborolan-2-yl)phenyl)-1,3,5-triazine instead of 4-([1,1'-biphenyl]-3-yl)-2-phenyl-6-(3-(4,4,5,5-tetramethyl-1,3,2-dioxaborolan-2-yl)phenyl)pyrimidine used in Synthesis Example 6, was carried out to afford the target organic compound (23 g, yield: 71 %).
GC-Mass (Cacld.: 689.86 g/mol, Found: 689 g/mol)

### [SYNTHESIS EXAMPLE 61] - Synthesis of Compound M16

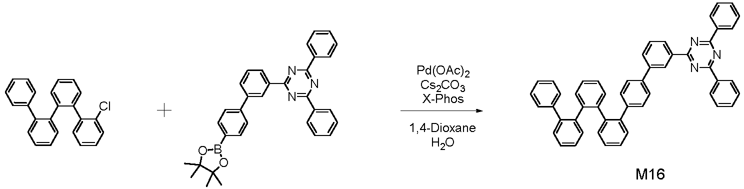

The same procedure as in Synthesis Example 1, with the exception of using 2,4-diphenyl-6-(4'-(4,4,5,5-tetramethyl-1,3,2-dioxaborolan-2-yl)-[1,1'-biphenyl]-3-yl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (28 g, yield: 71 %).
GC-Mass (Cacld.: 689.86 g/mol, Found: 689 g/mol)

### [SYNTHESIS EXAMPLE 62] - Synthesis of Compound M22

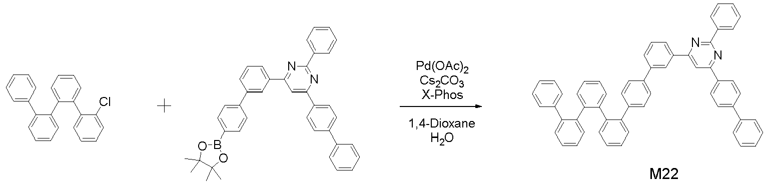

The same procedure as in Synthesis Example 1, with the exception of using 4-([1,1'-biphenyl]-4-yl)-2-phenyl-6-(4'-(4,4,5,5-tetramethyl-1,3,2-dioxaborolan-2-yl)-[1,1'-biphenyl]-3-yl)pyrimidine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (36 g, yield: 81 %).
GC-Mass (Cacld.: 764.97 g/mol, Found: 764 g/mol)

### [SYNTHESIS EXAMPLE 63] - Synthesis of Compound M31

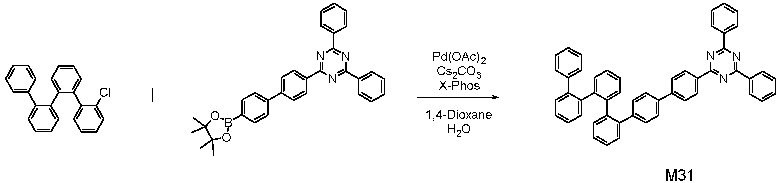

The same procedure as in Synthesis Example 1, with the exception of using 2,4-diphenyl-6-(4'-(4,4,5,5-tetramethyl-1,3,2-dioxaborolan-2-yl)-[1,1'-biphenyl]-4-yl)-1,3,5-triazine instead of 2,4-diphenyl-6-(3-(4,4,5,5-tetramethyl-1,3,2-dioxaborolan-2-yl)phenyl)-1,3,5-triazine used in Synthesis Example 1, was carried out to afford the target organic compound (31 g, yield: 78 %).
GC-Mass (Cacld.: 689.86 g/mol, Found: 689 g/mol)

### [SYNTHESIS EXAMPLE 64] - Synthesis of Compound O2

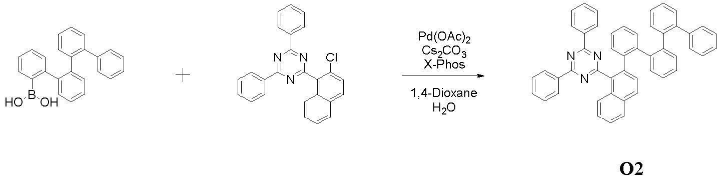

Under nitrogen stream, [1,1':2',1":2'',1‴-quaterphenyl]-2-ylboronic acid, (20.00 g, 57.11 mmol), 2-(2-chloronaphthalen-1-yl)-4,6-diphenyl-1,3,5-triazine (22.49 g, 57.11 mmol), Pd(OAc)₂ (0.38 g, 1.71 mmol), Cs₂CO₃ (37.21 g, 114.21 mmol), X-Phos (2.72g, 5.71 mmol), 1,4-dioxane (200 ml), and H₂O (50 ml) were mixed and then stirred at 110°C for 4 hours. After completion of the reaction, the reaction mixture was subjected to extraction with dichloromethane. The extract thus obtained was added with MgSO₄, followed by filtration. After removal of the solvent from the filtered organic layer, column chromatography afforded the target organic compound (24 g, yield: 65 %).
GC-Mass (Cacld.: 663.82 g/mol, Found: 663 g/mol)

### [SYNTHESIS EXAMPLE 65] - Synthesis of Compound O4

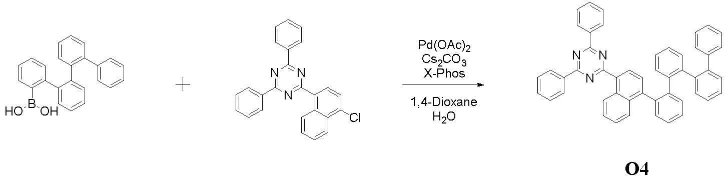

The same procedure as in Synthesis Example 64, with the exception of using 2-(4-chloronaphthalen-1-yl)-4,6-diphenyl-1,3,5-triazine instead of 2-(2-chloronaphthalen-1-yl)-4,6-diphenyl-1,3,5-triazine used in Synthesis Example 64, was carried out to afford the target organic compound (25 g, yield: 67 %).
GC-Mass (Cacld.: 663.82 g/mol, Found: 663 g/mol)

### [SYNTHESIS EXAMPLE 66] - Synthesis of Compound O35

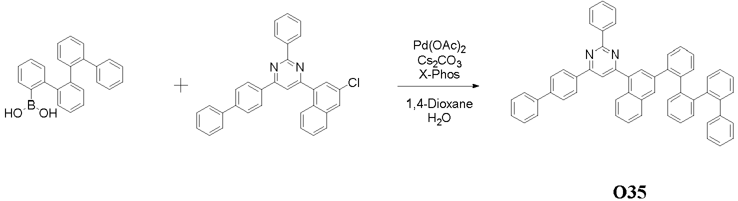

The same procedure as in Synthesis Example 64, with the exception of using 4-([1,1'-bipheny1]-4-yl)-6-(3-chloronaphthalen-1-yl)-2-phenylpyrimidine instead of 2-(2-chloronaphthalen-1-yl)-4,6-diphenyl-1,3,5-triazine used in Synthesis Example 64, was carried out to afford the target organic compound (30 g, yield: 72 %).
GC-Mass (Cacld.: 738.93 g/mol, Found: 738 g/mol)

### [SYNTHESIS EXAMPLE 67] - Synthesis of Compound N3

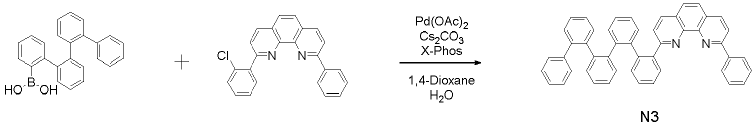

The same procedure as in Synthesis Example 64, with the exception of using 2-(2-chlorophenyl)-9-phenyl-1,10-phenanthroline instead of 2-(2-chloronaphthalen-1-yl)-4,6-diphenyl-1,3,5-triazine used in Synthesis Example 64, was carried out to afford the target organic compound (28 g, yield: 75 %).
GC-Mass (Cacld.: 636.80 g/mol, Found: 636 g/mol)

### [SYNTHESIS EXAMPLE 68] - Synthesis of Compound N5

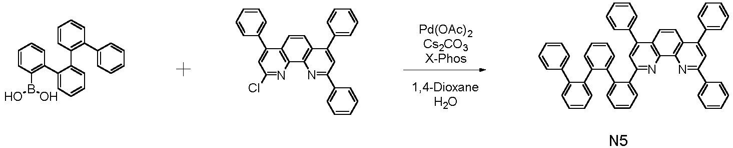

The same procedure as in Synthesis Example 64, with the exception of using 2-chloro-4,7,9-triphenyl-1,10-phenanthroline instead of 2-(2-chloronaphthalen-1-yl)-4,6-diphenyl-1,3,5-triazine used in Synthesis Example 64, was carried out to afford the target organic compound (31 g, yield: 77 %).
GC-Mass (Cacld.: 712.90 g/mol, Found: 712 g/mol)

### [EXAMPLES 1 TO 55] - Fabrication of Blue Organic Electroluminescent Devices

The compounds synthesized in the above Synthesis Examples was subjected to high-purity sublimation purification using a conventionally known method, and then a blue organic electroluminescent devices were fabricated according to the following process.

First, an ITO (indium tin oxide)-coated glass substrate with a thin film thickness of 1200 Å was cleaned using ultrasonic waves with distilled water. After distilled water cleaning, the substrate was ultrasonically cleaned using solvents such as isopropyl alcohol, acetone, and methanol, and then dried. Subsequently, the substrate was transferred to a UV OZONE cleaner (Power Sonic 405, Hwashin Tech), where UV treatment was performed for 5 minutes to clean the substrate before transferring it to a vacuum deposition system.

On the prepared ITO transparent electrode, the following layers were sequentially deposited to fabricate the organic electroluminescent device: HI + 2% HAT-CN6 (10 nm) / HI (140 nm) / EB (5 nm) / BH + 2% BD (20 nm) / Electron transport auxiliary layer material from Table 1 (5 nm) / ET + Liq (1:1) (30 nm) / LiF (1 nm) / Al (100 nm). The structures of HI, HAT-CN6, EB, BH, BD, ET, and Liq are as follows.

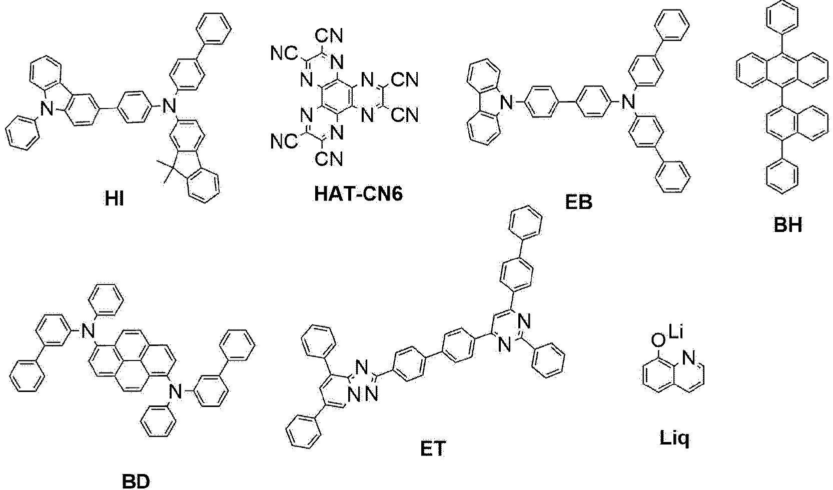

### [COMPARATIVE EXAMPLES 1 TO 4] Fabrication of Blue Organic Electroluminescent Devices

Blue organic electroluminescent devices were fabrication in the same manner as in Example 1 with the exception of using HB1, HB2, HB3, and HB4, instead of compound A1, as electron transport auxiliary layer materials. The structures of HB1, HB2, HB3, and HB4 are as follows:

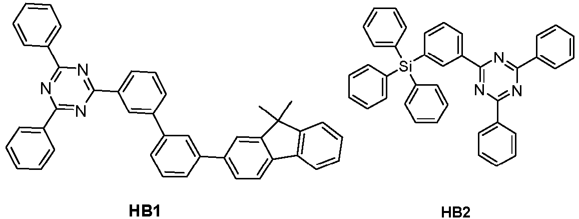

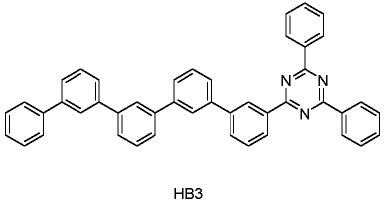

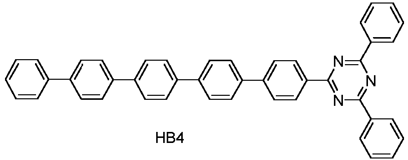

### [EVALUATION EXAMPLE 1]

The organic electroluminescent devices fabricated in Examples 1 to 55 and Comparative Examples 1 to 4 were measured for driving voltage, emission wavelength, current efficiency at a current density of 10 mA/cm², and the measurements are summarized in Table 1, below.

**TABLE 1**

| Sample | Electron Transport Auxiliary Layer Material | Driving Volt. (V) | Emission Peak (nm) | Current Efficiency (cd/A) |
|---|---|---|---|---|
| Ex. 1 | A1 | 4.0 | 454 | 7.9 |
| Ex. 2 | A6 | 3.9 | 455 | 8.1 |
| Ex. 3 | A7 | 4.5 | 456 | 8.0 |
| Ex. | A12 | 3.9 | 459 | 6.9 |
| Ex. 5 | A93 | 4.1 | 452 | 8.1 |
| Ex. 6 | B12 | 4.2 | 454 | 8.3 |
| Ex. 7 | B11 | 4.4 | 455 | 7.7 |
| Ex. 8 | C1 | 5.0 | 455 | 7.5 |
| Ex. 9 | C11 | 5.1 | 458 | 7.2 |
| Ex. 10 | C17 | 3.9 | 452 | 8.1 |
| Ex. 11 | D41 | 3.8 | 451 | 7.8 |
| Ex. 12 | D89 | 4.4 | 455 | 8.3 |
| Ex. 13 | D43 | 4.2 | 453 | 7.1 |
| Ex. 14 | D91 | 4.0 | 454 | 6.6 |
| Ex. 15 | E33 | 4.2 | 457 | 7.7 |
| Ex. 16 | G33 | 4.1 | 456 | 7.8 |
| Ex. 17 | E90 | 4.3 | 451 | 6.4 |
| Ex. 18 | E89 | 4.5 | 458 | 7.6 |
| Ex. 19 | A2 | 3.8 | 458 | 7.1 |
| Ex. 20 | A16 | 4.0 | 452 | 7.5 |
| Ex. 21 | A54 | 4.2 | 451 | 7.8 |
| Ex. 22 | A17 | 4.5 | 455 | 8.1 |
| Ex. 23 | A22 | 3.8 | 453 | 7.5 |
| Ex. 24 | A27 | 3.8 | 452 | 8.4 |
| Ex. 25 | A74 | 4.3 | 451 | 8.3 |
| Ex. 26 | A93 | 4.4 | 455 | 8.0 |
| Ex. 27 | B1 | 4.7 | 453 | 8.1 |
| Ex. 28 | B54 | 4.0 | 454 | 8.0 |
| Ex. 29 | B2 | 3.8 | 457 | 7.5 |
| Ex. 30 | B7 | 4.1 | 454 | 7.7 |
| Ex. 31 | B17 | 4.7 | 455 | 7.2 |
| Ex. 32 | B20 | 4.3 | 456 | 8.2 |
| Ex. 33 | D2 | 4.3 | 459 | 7.1 |
| Ex. 34 | D91 | 4.7 | 452 | 8.1 |
| Ex. 35 | D10 | 4.4 | 451 | 7.9 |
| Ex. 36 | D15 | 3.9 | 458 | 7.4 |
| Ex. 37 | D49 | 4.0 | 458 | 7.4 |
| Ex. 38 | D50 | 4.2 | 452 | 8.1 |
| Ex. 39 | F3 | 4.5 | 451 | 7.9 |
| Ex. 40 | F5 | 4.1 | 455 | 8.5 |
| Ex. 41 | F6 | 4.7 | 453 | 8.1 |
| Ex. 42 | F7 | 4.0 | 451 | 7.6 |
| Ex. 43 | W52 | 3.9 | 455 | 8.1 |
| Ex. 44 | H64 | 4.1 | 453 | 7.7 |
| Ex. 45 | I1 | 4.6 | 452 | 7.5 |
| Ex. 46 | I37 | 4.3 | 451 | 7.7 |
| Ex. 47 | J1 | 4.4 | 455 | 7.4 |
| Ex. 48 | J7 | 4.4 | 453 | 8.0 |
| Ex. 49 | J41 | 4.7 | 454 | 8.3 |
| Ex. 50 | M16 | 4.1 | 457 | 7.1 |
| Ex. 51 | M22 | 3.8 | 459 | 7.5 |
| Ex. 52 | M31 | 4.0 | 452 | 8.3 |
| Ex. 53 | O2 | 4.1 | 451 | 7.0 |
| Ex. 54 | O4 | 4.2 | 458 | 7.7 |
| Ex. 55 | O35 | 4.0 | 452 | 7.6 |
| C. Ex. 1 | HB1 | 5.1 | 458 | 5.9 |
| C. Ex. 2 | HB2 | 5.2 | 454 | 5.5 |
| C. Ex. 3 | HB3 | 5.5 | 452 | 5.1 |
| C. Ex. 4 | HB4 | 6.0 | 455 | 4.8 |

From Table 1, it was confirmed that the organic electroluminescent devices fabricated in Examples 1 to 55 exhibited superior performance in terms of driving voltage, emission peak, and current efficiency compared to the organic electroluminescent devices fabricated in Comparative Examples 1 to 4.

### [EXAMPLES 56 TO 101] Fabrication of Blue Organic Electroluminescent Device

The compounds synthesized in the above synthesis examples were subjected to high-purity sublimation purification using a conventionally known method, and then a blue organic electroluminescent (EL) device was fabricated according to the following process.

First, an ITO (indium tin oxide)-coated glass substrate with a thin film thickness of 1200 Å was cleaned using ultrasonic waves with distilled water. After distilled water cleaning, the substrate was ultrasonically cleaned using solvents such as isopropyl alcohol, acetone, and methanol, followed by drying. Subsequently, the substrate was transferred to a UV OZONE cleaner (Power Sonic 405, Hwashin Tech), where UV treatment was performed for 5 minutes to clean the substrate before transferring it to a vacuum deposition system.

On the prepared ITO transparent electrode, the following layers were sequentially deposited to fabricate the organic electroluminescent device: HI + 2% HAT-CN6 (10 nm) / HI (140 nm) / EB (5 nm) / BH + 2% BD (20 nm) / Electron transport layer material from Table 2 + Liq (1:1) (30 nm) / LiF (1 nm) / Al (100 nm). The structures of HI, HAT-CN6, EB, BH, BD, and Liq are as follows:

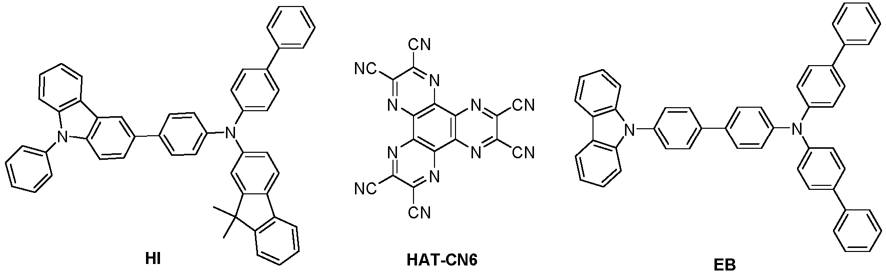

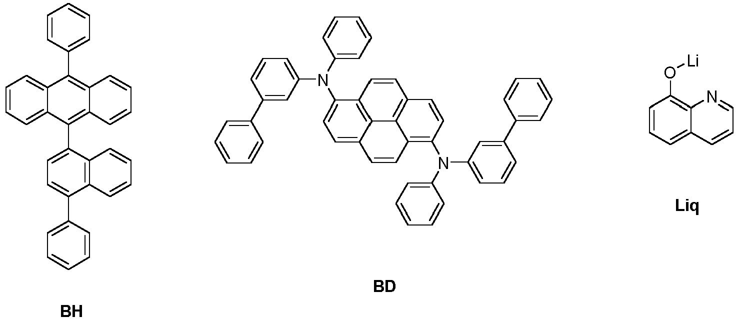

### [COMPARATIVE EXAMPLES 5 TO 10] Fabrication of Blue Organic Electroluminescent Devices

Blue organic electroluminescent devices were fabricated in the same manner as in Example 19, with the exception of using ET, HB2, HB3, HB4, ET-1, and ET-2, instead of compound A1, as electron transport layer materials. The structures of ET, HB2, HB3, and HB4 are as follows:

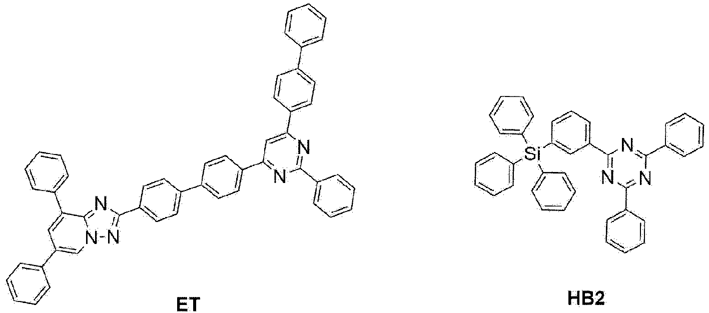

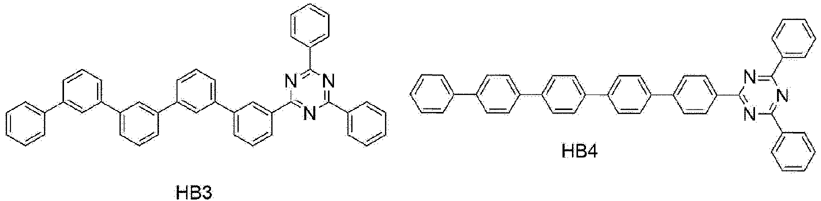

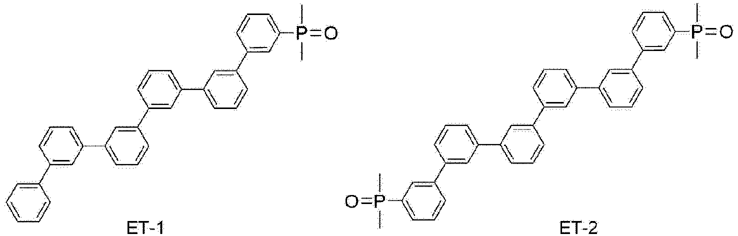

### [EVALUATION EXAMPLE 2]

The organic electroluminescent devices fabricated in Examples 56 to 101 and Comparative Examples 5 to 10 were measured for driving voltage, emission wavelength, current efficiency at a current density of 10 mA/cm², and the measurements are summarized in Table 2, below.

**TABLE 2**

| Sample | Electron Transport Auxiliary Layer Material | Driving Volt. (V) | Emission Peak (nm) | Current Efficiency (cd/A) |
|---|---|---|---|---|
| Ex. 56 | A1 | 3.2 | 454 | 8.2 |
| Ex. 57 | A6 | 3.7 | 455 | 8.5 |
| Ex. 58 | B11 | 3.5 | 455 | 7.7 |
| Ex. 59 | C1 | 3.5 | 455 | 8.8 |
| Ex. 60 | C11 | 3.7 | 458 | 9.1 |
| Ex. 61 | D41 | 3.8 | 451 | 8.5 |
| Ex. 62 | D43 | 3.5 | 453 | 8.5 |
| Ex. 63 | E33 | 3.8 | 457 | 7.8 |
| Ex. 64 | E90 | 3.2 | 451 | 8.4 |
| Ex. 65 | E89 | 3.9 | 458 | 7.9 |
| Ex. 66 | L2 | 4.1 | 455 | 9.1 |
| Ex. 67 | L3 | 4.1 | 453 | 9.0 |
| Ex. 68 | L21 | 3.8 | 455 | 8.7 |
| Ex. 69 | A2 | 4.2 | 453 | 9.1 |
| Ex. 70 | A21 | 4.0 | 457 | 8.5 |
| Ex. 71 | A16 | 3.5 | 451 | 8.5 |
| Ex. 72 | A26 | 3.3 | 458 | 7.8 |
| Ex. 73 | A54 | 4.0 | 455 | 8.4 |
| Ex. 74 | A27 | 3.5 | 455 | 7.9 |
| Ex. 75 | B11 | 3.7 | 455 | 7.5 |
| Ex. 76 | B6 | 3.5 | 458 | 8.6 |
| Ex. 77 | B26 | 3.5 | 451 | 7.7 |
| Ex. 78 | B7 | 4.1 | 453 | 7.9 |
| Ex. 79 | B27 | 3.7 | 457 | 8.6 |
| Ex. 80 | B20 | 3.9 | 451 | 7.9 |
| Ex. 81 | D1 | 3.6 | 455 | 8.4 |
| Ex. 82 | D15 | 4.0 | 453 | 8.6 |
| Ex. 83 | F3 | 3.6 | 457 | 9.0 |
| Ex. 84 | F5 | 4.1 | 451 | 8.7 |
| Ex. 85 | F6 | 4.0 | 458 | 9.1 |
| Ex. 86 | F7 | 3.7 | 455 | 8.5 |
| Ex. 87 | I1 | 3.6 | 455 | 8.5 |
| Ex. 88 | I37 | 3.8 | 455 | 7.8 |
| Ex. 89 | J1 | 4.0 | 455 | 7.9 |
| Ex. 90 | J7 | 3.5 | 458 | 8.4 |
| Ex. 91 | J25 | 3.7 | 451 | 8.6 |
| Ex. 92 | J21 | 3.4 | 453 | 9.0 |
| Ex. 93 | J41 | 3.8 | 457 | 8.7 |
| Ex. 94 | M16 | 3.5 | 451 | 9.1 |
| Ex. 95 | M22 | 4.0 | 458 | 8.5 |
| Ex. 96 | M31 | 3.7 | 455 | 8.8 |
| Ex. 97 | O2 | 3.6 | 455 | 9.1 |
| Ex. 98 | O4 | 3.8 | 455 | 8.5 |
| Ex. 99 | O35 | 4.0 | 455 | 8.5 |
| Ex. 100 | N3 | 3.5 | 458 | 7.8 |
| Ex. 101 | N5 | 3.7 | 455 | 9.0 |
| C. Ex. 5 | ET | 4.7 | 458 | 6.9 |
| C. Ex. 6 | HB2 | 5.2 | 454 | 6.5 |
| C. Ex. 7 | HB3 | 5.5 | 452 | 7.1 |
| C. Ex. 8 | HB4 | 4.8 | 455 | 6.8 |
| C. Ex. 9 | ET-1 | 5.4 | 458 | 7.0 |
| C. Ex. 10 | ET-2 | 5.2 | 451 | 7.1 |

From Table 2, it was confirmed that the organic electroluminescent devices fabricated in Examples 56 to 101 exhibited superior performance in terms of driving voltage, emission peak, and current efficiency compared to the organic electroluminescent devices fabricated in Comparative Examples 5 to 10.

## Claims

1. An organic compound represented by any one of the following Chemical Formulas 1 to 5: 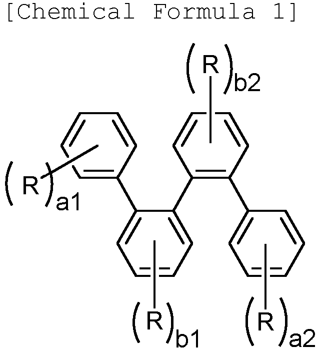 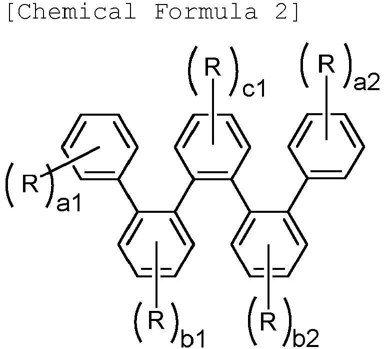 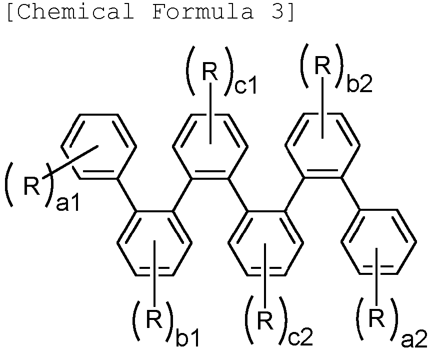 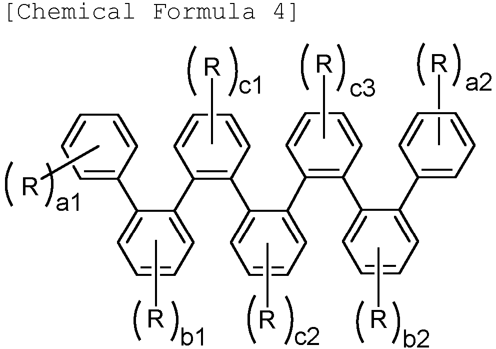 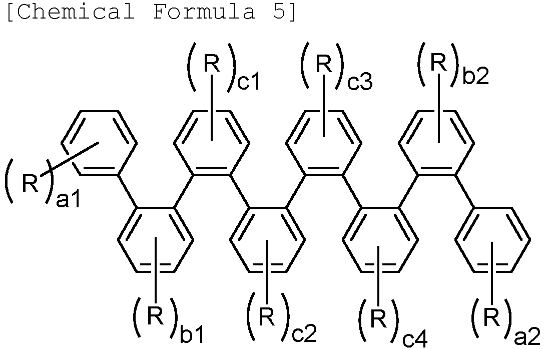 (wherein,
a1 and a2 are each an integer of 0 to 4,
b1 and b2 are each an integer of 0 to 3,
c1, c2, c3, and c4 are each an integer of 0 to 2,
with a proviso of a1+a2+b1+b2≥1, a1+a2+b1+b2+c1≥1, a1+a2+b1+b2+c1+c2≥1, a1+a2+b1+b2+c1+c2+c3≥1, and a1+a2+b1+b2+c1+c2+c3+c4≥1,
the plural R's, which are the same or different from each other, are each independently selected from the group consisting of a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, or may form a fused ring with an adjacent group,
with a proviso that at least one of the plural R's is a moiety A represented by the following Chemical Formula A1 or A2, 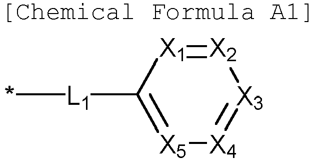 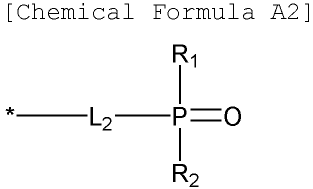
wherein,
L₁ and L₂ are each independently a single bond or a C₆-C₆₀ arylene group,
X₁ to X₅, which are the same or different from each other, are each independently N or C(R₃), with a proviso that at least one of X₁ to X₅ being N, wherein when two or more C(R₃) are present, the plural R₃'s are the same or different from each other,
R₁ to R₃, which are the same or different from each other, are each independently selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, or may form a fused ring with an adjacent group,
the alkyl group, alkenyl group, alkynyl group, cycloalkyl group, heterocycloalkyl group, aryl group, heteroaryl group, alkyloxy group, aryloxy group, alkylsilyl group, arylsilyl group, alkylboron group, arylboron group, alkylphosphine oxide group, arylphosphine group, alkylphosphine group, arylphosphine oxide group, and arylamine group and the fused ring in R and R₁ to R₃, and the arylene group in L₁ and L₂ are unsubstituted or substituted with at least one substituent selected from the group consisting of a deuterium atom, a halogen group, a cyano group, a nitro group, a C₂-C₄₀ alkenyl group, a C₂-C₄₀ alkynyl group, a C₃-C₄₀ cycloalkyl group, a heterocycloalkyl group having 3 to 40 nuclear atoms, a C₁-C₄₀ alkyl group, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group, wherein when the substituent is present in a plurality of numbers, a plurality of substituents are the same or different from each other).

2. The organic compound of Claim 1, wherein the organic compound represented by any one of Chemical Formulas 1 to 5 is represented by any one of the following Chemical Formulas 1a to 5k: 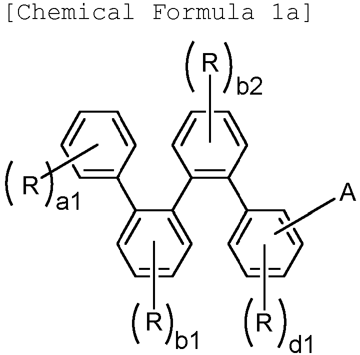 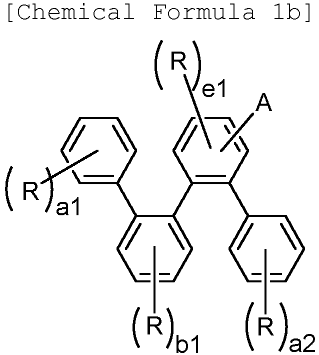 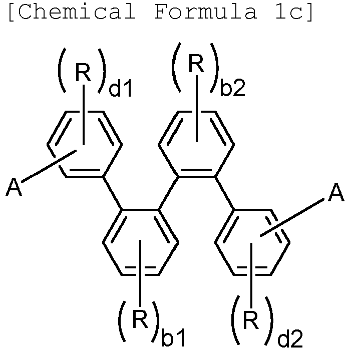 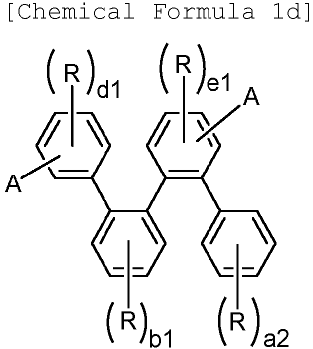 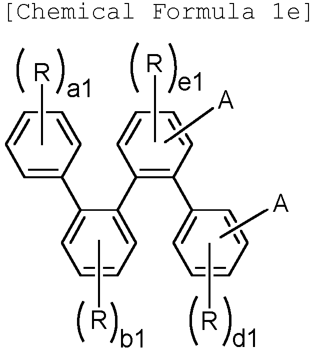 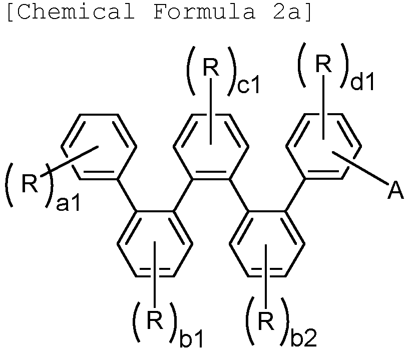 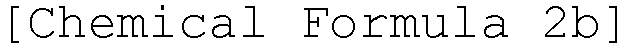 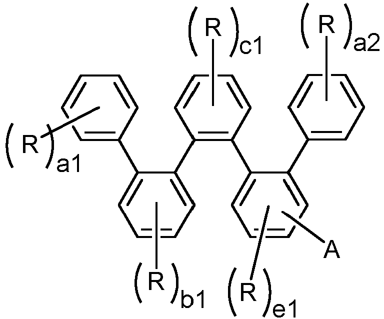 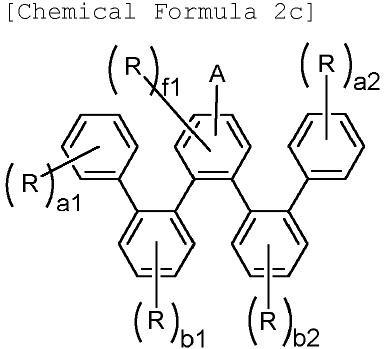 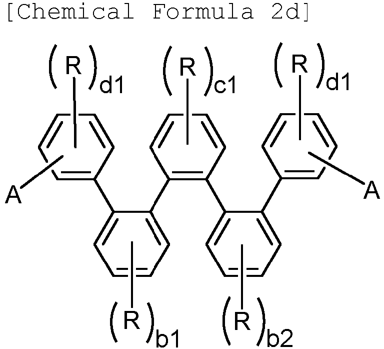 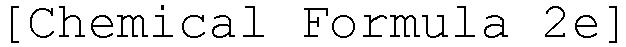 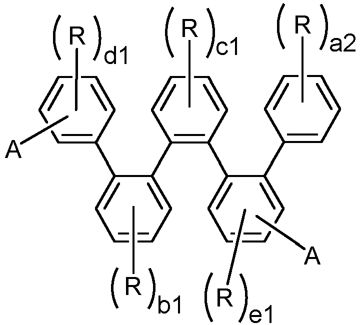 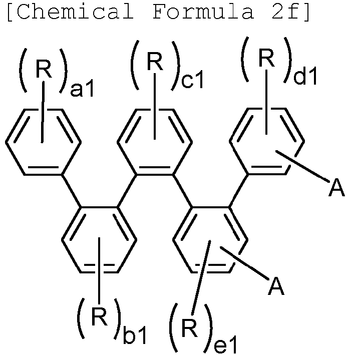 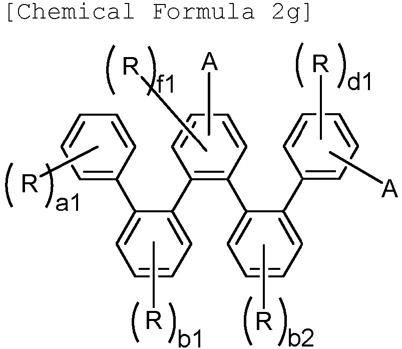 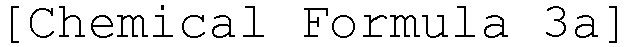 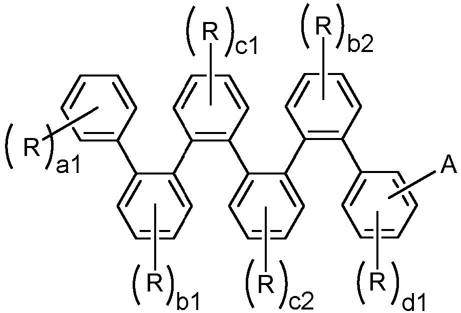 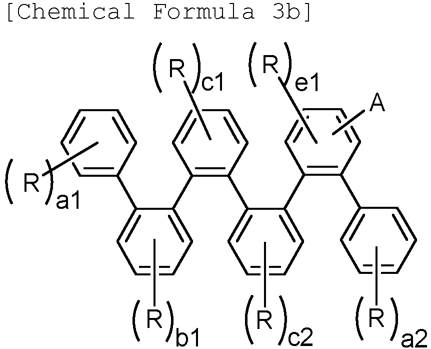 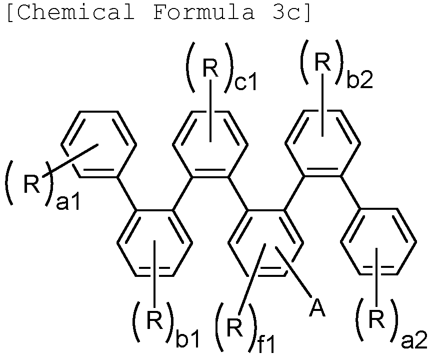 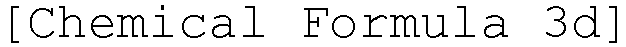 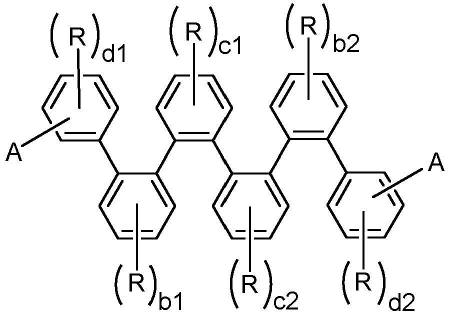 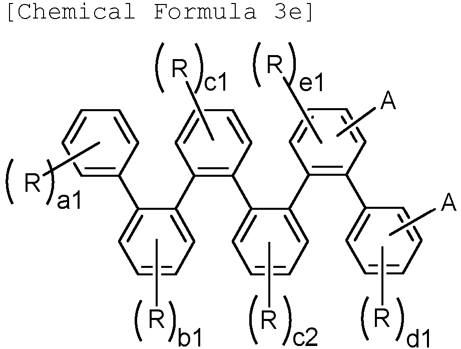 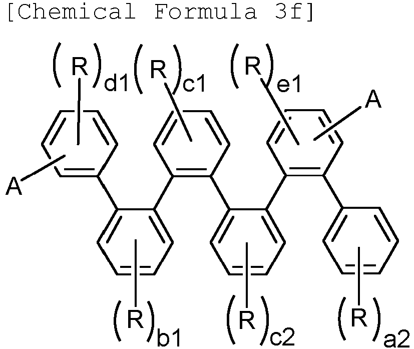 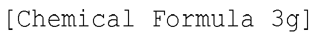 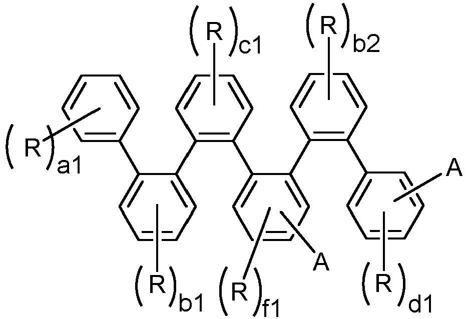 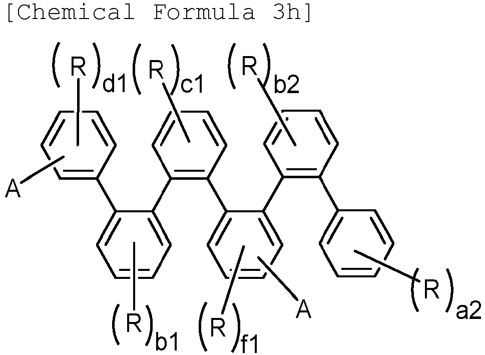 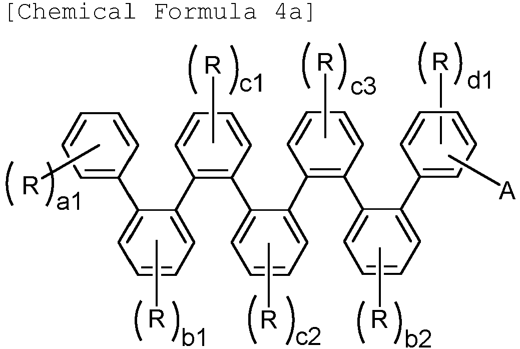 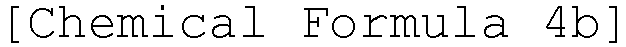 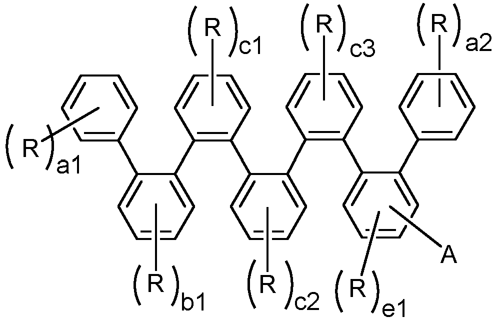 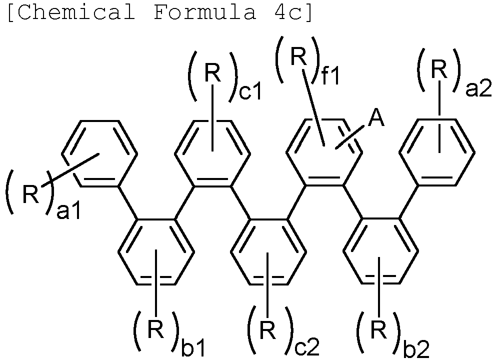 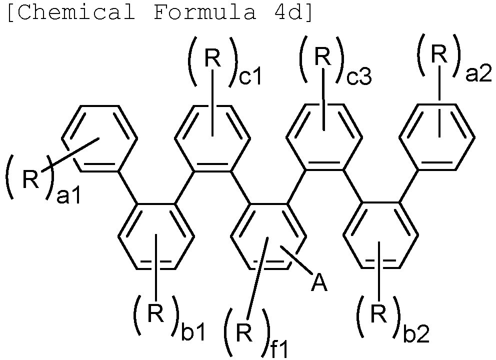 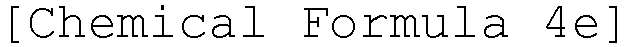 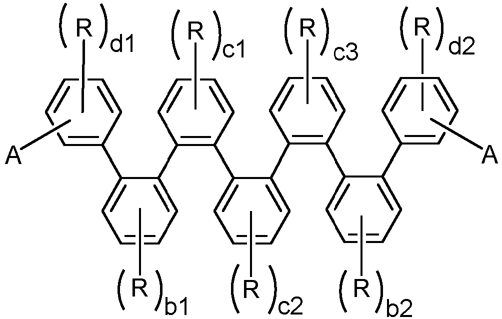 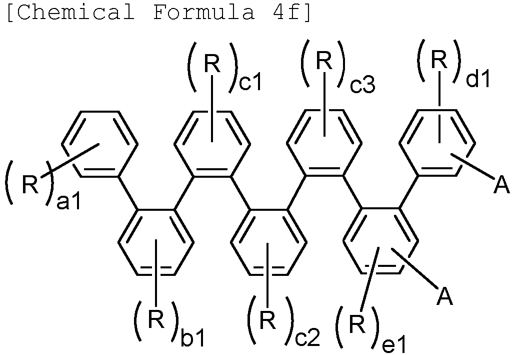 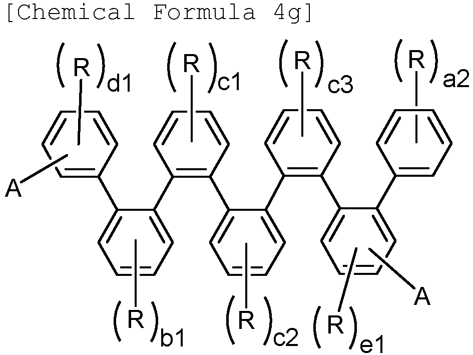 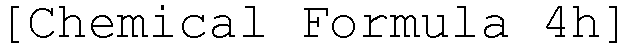 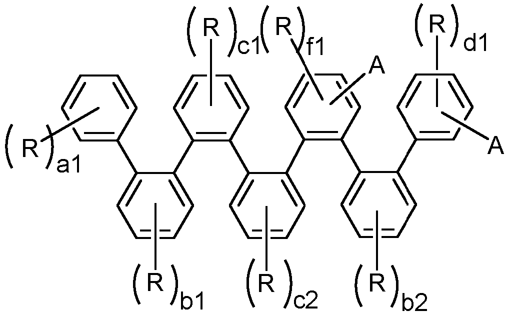 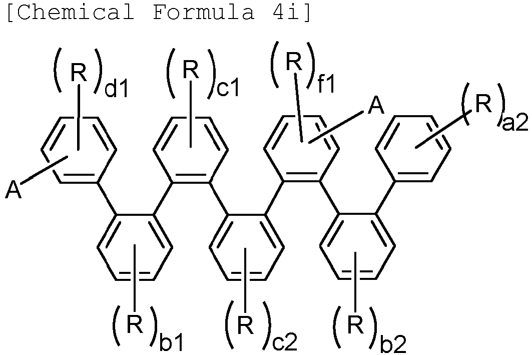 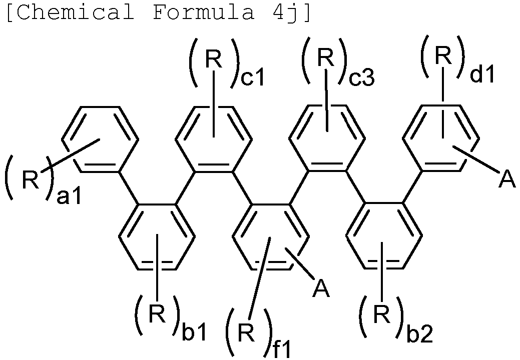 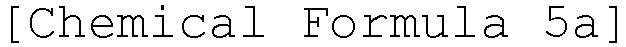 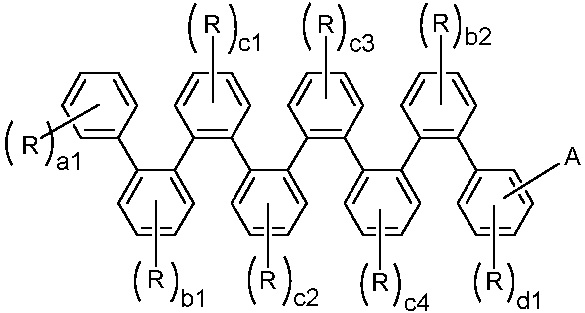 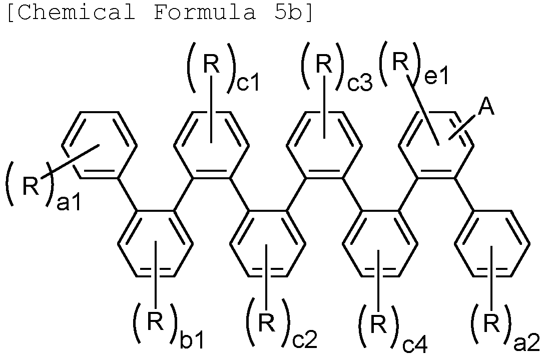 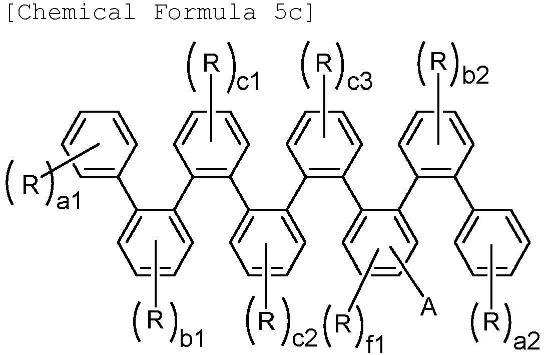 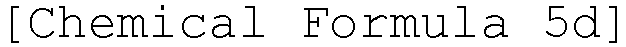 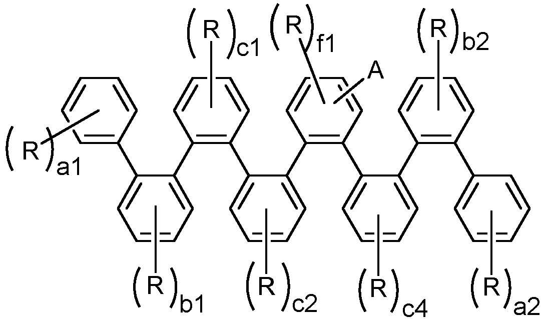 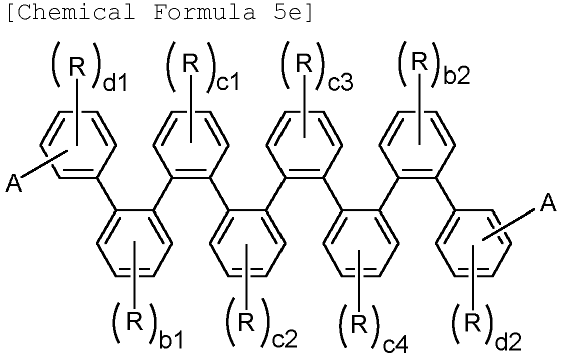 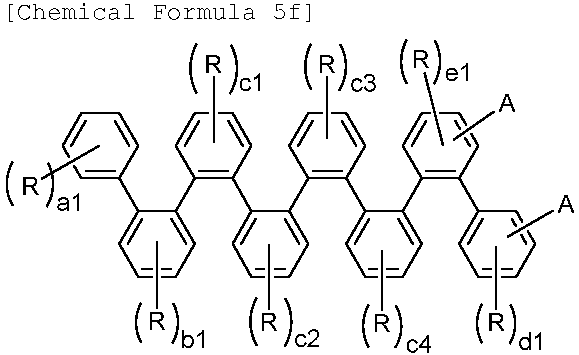 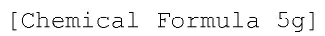 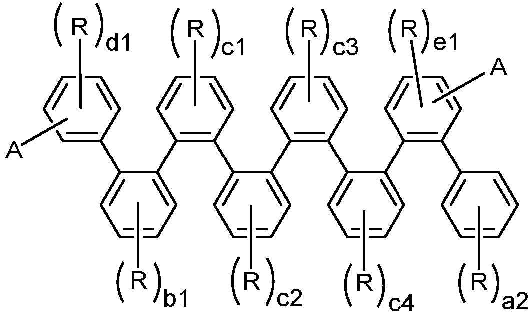 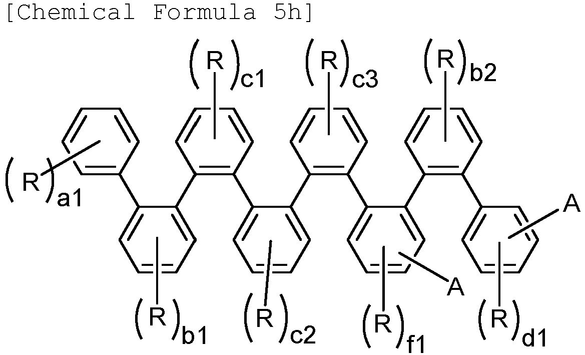 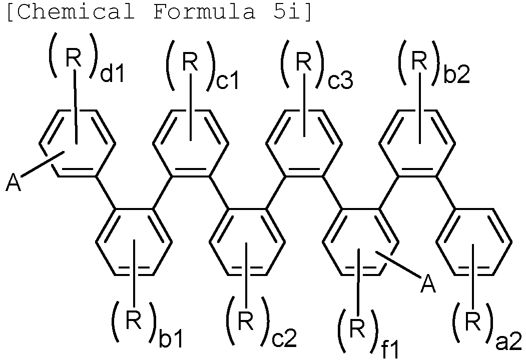 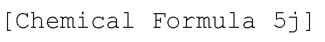 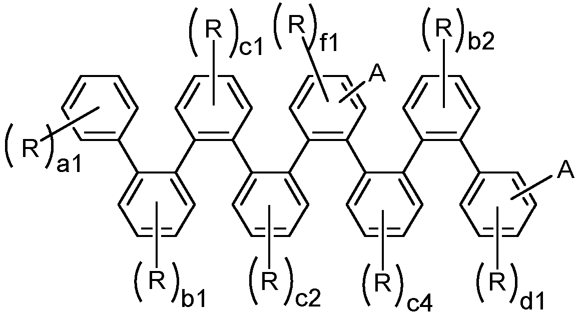 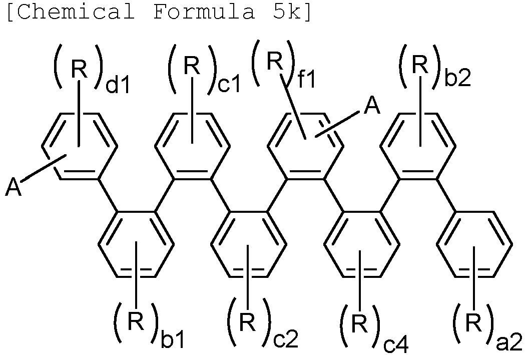 wherein,
A represents the moiety A, which is as defined in Claim 1, with plural A's being the same or different from each other,
a1, a2, b1, b2, c1, c2, c3, c4, and R are each as defined in Claim 1,
d1 and d2 are each independently an integer of 0 to 3,
e1 is an integer of 0 to 2, and
f1 is 0 or 1.

3. The organic compound of claim 1, wherein the plural R's are the same or different from each other and are each independently selected from the group consisting of a deuterium atom (D), a cyano group, a C₁-C₄₀ alkyl group, a C₆-C₆₀ aryl group, a heteroaryl group having 5 to 60 nuclear atoms, a C₁-C₄₀ alkylphosphine oxide group, and a C₆-C₆₀ arylphosphine oxide or can form a fused ring with an adjacent group.

4. The organic compound of claim 1, wherein the moiety A represented by Chemical Formula A1 is selected from the group consisting of the following moieties S1-1 to S1-10: 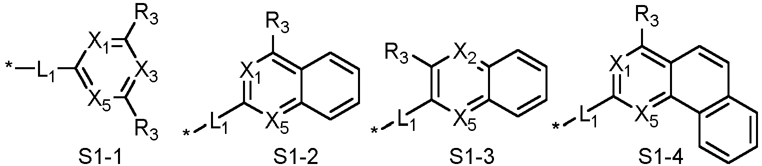 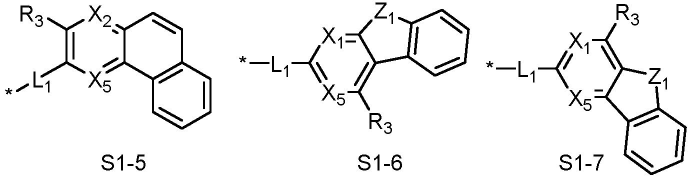 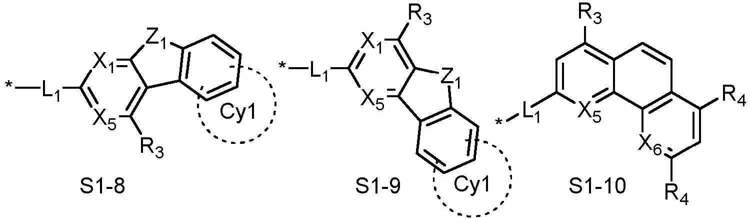 (in moiety S1-1,
X₁, X₃, and X₅, which are the same or different from each other, are each independently N or C(R₃), with a proviso that at least one of X₁, X₃, and X₅ is N,
in moieties S1-2, S1-4, and S1-6 to S1-9,
X₁ and X₅, which are the same or different from each other, are each independently N or C(R₃), with a proviso that at least one of X₁ and X₅ is N,
in moieties S1-3 and S1-5,
X₂ and X₅, which are the same or different from each other, are each independently N or C(R₃), with a proviso that at least one of X₂ and X₅ is N,
Z₁ is O or S,
Cy1 is a C₆-C₃₀ fused aromatic ring,
in moiety S1-10, X₅ and X₆, which are the same or different from each other, are each independently N or C(R₃), with a proviso that at least one of X₅ and X₆ is N,
L₁ and R₃ are each as defined in Claim 1,
the plural R₄'s are the same or different from each other,
R₄ is selected from the group consisting of a hydrogen atom, a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group or can form a fused ring with an adjacent group).

5. The organic compound of Claim 1, wherein L₁ and L₂ are each independently a single bond or selected from the group consisting of the following linkers L-1 to L-19: 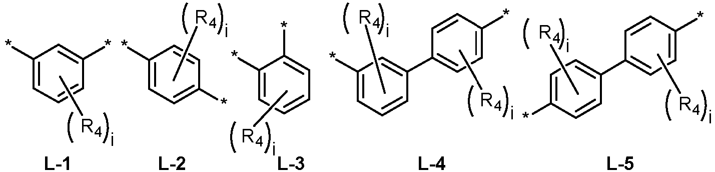 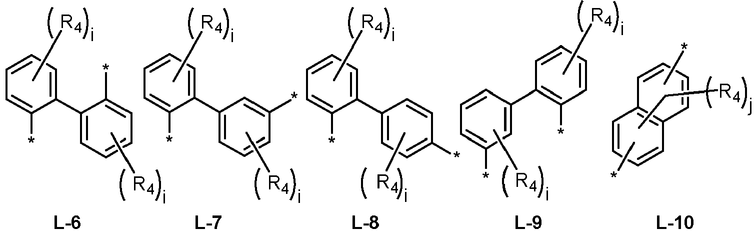 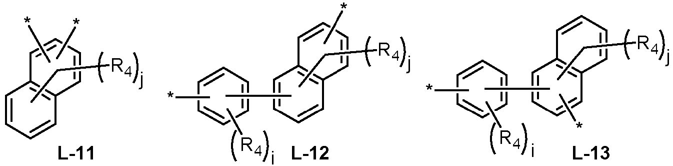 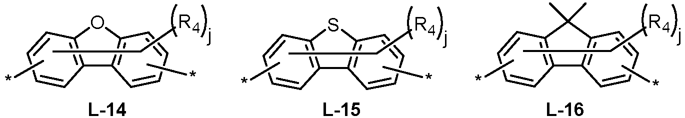 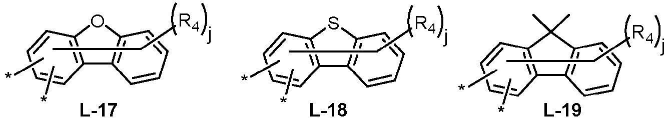 (wherein,
i is an integer of 0 to 4,
j is an integer of 0 to 6,
plural R₄'s are the same or different from each other,
R₄ is selected from the group consisting of a deuterium atom (D), a halogen group, a cyano group, a nitro group, an amino group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, a heteroaryl group having 5 to 60 nuclear atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a phosphine oxide group, a C₁-C₄₀ alkylphosphine group, a C₆-C₆₀ arylphosphine group, a C₁-C₄₀ alkylphosphine oxide group, a C₆-C₆₀ arylphosphine oxide group, and a C₆-C₆₀ arylamine group or can form a fused ring with an adjacent group).

6. The organic compound of claim 1, wherein the plural R₃'s, which are the same or different from each other, are each independently selected from the group consisting of the following substituents S1 to S23:

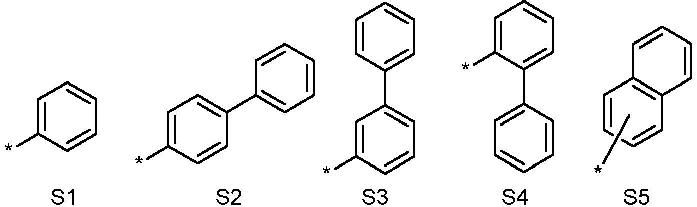

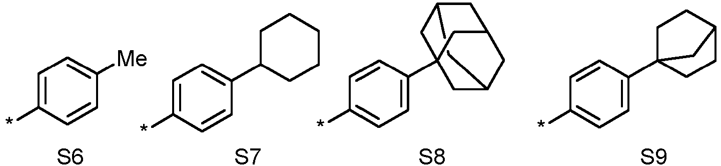

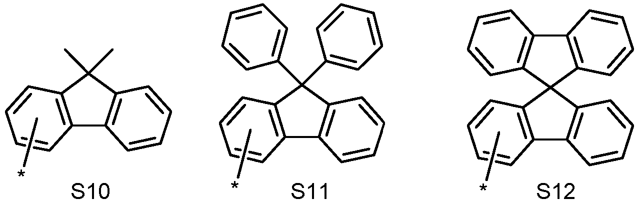

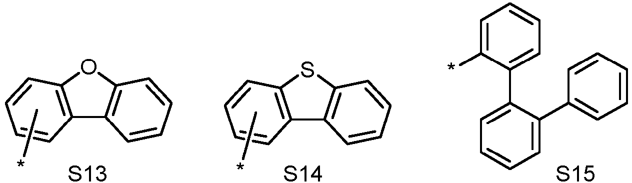

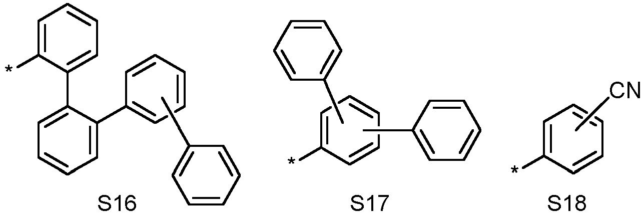

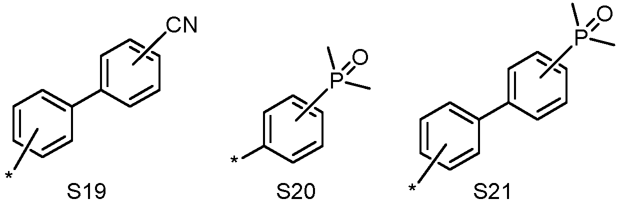

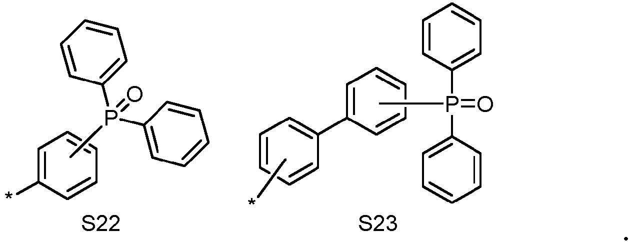

7. The organic compound of claim 1, wherein the organic compound represented by any one of Chemical Formulas 1 to 5 is represented by any one of the following Chemical Formulas 6 to 58: 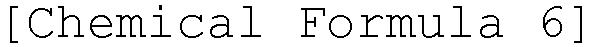 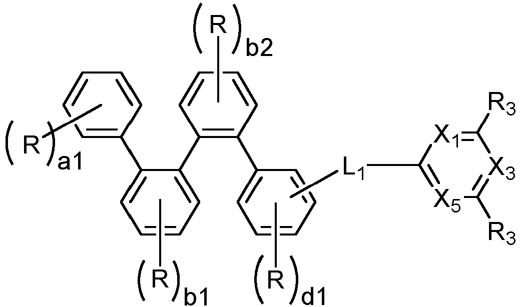 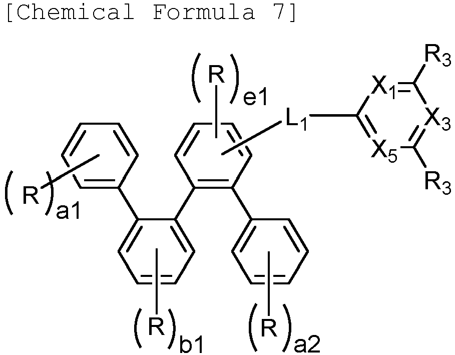 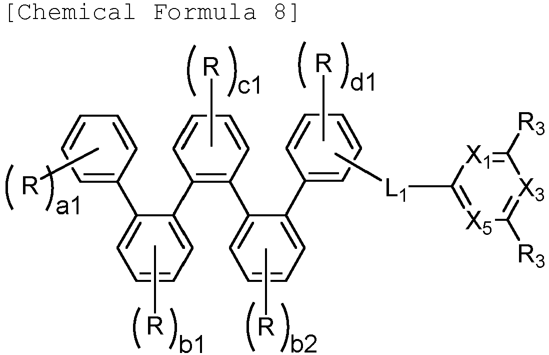 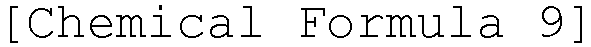 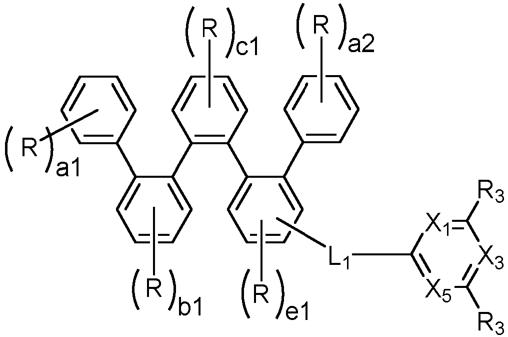 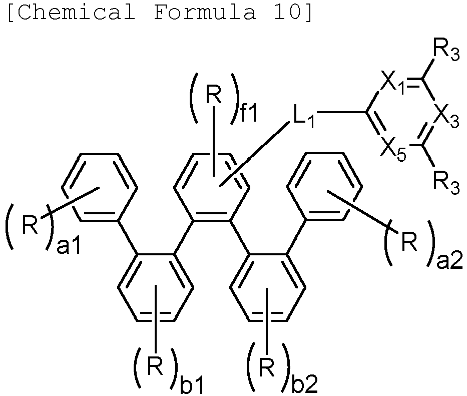 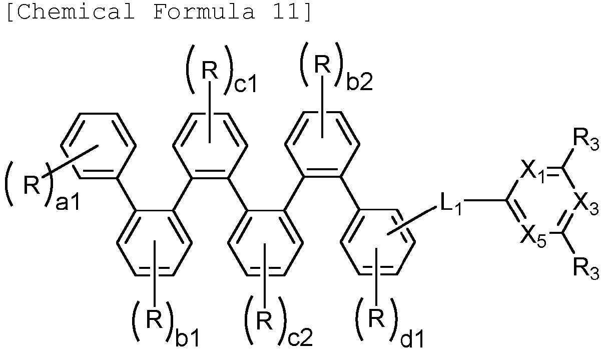 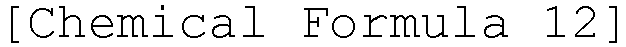 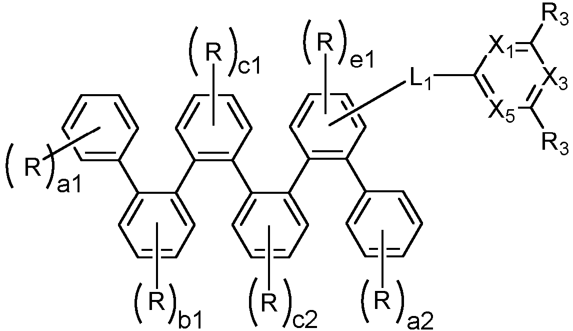 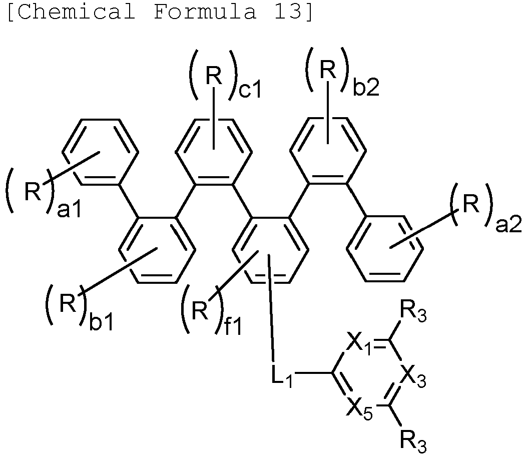 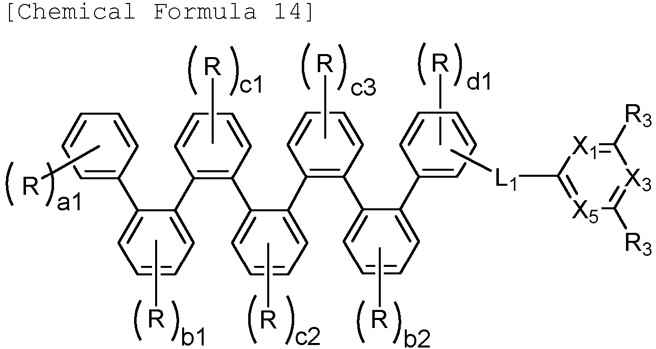 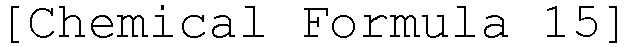 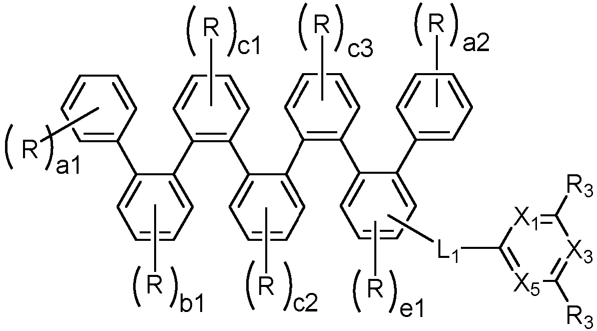 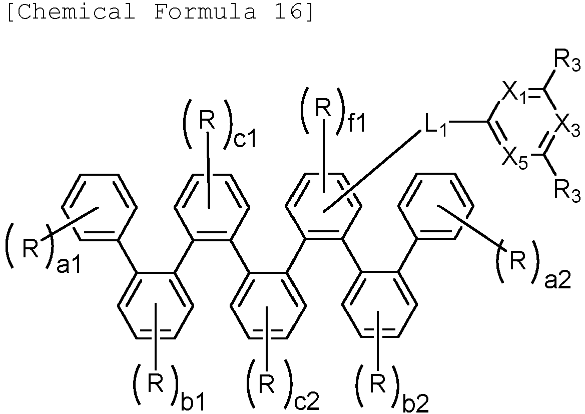 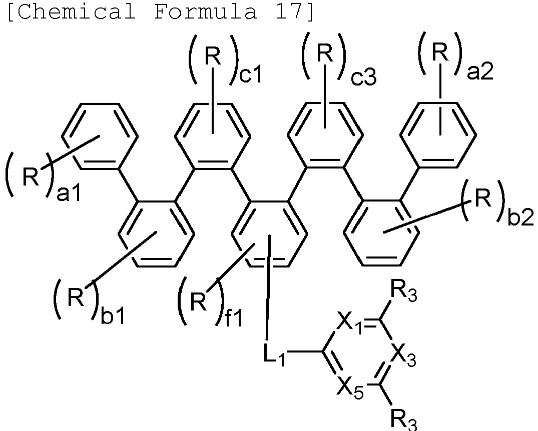 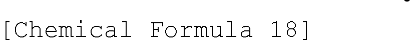 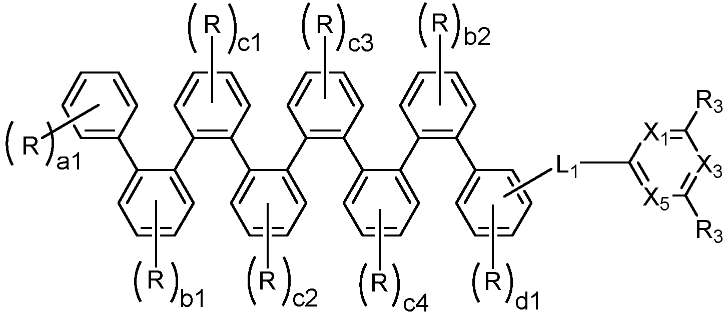 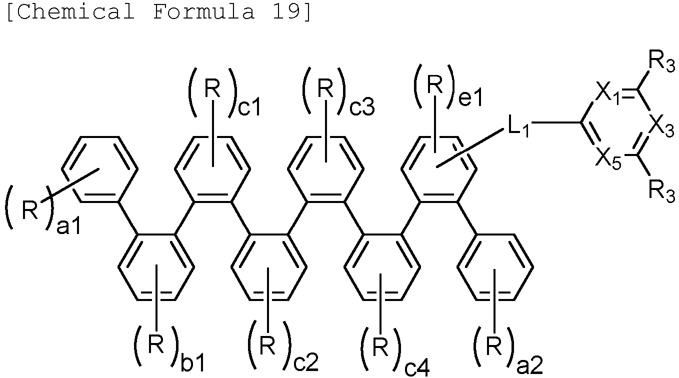 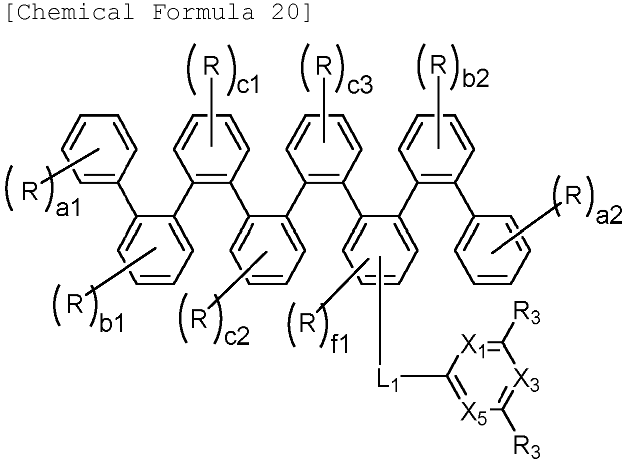 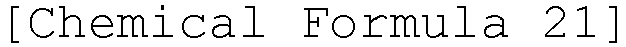 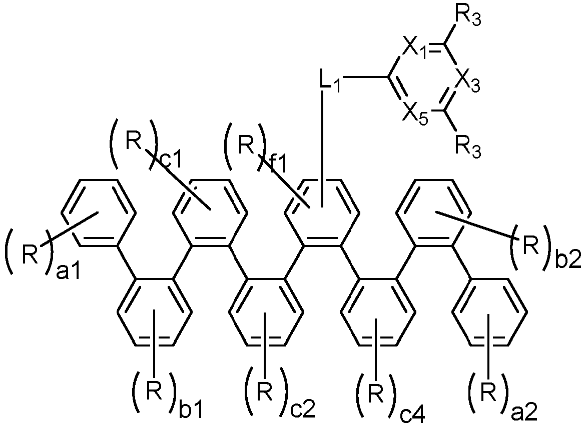 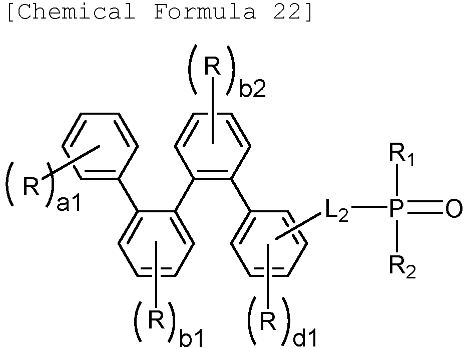 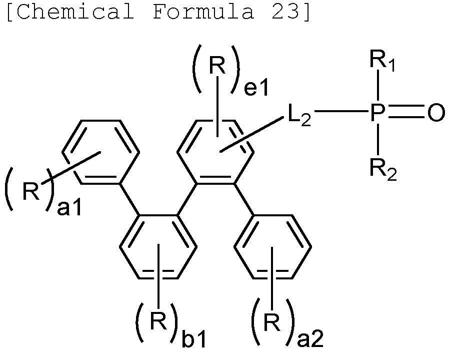 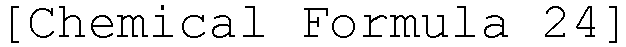 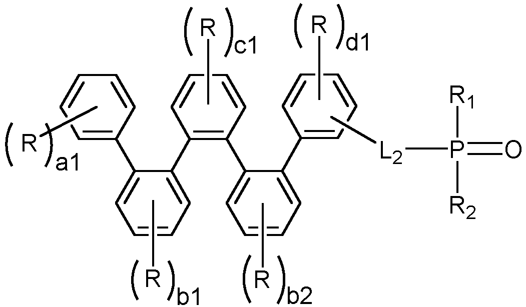 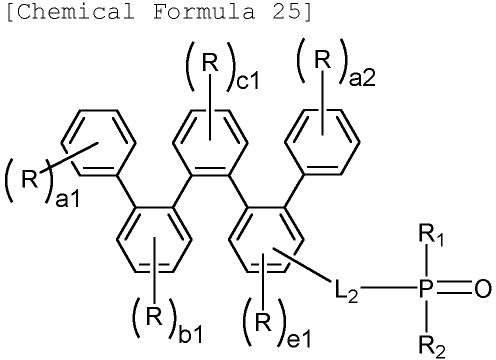 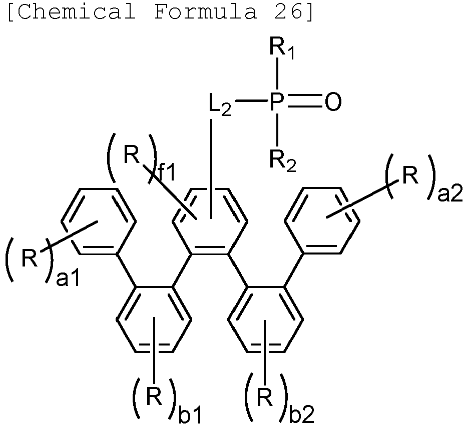 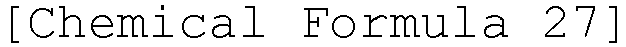 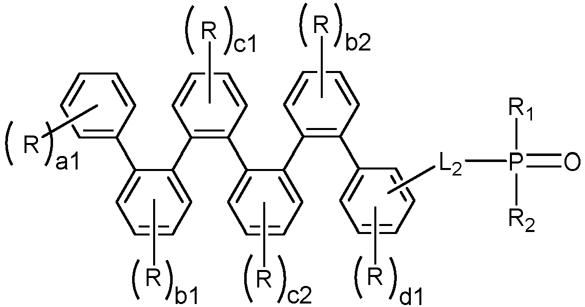 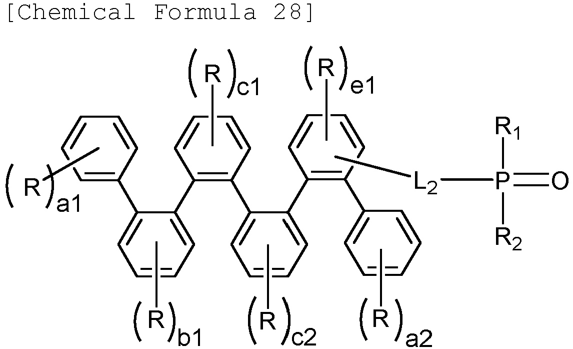 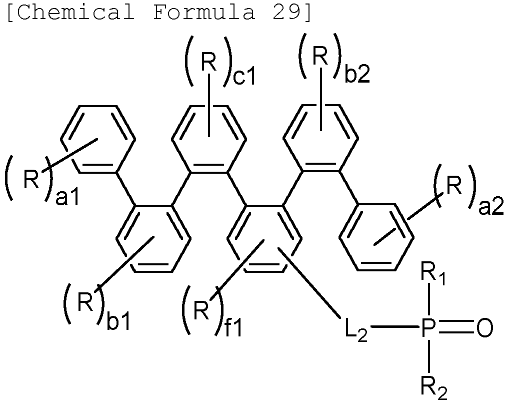 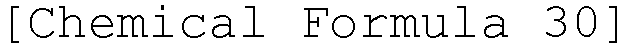 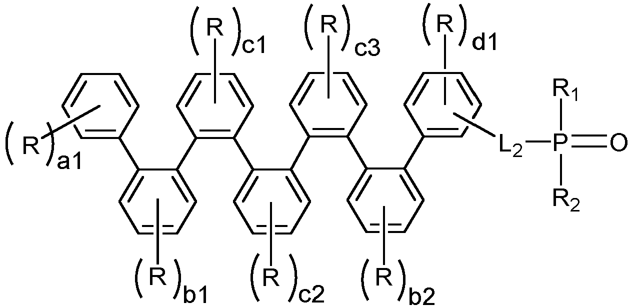 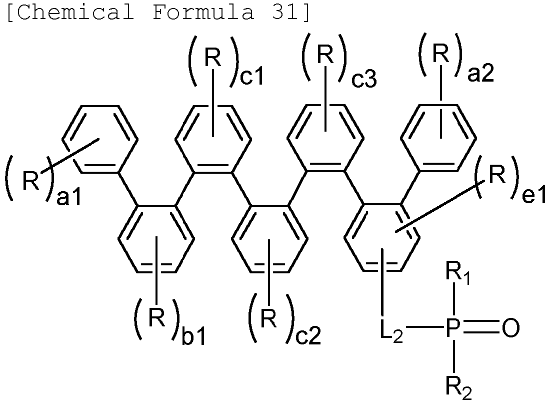 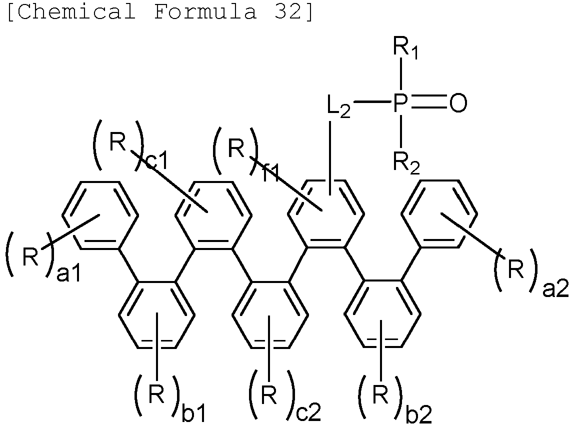 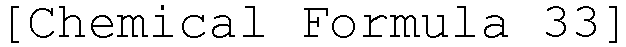 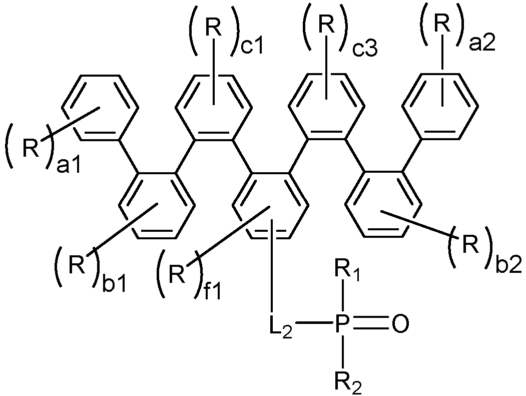 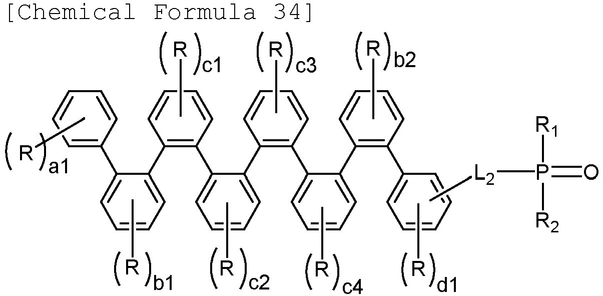 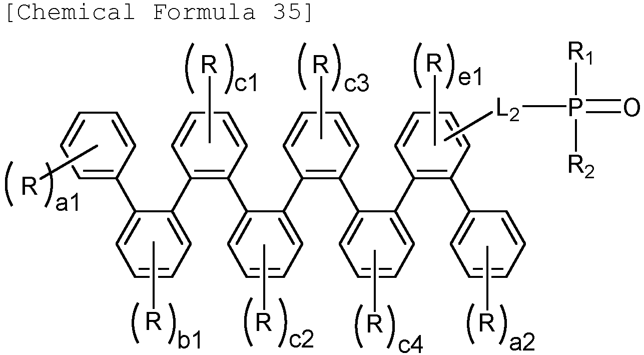 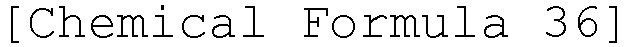 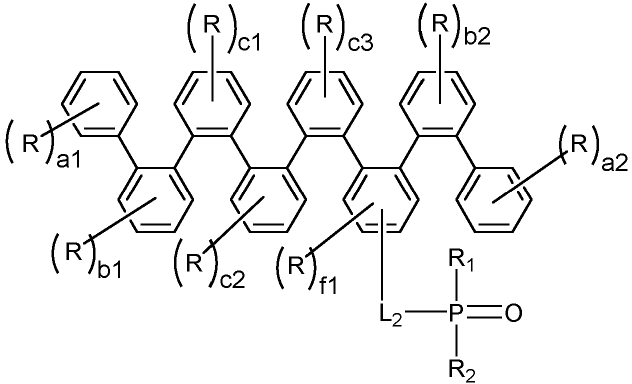 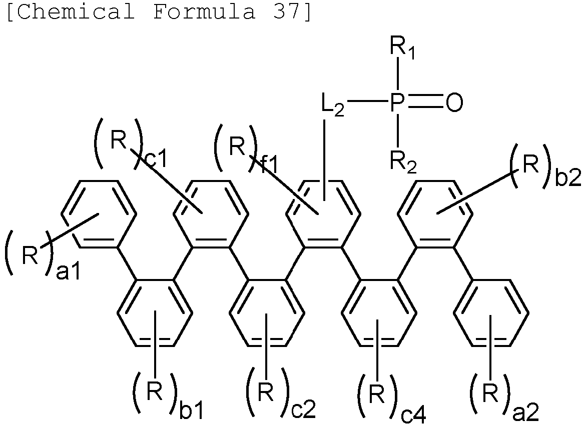 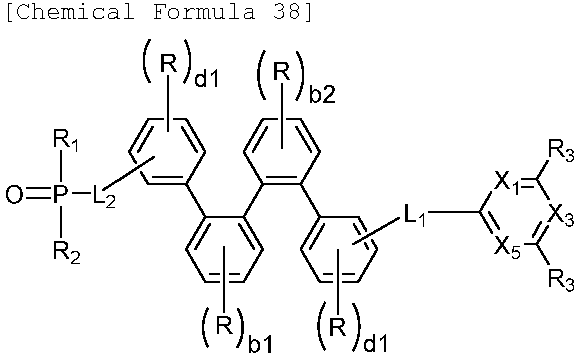 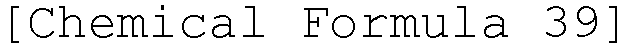 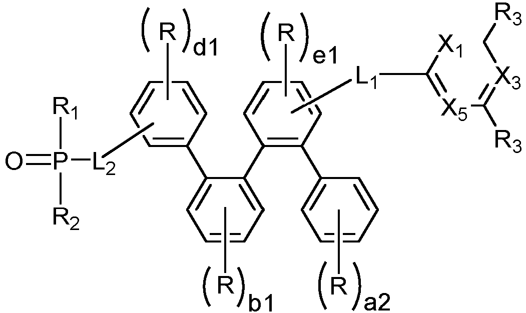 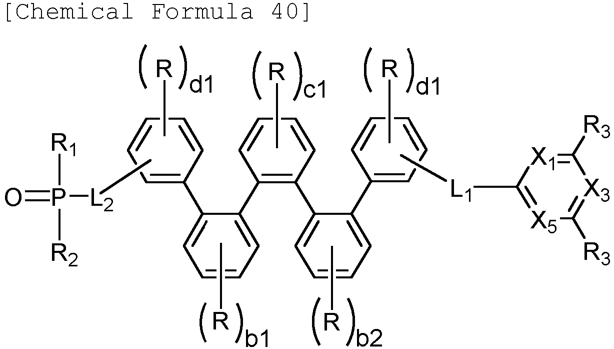 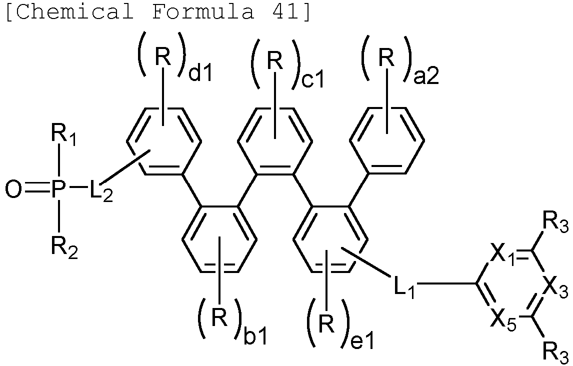 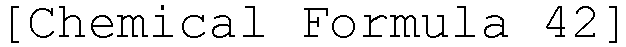 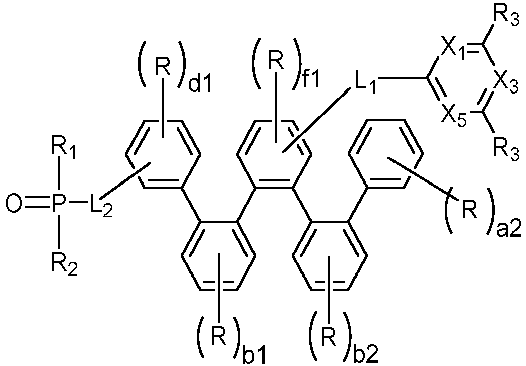 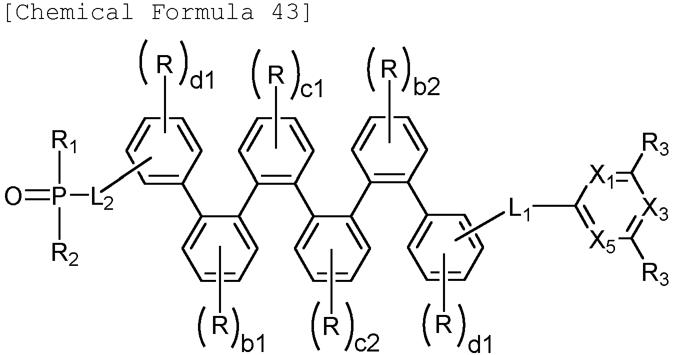 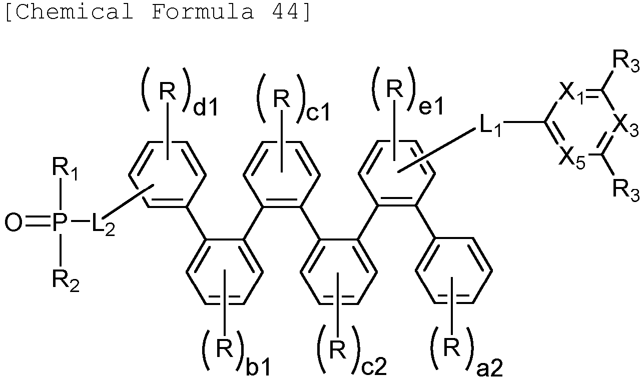 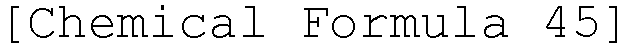 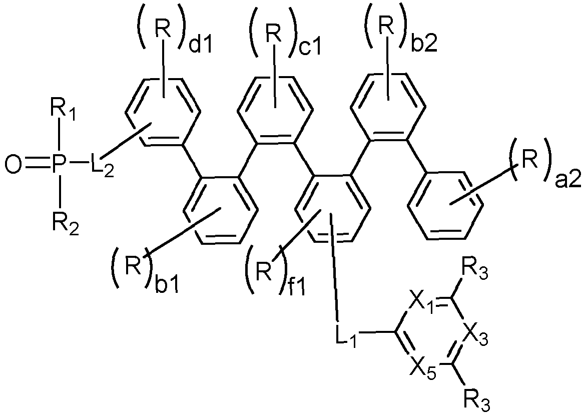 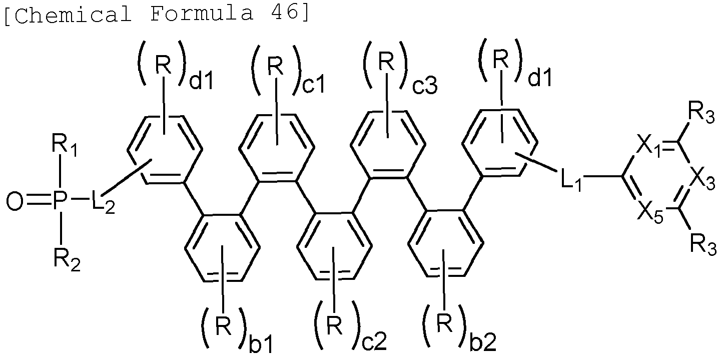 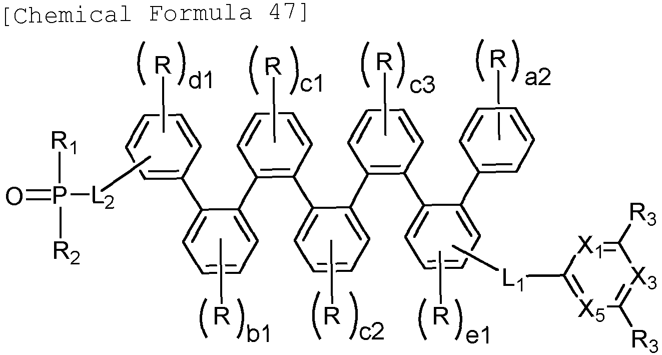 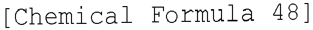 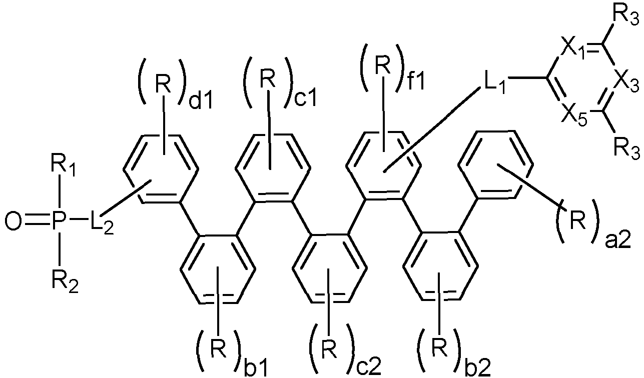 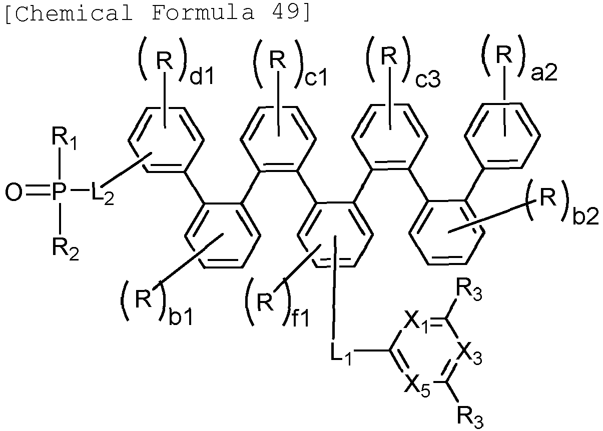 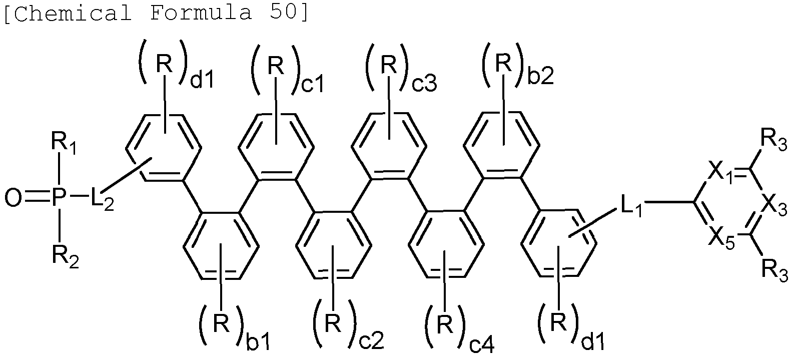 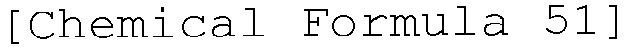 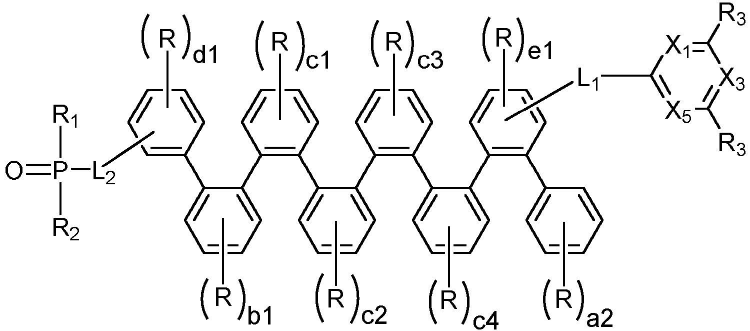 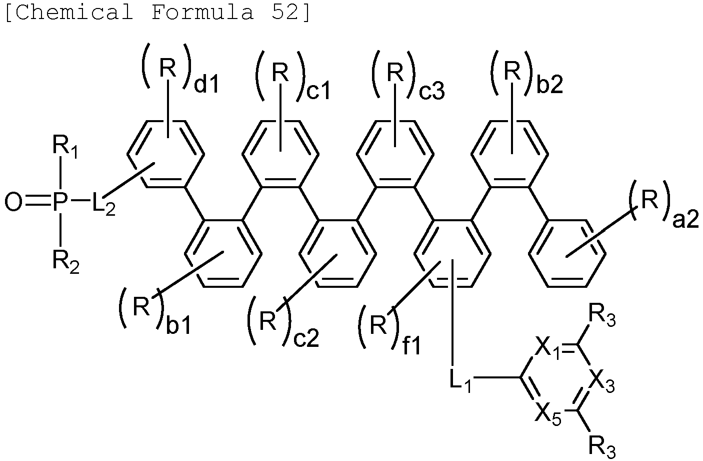 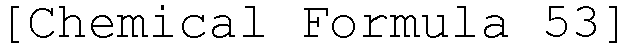 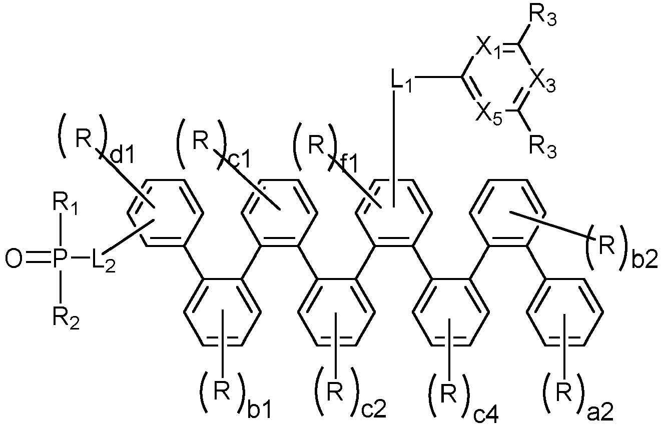 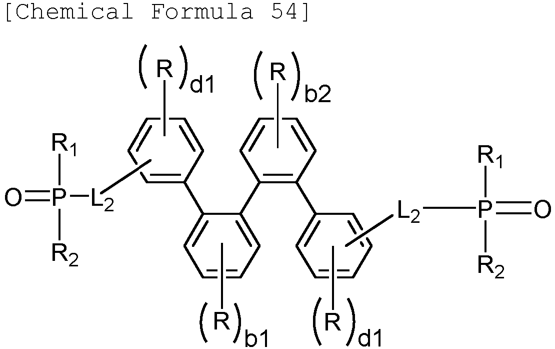 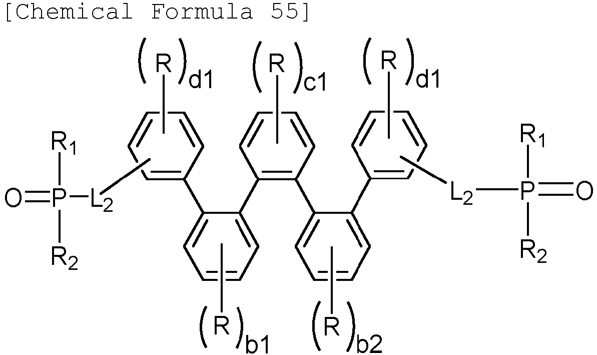 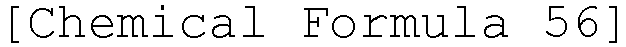 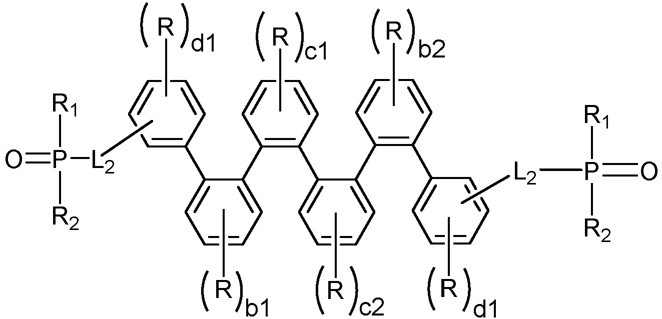 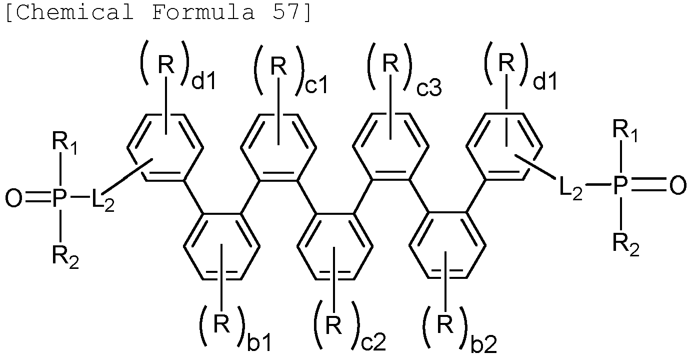 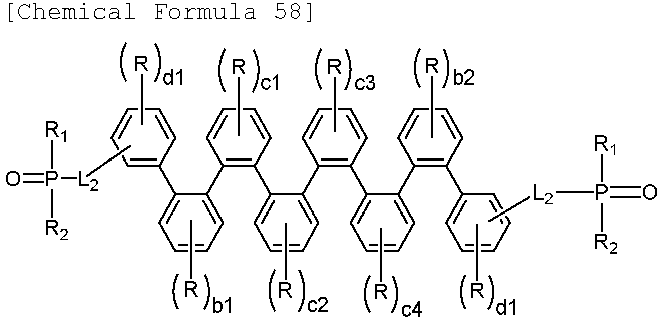 (wherein,
a1, b1, b2, c1, c2, c3, c4, R, R₁, R₂, L₁, and L₂ are each as defined in Claim 1,
d1 is an integer of 0 to 3,
e1 is an integer of 0 to 2,
f1 is 0 or 1,
X₁, X₃, and X₅, which are the same or different from each other, are each independently N or C(R₃), with a proviso that at least one of X₁, X₃, and X₅ is N, and
R₃ is as defined in claim 1).

8. The organic compound of claim 1, wherein the organic compound represented by any one of Chemical Formulas 1 to 5 is represented by any one of the following Chemical Formulas 59 to 126: 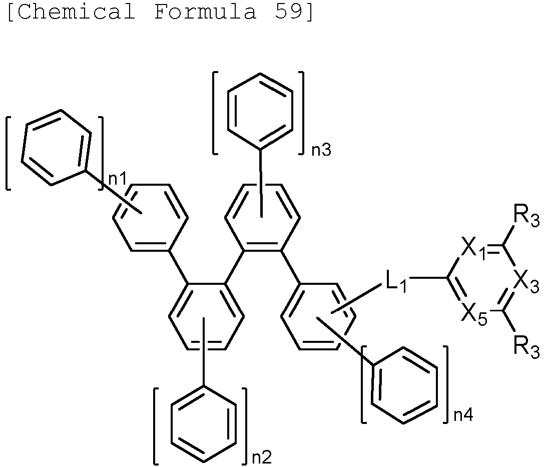 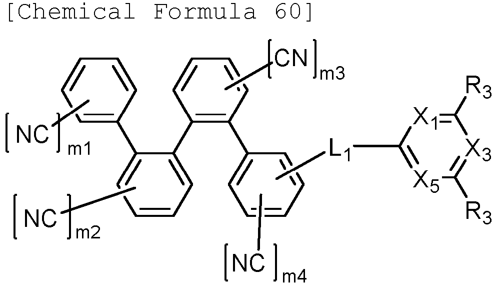 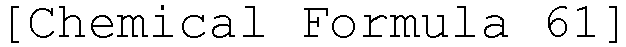 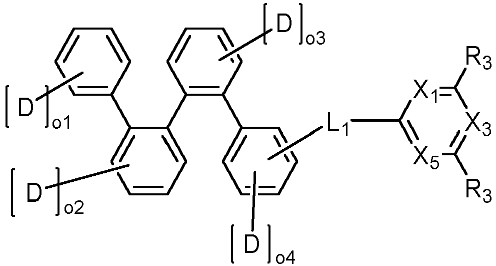 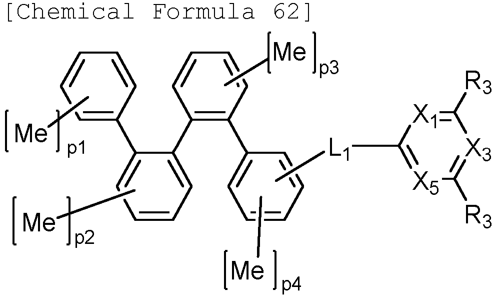 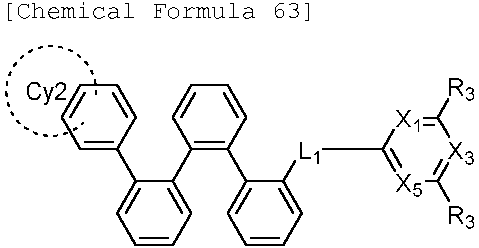 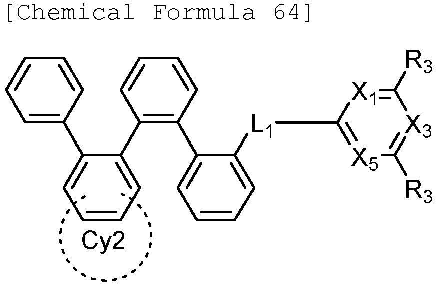 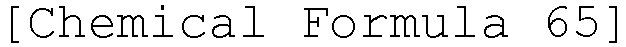 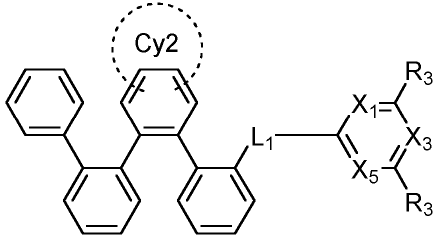 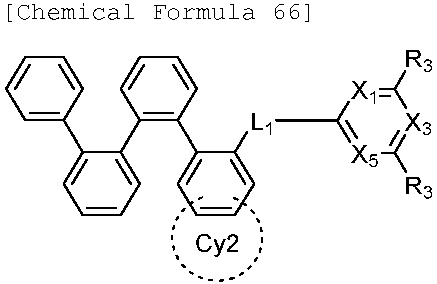 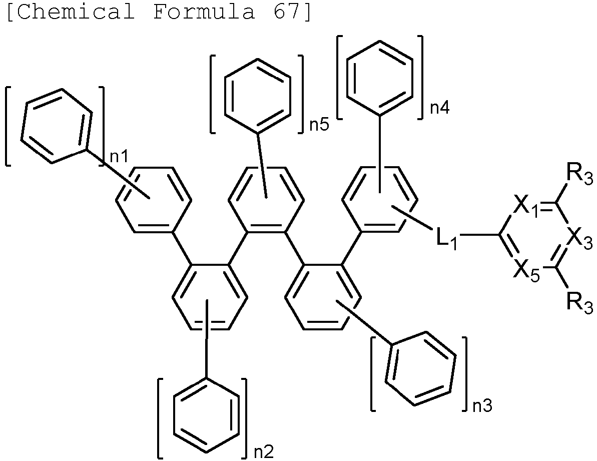 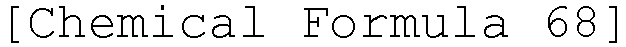 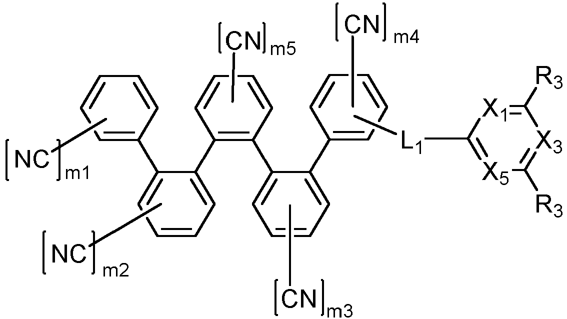 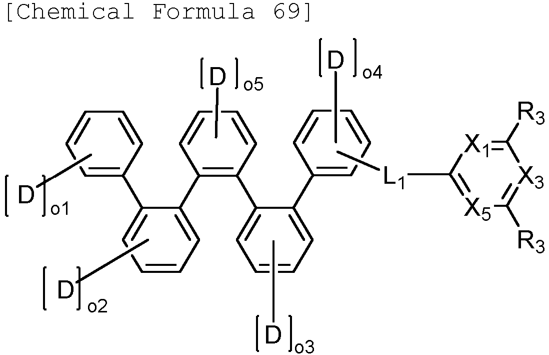 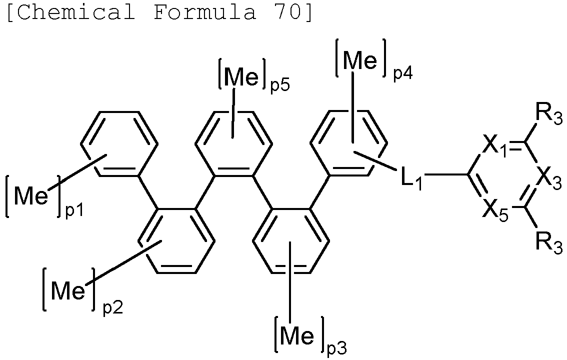 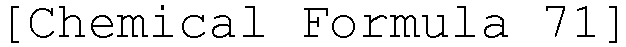 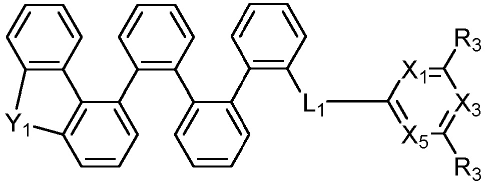 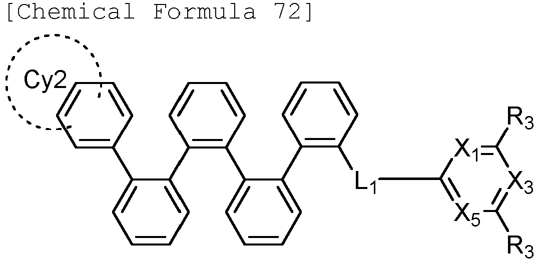 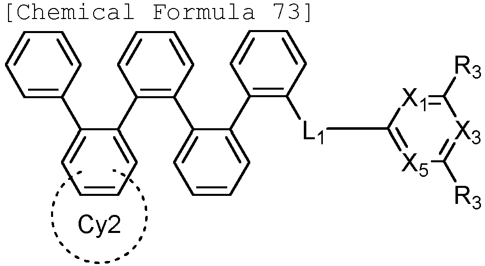 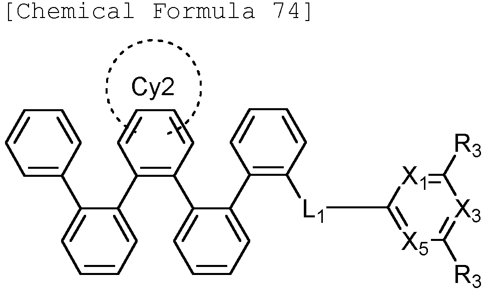 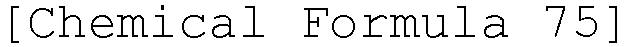 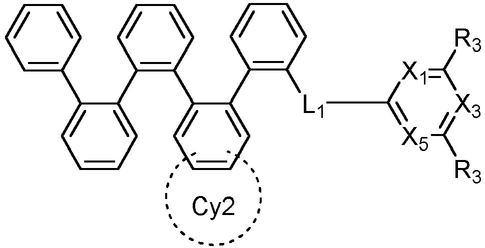 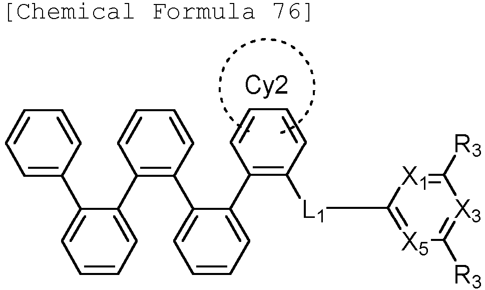 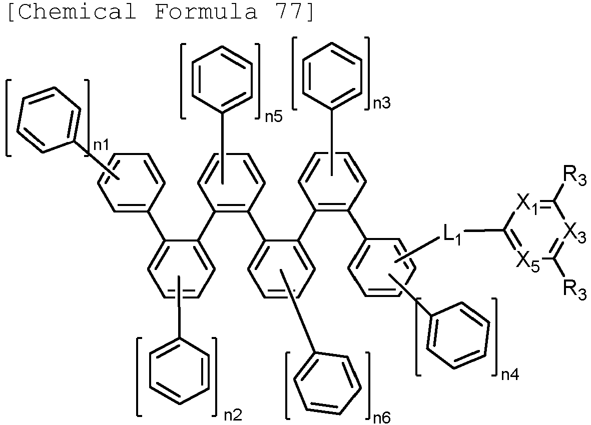 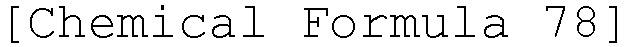 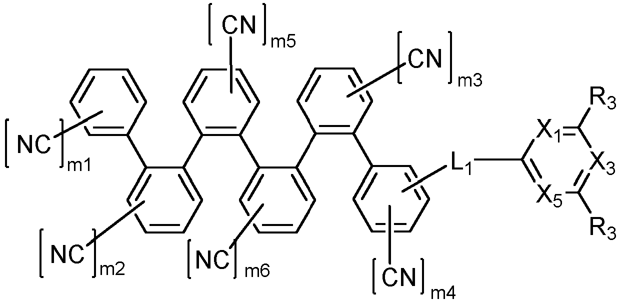 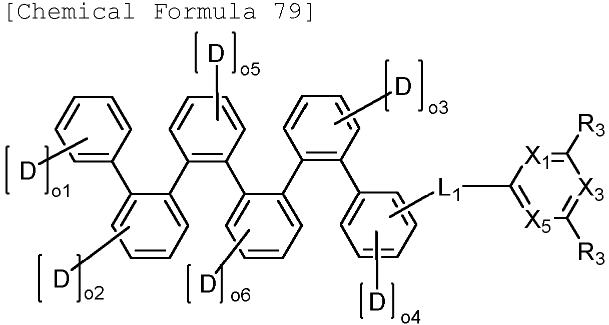 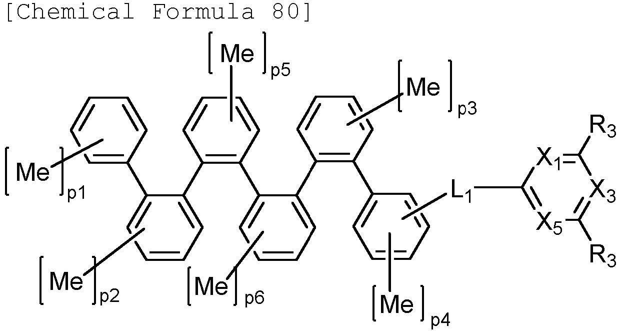 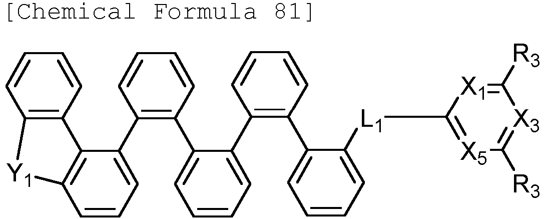 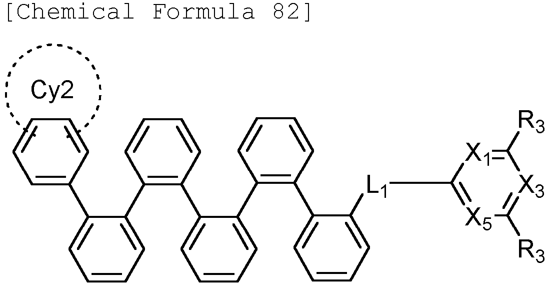 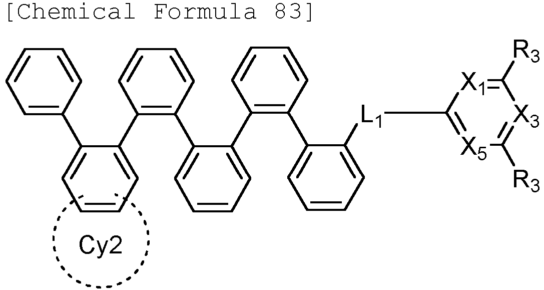 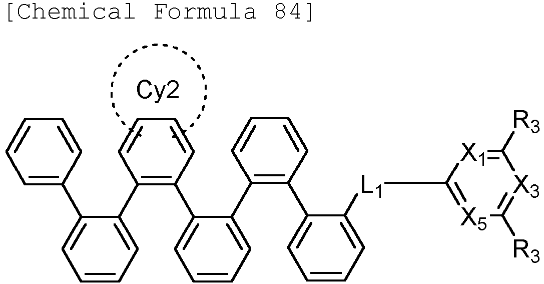 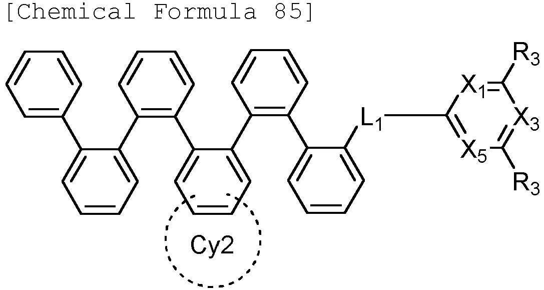 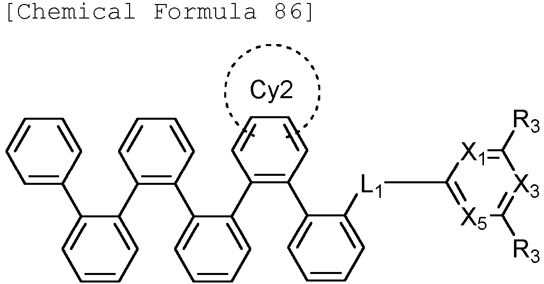 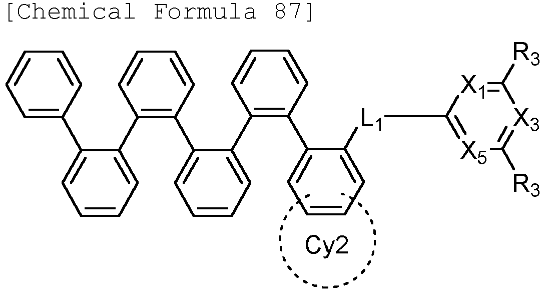 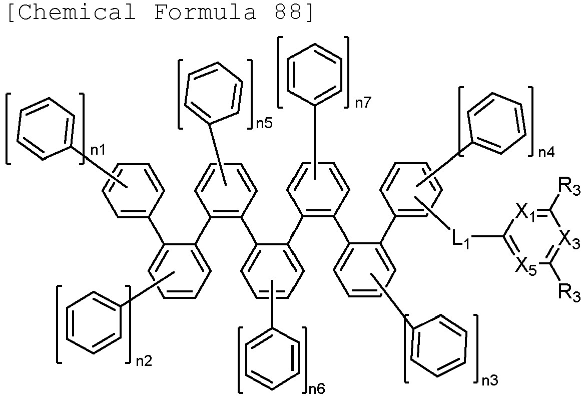 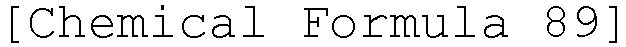 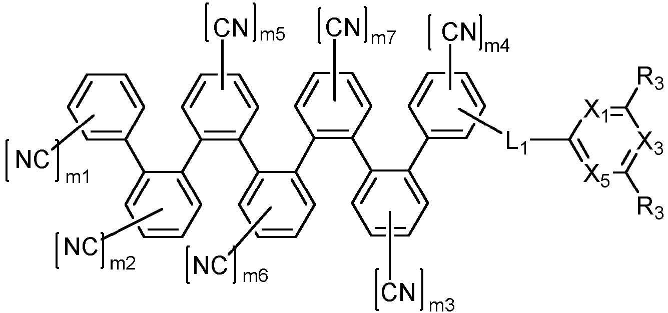 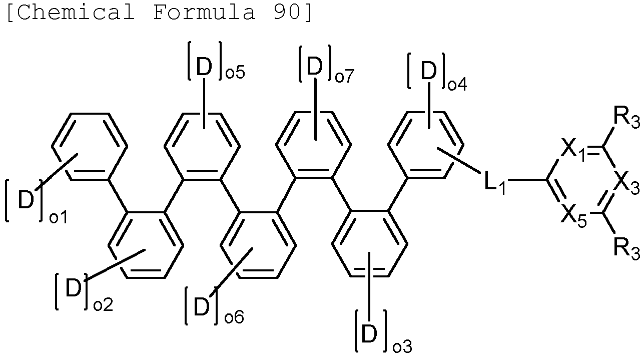 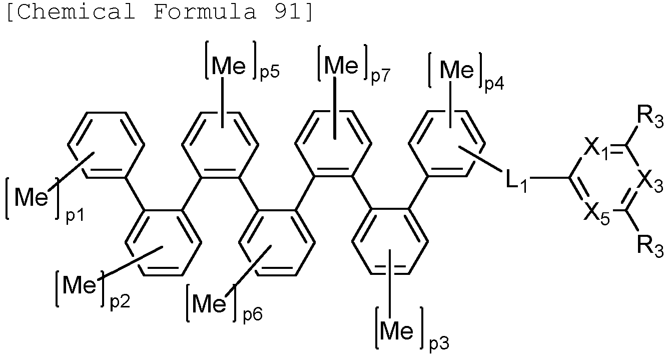 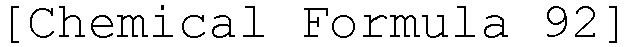 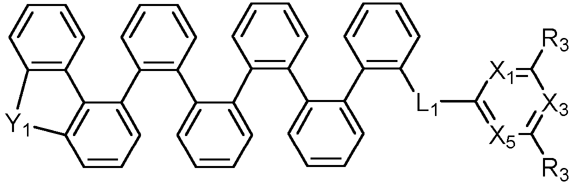 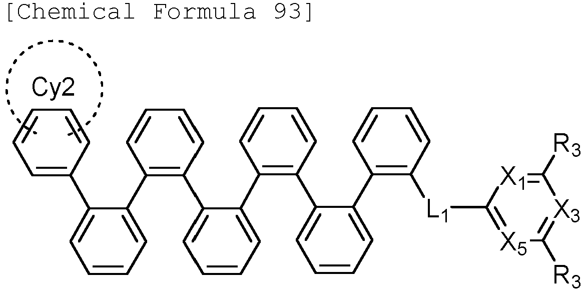 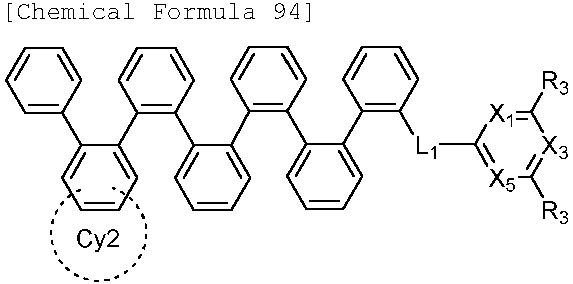 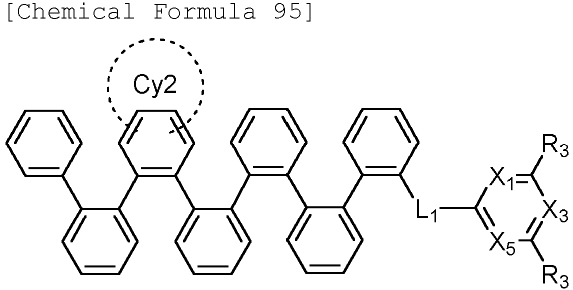 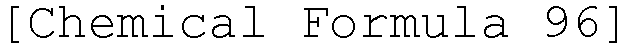 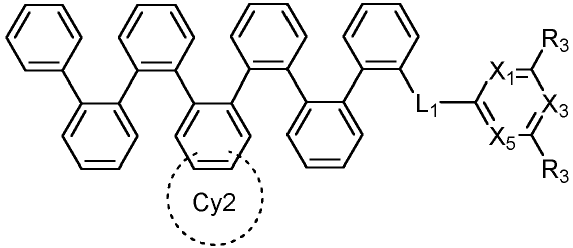 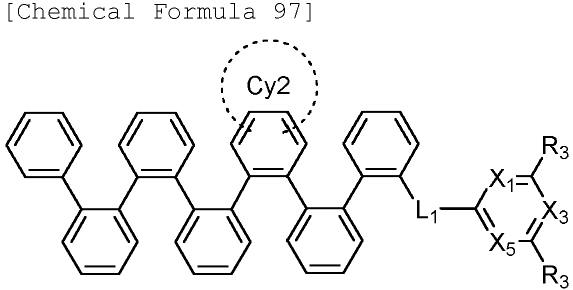 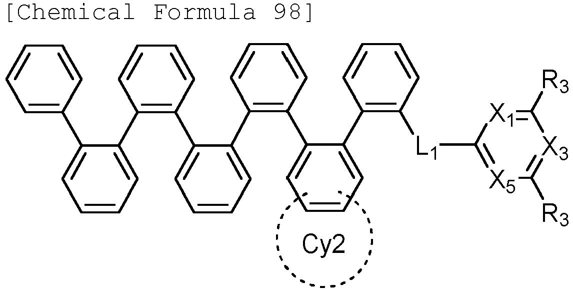 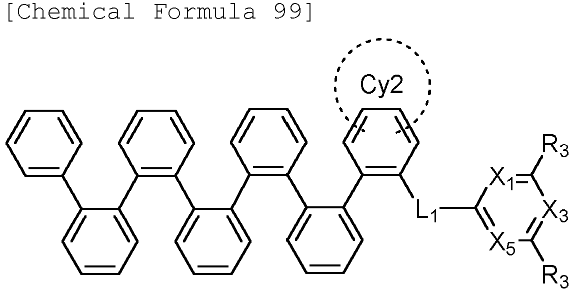 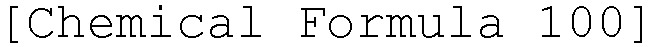 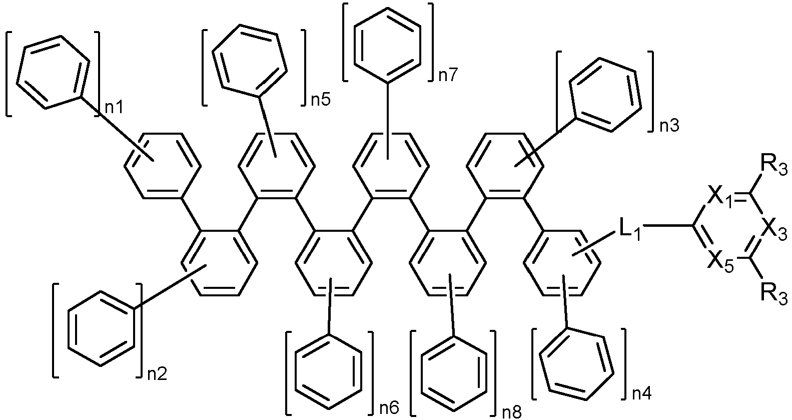 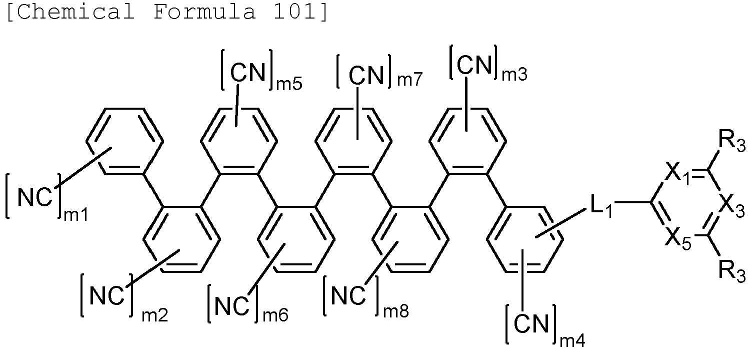 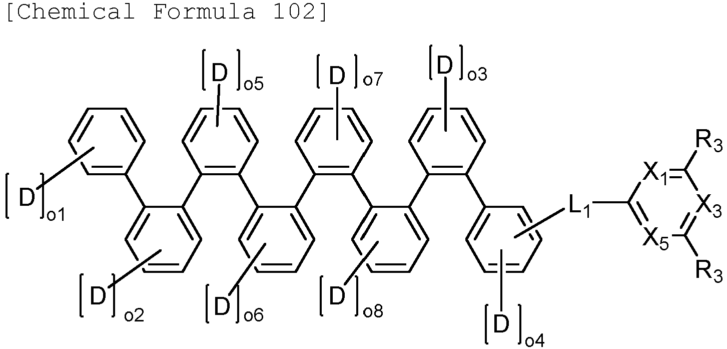 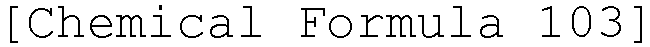 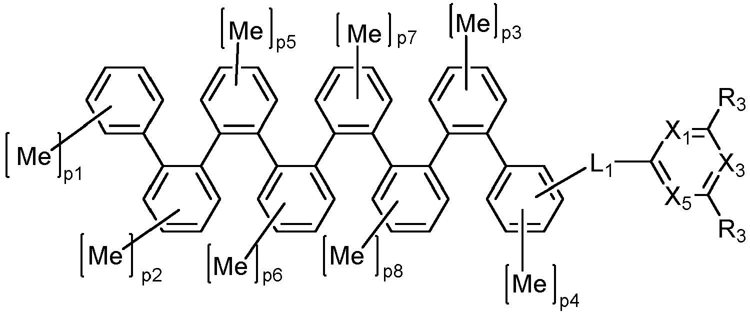 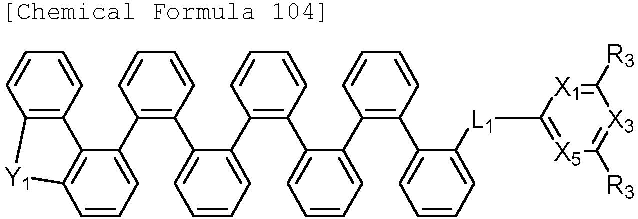 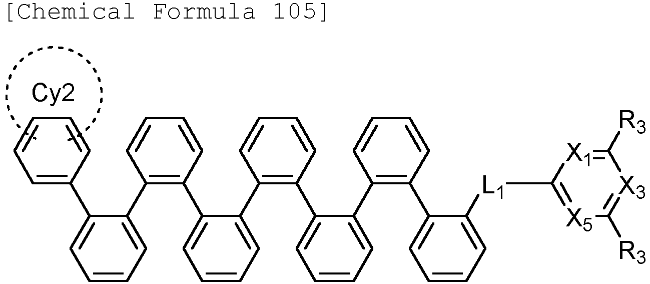 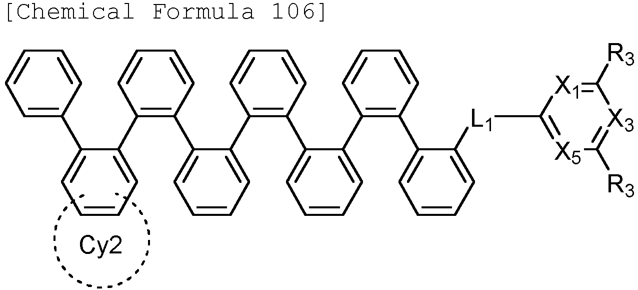 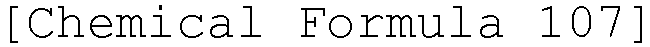 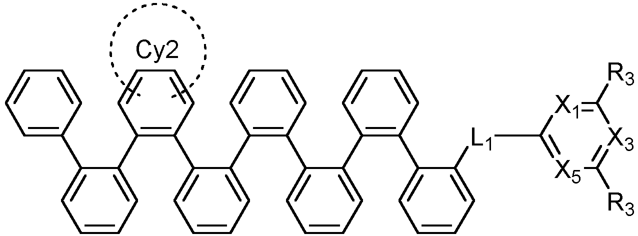 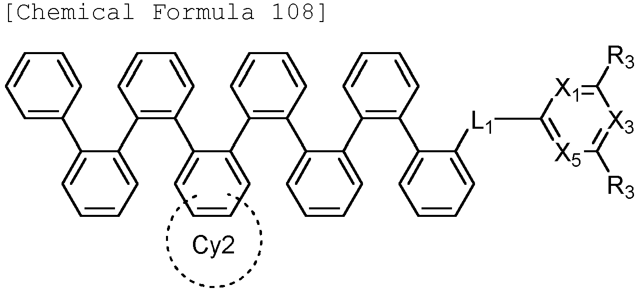 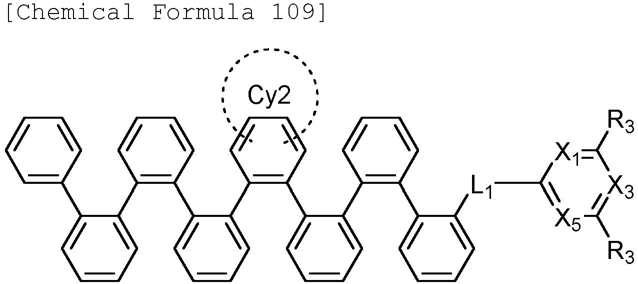 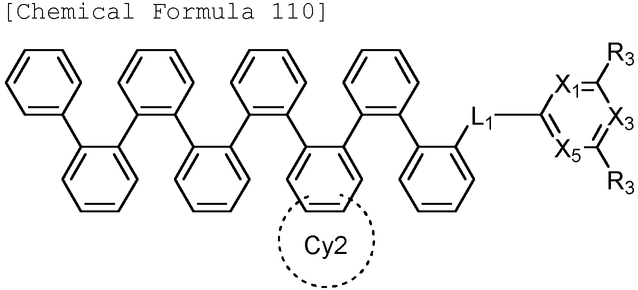 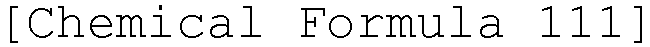 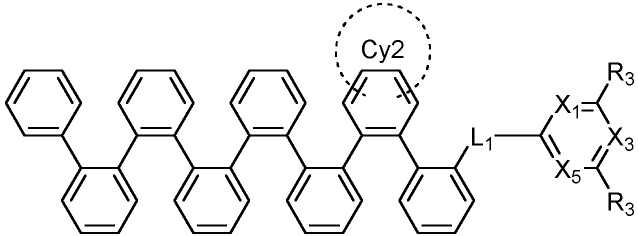 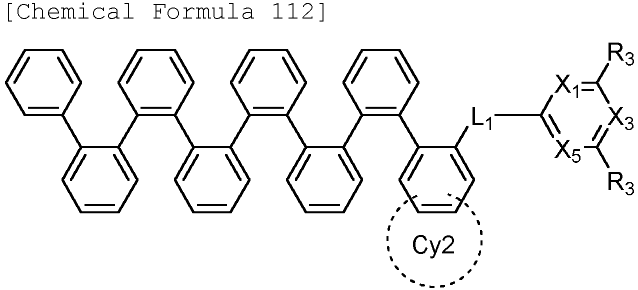 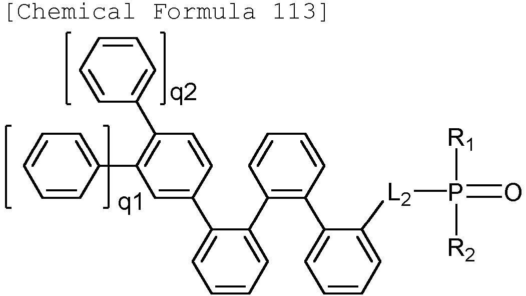 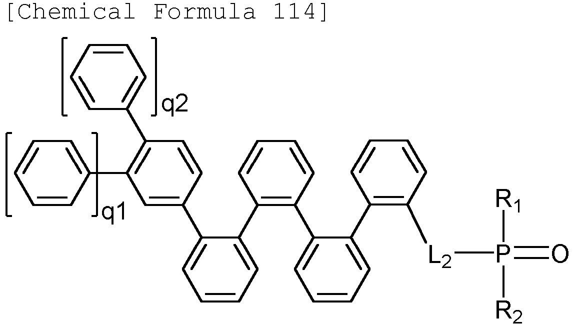 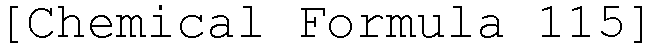 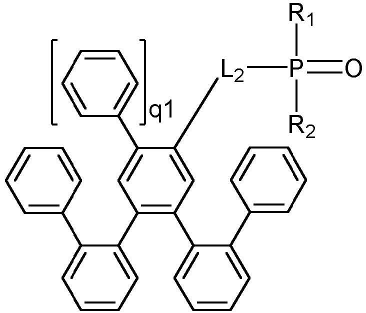 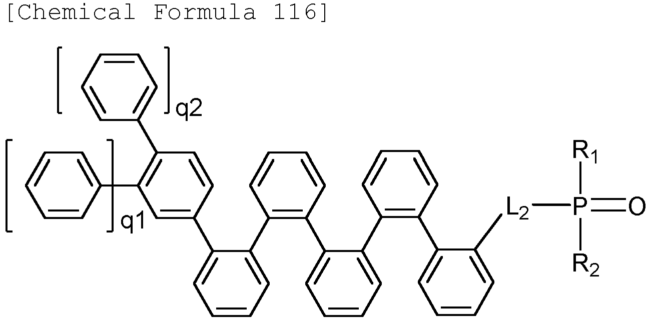 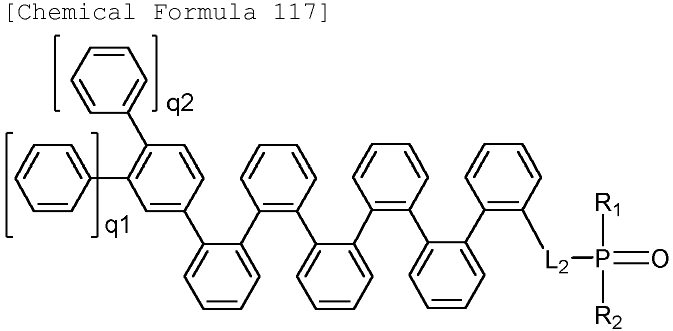 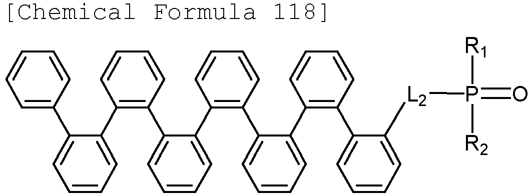 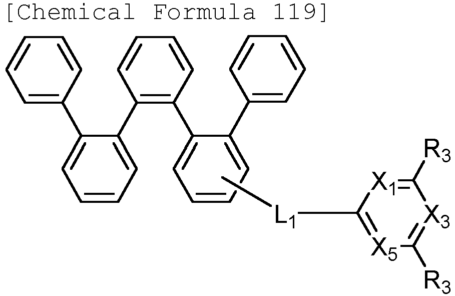 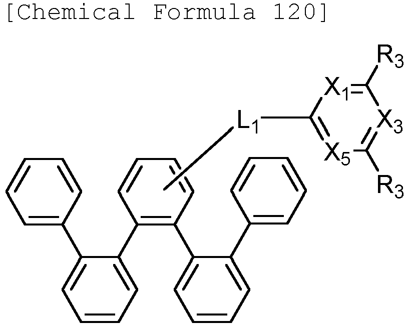 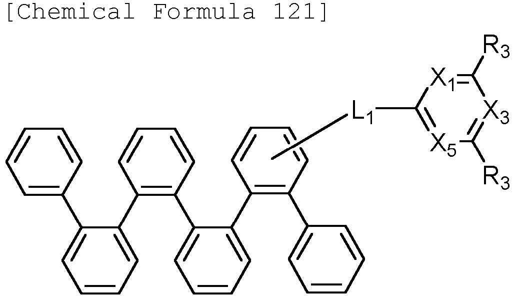 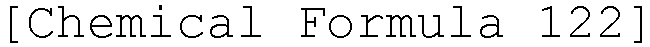 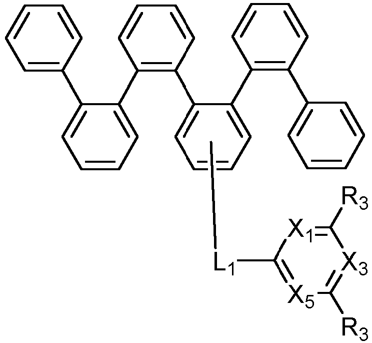 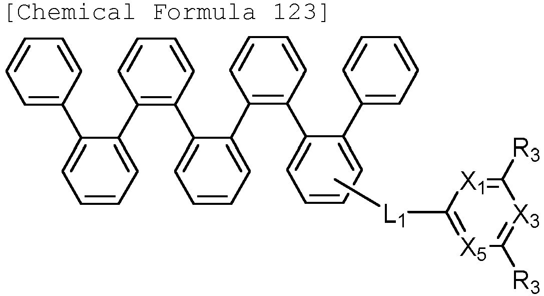 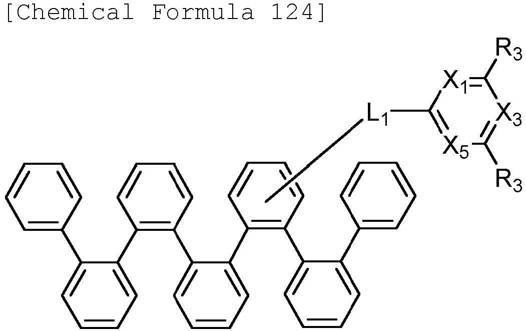 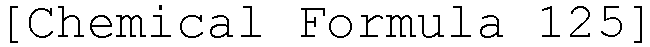 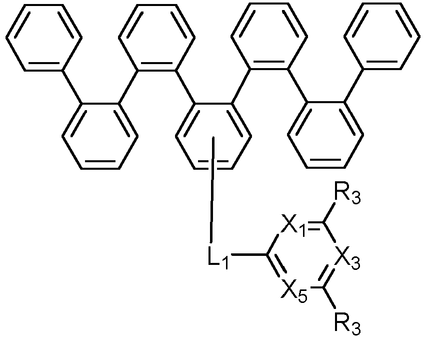 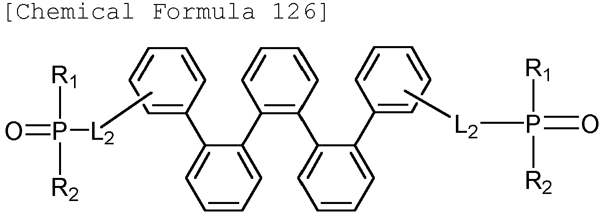 (wherein,
n1, m1, o1, and p1 are each independently an integer of 0 to 4,
n2, n3, n4, m2, m3, m4, o2, o3, o4, p2, p3, and p4 are each independently an integer of 0 to 3,
n5, n6, n7, n8, m5, m6, m7, m8, o5, o6, o7, o8, p5, p6, p7, and p8 are each independently an integer of 0 to 2,
CN represents a cyano group,
D represents a deuterium atom,
Me represents a methyl group,
q1 and q2 are each independently 0 or 1,
Y₁ is O, S, or C(R₅)(R₆),
Cy2 is selected from the group consisting of the following fused rings Cy2-1 to Cy2-7, 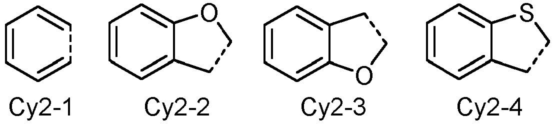 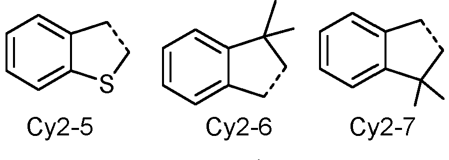
X₁, X₃, and X₅, which are the same or different from each other, are each independently N or C(R₃), with a proviso that at least one of X₁, X₃, and X₅ is N,
L₁, L₂, R₁, R₂, and R₃ are each as defined in Claim 1,
R₅ and R₆, which are the same or different from each other, are each independently selected from the group consisting of a hydrogen atom, a deuterium atom (D), a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 nuclear atoms, a C₆-C₆₀ aryl group, and a heteroaryl group having 5 to 60 nuclear atoms or can form a fused ring with an adjacent group).

9. The organic compound of claim 1, wherein the organic compound represented by any one of Chemical Formulas 1 to 5 is selected from the group consisting of the following organic compounds A1-A120, B1-B120, C1-C120, D1-D104, E1-E100, F1-F74, G1-G88, H1-H90, I1-I58, J1-J120, K1-K80, L1-L39, M1-M45, N1-N50, and O1-O64:

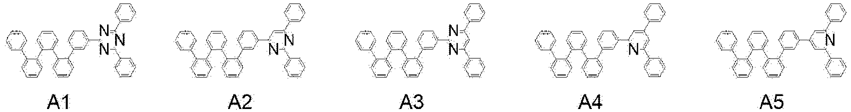

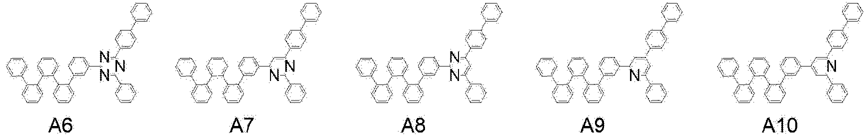

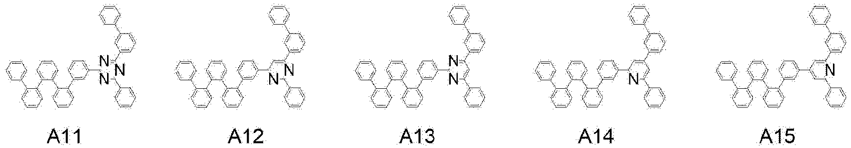

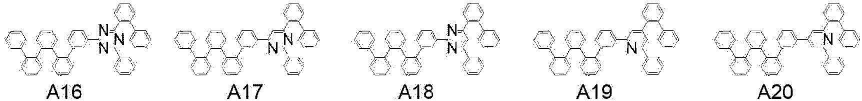

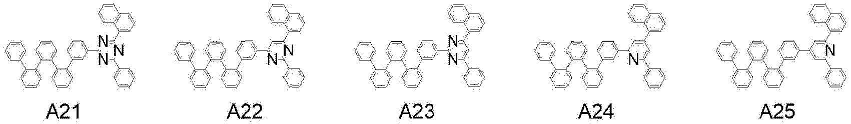

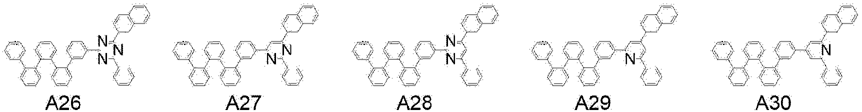

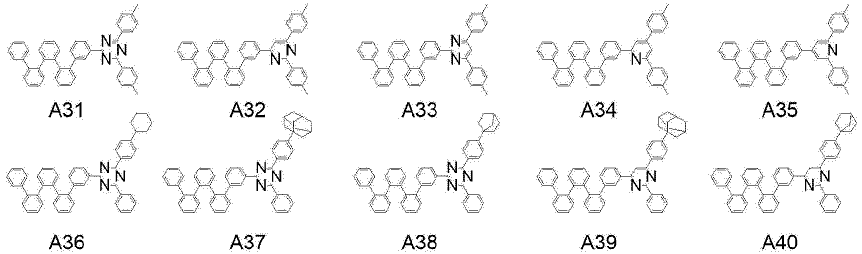

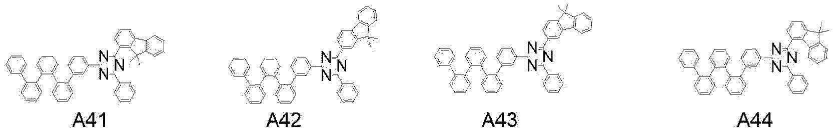

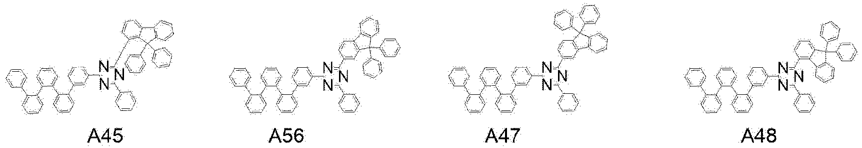

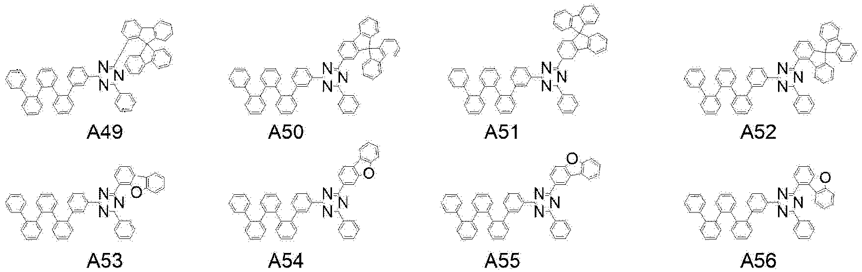

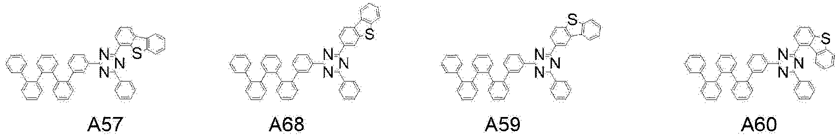

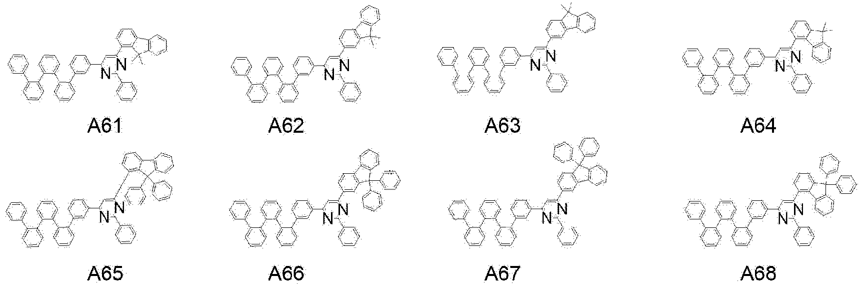

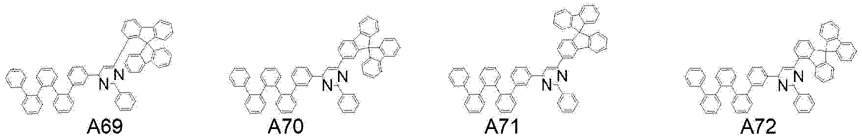

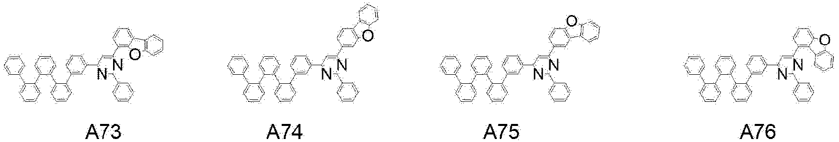

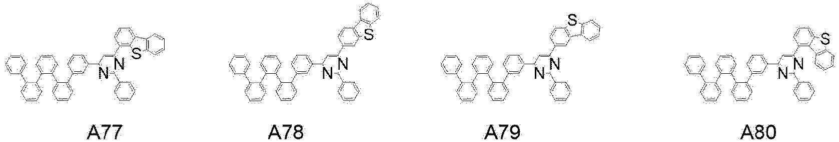

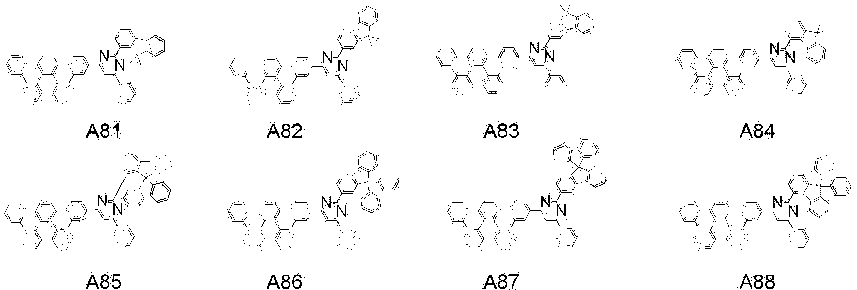

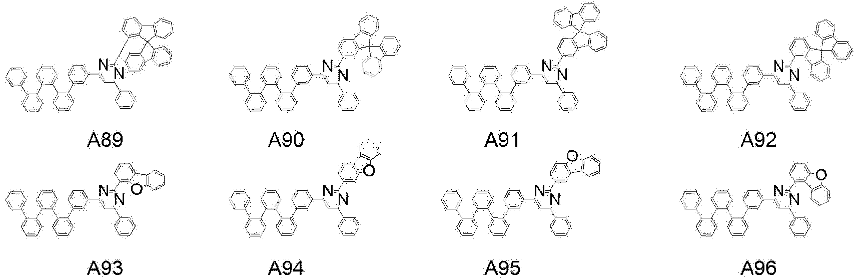

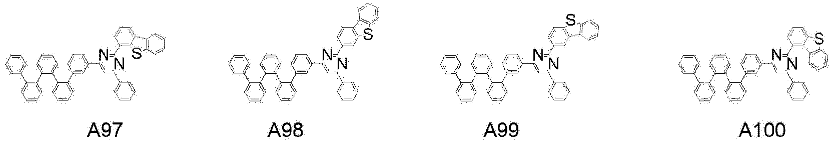

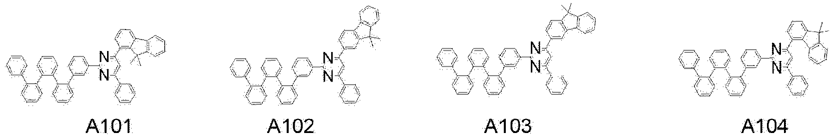

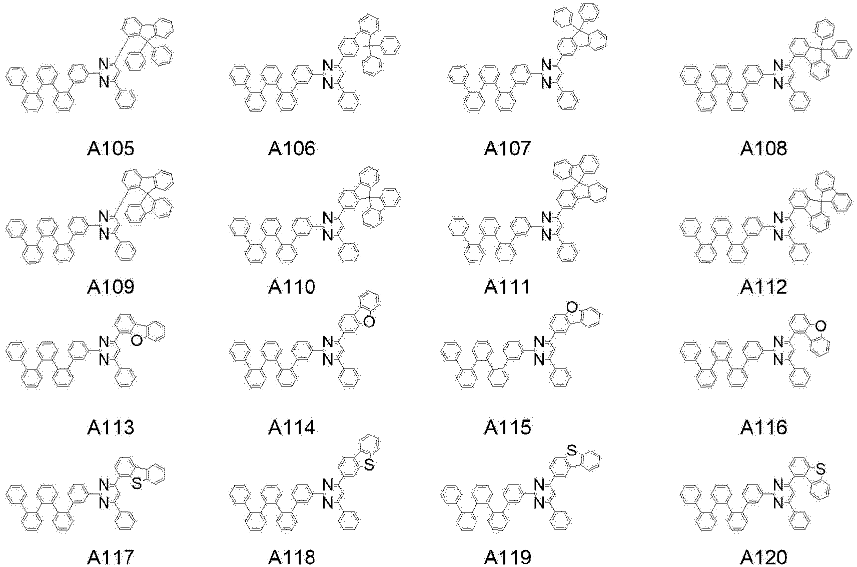

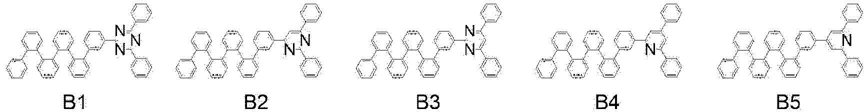

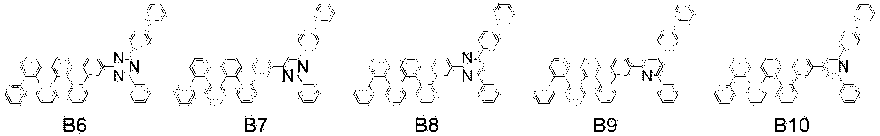

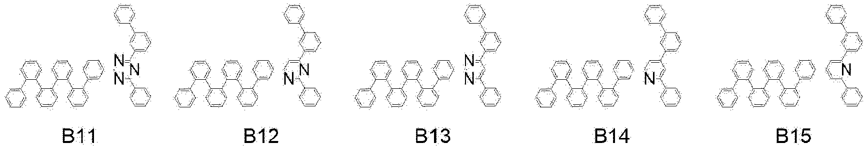

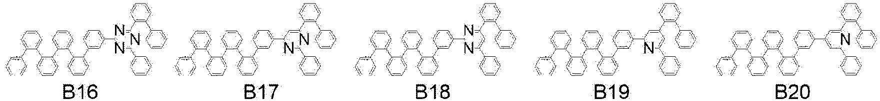

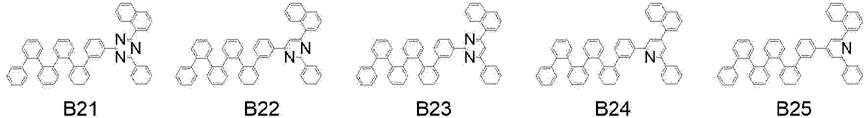

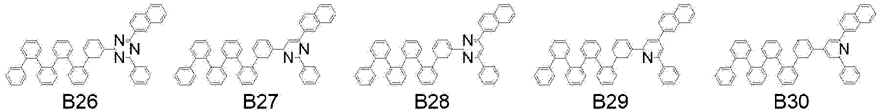

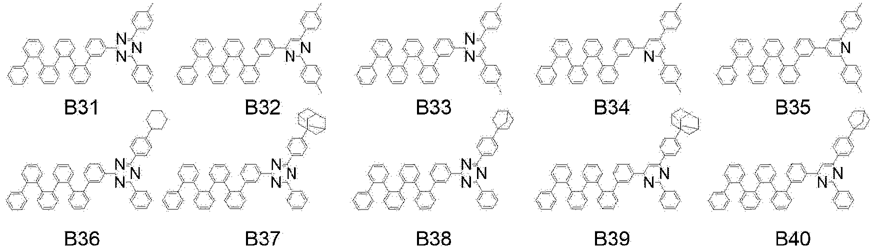

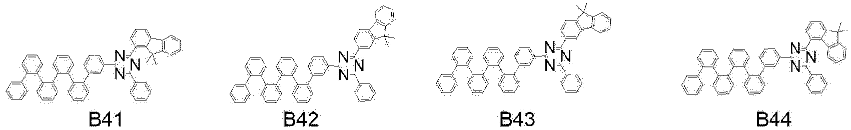

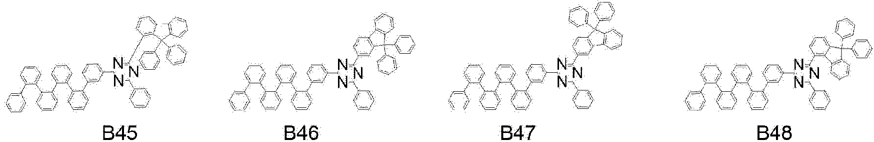

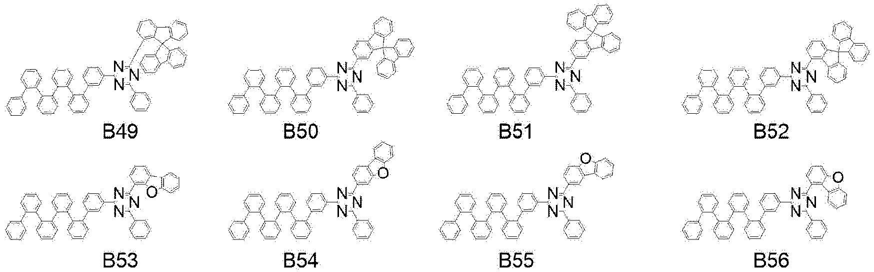

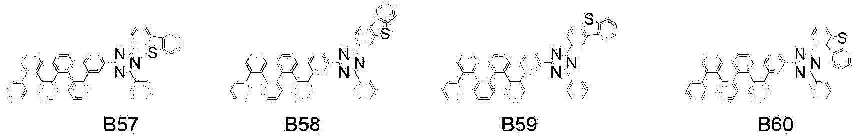

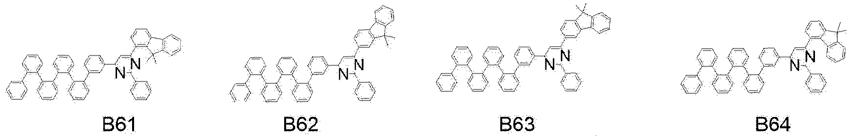

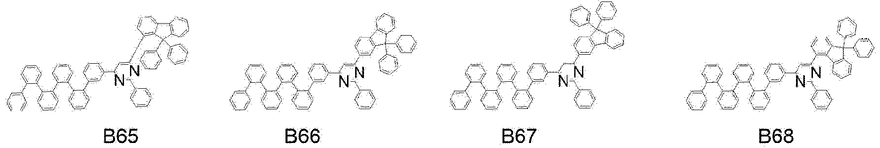

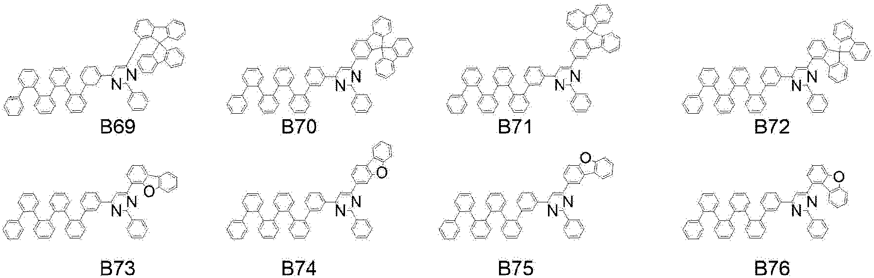

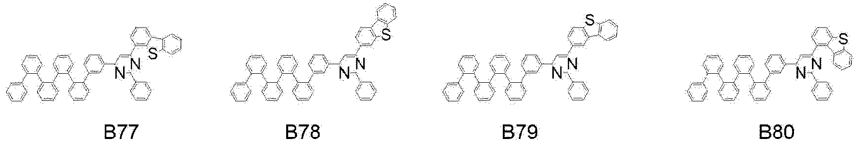

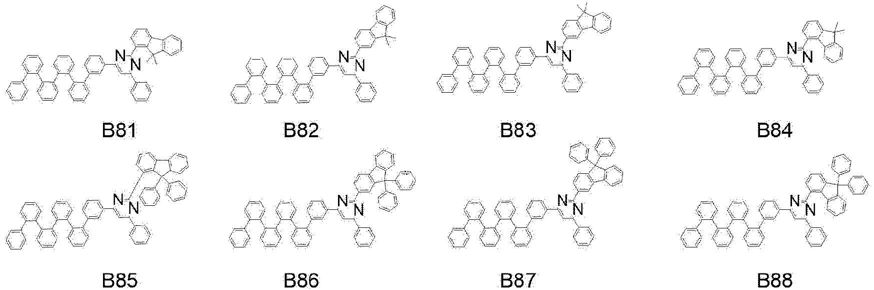

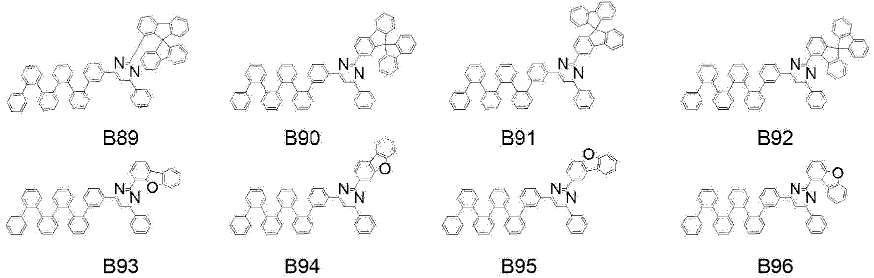

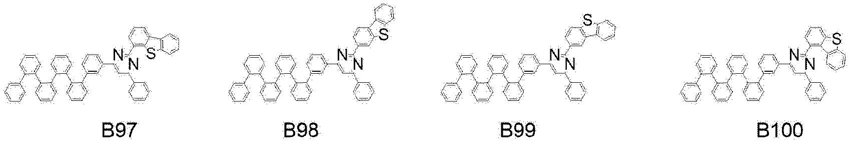

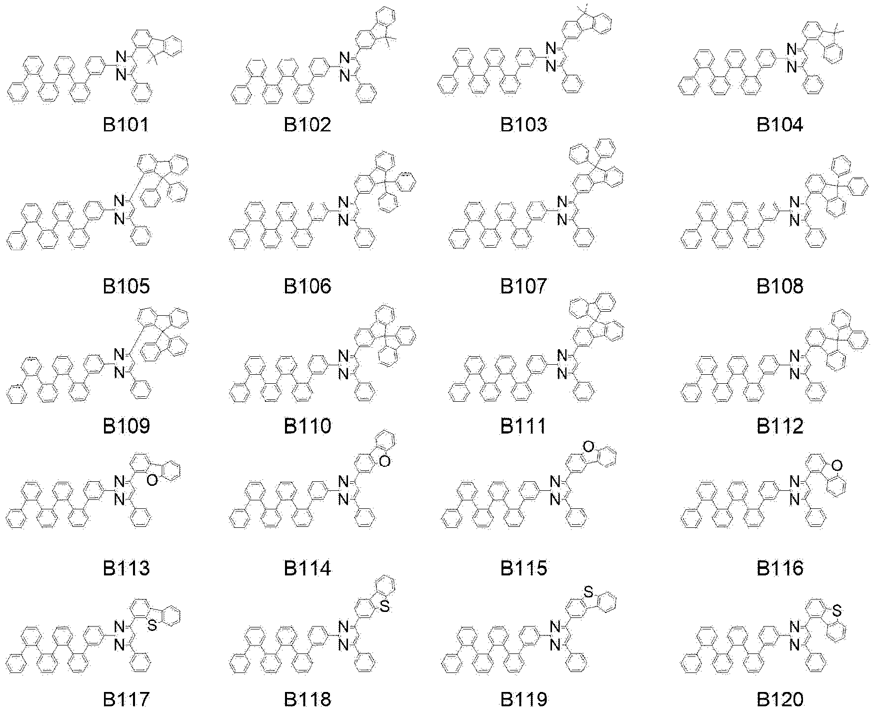

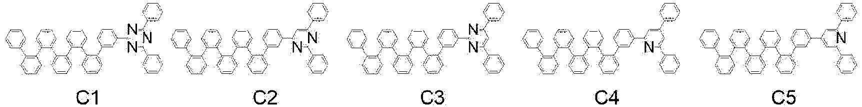

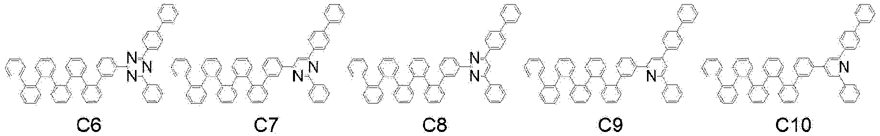

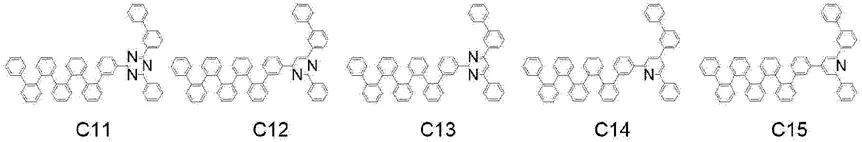

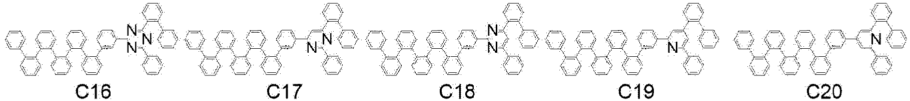

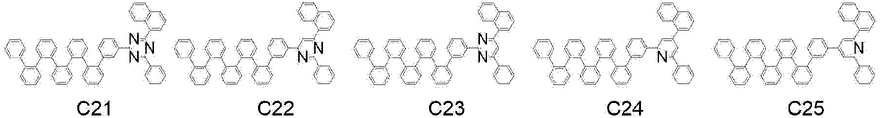

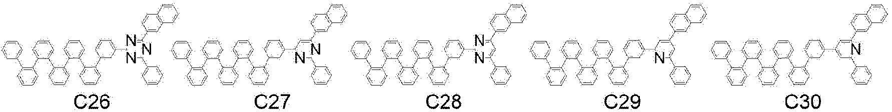

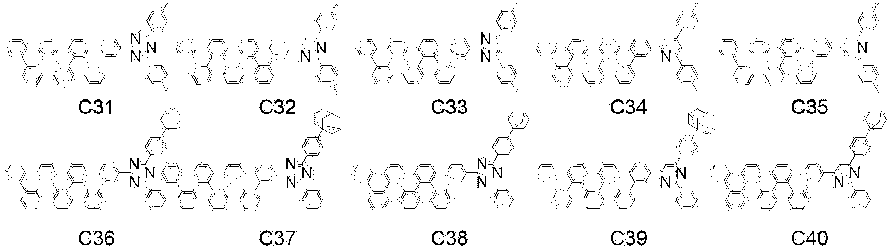

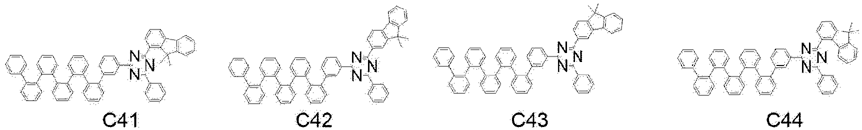

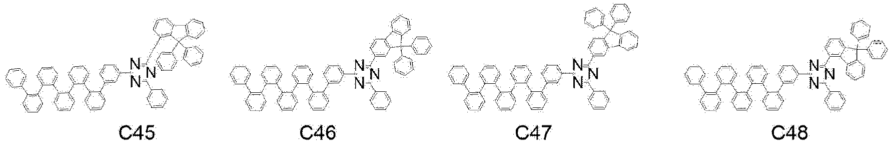

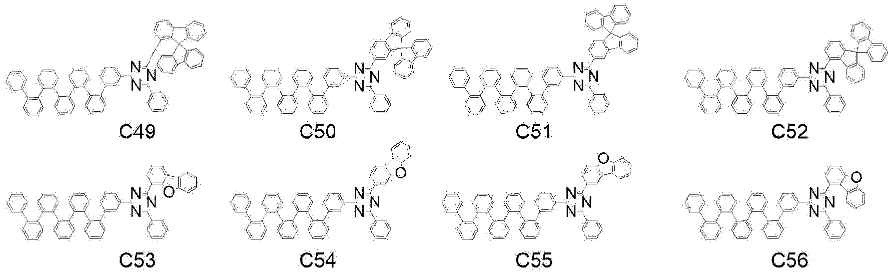

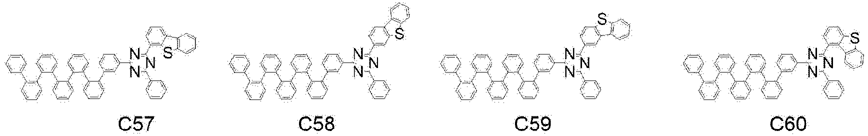

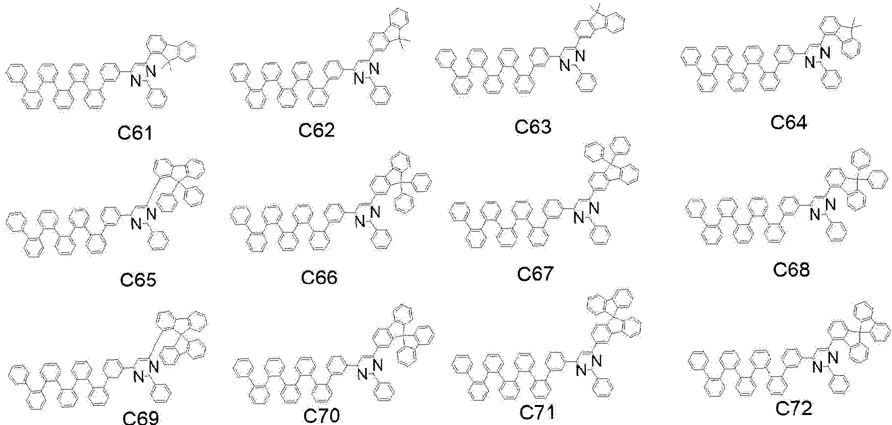

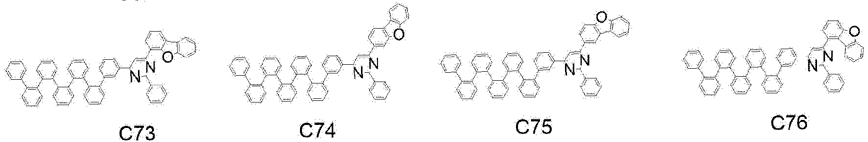

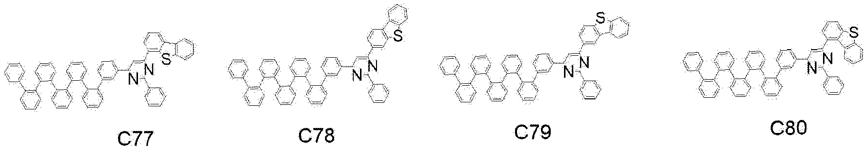

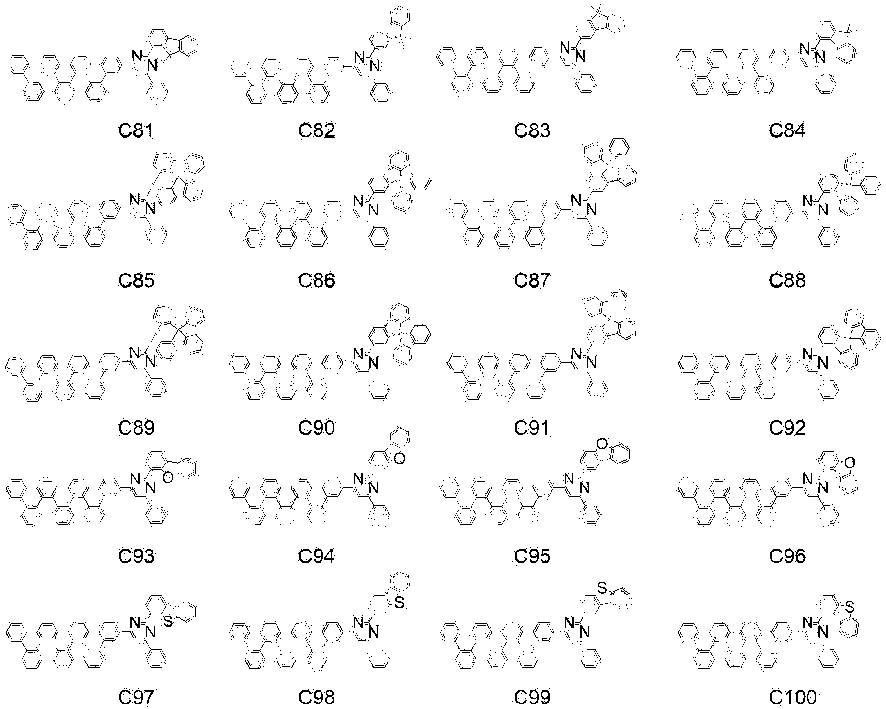

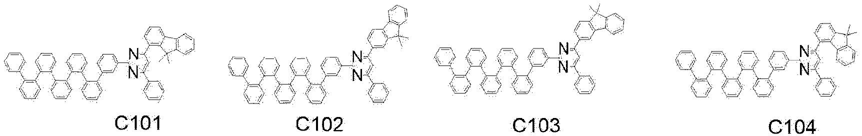

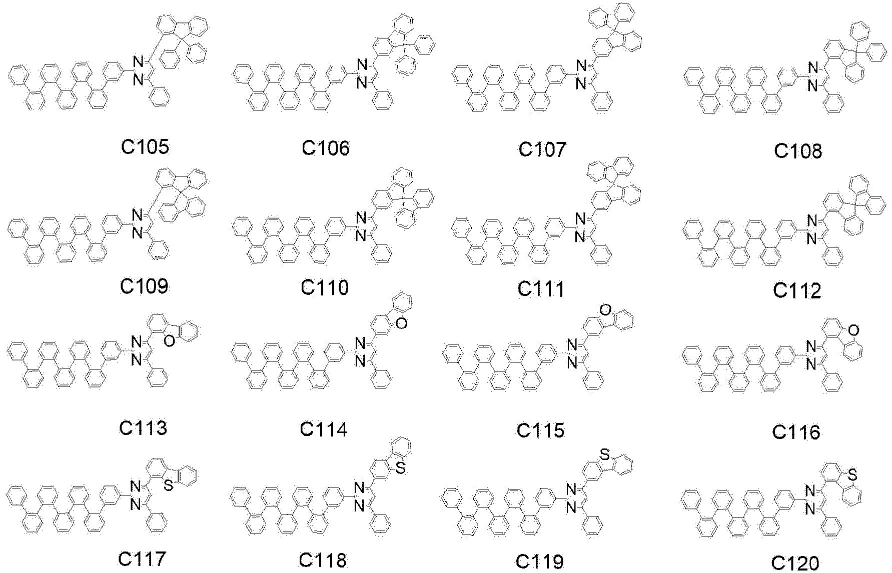

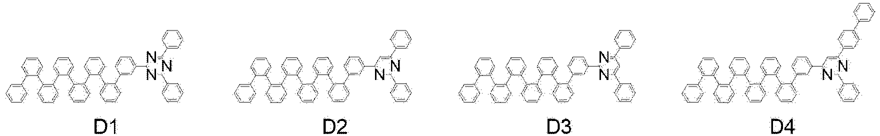

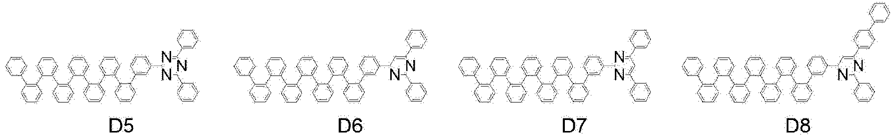

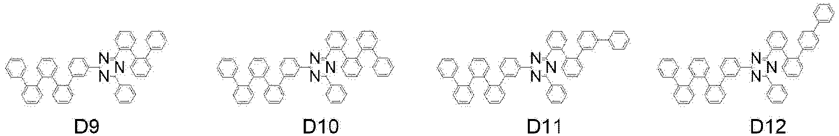

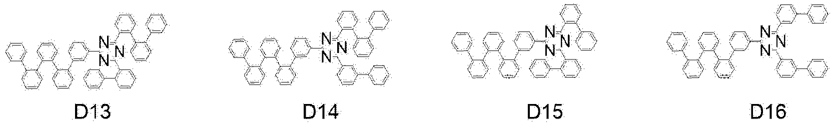

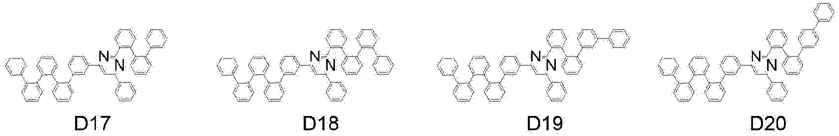

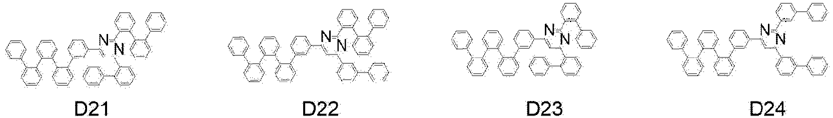

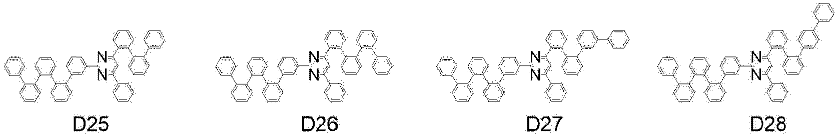

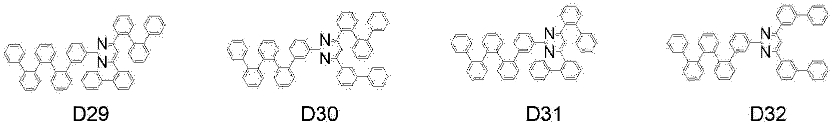

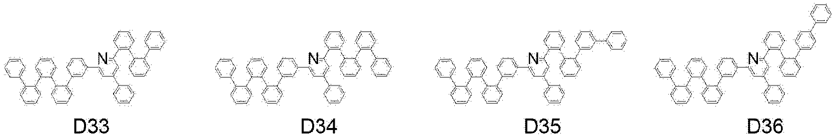

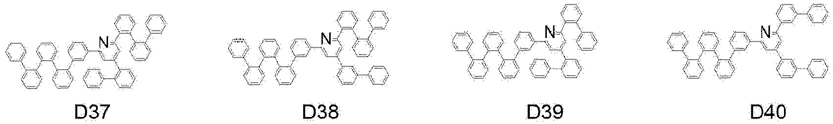

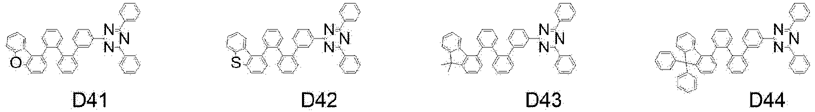

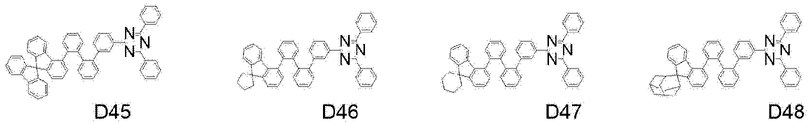

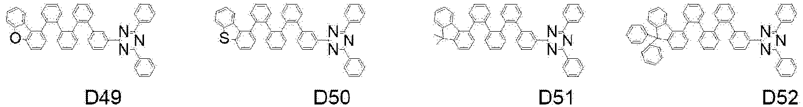

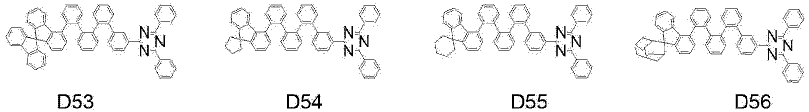

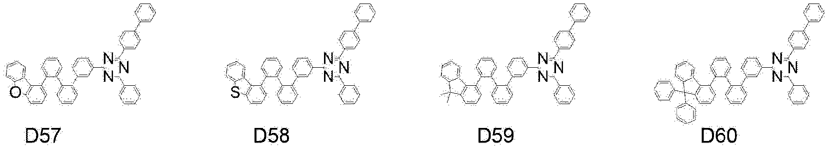

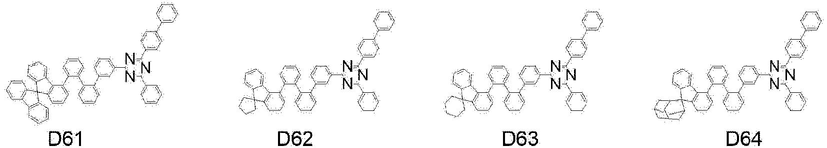

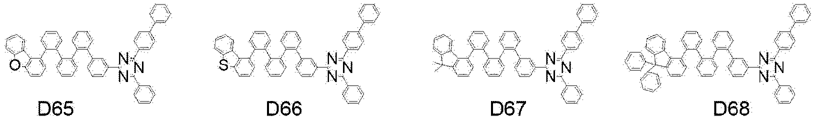

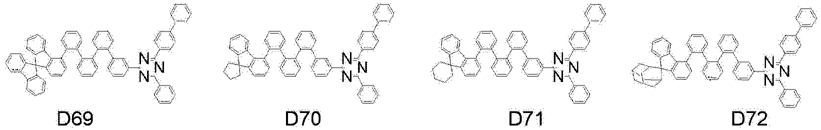

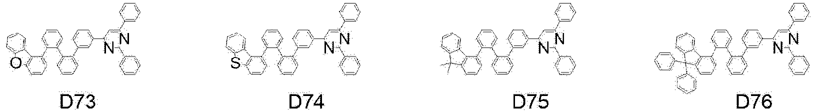

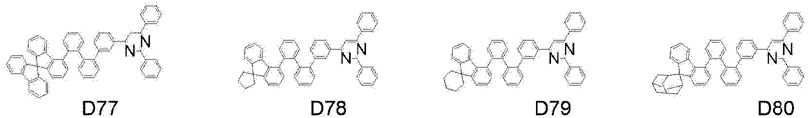

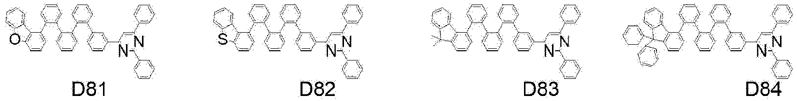

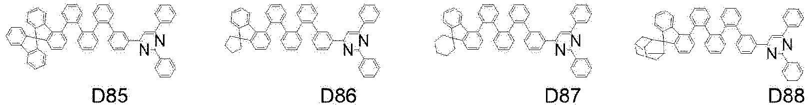

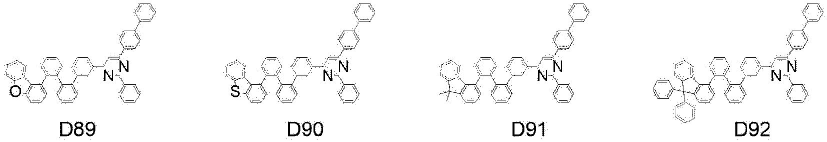

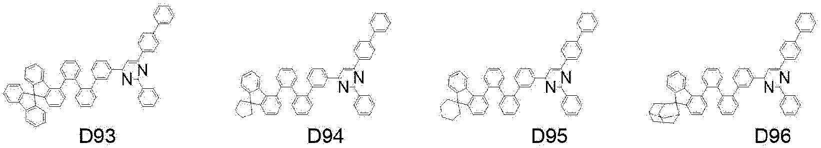

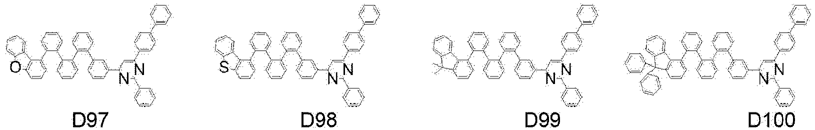

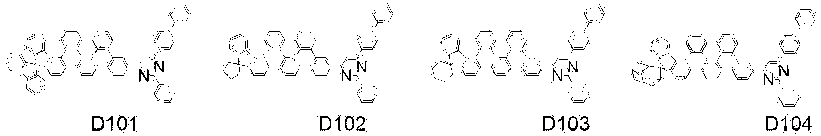

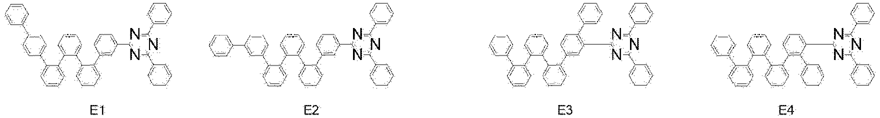

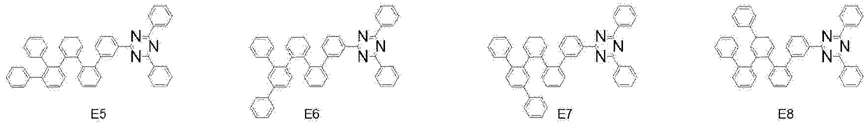

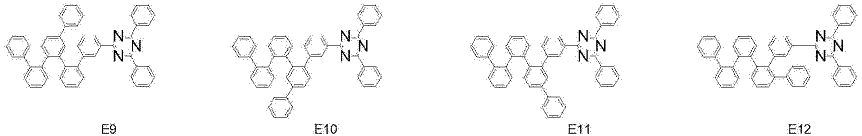

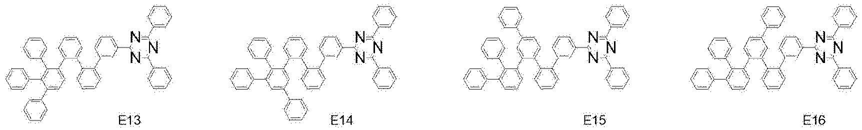

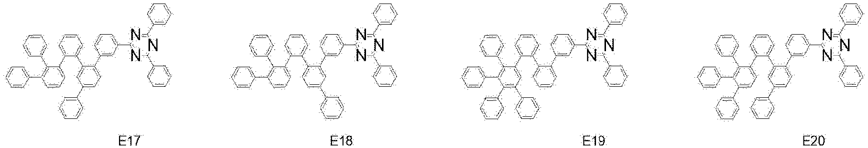

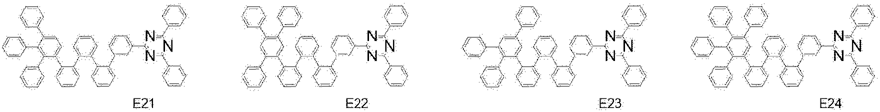

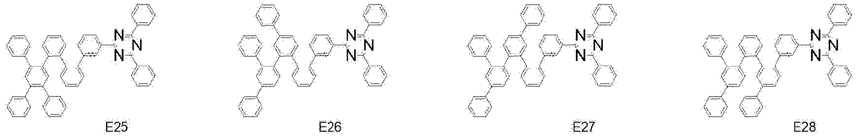

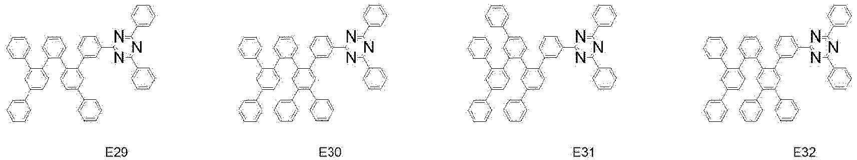

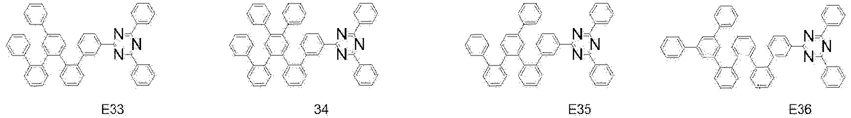

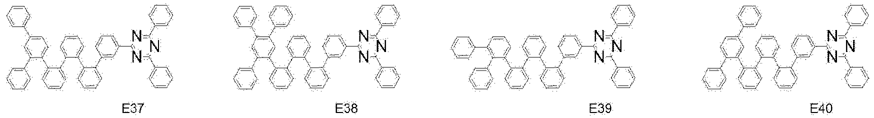

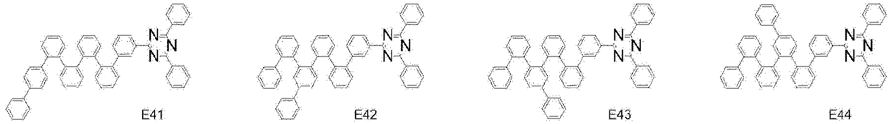

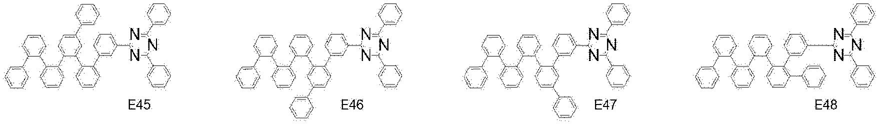

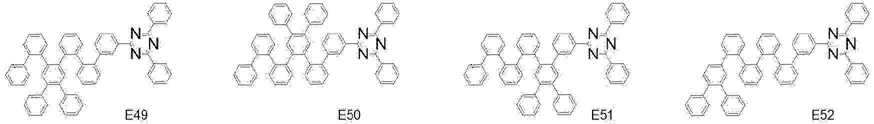

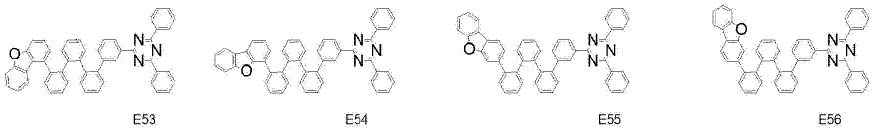

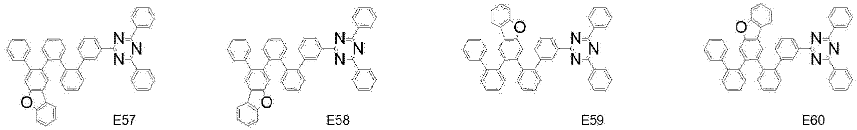

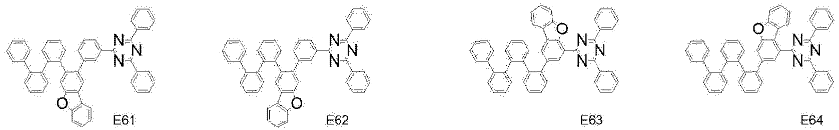

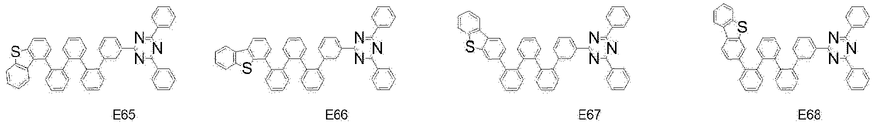

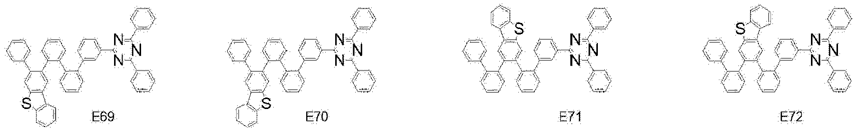

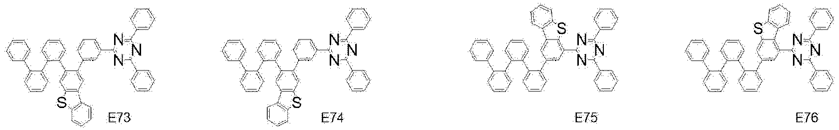

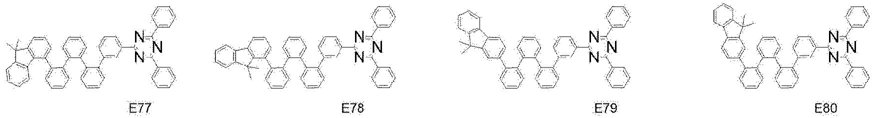

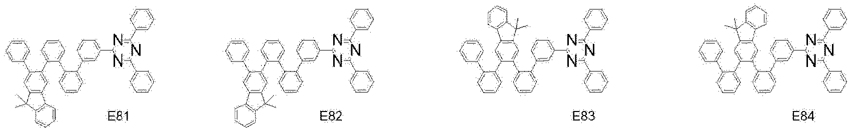

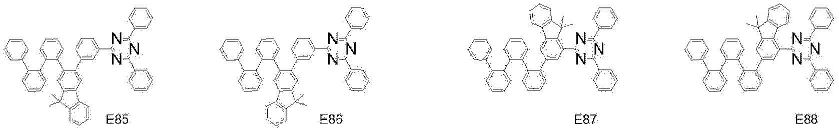

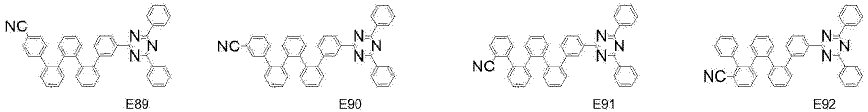

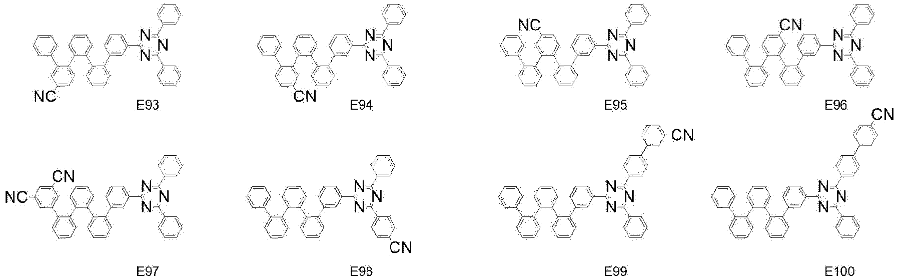

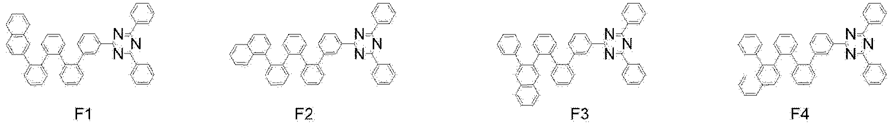

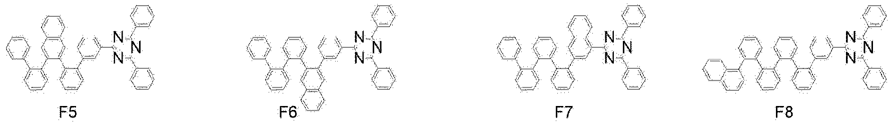

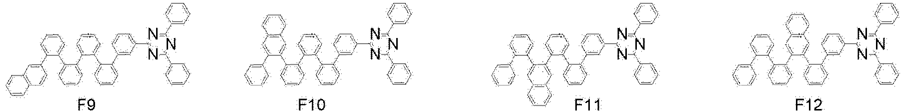

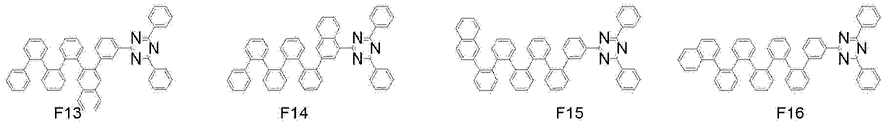

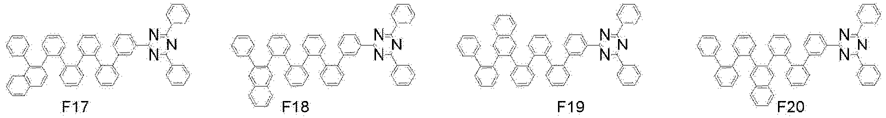

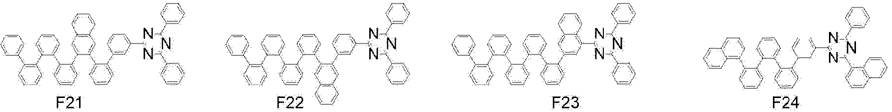

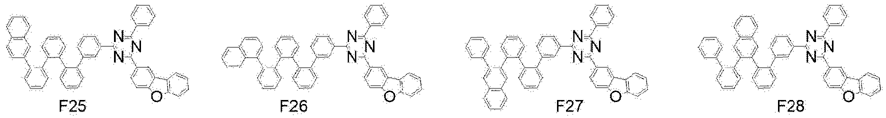

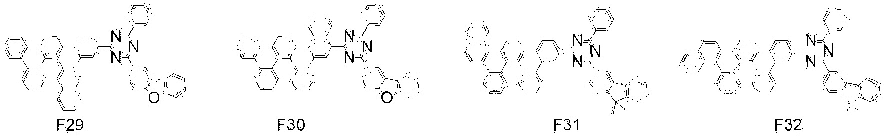

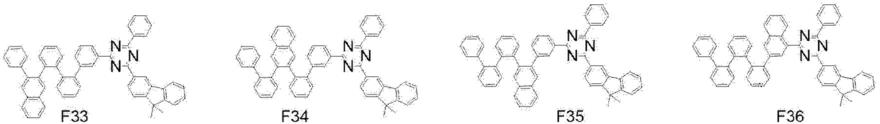

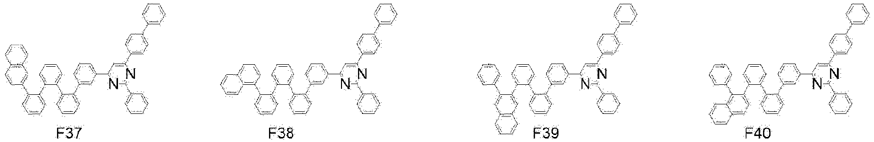

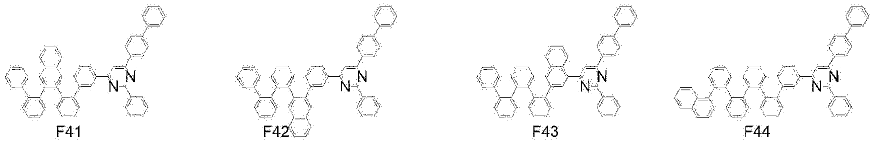

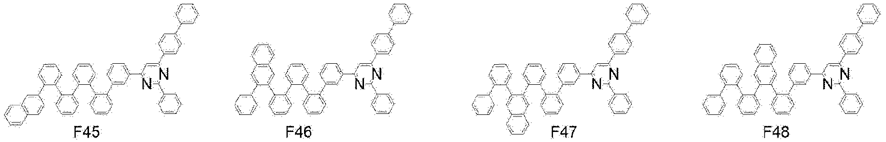

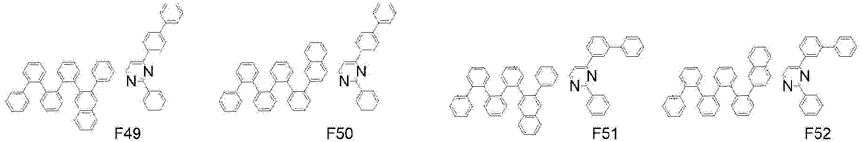

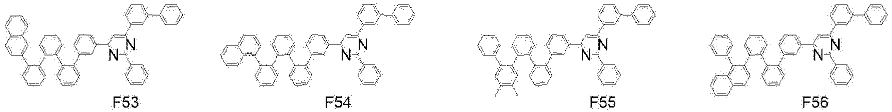

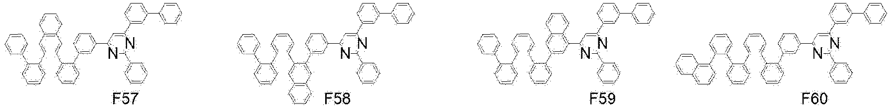

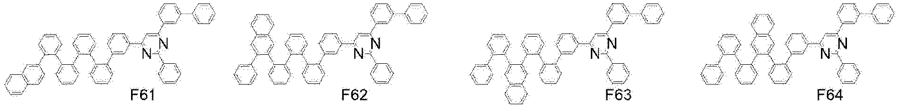

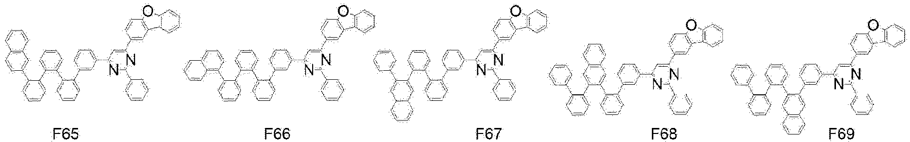

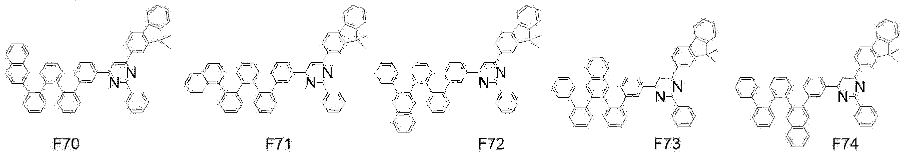

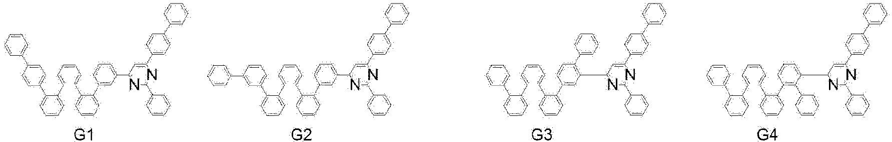

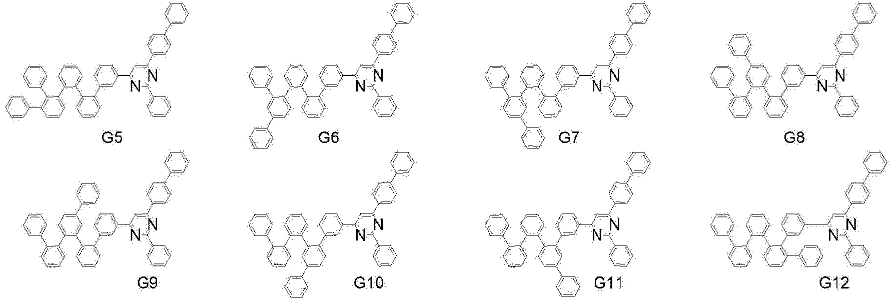

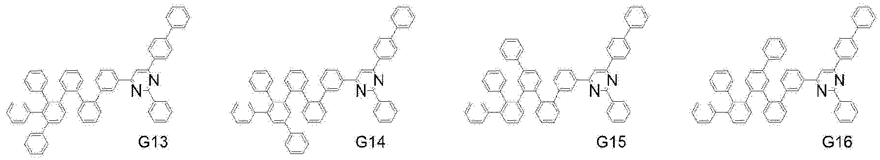

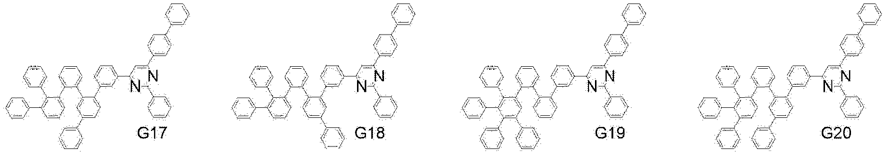

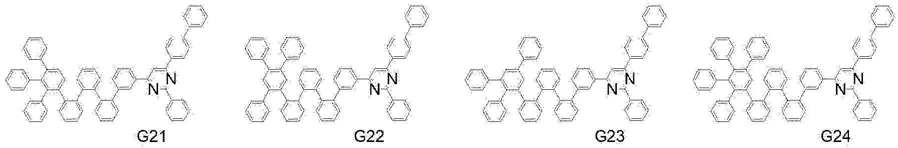

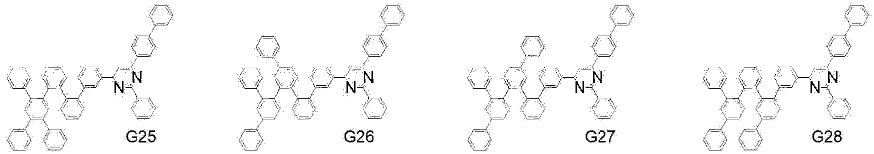

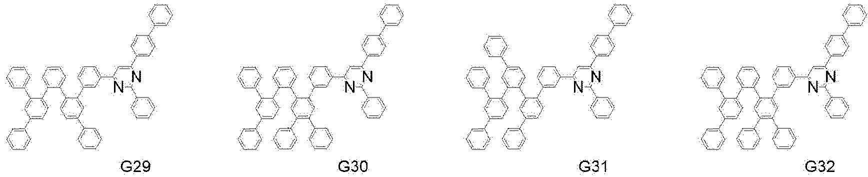

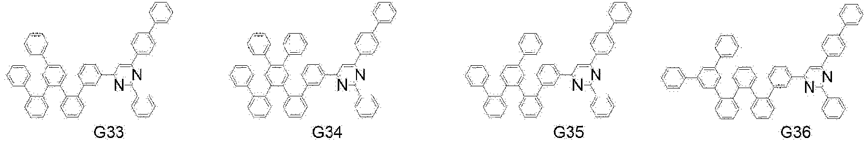

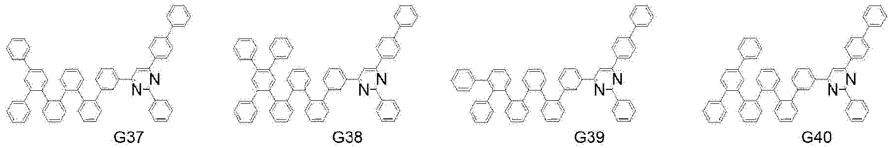

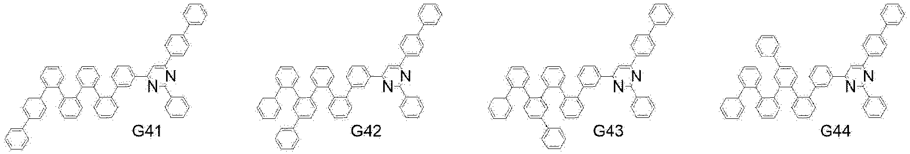

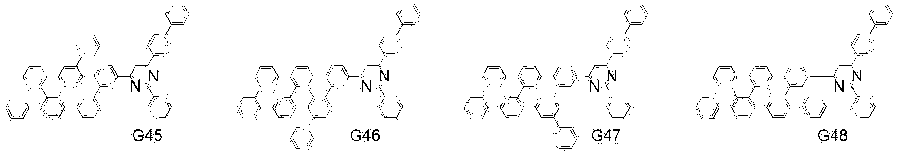

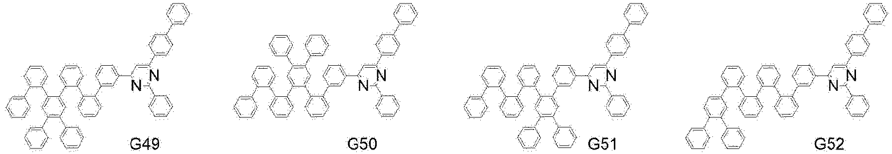

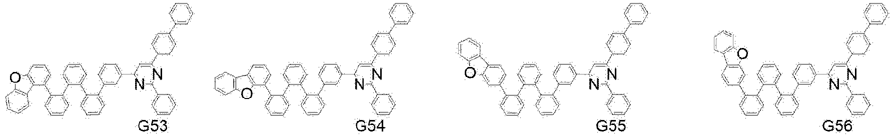

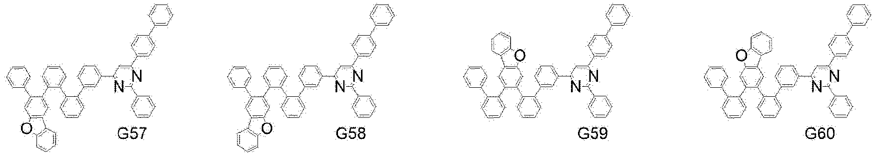

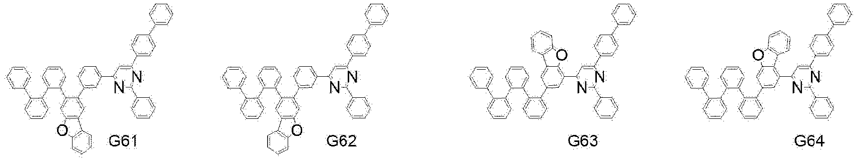

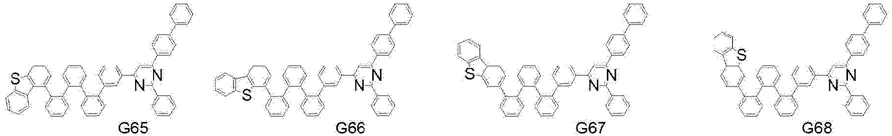

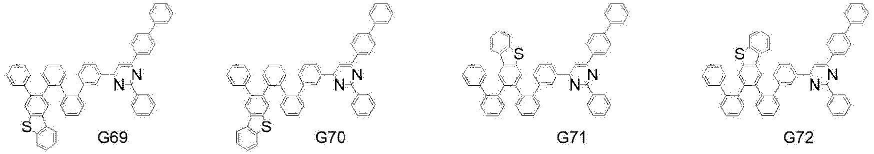

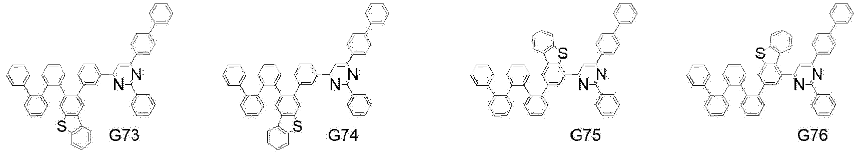

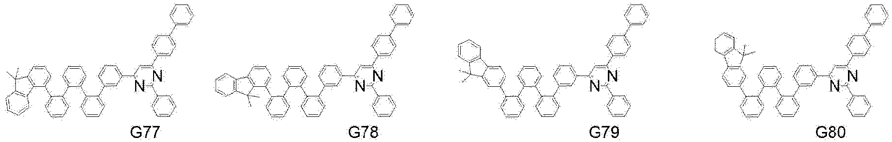

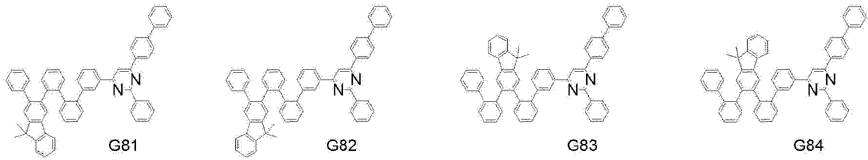

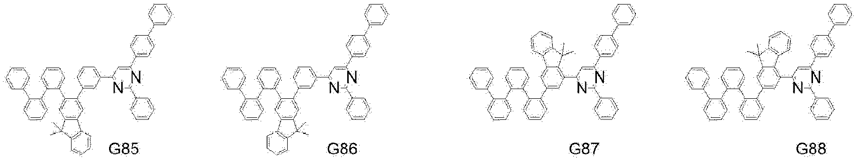

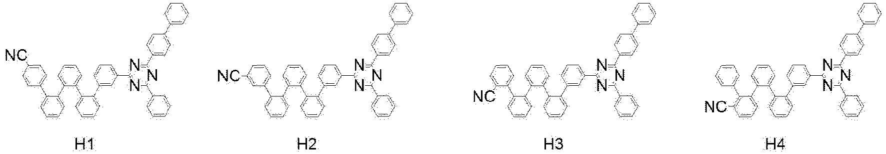

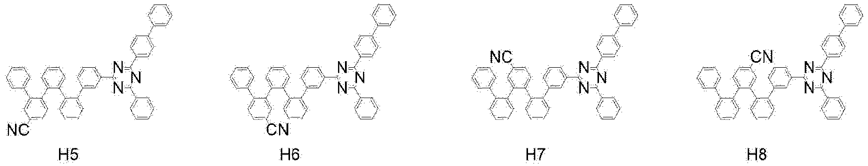

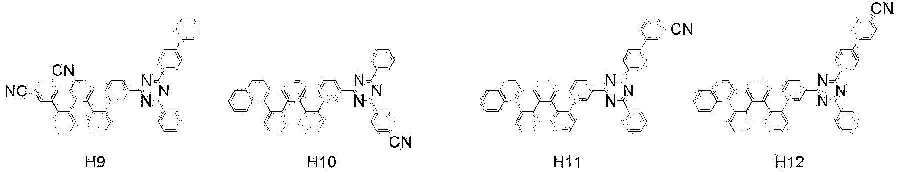

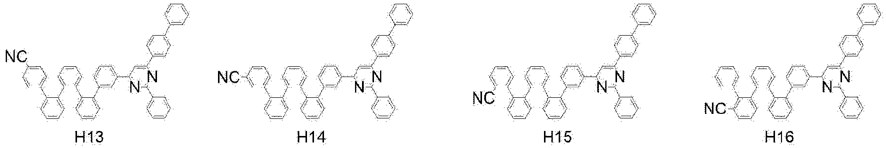

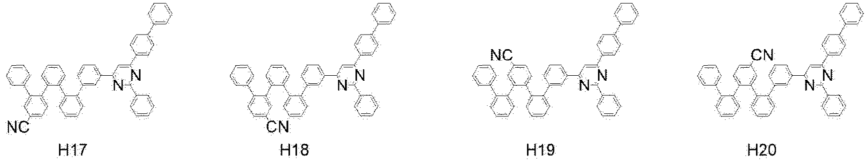

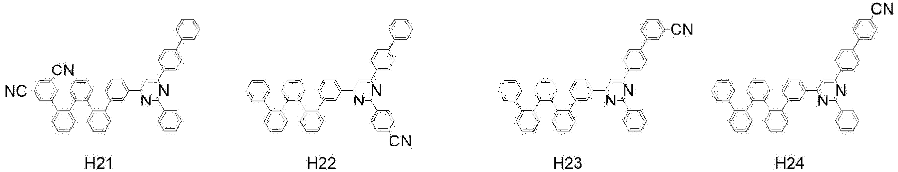

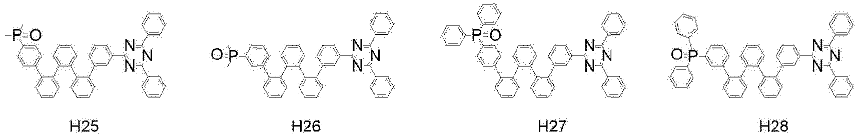

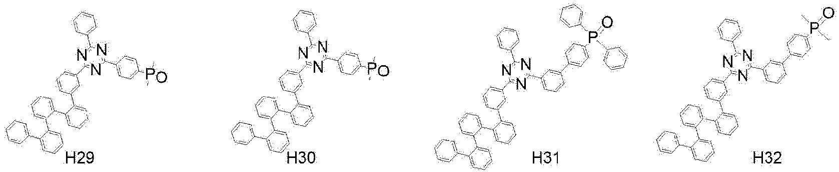

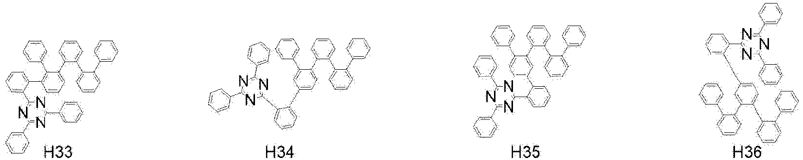

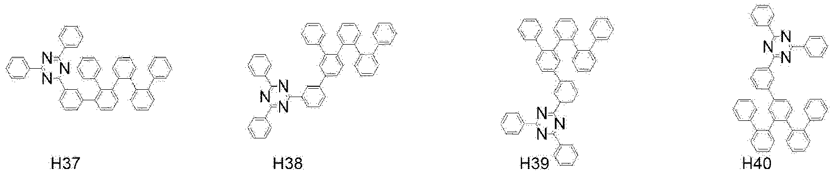

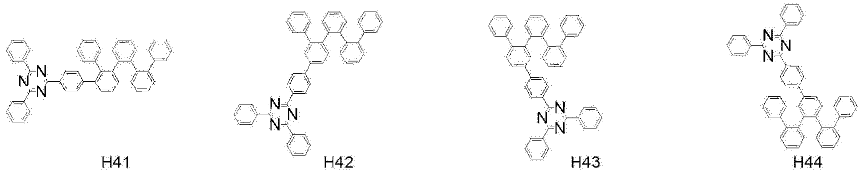

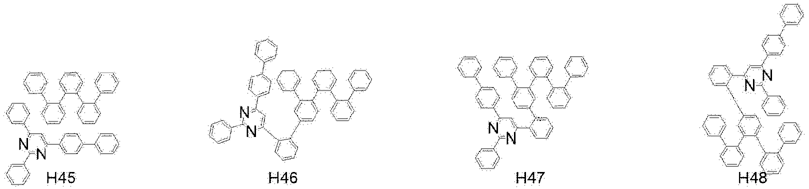

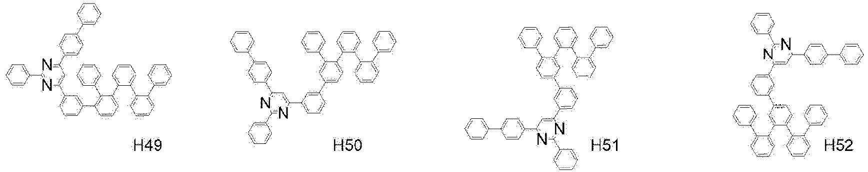

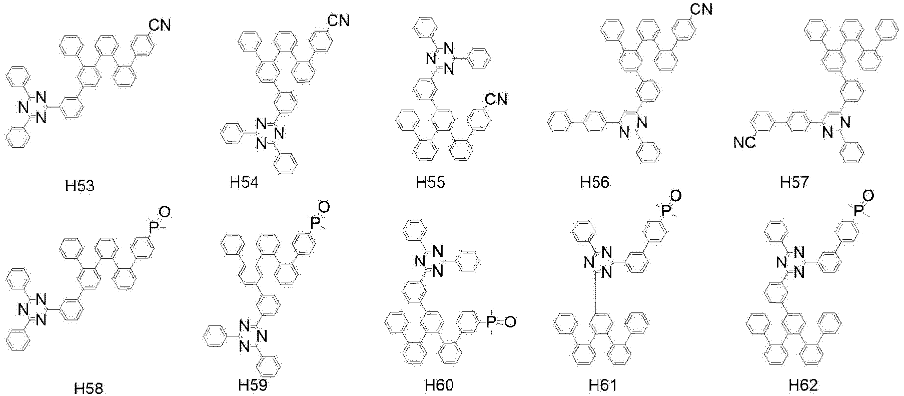

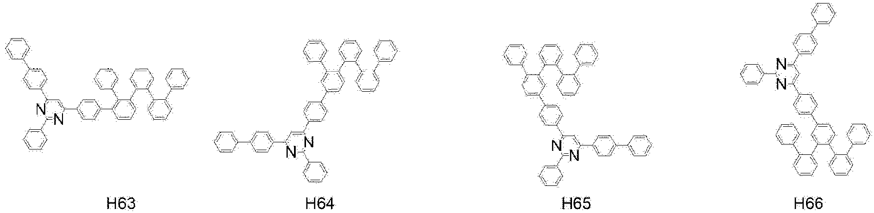

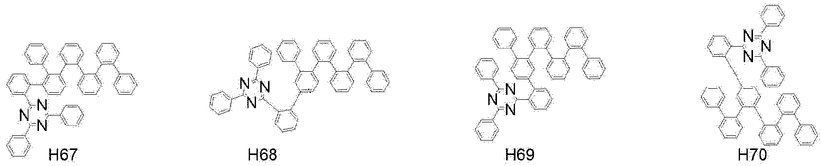

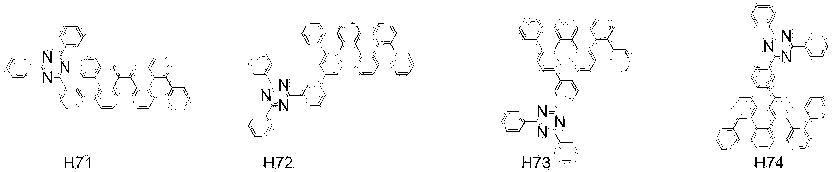

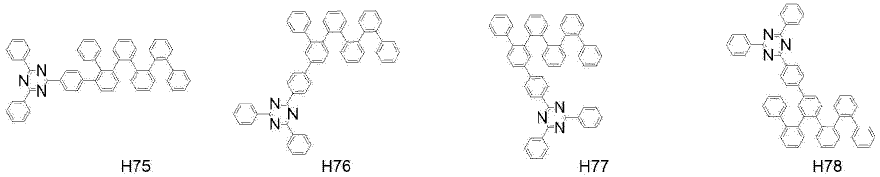

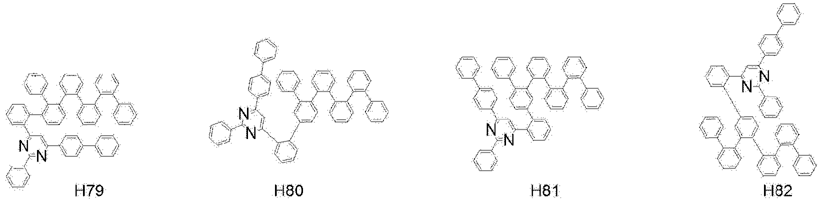

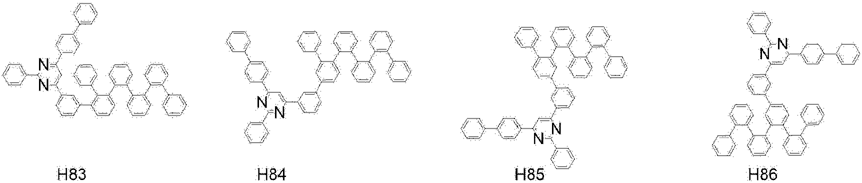

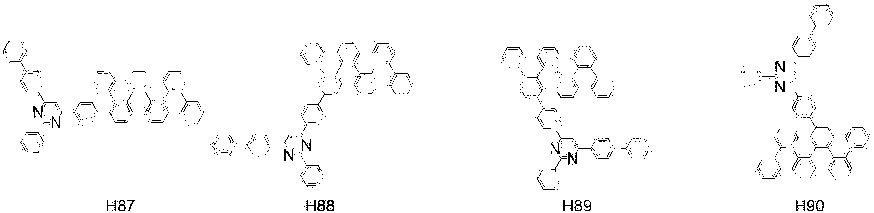

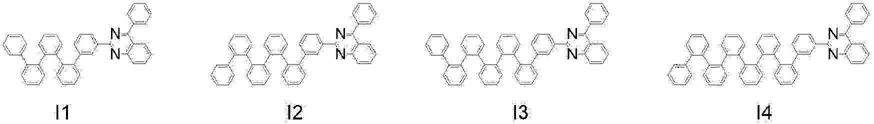

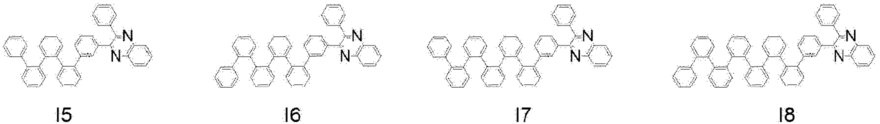

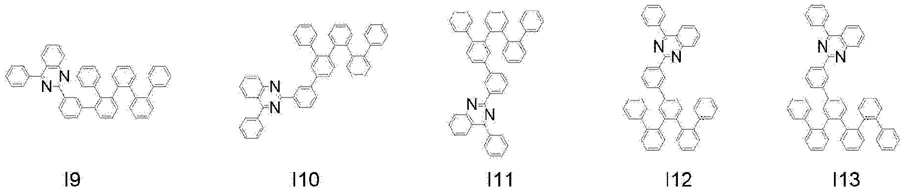

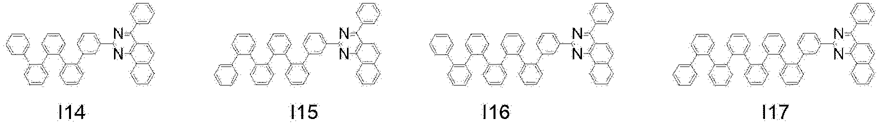

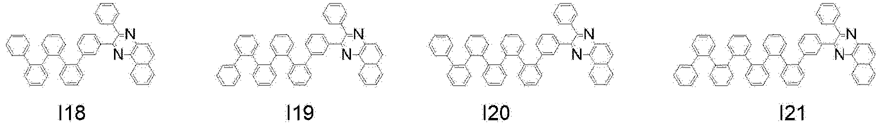

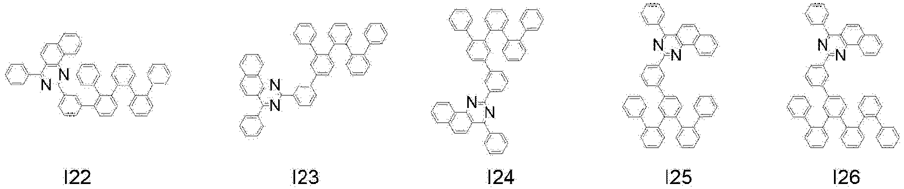

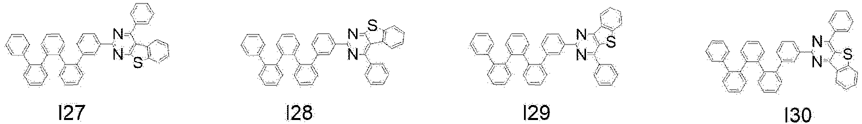

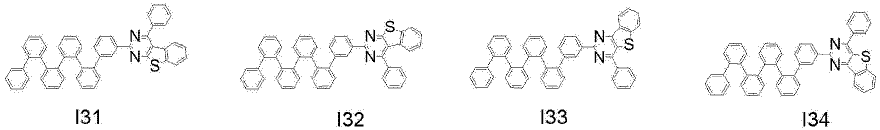

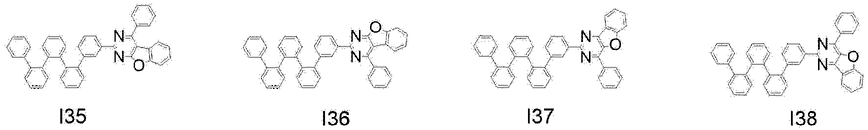

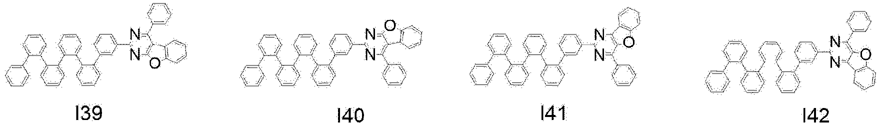

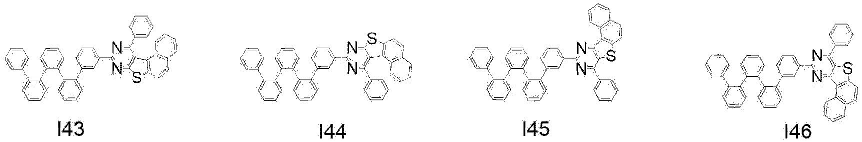

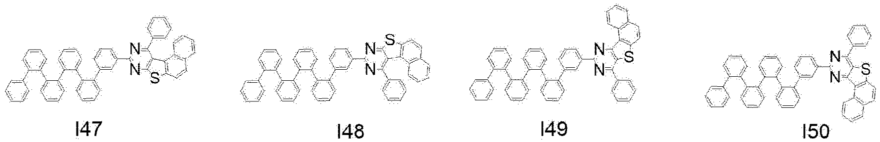

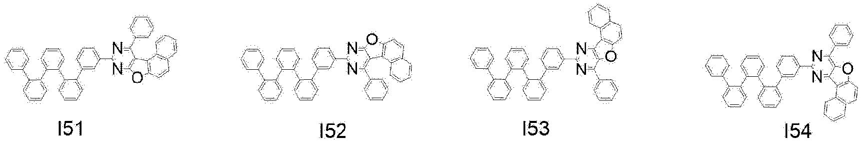

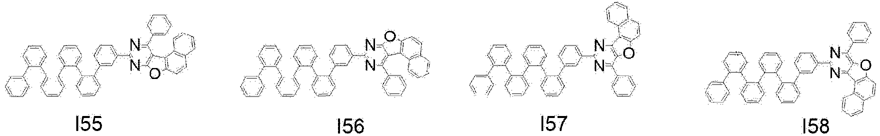

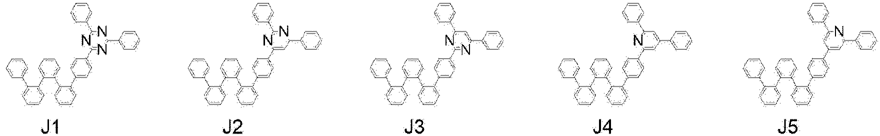

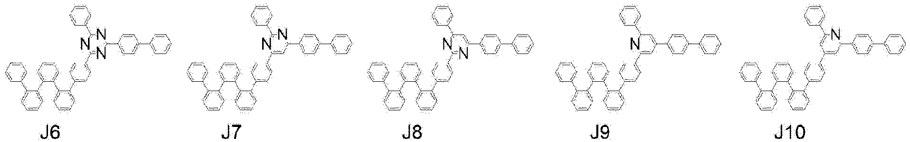

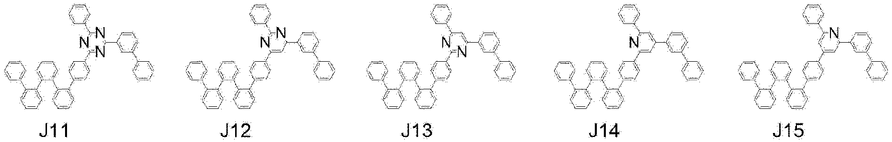

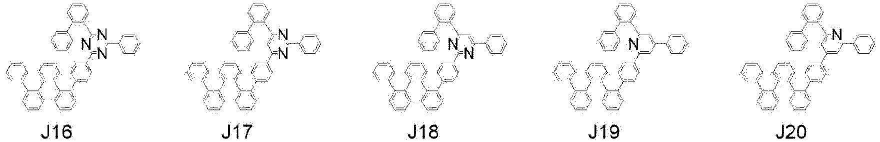

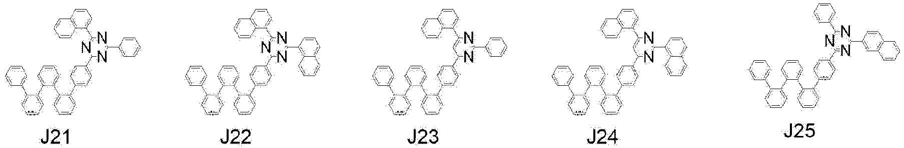

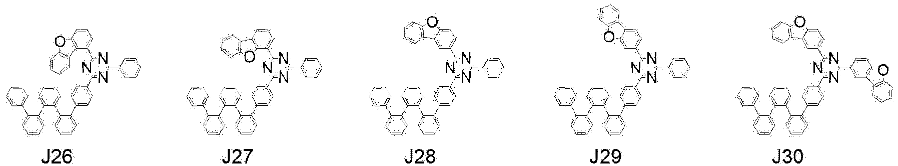

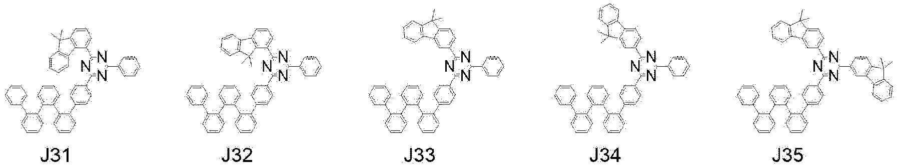

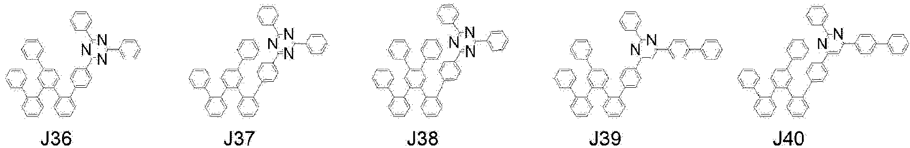

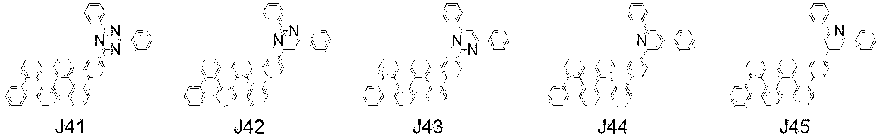

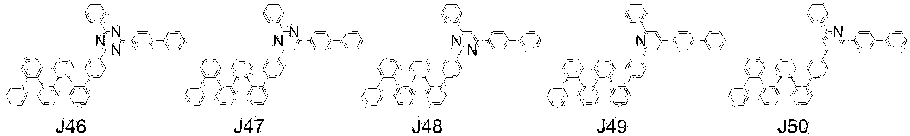

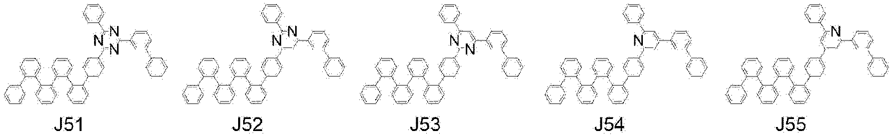

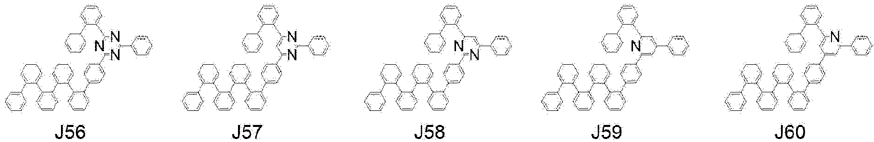

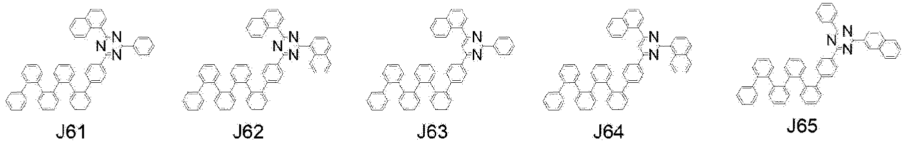

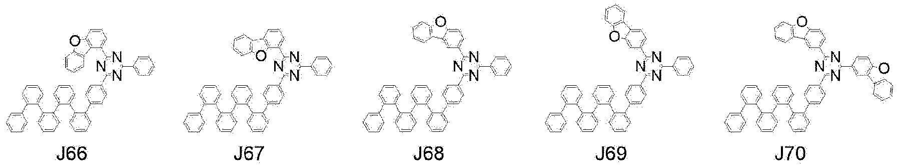

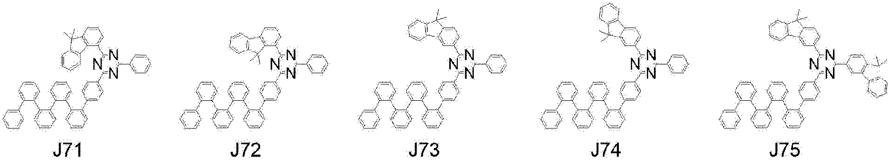

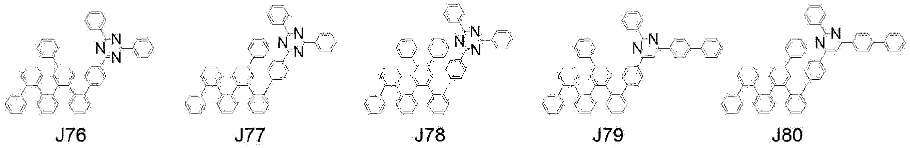

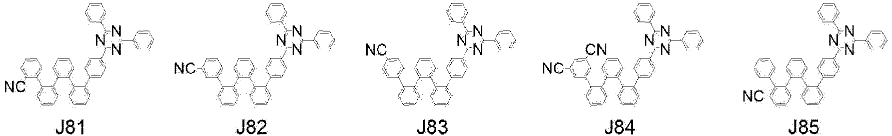

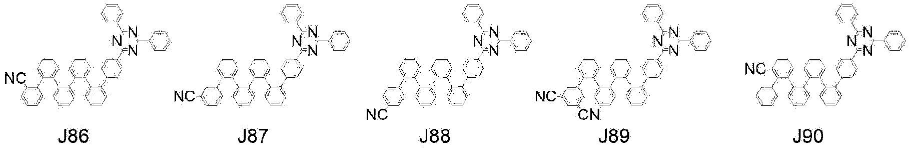

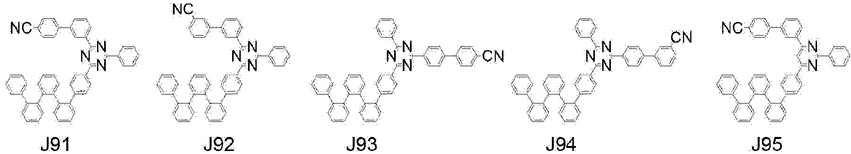

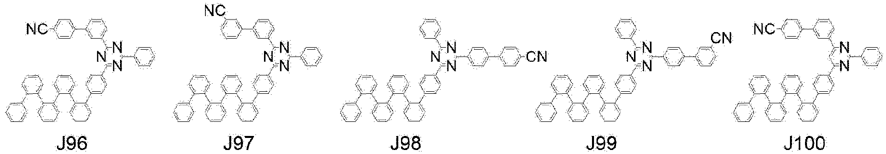

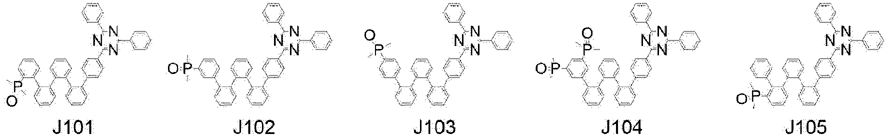

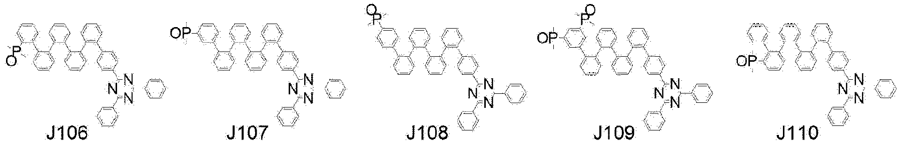

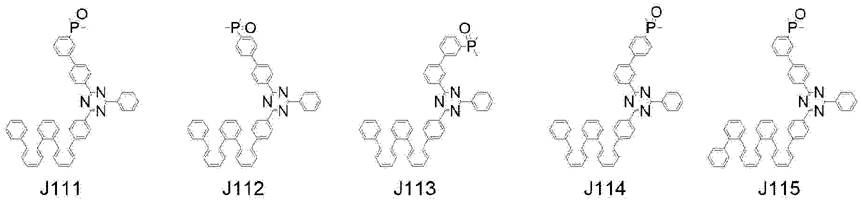

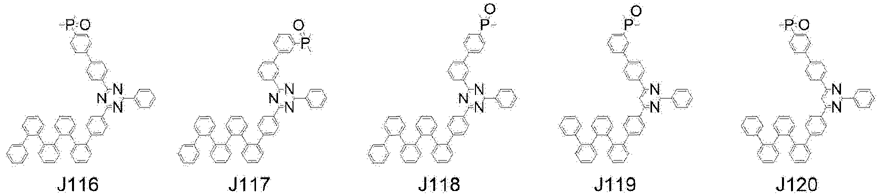

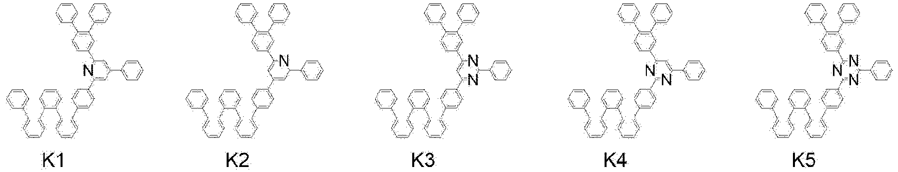

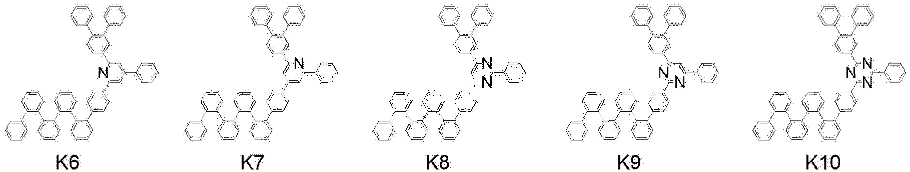

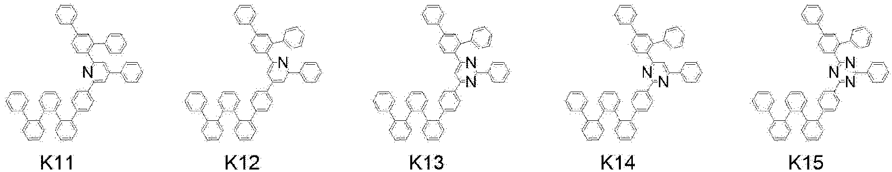

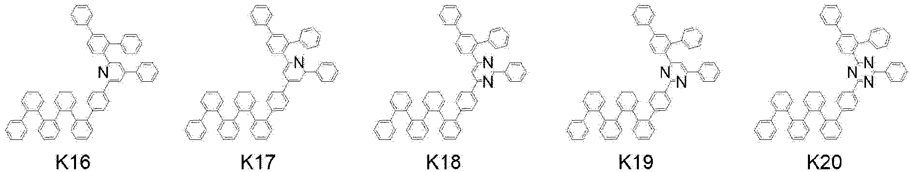

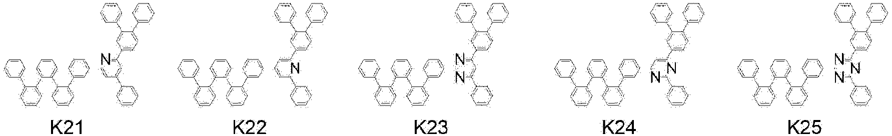

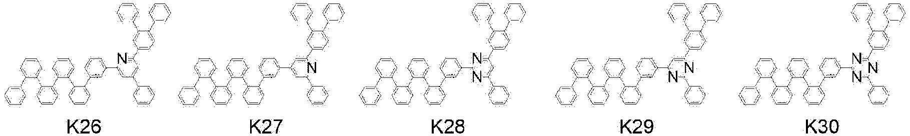

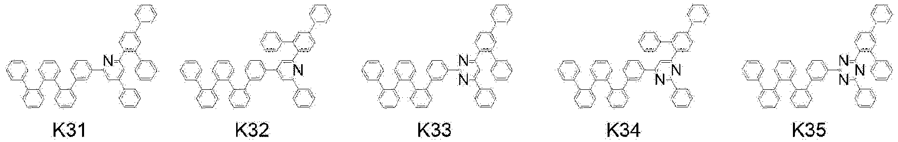

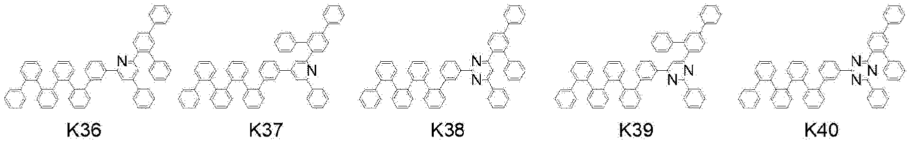

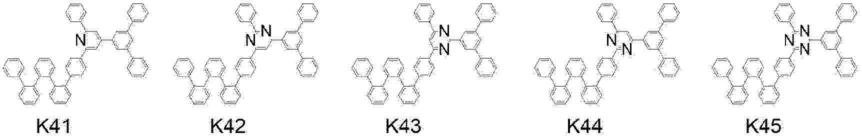

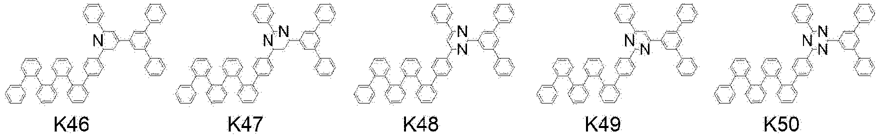

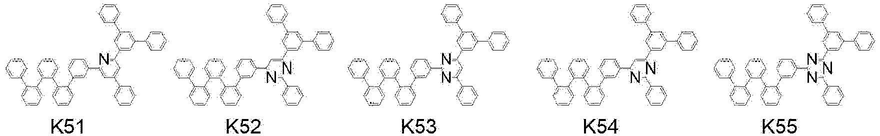

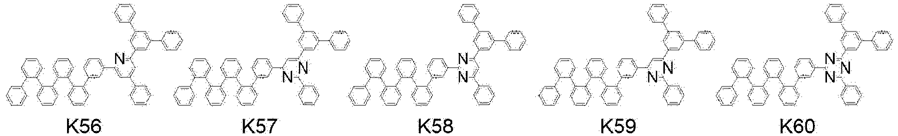

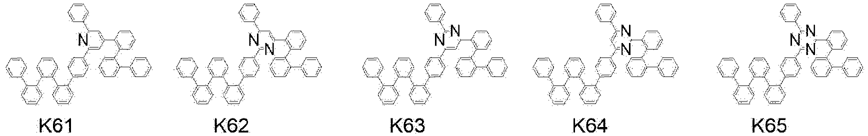

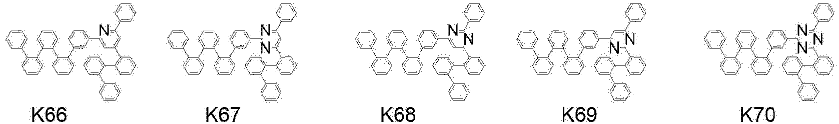

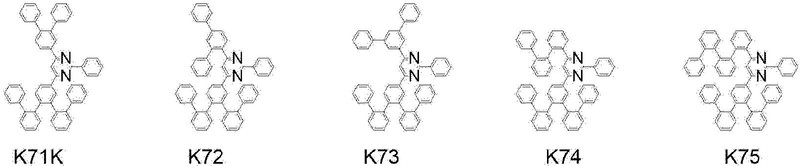

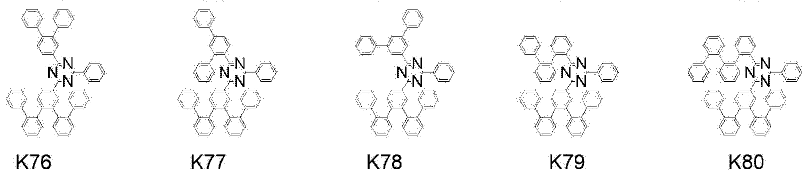

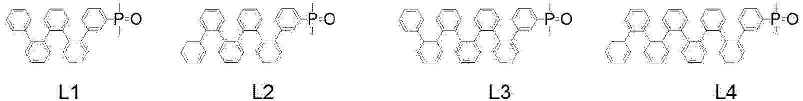

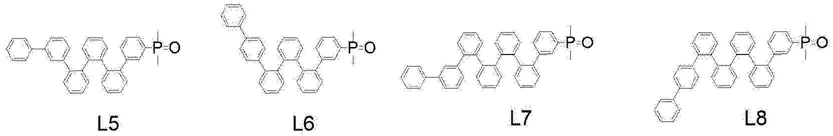

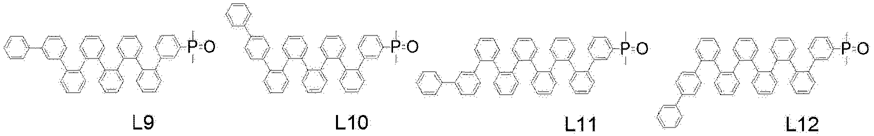

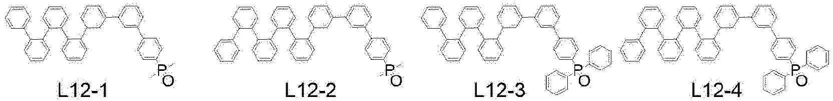

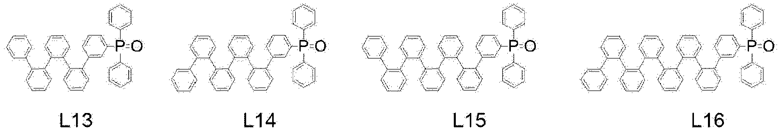

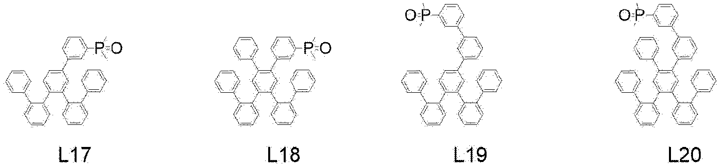

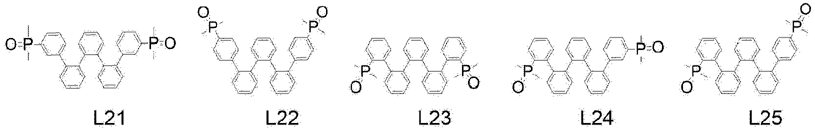

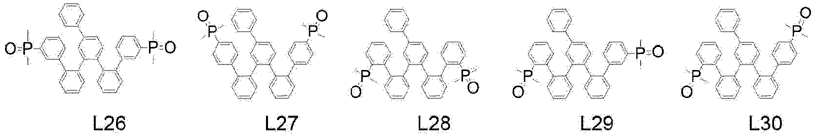

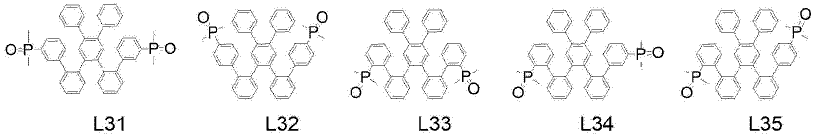

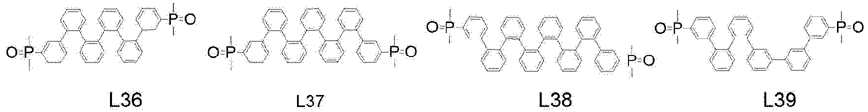

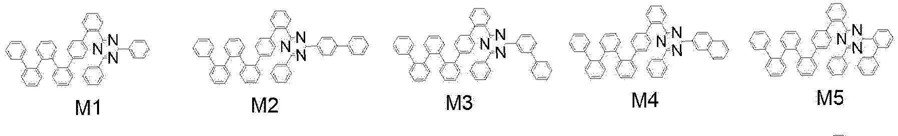

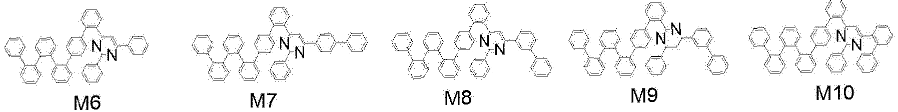

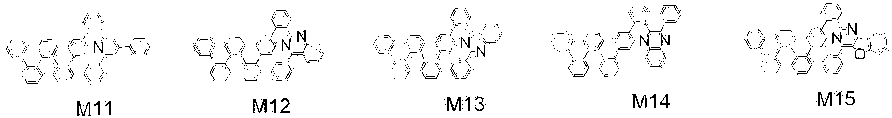

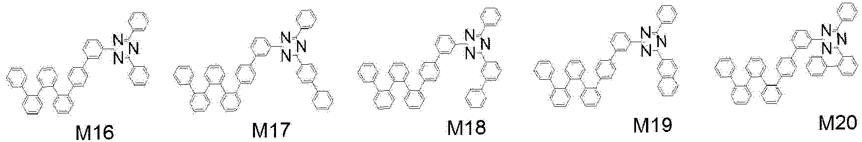

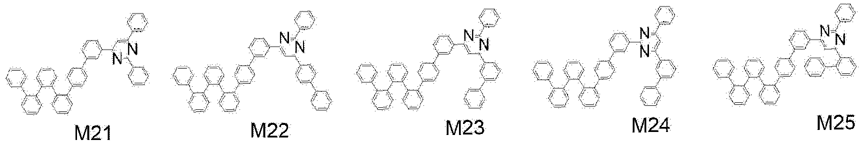

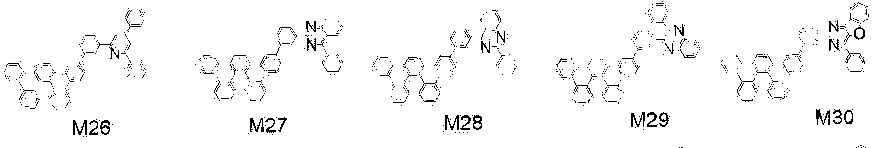

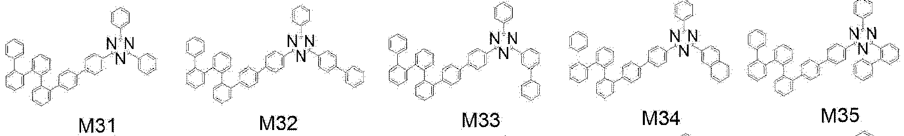

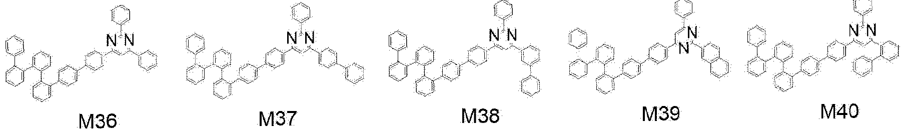

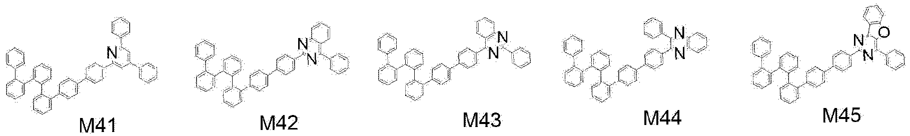

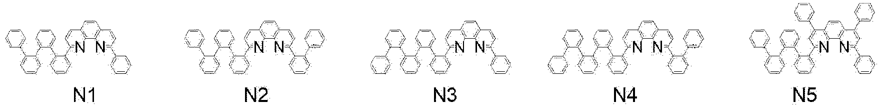

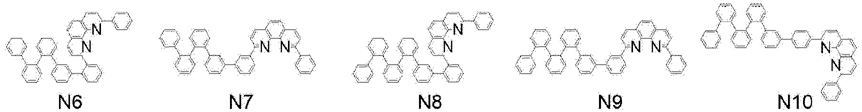

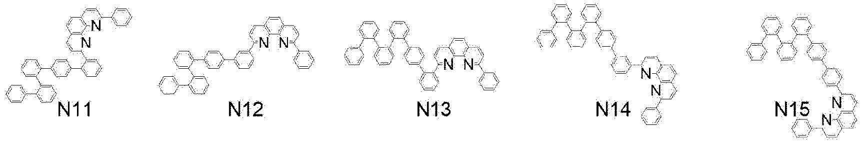

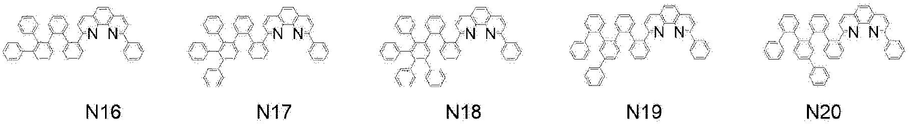

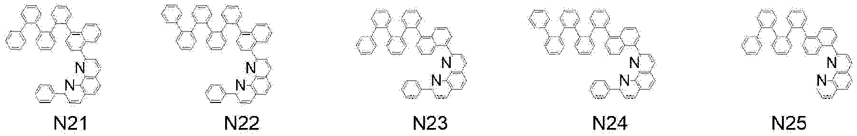

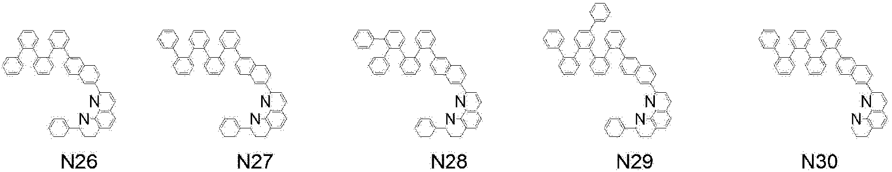

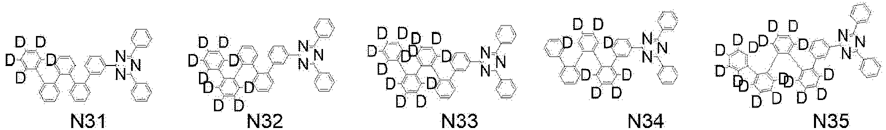

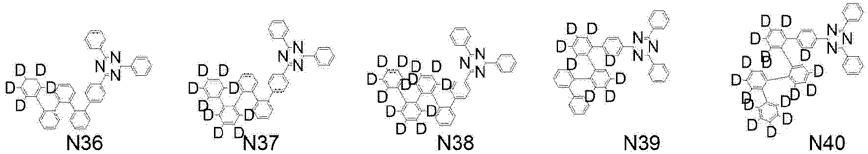

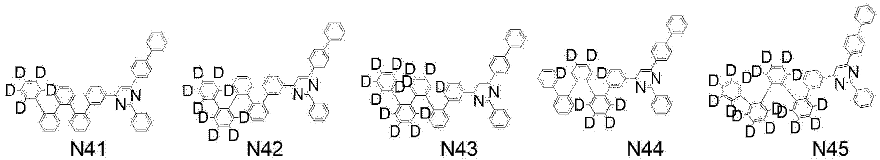

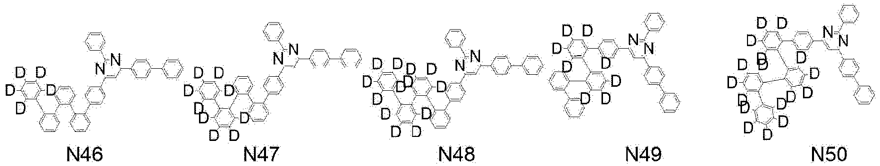

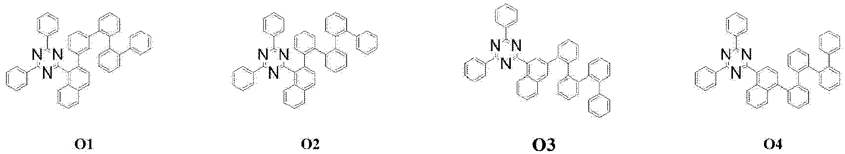

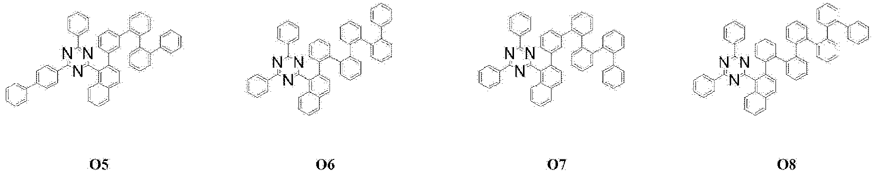

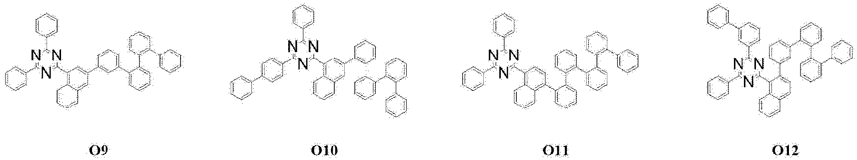

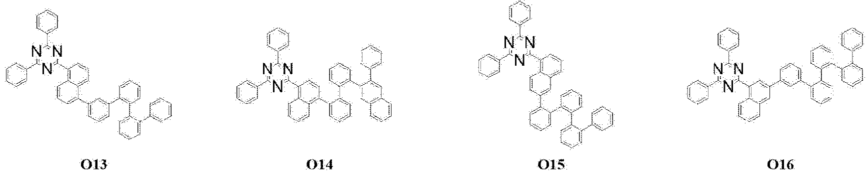

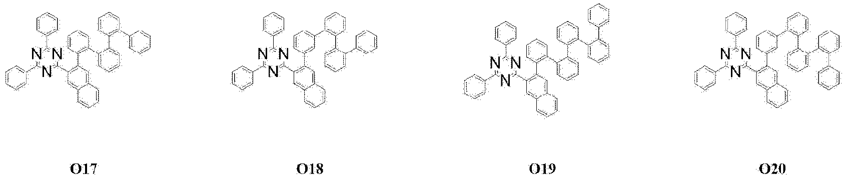

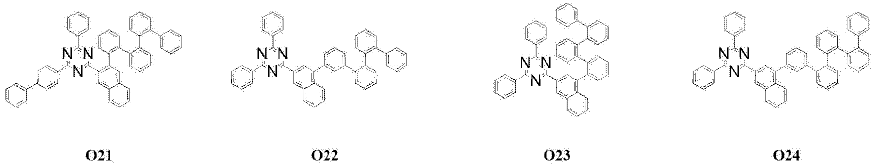

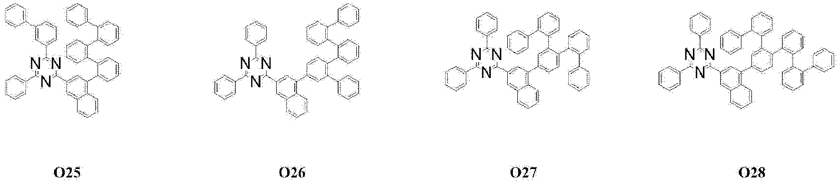

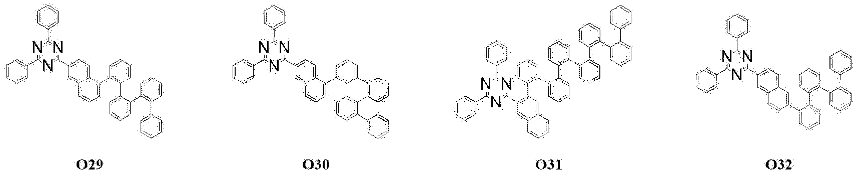

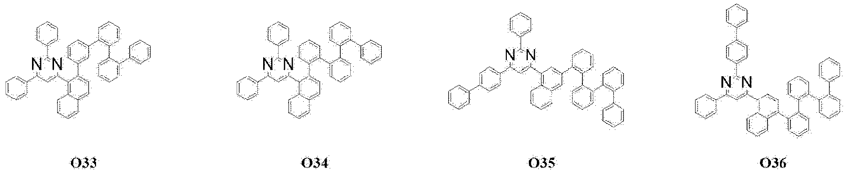

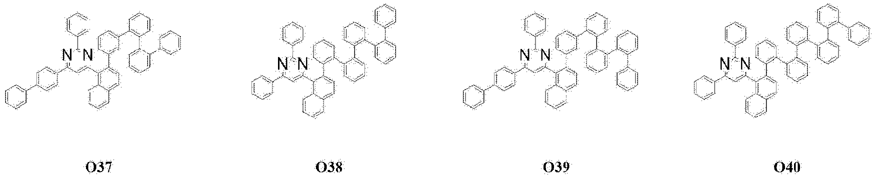

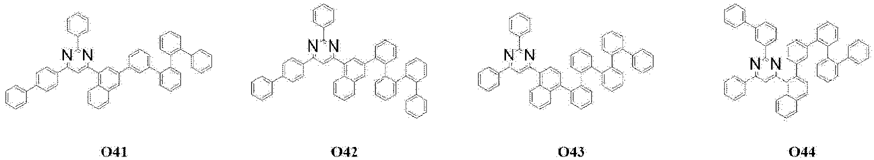

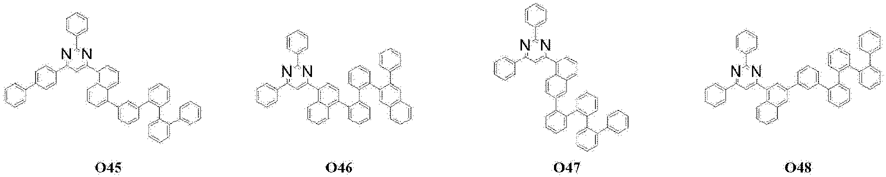

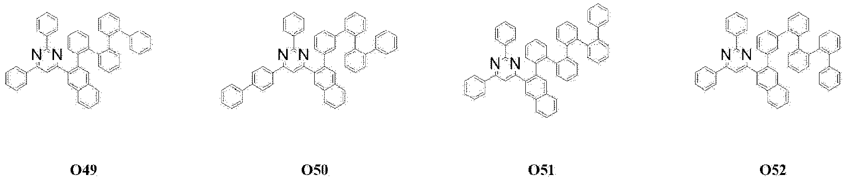

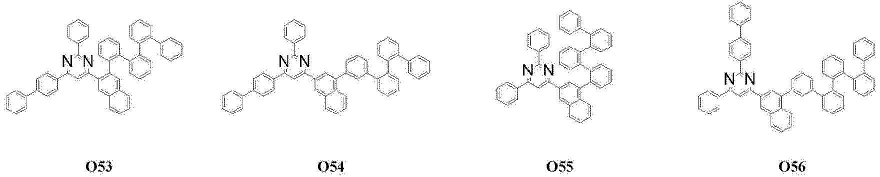

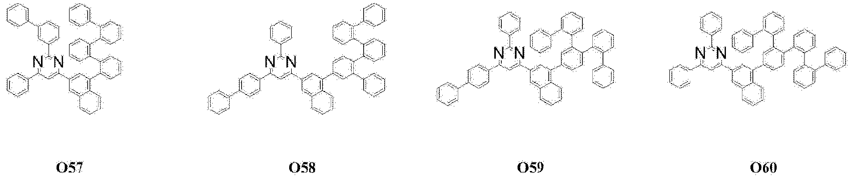

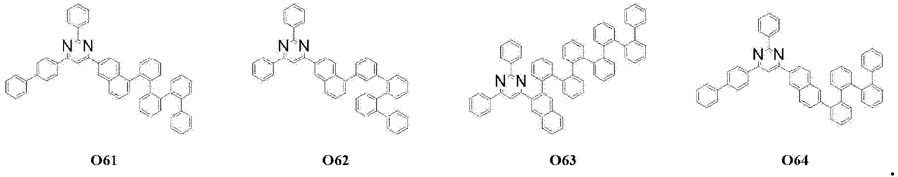

10. An organic electroluminescent device, comprising: an anode; a cathode; and one or more organic layers interposed between the anode and the cathode,
wherein at least one of the one or more organic layers contains the organic compound of any one of claims 1 to 9.

11. The organic electroluminescent device of claim 9, wherein the organic layer containing the organic compound is an electron transport layer or an electron transport auxiliary layer.
